# EUROPEAN PATENT SPECIFICATION

(11) **EP 4 540 267 B1**
(45) Date of publication and mention of the grant of the patent: **15.04.2026**
(21) Application number: 23738338.5
(22) Date of filing: 13.06.2023
(51) Int. Cl.: C07K 7/64, A61P 9/10, A61K 38/00

(54) **CYCLIC PEPTIDES FOR TRAPPING INTERLEUKIN-1 BETA**
ZYKLISCHE PEPTIDE ZUM EINFANGEN VON INTERLEUKIN-1 BETA
PEPTIDES CYCLIQUES POUR PIÉGER L'INTERLEUKINE-1 BÊTA

(30) Priority: 15.06.2022 US 202263352283 P; 14.04.2023 US 202363496265 P
(43) Date of publication of application: 23.04.2025
(60) Divisional application: 26162664.2
(73) Proprietor: Merck Sharp & Dohme LLC, Rahway, New Jersey 07065 (US)
(72) Inventor: CRUZ, Faben A., San Francisco, California 94080 (US); FENG, Danqing, Kenilworth, New Jersey 07033 (US); GUO, Zhuyan, Scotch Plains, New Jersey 07076 (US); HANISAK, Jennifer, Kenilworth, New Jersey 07033 (US); HICKEY, Jennifer L., Kenilworth, New Jersey 07033 (US); JAYNE, Charles Lee, Kenilworth, New Jersey 07033 (US); KEKEC, Ahmet, Kenilworth, New Jersey 07033 (US); LO, Michael Man-Chu, Kenilworth, New Jersey 07033 (US); PLUMMER, Christopher W., Kenilworth, New Jersey 07033 (US); BIANCHI, Elisabetta, 00040 Pomezia (RM) (IT); COLARUSSO, Stefania, 00040 Pomezia (RM) (IT); NIZI, Emanuela, 00040 Pomezia (RM) (IT); PAVONE, Francesca, 00040 Pomezia (RM) (IT); YOUSIF, Ali Munaim, 00040 Pomezia (RM) (IT)
(74) Representative: Merck Sharp & Dohme LLC
(86) International application number: PCT/US2023/025105
(87) International publication number: WO 2023/244554

(56) References cited:
- WO-A1-2020/110011
- WO-A2-2010/106441
- BORIS KLEMENTIEV ET AL: "Anti-inflammatory properties of a novel peptide interleukin 1 receptor antagonist", JOURNAL OF NEUROINFLAMMATION, BIOMED CENTRAL LTD., LONDON, GB, vol. 11, no. 1, 3 February 2014 (2014-02-03), pages 27, XP021176071, ISSN: 1742-2094, DOI: 10.1186/1742-2094-11-27
- CHARLES A. DINARELLO ET AL: "Treating inflammation by blocking interleukin-1 in a broad spectrum of diseases", NATURE REVIEWS DRUG DISCOVERY, vol. 11, no. 8, 1 August 2012 (2012-08-01), pages 633 - 652, XP055051238, ISSN: 1474-1776, DOI: 10.1038/nrd3800

## Description

### FIELD OF THE INVENTION

The present disclosure relates to certain cyclic peptides that trap interleukin-1β (IL-1β), pharmaceutical compositions comprising such peptides, and methods for using the compounds for treating, inhibiting, or ameliorating one or more cardiovascular disease states that could benefit from trapping IL-1β, including atherosclerosis.

### BACKGROUND OF THE INVENTION

Atherosclerosis is a disease of the arteries characterized by the accumulation of cholesterol plaques on the interior wall of the artery. Progression of atherosclerosis can result in hardening or narrowing of the arteries and increases the risk of plaque ruptures. These ruptures release cholesterol globules and other material into the bloodstream which may result in blockage of blood flow to the brain, heart, or other organ. Medically, these are known as Major Adverse Cardiac Events (MACE).

Risk factors for the development and progression of Atherosclerotic Cardiovascular Disease (ASCVD) include high cholesterol, high blood pressure, diet high in saturated fat, smoking, obesity, diabetes, lack of exercise, and elevated levels of C-reactive protein (CRP), a marker of inflammation.

The first line of treatment to prevent the progression of ASCVD is a healthy diet and exercise, however, compliance is generally poor. Pharmacological treatments for ASCVD have largely focused on cholesterol-lowering medications such as statins, cholesterol absorption inhibitors, and low-density lipoprotein (LDL) receptor inhibitors. These medications are highly effective at reducing the buildup of fatty acid deposits and improving arterial health. Other medications that are prescribed for ASCVD which do not ameliorate the disease state include blood thinners, such as aspirin, to prevent clumping of platelets in narrow arteries, and blood pressure medications to reduce the risk and severity of heart attacks. Surgical options for more aggressive intervention in advanced cases of ASCVD include angioplasty, stent placement, endarterectomy (surgical removal of plaques), and bypass surgery.

While cholesterol-lowering medications have served as an important standard of care for slowing the progression of atherosclerosis, clinical data support an additional critical role for inflammation in the progression of ASCVD that has remained untreated. Biomarkers of inflammation such as CRP are associated with increased risk of cardiovascular events, independent of cholesterol levels. The Canakinumab Anti-inflammatory Thrombosis Outcomes Study (CANTOS) was the first clinical trial to show that reducing vascular inflammation in the absence of concomitant lipid lowering reduces the rates of cardiovascular events. N Engl J Med 2017; 377:1119-1131. Canakinumab is an anti-interleukin-1 beta (IL-1β) human monoclonal antibody approved for clinical use in rheumatologic disorders. IL-1β is a proinflammatory cytokine that induces IL-6 and thereby elevates the downstream inflammatory biomarker high sensitivity CRP (hsCRP). Therefore, CANTOS provides proof of concept that therapies targeting IL-1β could reduce rates of MACE in certain patients in a manner that is complimentary and potentially additive to the LDL-lowering standard of care.

BORIS KLEMENTIEV ET AL: "Anti-inflammatory properties of a novel peptide interleukin 1 receptor antagonist", JOURNAL OF NEUROINFLAMMATION, vol. 11, no. 27, 3 February 2014, pages 1-18, disclose a peptide which antagonizes interleukin-I receptor and has anti-inflammatory activity.

There is a need for additional, non-surgical therapeutic approaches beyond the standard of care cholesterol-lowering medications for slowing the progression of atherosclerosis and decreasing the risk of MACE. In addition, patients suffering from inflammatory disorders would benefit from orally administered agents which block the same cytokine, IL-1β, as canakinumab.

### SUMMARY OF THE DISCLOSURE

The scope of the invention is defined in the appended set of claims. The present disclosure provides certain cyclic peptides that reduce inflammation by binding to the IL-1β cytokine and prevent engagement with the IL-1 receptor, resulting in inhibition of downstream pro-inflammatory signaling. These cyclic peptides can be valuable pharmaceutically active compounds for the treatment of cardiovascular diseases and inflammatory disorders. In one aspect, the present disclosure provides compounds of Formula (I) 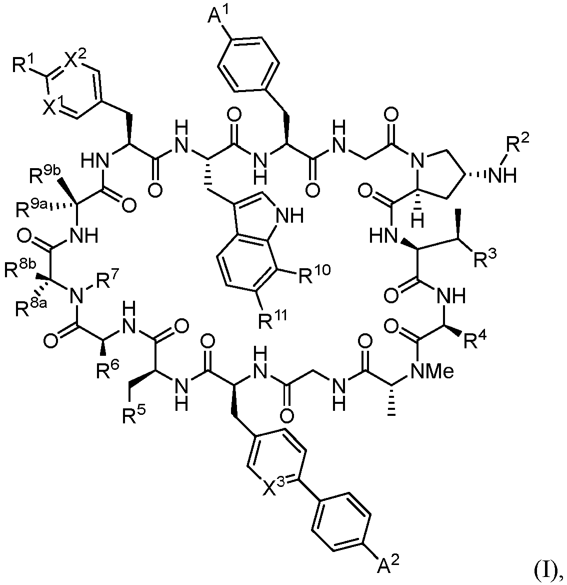 and their pharmaceutically acceptable salts.

The references to methods of treatment in the subsequent paragraphs of this description are to be interpreted as references to the compounds, pharmaceutical compositions and medicaments of the present invention for use in a method for treatment of the human (or animal) body by therapy.

The compounds can trap IL-1β and thereby affect the downstream pro-inflammatory signaling pathway which may be associated with cardiovascular disorders. Accordingly, in another aspect, the present disclosure provides a method for treating cardiovascular disorder (*e.g*., atherosclerosis, vascular inflammation) comprising administering a therapeutically effective amount of the compound of the disclosure to a subject in need thereof. In some embodiments, the administration comprises an oral administration of the compound.

The disclosure furthermore provides processes for preparing compounds of the disclosure and pharmaceutical compositions which comprise compounds of the disclosure and a pharmaceutically acceptable carrier.

### DETAILED DESCRIPTION OF THE INVENTION

### Compounds of the Disclosure

In one embodiment, the present disclosure provides a compound having structural Formula (I) as shown above wherein:
R¹ is CH₃C(O)NH-CH₂CH₂-O- or C¹;
   C¹ is:
   (i) a 5- to 6-membered monocyclic aryl or heteroaryl, wherein said heteroaryl contains 1 to 2 heteroatoms selected from the group consisting of N, O, and S; or
   (ii) a 5- to 6-membered mono- or bicyclic, saturated cycloalkyl or heterocycloalkyl containing 1 to 2 heteroatoms selected from the group consisting of N, O, and S; or
   (iii) a 5- to 6-membered mono- or bicyclic cycloalkyl;
      wherein C¹ is unsubstituted or substituted by 1 to 3 R^{C1} substituents independently selected from the group consisting of halo, C1-C3 alkyl, C1-C3 fluoroalkyl, carboxy, C1-C3 alkoxy, and C2-C3 acyl;
R² is H, C1-C3 alkyl, benzyl, or phenyl-CH₂CH₂-;
R³ is a 9- to 10-membered bicyclic aryl or heteroaryl, wherein said heteroaryl contains 1 to 2 heteroatoms selected from the group consisting of N, O, and S;
   wherein R³ is unsubstituted or substituted by 1 to 3 R^{3a} substituents independently selected from the group consisting of halo, C1-C3 alkyl, C1-C3 fluoroalkyl, hydroxy and C1-C3 alkoxy;
R⁴ is C1-C3 alkyl, HO₂C-(CH₂)ₘ-, H₂NC(O)-(CH₂)ₘ-, (CH₃)₂NC(O)-(CH₂)ₘ-, or tetrazolyl-(CH₂)ₘ-;
R⁵ is amino, H₂N(CH₂)ₙ-, H₂NC(O)-(CH₂)ₙ-, CH₃C(O)NH-, CH₃C(O)NH(CH₂)ₙ-, C⁵, or C⁵-CH₂-,
   C⁵ is:
   (i) a 5- to 6-membered monocyclic aryl or heteroaryl, wherein said heteroaryl contains 1 to 2 heteroatoms selected from the group consisting of N, O, and S;
   (ii) a 9- to 10-membered bicyclic aryl or heteroaryl, wherein said bicyclic heteroaryl contains 1 to 3 heteroatoms selected from the group consisting of N, O, and S;
   (iii) a 5- to 6-memberered monocyclic or 9-to 10-membered heterocycloalkyl, wherein said heterocycloalkyl is saturated or partially unsaturated, and contains 1 to 2 heteroatoms selected from the group consisting of N, O, and S;
   (iv) a 5- to 6-membered monocyclic cycloalkyl;
   (v) 2,3-dihydroindolyl;
      wherein C⁵ is unsubstituted or substituted by 1 to 3 R^{C5} substituents independently selected from the group consisting of halo, amino, hydroxy, C1-C3 alkyl, C1-C3 fluoroalkyl, C1-C3 alkoxy, H₂N-(CH₂)ₖ-, H₂NC(O)-(CH₂)ₖ-, H₂C=CH-CH₂O-, and phenyl;
R⁶ is H, C1-C5 alkyl, H₂N(CH₂)ₚ-, HOCH₂-, (CH₃)₂NCH₂-, H₃CO-(CH₂)_{q}-, or C⁶-CH₂-;
   C⁶ is 5- or 6-membered monocyclic, saturated heterocycloalkyl containing 1 to 2 heteroatoms selected from the group consisting of N, O, and S; and wherein C⁶ is unsubstituted or substituted by 1 to 3 R^{C6} substituents independently selected from the group consisting of halo, C1-C3 alkyl, C1-C3 fluoroalkyl, hydroxy and C1-C3 alkoxy;
R⁷ is H or C1-C3 alkyl;
R^{8a} is H, C1-C5 alkyl, HOCH₂-, H₂N(CH₂)ᵣ-, (CH₃)₃N⁺(CH₂)ᵣ-,or CH₃C(O)NH(CH₂)ᵣ-;
R8b is H or C1-C3 alkyl;
R9a is H or C1-C3 alkyl;
R9b is H, C1-C5 alkyl, C⁹-CH₂-, or C⁹-CH₂CH₂-;
   C⁹ is:
   (i) a 5- to 6-membered monocyclic aryl or heteroaryl, wherein said heteroaryl contains 1 to 2 heteroatoms selected from the group consisting of N, O, and S; or
   (ii) a 5- to 6-membered monocyclic, saturated cycloalkyl or heterocycloalkyl, wherein the heterocycloalkyl contains 1 to 2 heteroatoms selected from the group consisting of N, O, and S;
      wherein C⁹ is unsubstituted or substituted by 1 to 3 R^{C9} substituents independently selected from the group consisting of halo, amino, hydroxy, cyano, C1-C3 alkyl, C1-C3 fluoroalkyl, C1-C3 alkoxy, H2N-(CH2)k-, H2NC(O)-(CH2)k-, H₂NCH₂CH₂O-, CH₃C(O)NH-CH₂CH₂O-, and morpholinyl-CH₂CH₂O-;
R¹⁰ is H, halo, or C1-C3 alkyl;
R¹¹ is H, halo, or C1-C3 alkyl;
each occurrence of subscript k is independently 1 or 2;
subscript m is 1 or 2;
subscript n is 1, 2, 3, or 4;
subscript p is 1, 2, 3, or 4;
subscript q is 1 or 2;
subscript r is 1, 2, 3, or 4;
X¹, X², and X³ are independently C(H) or N; and
A¹ and A² are independently selected from the group consisting of HO2C-, H₂NC(O)-, CH3C(O)N(H)-, H₂NS(O)₂-, CH₃S(O)₂N(H)-, tetrazolyl, and 5-oxo oxadiazolyl; or
a pharmaceutically acceptable salt thereof.

In another embodiment, the present disclosure provides a compound Formula (I), wherein:
R⁵ is amino, H₂N(CH₂)ₙ-, H₂NC(O)-(CH₂)ₙ-, C⁵, or C⁵-CH₂-,
   C⁵ is:
   (i) a 5- to 6-membered monocyclic aryl or heteroaryl, wherein said heteroaryl contains 1 to 2 heteroatoms selected from the group consisting of N, O, and S:
   (ii) a 9- to 10-membered bicyclic aryl or heteroaryl, wherein said bicyclic heteroaryl contains 1 to 3 heteroatoms selected from the group consisting of N, O, and S;
   (iii) a 5- to 6-memberered monocyclic or 9-to 10-membered heterocycloalkyl, wherein said heterocycloalkyl is saturated or partially unsaturated, and contains 1 to 2 heteroatoms selected from the group consisting of N, O, and S; or
   (iv) 2,3-dihydroindolyl;
      wherein C⁵ is unsubstituted or substituted by 1 to 3 R^{C5} substituents independently selected from the group consisting of halo, amino, hydroxy, C1-C3 alkyl, C1-C3 fluoroalkyl, C1-C3 alkoxy, H₂N-(CH₂)ₖ-, H₂NC(O)-(CH2)ₖ-, H₂C=CH-CH₂O-, and phenyl;
R⁶ is H, C2-C5 alkyl, H₂N(CH₂)ₚ-, HOCH₂-, (CH₃)₂NCH₂-, H₃CO-(CH₂)_{q}-, or C⁶-CH₂-;
R^{8a} is H, C1-C3 alkyl, HOCH₂-, or H₂N(CH₂)ᵣ-;
R9a is H;
R9b is H, C1-C3 alkyl, C⁹-CH₂-, or C⁹-CH₂CH₂-;
R10 is H;
R11 is H;
subscript p is 1, 2, or 3; and
subscript r is 2, 3, or 4.

In one embodiment, the compound of Formula (I) has the structural Formula (IA)

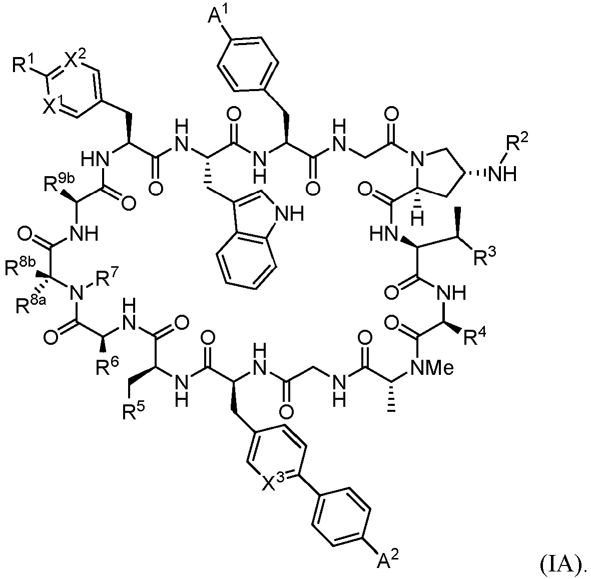

In another embodiment, the present disclosure provides a compound Formula (I),
wherein:
C¹ is phenyl, pyrimidyl, or piperazinyl, wherein C¹ is unsubstituted or substituted by 1 to 2 R^{C1} substituents;
R³ is naphthyl or indolyl, wherein R³ is unsubstituted or substituted by 1 to 2 R^{3a} substituents;
C⁵ is phenyl, pyridyl, pyrimidyl, naphthyl, indolyl, 7-azaindolyl, indazolyl, 2,3-dihydroindolyl, piperidinyl, tetrahydropyranyl, or cyclohexyl, wherein C⁵ is unsubstituted or substituted by 1 to 2 R^{C5};
C⁶ is tetrahydropyranyl or morpholinyl; wherein C⁶ is unsubstituted or substituted by 1 to 2 RC6; and
R9a is H or methyl;
C⁹ is phenyl, pyridyl, cyclohexyl, morpholinyl, or piperidinyl, wherein C⁹ is unsubstituted or substituted by 1 to 2 R^{C9}.

In another embodiment, the present disclosure provides a compound of Formula (I),
wherein:
X¹ and X² are C(H); and
R¹ is phenyl substituted by carboxy.

In another embodiment, the present disclosure provides a compound of Formula (I), wherein:
X¹ and X² are C(H); and
R¹ is CH₃C(O)NH-CH₂CH₂-O-.

In another embodiment, the present disclosure provides a compound of Formula (I), wherein R² is H.

In another embodiment, the present disclosure provides a compound of Formula (I), wherein R³ is indolyl substituted by one halo.

In another embodiment, the present disclosure provides a compound of Formula (I), wherein R³ is naphthyl.

In another embodiment, the present disclosure provides a compound of Formula (I), wherein R⁴ is HO₂C-(CH₂)ₘ-.

In another embodiment, the present disclosure provides a compound of Formula (I), wherein X³ is C(H).

In another embodiment, the present disclosure provides a compound of Formula (I), wherein R⁵ is H₂N(CH₂)ₙ-, indole, 7-azaindole, naphthyl or pyridyl.

In another embodiment, the present disclosure provides a compound of Formula (I), wherein R⁵ is H₂N(CH₂)ₙ-, indole, naphthyl or pyridyl.

In another embodiment, the present disclosure provides a compound of Formula (I), wherein R⁶ is H, HOCH₂-, C2-C5 alkyl or H₂N(CH₂)ₚ-.

In another embodiment, the present disclosure provides a compound of Formula (I), wherein R⁶ is C2-C5 alkyl or H₂N(CH₂)ₚ-.

In another embodiment, the present disclosure provides a compound of Formula (I), wherein R⁷ is H.

In another embodiment, the present disclosure provides a compound of Formula (I), wherein:
R^{8a} is methyl or H2NCH2CH2-; and
R8b is H.

In another embodiment, the present disclosure provides a compound of Formula (I), wherein R9b is

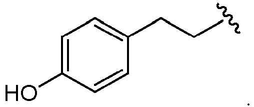

In another embodiment, the present disclosure provides a compound of Formula (I), wherein R9b is

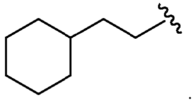

In one embodiment, the present disclosure provides a compound of Formula (I), wherein A¹ is selected from the group consisting of HO2C-, H₂NC(O)-, CH₃C(O)N(H)-, H₂NS(O)₂-, CH₃S(O)₂N(H)-, tetrazolyl, and 5-oxo oxadiazolyl. Persons skilled in chemistry will recognize that such moieties in the amino acid residue can be shown by the following substructures:

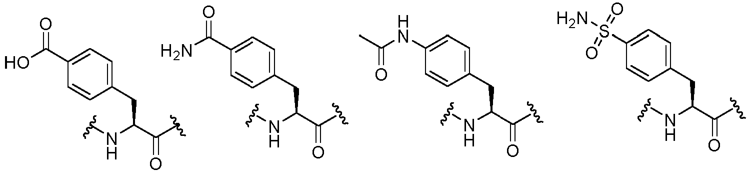

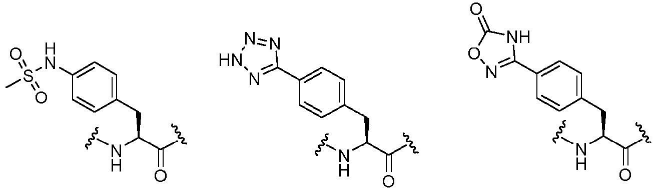

In one embodiment, the present disclosure provides a compound of Formula (I), wherein A² is selected from the group consisting of HO2C-, H₂NC(O)-, CH₃C(O)N(H)-, H₂NS(O)₂-, CH₃S(O)₂N(H)-, tetrazolyl, and 5-oxo oxadiazolyl. Persons skilled in chemistry will recognize that such moieties in the amino acid residue can be shown by the following substructures:

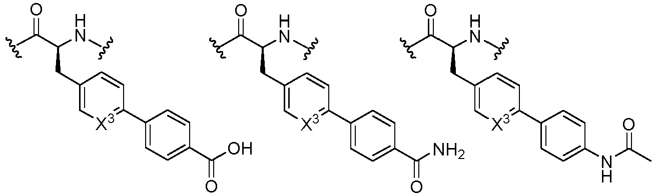

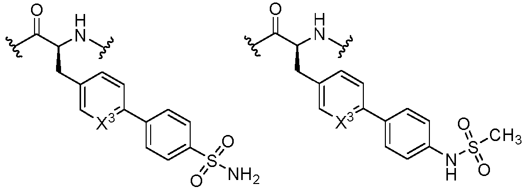

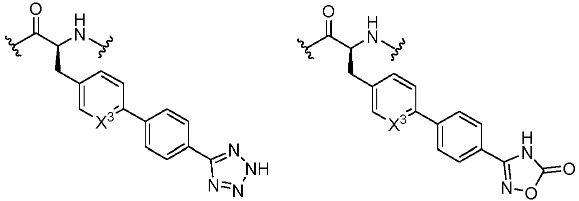

In another embodiment, the present disclosure provides a compound of Formula (I), wherein:
R10 is H; and
R11 is H, F or Cl.

In another embodiment, the present disclosure provides a compound of Formula (I), wherein A¹ and A² are both HO2C- (*i.e.,* carboxy).

In some embodiments, the present disclosure provides a compound of Formula (I), wherein:
R¹ is 4-CH₃C(O)-piperazin-1-yl, CH₃C(O)NH-CH₂CH₂-O-, 5-CO₂H-pyrimidin-2-yl, or 4-CO₂H-phenyl;
R² is H, ethyl, benzyl, or phenyl-CH₂CH₂-;
R³ is naphth-1-yl, 4-fluoroindol-3-yl, or 4-chloroindol-3-yl;
R⁴ is methyl, HO₂C-(CH₂)ₘ-, or H₂NC(O)-(CH₂)ₘ-;
R⁵ is amino, H₂N(CH₂)ₙ-, naphth-1-yl, indol-3-yl, 7-aza-indol-3-yl, indazol-1-yl, 2,3-dihydroindol-1-yl, pyrid-3-yl, pyrid-4-yl, piperidin-4-yl, 3-aminomethylphenyl, 4-aminomethylphenyl, 3-aminophenyl, 4-aminophenyl, phenyl, pyrid-4-yl-CH₂-, pyrimidin-5-yl, tetrahydropyran-4-yl-, H₂NC(O)-(CH₂)₂-, 3-biphenyl, 3-CH₂=CH-CH₂O-phenyl, CH₃C(O)NH-, CH₃C(O)NH(CH₂)₃-, or cyclohexyl;
R⁶ is H, (CH₃)₂CHCH₂-, (CH₃)₃CCH₂-, H₂N(CH₂)ₚ-, HOCH₂-, H₃CCH₂CH₂-, morpholin-4-yl-CH₂-, tetrahydropyran-4-yl-CH₂-, (CH₃)₂NCH₂-, or H₃CO-(CH₂)_{q}-.
R⁷ is H or methyl;
R^{8a} is H, methyl, HOCH₂-, H₂N(CH₂)ᵣ-, (CH₃)₃NCH₂CH₂-, or CH3C(O)NH(CH2)4-;
R8b is H or methyl;
R9a is H or methyl;
R^{9b} is H, methyl, H₃CCH₂CH₂CH₂-, 4-HO-phenyl-CH₂CH₂-, phenyl-CH₂CH₂-, cyclohexyl-CH₂CH₂-, 5-NC-pyrid-3-yl-CH₂CH₂-, 4-F₃C-phenyl-CH₂-, 3-F₃C-phenyl-CH₂-, 2-F₃C-phenyl-CH₂-, morpholin-4-yl-CH₂CH₂O-phenyl-CH₂-, 4-aminophenyl-CH₂-, 4,4-difluorocyclohexyl-CH₂-, 4-H₂NCH₂CH₂O-phenyl-CH₂-, 4-H₂NCH₂CH₂O-pyrid-3-yl-CH₂-, piperidin-4-yl-CH₂-, or 4-CH₃C(O)NH-CH₂CH₂O-phenyl-CH₂-;
R¹⁰ is H, fluoro, or methyl;
R¹¹ is H, fluoro, or chloro;
A¹ and A² are both HO2C-; and
X¹, X², and X³ are C(H).

In certain embodiments, the present disclosure provides a compound of Formula (I), wherein:
R¹ is 4-CH₃C(O)-piperazin-1-yl, CH₃C(O)NH-CH₂CH₂-O-, 5-CO₂H-pyrimidin-2-yl, or 4-CO₂H-phenyl;
R² is H, ethyl, benzyl, or phenyl-CH₂CH₂-;
R³ is naphth-1-yl, 4-fluoroindol-3-yl, or 4-chloroindol-3-yl;
R⁴ is methyl, HO₂C-(CH₂)ₘ-, or H₂N(O)C-(CH₂)ₘ-;
R⁵ is amino, H₂N(CH₂)ₙ-, naphth-1-yl, indol-3-yl, 7-aza-indol-3-yl, indazol-1-yl, 2,3-dihydroindol-1-yl, pyrid-3-yl, pyrid-4-yl, piperidin-4-yl, 3-aminomethylphenyl, 4-aminomethylphenyl, 3-aminophenyl, 4-aminophenyl, phenyl, pyrid-4-yl-CH₂-, pyrimidin-5-yl, tetrahydropyran-4-yl-, H₂N(O)C-(CH₂)₂-, 3-biphenyl, or 3-CH₂=CH-CH₂O-phenyl,
R⁶ is H, (CH₃)₂CHCH₂-, H₂N(CH₂)ₚ-, HOCH₂-, H₃CCH₂CH₂-, morpholin-4-yl-CH₂-, tetrahydropyran-4-yl-CH₂-, (CH₃)₂NCH₂-, or H₃CO-(CH₂)_{q}-.
R⁷ is H or methyl;
R8a is H, methyl, HOCH₂-, or H₂N(CH₂)ᵣ-;
R8b is H or methyl;
R9a is H;
R^{9b} is H, methyl, 4-HO-phenyl-CH₂CH₂-, phenyl-CH₂CH₂-, 5-NC-pyrid-3-yl-CH2CH2-, 4-F₃C-phenyl-CH₂-, 3-F₃C-phenyl-CH₂-, 2-F₃C-phenyl-CH₂-, morpholin-4-yl-CH₂CH₂O-phenyl-CH₂-, 4-aminophenyl-CH₂-, 4,4-difluorocyclohexyl-CH₂-, 4-H₂NCH₂CH₂O-phenyl-CH₂-, 4-H₂NCH₂CH₂O-pyrid-3-yl-CH₂-, piperidin-4-yl-CH₂-, or 4-CH₃C(O)NH-CH₂CH₂O-phenyl-CH₂-;
R10 and R¹¹ are both H; and
A¹ and A² are both HO2C-.

In some embodiments, the present disclosure provides a compound of Formula (I), wherein the compound is selected from the group consisting of SEQ ID NOS: 1-215 as set forth in Table 1. In certain embodiments, the present disclosure provides a compound of Formula (I), wherein the compound is selected from the group consisting of SEQ ID NOS: 1-173 as set forth in Table 1.

In specific embodiments, the present disclosure provides a compound of Formula (I), wherein the compound is selected from the group consisting of (SEQ ID NOS 78, 71-72, 67, 65, 70, 69, 68, 66, 64, 77, 79, 209, 193, 215, 212, and 210 respectively, in order of appearance): 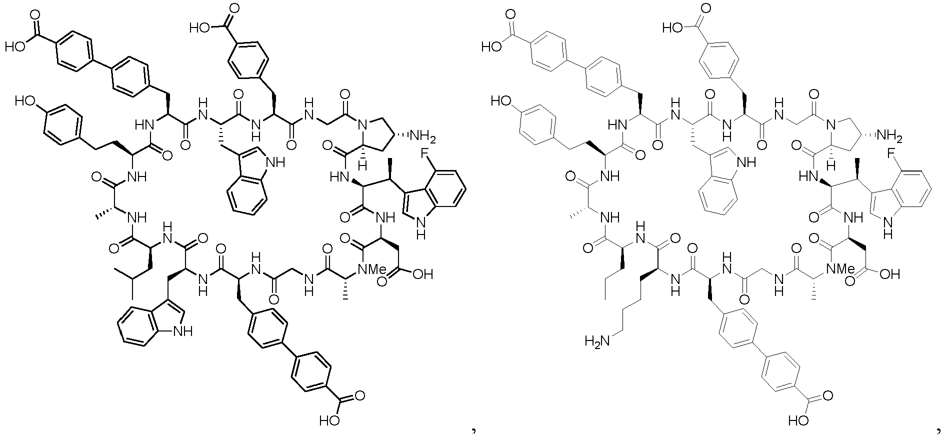 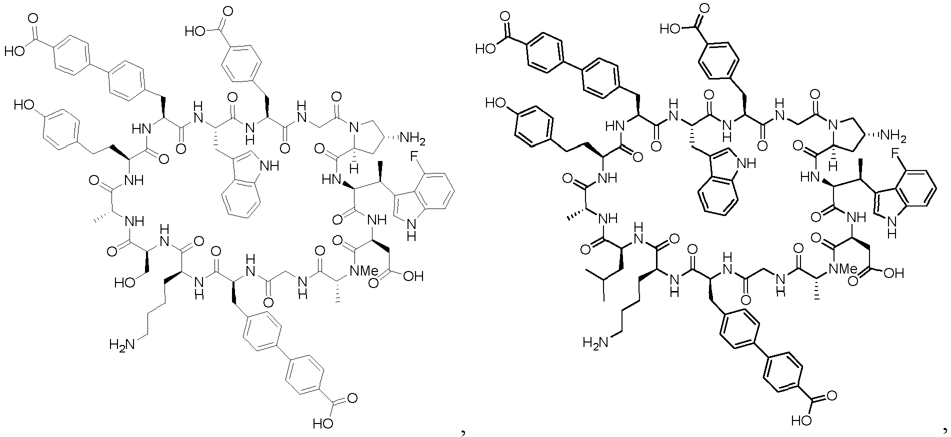 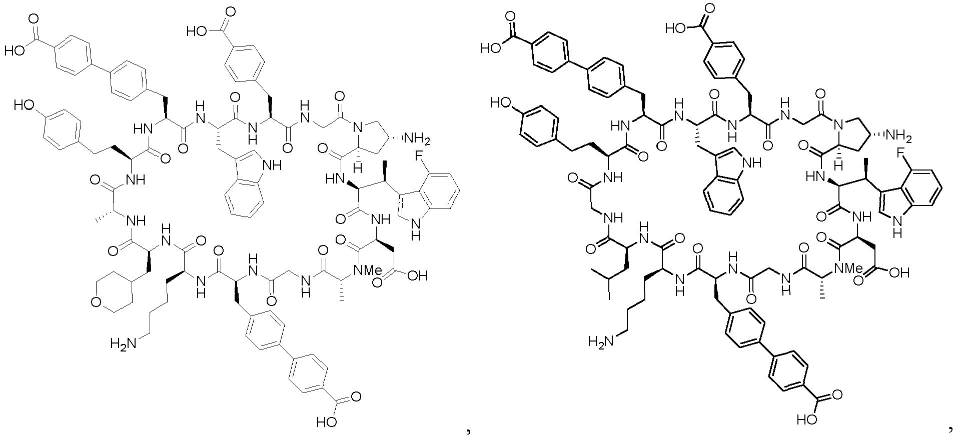 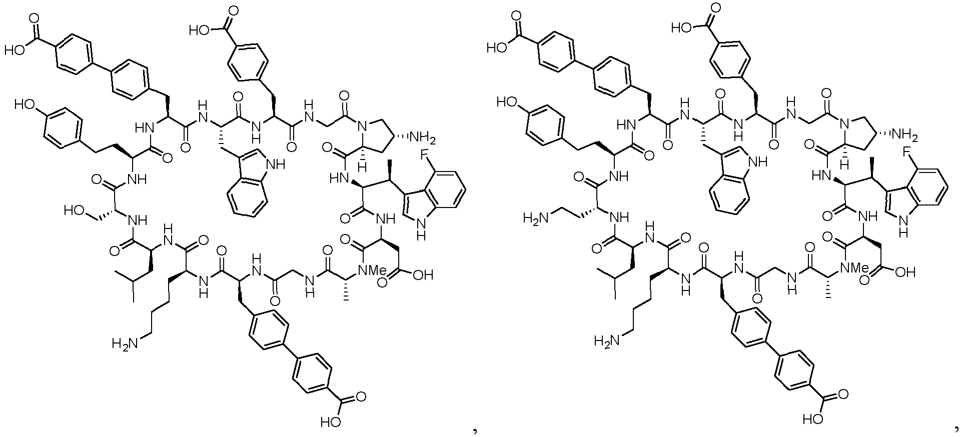 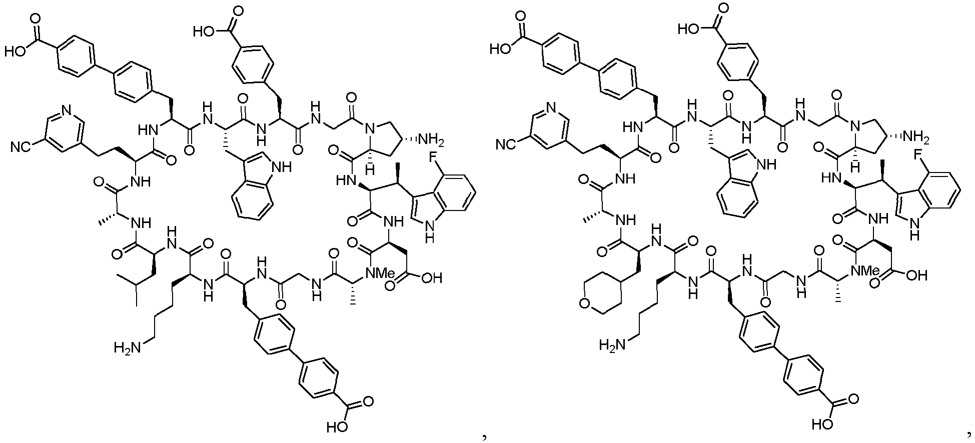 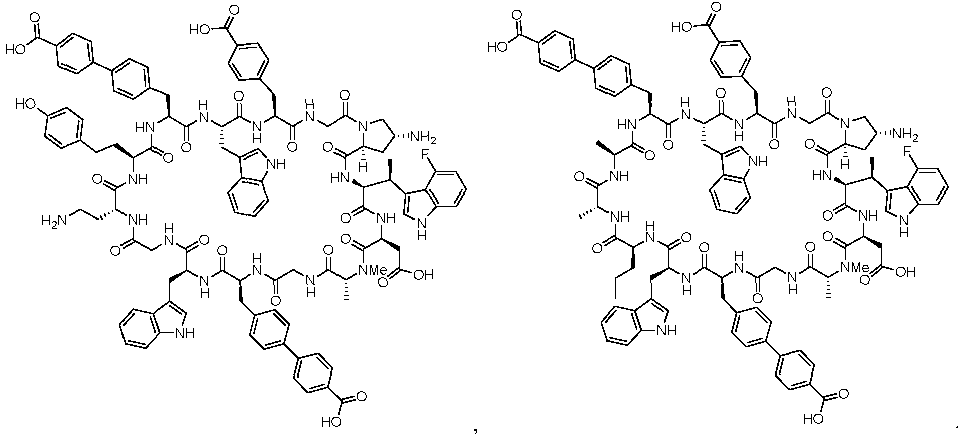 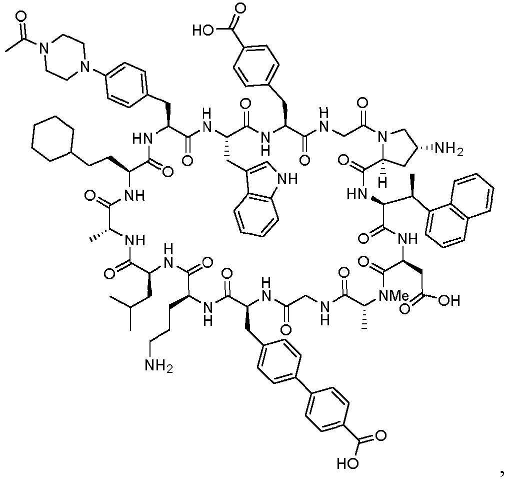 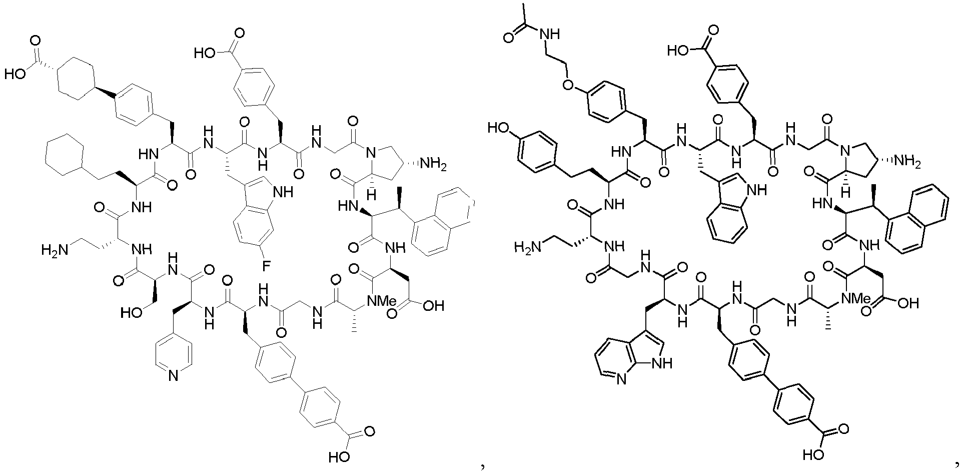 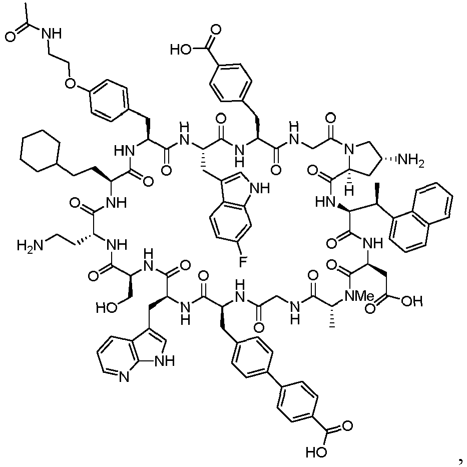 and

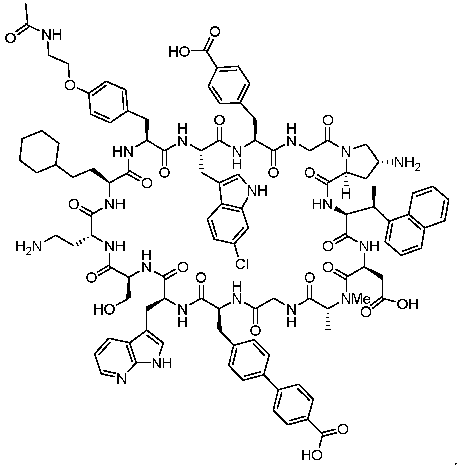

In some embodiments, the present disclosure provides a compound of Formula (I), wherein the compound is selected from the group consisting of SEQ ID NOS: 78, 71-72, 67, 65, 70, 69, 68, 66, 64, 77, and 79.

While not being bound by any specific theory, the Applicants believe that the compounds of the disclosure trap interleukin-1β, prevent signaling through the IL-1 receptor and hence reduce the downstream markers IL-6 and CRP. Hence the compounds can be useful to treat the inflammatory components of cardiovascular diseases such as ASCVD and heart failure with preserved ejection fraction (HFpEF). The compounds can also be useful to treat inflammatory disorders such as hidradenitis suppurativa (acne inversa), inflammatory bowel disease, and osteoarthritis.

### Definitions

Unless defined otherwise, all technical and scientific terms used herein have the same meaning as is commonly understood by one of skill in the art to which this invention belongs.

As used throughout this disclosure, "a compound of the disclosure", "a compound of the present disclosure" and "a compound disclosed herein" are used interchangeably are to be understood to include the disclosed cyclic peptides and compounds of Formula (I). Reference to the compounds of Formula (I) includes the compounds of other generic formulas that fall within the scope of Formula (I) including but not limited to the compounds of Formula (IA). The compounds of Formula (I) can form salts which are also within the scope of the present disclosure. Reference to a compound of the disclosure (or compound of Formula (I)) herein is understood to include reference to salts thereof, unless otherwise indicated. The term "salt(s)", as employed herein, denotes acidic salts formed with inorganic and/or organic acids, as well as basic salts formed with inorganic and/or organic bases. In addition, when a compound of Formula (I) contains both a basic moiety, such as, but not limited to an amino group, pyrrolidine or imidazole, and an acidic moiety, such as, but not limited to a carboxylic acid, zwitterions ("inner salts") may be formed and are included within the term "salt(s)" as used herein. In one embodiment, the salt is a pharmaceutically acceptable (*i.e.,* non-toxic, physiologically acceptable) salt. In another embodiment, the salt is other than a pharmaceutically acceptable salt. Salts of the compounds of Formula (I) may be formed, for example, by reacting a compound of Formula (I) with an amount of acid or base, such as an equivalent amount, in a medium such as one in which the salt precipitates or in an aqueous medium followed by lyophilization.

"Acyl" means an alkyl-C(O)- group, wherein alkyl is as defined below. The bond to the parent group is through the carbon atom of the carbonyl group.

"Alkyl", as well as other groups having the prefix "alk", such as alkoxy, and the like, means carbon chains which may be linear or branched, or combinations thereof, containing the indicated number of carbon atoms. For instance, a C₁-C₆ alkyl means an alkyl group having one (*i.e.,* methyl) up to 6 carbon atoms (*i.e.,* hexyl). In particular embodiments, linear alkyl groups have 1-6 carbon atoms and branched alkyl groups have 3-7 carbon atoms. Examples of alkyl groups include methyl, ethyl, propyl, isopropyl, butyl, *sec-* and *tert*-butyl, pentyl, hexyl, heptyl, octyl, nonyl and the like.

"Alkoxy" and "alkyl-O-" are used interchangeably and refer to an alkyl group linked to oxygen.

"Amino" means a H₂N- group. The bond to the parent group is through the nitrogen atom.

"Amino acid" refers to naturally-occurring α-amino acids and their stereoisomers, as well as unnatural amino acids (such as β-amino acids and substituted amino acids) and their stereoisomers. In the sequences given for the peptides (compounds) according to the present disclosure, the amino acid residues have their conventional meaning. Thus, "G" is glycine, "W" is tryptophan, "A" is alanine, "S" is serine, and so on. It is to be understood that "D" isomers are designated by a "d" before the one letter code or amino acid name, such that for example dA is the D isomer of L-alanine. Amino acid residues not encompassed by the foregoing have the definitions provided in the Table in the Examples section below.

"Aryl", as used herein, represents a monocyclic 6-membered or bicyclic 10-membered ring system, wherein at least one ring is aromatic, and all the ring atoms are carbon.

"Bicyclic ring system" refers to two joined rings. The rings may be fused, *i.e.,* share two adjacent atoms, or "spirocyclic", *i.e.,* share only a single atom.

"Carboxy" means a HO2C- group. The bond to the parent group is through the carbon atom of the carbonyl component.

"Cycloalkyl" means a saturated cyclic hydrocarbon radical. In particular embodiments, the cycloalkyl group has 3-12 carbon atoms, forming 1-3 carbocyclic rings that are fused. Examples of cycloalkyl include cyclopropyl, cyclobutyl, cyclopentyl, cyclohexyl, cycloheptyl, adamantyl, and the like.

"Fluoroalkyl" includes mono-substituted as well as multiple fluoro-substituted alkyl groups, up to perfluoro substituted alkyl. For example, fluoromethyl, 1,1-difluoroethyl, trifluoromethyl or 1,1,1,2,2-pentafluorobutyl are included.

"Halogen" or "halo", unless otherwise indicated, includes fluorine (fluoro), chlorine (chloro), bromine (bromo) and iodine (iodo). In one embodiment, halo is fluoro (-F) or chloro (-Cl).

"Heterocycloalkyl" means a non-aromatic monocyclic, bicyclic or tricyclic ring system comprising about 3 to about 10 ring atoms, preferably about 5 to about 10 ring atoms, in which one or more of the atoms in the ring system is an element other than carbon, for example nitrogen, oxygen or sulfur, alone or in combination. There are no adjacent oxygen and/or sulfur atoms present in the ring system. In some embodiments, heterocycloalkyls contain about 5 to about 6 ring atoms. The prefix aza, oxa or thia before the heterocyclyl root name means that at least a nitrogen, oxygen or sulfur atom respectively is present as a ring atom. In some embodiments, the nitrogen or sulfur atom of the heterocycloalkyl can be optionally oxidized to the corresponding N-oxide, S-oxide or S,S-dioxide. Non-limiting examples of suitable monocyclic heterocyclyl rings include piperidyl, pyrrolidinyl, piperazinyl, morpholinyl, thiomorpholinyl, thiazolidinyl, 1,4-dioxanyl, tetrahydrofuranyl, tetrahydropyranyl, tetrahydrothiophenyl, and the like.

"Heteroaryl" refers to aromatic monocyclic, bicyclic and tricyclic ring structures in which one or more atoms in the ring, the heteroatom(s), is an element other than carbon. Heteroatoms are typically O, S, or N atoms. Examples of heteroaromatic groups include pyridinyl, pyrimidinyl, pyrrolyl, pyridazinyl, isoxazolyl, thiazolyl, oxazolyl, indolyl, benzoxazolyl, benzothiazolyl, and imidazolyl.

When any variable (*e.g*., R^{C1}) occurs more than one time in any constituent or in Formula (I) or other generic formulas herein, its definition on each occurrence is independent of its definition at every other occurrence. Combinations of substituents and/or variables are permissible only if such combinations result in stable compounds. In choosing compounds of the present disclosure, one of ordinary skill in the art will recognize that the various substituents, *e.g.,* R^{C9}, are to be chosen in conformity with well-known principles of chemical structure connectivity and stability. Unless expressly stated to the contrary, substitution by a named substituent is permitted on any atom in a ring (*e.g.,* aryl, a heteroaryl ring, or a saturated heteroaryl ring) provided such ring substitution is chemically allowed and results in a stable compound. A "stable" compound is a compound which can be prepared and isolated and whose structure and properties remain or can be caused to remain essentially unchanged for a period of time sufficient to allow use of the compound for the purposes described herein (*e.g*., therapeutic or prophylactic administration to a subject).

The term "substituted" shall be deemed to include multiple degrees of substitution by a named substituent. Where multiple substituent moieties are disclosed or claimed, the substituted compound can be independently substituted by one or more of the disclosed or claimed substituent moieties, singly or plurally. By independently substituted, it is meant that the (two or more) substituents can be the same or different.

Unless expressly depicted or described otherwise, variables depicted in a structural formula with a "floating" bond, are permitted on any available carbon atom in the ring to which the variable is attached. When a moiety is noted as being "optionally substituted" in Formula (I) or any embodiment thereof, it means that Formula (I) or the embodiment thereof encompasses compounds that contain the noted substituent (or substituents) on the moiety and also compounds that do not contain the noted substituent (or substituents) on the moiety. The wavy line 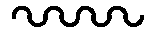 , as used herein, indicates a point of attachment to the rest of the compound.

Some of the compounds described herein may exist as tautomers which have different points of attachment of hydrogen accompanied by one or more double bond shifts. For example, a ketone and its enol form are keto-enol tautomers. The individual tautomers as well as mixtures thereof are encompassed with compounds of the present disclosure.

In the compounds of the disclosure, the atoms may exhibit their natural isotopic abundances, or one or more of the atoms may be artificially enriched in a particular isotope having the same atomic number, but an atomic mass or mass number different from the atomic mass or mass number predominantly found in nature. The present disclosure as described and claimed herein is meant to include all suitable isotopic variations of the compounds of the disclosure and embodiments thereof. For example, different isotopic forms of hydrogen (H) include protium (¹H) and deuterium (²H, also denoted herein as D). Protium is the predominant hydrogen isotope found in nature. Enriching for deuterium may afford certain therapeutic advantages, such as increasing *in vivo* half-life or reducing dosage requirements or may provide a compound useful as a standard for characterization of biological samples. Isotopically-enriched compounds of the disclosure, can be prepared without undue experimentation by conventional techniques well known to those skilled in the art or by processes analogous to those described in the Schemes and Examples herein using appropriate isotopically-enriched reagents and/or intermediates.

The term "pharmaceutically acceptable salts" refers to salts prepared from pharmaceutically acceptable non-toxic bases or acids. When the compound of the present disclosure is acidic (or has a functional group which may be anionic), its corresponding salt can be conveniently prepared from pharmaceutically acceptable non-toxic bases, including inorganic bases and organic bases. Examples of suitable inorganic cations include, but are not limited to, alkali metal ions such as Li⁺, Na⁺, and K⁺, alkaline earth metal cations such as Ca2⁺, and Mg2⁺, and other cations such as Al³⁺ and Zn⁺. Examples of suitable organic cations include, but are not limited to, ammonium ion (*i.e.,* NH₄⁺) and substituted ammonium ions. Examples of suitable substituted ammonium ions are those derived from methylamine, ethylamine, diethylamine, triethylamine and ethylenediamine. When a compound of the present disclosure is basic, its corresponding salt can be conveniently prepared from pharmaceutically acceptable non-toxic acids, including inorganic acids and organic acids. Example of such acid addition salts include salts formed from hydrohalic acids (*e.g*., hydrochloric, hydrobromic, hydroiodic), formic acid, acetic acid, capric acid, and citric acids. Salts containing acetate, formate, caprate, chloride, or sodium salts are typical for use with the compounds of the present disclosure. In some embodiments, salts of compounds of the present disclosure can be formed by exchange well-known to those of ordinary skill in the art, such as by anion exchange, *e.g*., replacement of trifluoroacetate ions with chloride ions.

Furthermore, compounds of the present disclosure may exist in amorphous form and/or one or more crystalline forms, and as such all amorphous and crystalline forms and mixtures thereof of the compounds of Formula (I), including the Examples, are intended to be included within the scope of the present disclosure. In addition, some of the compounds of the instant disclosure may form solvates with water (*i.e.,* a hydrate) or common organic solvents such as, but not limited to, acetic acid or acetonitrile. Such solvates and hydrates, particularly the pharmaceutically acceptable solvates and hydrates, of the instant compounds are likewise encompassed within the scope of this disclosure, along with un-solvated and anhydrous forms.

Any pharmaceutically acceptable pro-drug modification of a compound of this disclosure which results in conversion *in vivo* to a compound within the scope of this disclosure is also within the scope of this disclosure.

The present disclosure also relates to processes for the preparation of the compounds of Formula (I) which are described in the following Examples and by which the compounds of the disclosure are obtainable.

"Treatment" and "treating" refer to all processes in which there may be a slowing, interrupting, arresting, controlling, or stopping of the progression of a disease or disorder described herein. The terms do not necessarily indicate a total elimination of all disease or disorder symptoms.

"Preventing," or "prophylaxis," as used herein, refers to reducing the likelihood of contracting disease or disorder described herein, or reducing the severity of a disease or disorder described herein.

The terms "therapeutically effective (or efficacious) amount" and similar descriptions such as "an amount efficacious for treatment" or "an effective dose" are intended to mean that amount of a compound of the disclosure that will elicit the biological or medical response of a tissue, a system, animal or human that is being sought by a researcher, veterinarian, medical doctor or other clinician. In a preferred embodiment, the term "therapeutically effective amount" means an amount of a compound of the disclosure that alleviates at least one clinical symptom in a human patient. The terms "prophylactically effective (or efficacious) amount" and similar descriptions such as "an amount efficacious for prevention" are intended to mean that amount of a compound of the disclosure that will prevent or reduce the risk of occurrence of the biological or medical event that is sought to be prevented in a tissue, a system, animal or human by a researcher, veterinarian, medical doctor or other clinician.

### Dosages of the Compounds of the Present Disclosure

The dosage regimen utilizing a compound of the present disclosure is selected in accordance with a variety of factors including type, species, age, weight, sex and medical condition of the patient; the severity of the condition to be treated; the potency of the compound chosen to be administered; the route of administration; and the renal and hepatic function of the patient. A consideration of these factors is well within the purview of the ordinarily skilled clinician for the purpose of determining the therapeutically effective or prophylactically effective dosage amount needed to prevent, counter, or arrest the progress of the condition. It is understood that a specific daily dosage amount can simultaneously be both a therapeutically effective amount, *e.g*., for treatment of an oncological condition, and a prophylactically effective amount, *e.g.,* for prevention of an oncological condition.

While individual needs vary, determination of optimal ranges of effective amounts of the compound of the present disclosure is within the skill of the art. For administration to a human in the curative or prophylactic treatment of the conditions and disorders identified herein, for example, typical dosages of the compounds of the present disclosure can be about 0.05 mg/kg/day to about 50 mg/kg/day. In some embodiments, a patient is administered from about 5 mg/day to about 120 mg/day, such as from 10 mg/day, 20 mg/day, 30 mg/day, 40 mg/day, 50 mg/day, 60 mg/day, 70 mg/day, 80 mg/day, mg/day, 90 mg/day, or 100 mg/day of a compound of the present disclosure. In certain embodiments, a patient is administered from about 0.2 mg/kg to about 5 mg/kg, such as from 0.5 mg/kg, 0.75 mg/kg, 1.0 mg/kg, 1.25 mg/kg, or 1.5 mg/kg of a compound of the present disclosure. Such doses may be administered in a single dose or may be divided into multiple doses.

### Pharmaceutical Compositions

The compounds of the disclosure and their pharmaceutically acceptable salts can be administered to animals, preferably to mammals, and particularly to humans, as pharmaceuticals by themselves, in mixtures with one another or in the form of pharmaceutical compositions. The term "subject" or "patient" includes animals, preferably mammals and especially humans, who use the instant active agents for the prevention or treatment of a medical condition. Administering of the drug to the subject includes both self-administration and administration to the patient by another person. The subject may be in need of, or desire, treatment for an existing disease or medical condition, or may be in need of or desire prophylactic treatment to prevent or reduce the risk of occurrence of the disease or medical condition. As used herein, a subject "in need" of treatment of an existing condition or of prophylactic treatment encompasses both a determination of need by a medical professional as well as the desire of a patient for such treatment.

The present disclosure therefore also provides the compounds of the disclosure and their pharmaceutically acceptable salts for use as pharmaceuticals, their use for modulating the activity of the cytokine IL-1β, and in particular, their use in the therapy and prophylaxis of the below-mentioned diseases or disorders as well as their use for preparing medicaments for these purposes. In certain embodiments, the compounds of the disclosure and their pharmaceutically acceptable salts trap IL-1β.

Furthermore, the present disclosure provides pharmaceutical compositions which comprise as active component an effective dose of at least one compound of the disclosure and/or a pharmaceutically acceptable salt thereof and a customary pharmaceutically acceptable carrier, *i.e.,* one or more pharmaceutically acceptable carrier substances and/or additives.

Thus, the present disclosure provides, for example, said compound and its pharmaceutically acceptable salts for use as pharmaceutical compositions which comprise as active component an effective dose of the compound of the disclosure and/or a pharmaceutically acceptable salt thereof and a customary pharmaceutically acceptable carrier, and the uses of said compound and/or a pharmaceutically acceptable salt thereof in the therapy or prophylaxis of the below-mentioned diseases or disorders, *e.g*., atherosclerosis, as well as their use for preparing medicaments for these purposes.

The pharmaceutical compositions according to the disclosure can be administered orally, for example, in the form of pills, tablets, lacquered tablets, sugar-coated tablets, granules, hard and soft gelatin capsules, aqueous, alcoholic or oily solutions, syrups, emulsions or suspensions, or rectally, for example, in the form of suppositories. Administration can also be carried out parenterally, for example, subcutaneously, intramuscularly or intravenously in the form of solutions for injection or infusion.

Other suitable administration forms are, for example, percutaneous or topical administration, for example, in the form of ointments, tinctures, sprays or transdermal therapeutic systems, or, for example, microcapsules, implants or rods. The preferred administration form depends, for example, on the disease to be treated and on its severity.

The present disclosure also provides pharmaceutical compositions comprising a compound of Formula (I). The compound of Formula (I) can be used in combination with any suitable pharmaceutical carrier or excipient. Such pharmaceutical compositions comprise a therapeutically effective amount of one or more compounds of Formula (I), and pharmaceutically acceptable excipient(s) and/or carrier(s). The specific pharmaceutic composition will suit the mode of administration. In particular aspects, the pharmaceutical acceptable carrier may be water or a buffered solution.

Excipients included in the pharmaceutical compositions have different purposes depending, for example on the nature of the drug, and the mode of administration. Examples of generally used excipients include, without limitation: saline, buffered saline, dextrose, water-for-infection, glycerol, ethanol, and combinations thereof, stabilizing agents, solubilizing agents and surfactants, buffers and preservatives, tonicity agents, bulking agents, lubricating agents (such as talc or silica, and fats, such as vegetable stearin, magnesium stearate or stearic acid), emulsifiers, suspending or viscosity agents, inert diluents, fillers (such as cellulose, dibasic calcium phosphate, vegetable fats and oils, lactose, sucrose, glucose, mannitol, sorbitol, calcium carbonate, and magnesium stearate), disintegrating agents (such as crosslinked polyvinyl pyrrolidone, sodium starch glycolate, cross-linked sodium carboxymethyl cellulose), binding agents (such as starches, gelatin, cellulose, methyl cellulose or modified cellulose such as microcrystalline cellulose, hydroxypropyl cellulose, sugars such as sucrose and lactose, or sugar alcohols such as xylitol, sorbitol or maltitol, polyvinylpyrrolidone and polyethylene glycol), wetting agents, antibacterials, chelating agents, coatings (such as a cellulose film coating, synthetic polymers, shellac, corn protein zein or other polysaccharides, and gelatin), preservatives (including vitamin A, vitamin E, vitamin C, retinyl palmitate, and selenium, cysteine, methionine, citric acid and sodium citrate, and synthetic preservatives, including methyl paraben and propyl paraben), sweeteners, perfuming agents, flavoring agents, coloring agents, absorption enhancers, administration aids, and combinations thereof.

Carriers are compounds and substances that improve and/or prolong the delivery of an active ingredient to a subject in the context of a pharmaceutical composition. Carriers may serve to prolong the *in vivo* activity of a drug or slow the release of the drug in a subject, using controlled-release technologies. Carriers may also decrease drug metabolism in a subject and/or reduce the toxicity of the drug. Carriers can also be used to target the delivery of the drug to particular cells or tissues in a subject. Common carriers (both hydrophilic and hydrophobic carriers) include fat emulsions, lipids, PEGylated phospholipids, PEGylated liposomes, PEGylated liposomes coated via a PEG spacer with a cyclic RGD peptide, liposomes and lipospheres, microspheres (including those made of biodegradable polymers or albumin), polymer matrices, biocompatible polymers, protein-DNA complexes, protein conjugates, erythrocytes, vesicles, nanoparticles, and side-chains for hydro-carbon stapling. The aforementioned carriers can also be used to increase cell membrane permeability of the compounds of Formula (I). In addition to their use in the pharmaceutical compositions of the present disclosure, carriers may also be used in compositions for other uses, such as research uses *in vitro* (*e.g.,* for delivery to cultured cells) and/or *in vivo.*

Pharmaceutical compositions adapted for oral administration may be presented as discrete units such as capsules or tablets; as powders or granules; as solutions, syrups or suspensions (in aqueous or non-aqueous liquids; or as edible foams or whips; or as emulsions). Suitable excipients for tablets or hard gelatin capsules include lactose, maize starch or derivatives thereof, stearic acid or salts thereof. Suitable excipients for use with soft gelatin capsules include for example vegetable oils, waxes, fats, semi-solid, or liquid polyols etc. For the preparation of solutions and syrups, excipients which may be used include for example water, polyols and sugars. For the preparation of suspensions oils, *e.g*., vegetable oils, may be used to provide oil-in-water or water in oil suspensions. Excipients which promote absorption from the gastrointestinal tract, *e.g*., permeation enhancers, such as sodium caprate can be included. In certain situations, delayed release preparations may be advantageous and compositions which can deliver the compounds of the present disclosure in a delayed or controlled release manner may also be prepared. Prolonged gastric residence brings with it the problem of degradation by the enzymes present in the stomach and so enteric-coated capsules may also be prepared by standard techniques in the art where the active substance for release lower down in the gastro-intestinal tract.

Pharmaceutical compositions adapted for transdermal administration may be presented as discrete patches intended to remain in intimate contact with the epidermis of the recipient for a prolonged period of time. For example, the active ingredient may be delivered from the patch by iontophoresis as generally described in Pharmaceutical Research, 3(6):318 (1986).

Pharmaceutical compositions adapted for topical administration may be formulated as ointments, creams, suspensions, lotions, powders, solutions, pastes, gels, sprays, aerosols or oils. When formulated in an ointment, the active ingredient may be employed with either a paraffinic or a water-miscible ointment base. Alternatively, the active ingredient may be formulated in a cream with an oil-in-water cream base or a water-in-oil base. Pharmaceutical compositions adapted for topical administration to the eye include eye drops wherein the active ingredient is dissolved or suspended in a suitable carrier, especially an aqueous solvent. Pharmaceutical compositions adapted for topical administration in the mouth include lozenges, pastilles and mouth washes.

Pharmaceutical compositions adapted for rectal administration may be presented as suppositories or enemas.

Pharmaceutical compositions adapted for nasal administration wherein the carrier is a solid include a coarse powder having a particle size for example in the range 20 to 500 microns which is administered in the manner in which snuff is taken, *i.e.,* by rapid inhalation through the nasal passage from a container of the powder held close up to the nose. Suitable compositions wherein the carrier is a liquid, for administration as a nasal spray or as nasal drops, include aqueous or oil solutions of the active ingredient.

Pharmaceutical compositions adapted for administration by inhalation include fine particle dusts or mists which may be generated by means of various types of metered dose pressurized aerosols, nebulizers or insufflators.

Pharmaceutical compositions adapted for vaginal administration may be presented as pessaries, tampons, creams, gels, pastes, foams or spray formulations.

Pharmaceutical compositions adapted for parenteral administration include aqueous and non-aqueous sterile injection solution which may contain anti-oxidants, buffers, bacteriostats and solutes which render the formulation substantially isotonic with the blood of the intended recipient; and aqueous and non-aqueous sterile suspensions which may include suspending agents and thickening agents. Excipients which may be used for injectable solutions include water-for-injection, alcohols, polyols, glycerin and vegetable oils, for example. The compositions may be presented in unit-dose or multi-dose containers, for example sealed ampoules and vials, and may be stored in a freeze-dried (lyophilized) condition requiring only the addition of the sterile liquid carrier, for example water or saline for injections, immediately prior to use. Extemporaneous injection solutions and suspensions may be prepared from sterile powders, granules and tablets. The pharmaceutical compositions may contain preserving agents, solubilizing agents, stabilizing agents, wetting agents, emulsifiers, sweeteners, colorants, odorants, salts (substances of the present disclosure may themselves be provided in the form of a pharmaceutically acceptable salt), buffers, coating agents or antioxidants. They may also contain therapeutically-active agents in addition to the compounds of the present disclosure.

### Methods of Using the Compounds of the Disclosure

The present application provides a method of IL-1 mediated cell signaling comprising contacting a cell with a compound of the disclosure or a pharmaceutically acceptable salt thereof. Inhibition of IL-1 mediated cell signaling can be assessed by detecting decreases in the levels of downstream biomarker IL-6 and CRP (*e.g.,* hsCRP).

The present application also provides methods of using the compounds of the disclosure (or their pharmaceutically acceptable salts) or pharmaceutical compositions containing such compounds to treat disease conditions, including but not limited to, conditions implicated by IL-1β.

In some embodiments, the present disclosure provides a method of treating-cardiovascular disease, the method comprising administering a therapeutically effective amount a compound of the disclosure (or a pharmaceutically acceptable salt thereof) or any of the foregoing pharmaceutical compositions comprising such a compound to a subject in need of such treatment. In some embodiments, the cardiovascular disease is vascular inflammation. In some embodiments, the cardiovascular disease is atherosclerosis. In some embodiments, the cardiovascular disease is heart failure with preserved ejection fraction (HFpEF). In other embodiments the cardiovascular disease is heart failure with reduced ejection fraction (HFrEF).

In some embodiments, the present disclosure provides a method of treating a chronic kidney disease, the method comprising administering a therapeutically effective amount a compound of the disclosure (or a pharmaceutically acceptable salt thereof) or any of the foregoing pharmaceutical compositions comprising such a compound to a subject in need of such treatment.

In some embodiments, the present disclosure provides a method of treating inflammatory disorders, the method comprising administering a therapeutically effective amount of a compound of the disclosure (or a pharmaceutically acceptable salt thereof) or any of the foregoing pharmaceutical compositions comprising such a compound to a subject in need of such treatment. In certain embodiments, the inflammatory disorder is selected from the group consisting of hidradenitis suppurativa (acne inversa), inflammatory bowel disease, arthritis, and nonalcoholic steatohepatitis (NASH).

In some embodiments, the inflammatory disorder is hidradenitis suppurativa (acne inversa).

In certain embodiments, the inflammatory disorder is inflammatory bowel disease, such as Crohn's disease or ulcerative colitis.

In some embodiments, the inflammatory disorder is arthritis, e.g., osteoarthritis, rheumatoid arthritis, psoriatic arthritis, or gouty arthritis.

In other embodiments, the inflammatory disorder is nonalcoholic steatohepatitis (NASH).

### Combination Therapies

One or more additional pharmacologically active agents may be administered in combination with a compound of the disclosure. An additional active agent (or agents) is intended to mean a pharmaceutically active agent (or agents) that is active in the body, including pro-drugs that convert to pharmaceutically active form after administration, which are different from the compound of Formula I, and also includes free-acid, free-base and pharmaceutically acceptable salts of said additional active agents. Generally, any suitable additional active agent or agents, including but not limited to anti-hypertensive agents, anti-atherosclerotic agents such as a lipid modifying compound, anti-diabetic agents and/or anti-obesity agents, anti-inflammatory agents, may be used in any combination with the compound of the disclosure in a single dosage formulation (a fixed dose drug combination), or may be administered to the subject in one or more separate dosage formulations which allows for concurrent or sequential administration of the active agents (co-administration of the separate active agents).

Examples of additional active agents which may be employed in treating cardiovascular disorders include but are not limited to angiotensin converting enzyme inhibitors (*e.g*., alacepril, benazepril, captopril, ceronapril, cilazapril, delapril, enalapril, enalaprilat, fosinopril, imidapril, lisinopril, moveltipril, perindopril, quinapril, ramipril, spirapril, temocapril, or trandolapril), angiotensin II receptor antagonists (*e.g.,* losartan, *i.e.,* COZAAR^{®}, valsartan (including combinations with sacubitril), candesartan, olmesartan, telmesartan and any of these drugs used in combination with hydrochlorothiazide such as HYZAAR^{®}); sGC activators (*e.g*., riociguat and vericiguat), PCSK9 inhibitors (*e.g*., evolocumab, alirocumab, MK-0616 and those disclosed in WO2019/246349), neutral endopeptidase inhibitors (*e.g*., thiorphan and phosphoramidon), aldosterone antagonists, aldosterone synthase inhibitors, renin inhibitors, endothelin receptor antagonists, phosphodiesterase-5 inhibitors (*e.g*., sildenafil, tadalafil and vardenafil), vasodilators, calcium channel blockers (*e.g*., amlodipine, nifedipine, verapamil, diltiazem, gallopamil, niludipine, nimodipins, nicardipine), potassium channel activators (*e.g*., nicorandil, pinacidil, cromakalim, minoxidil, aprilkalim, loprazolam), diuretics (*e.g*., hydrochlorothiazide), sympatholitics, beta-adrenergic blocking drugs (*e.g*., propranolol, atenolol, bisoprolol, carvedilol, metoprolol, or metoprolol tartate), alpha adrenergic blocking drugs (*e.g*., doxazocin, prazocin or alpha methyldopa) central alpha adrenergic agonists, peripheral vasodilators (*e.g*., hydralazine); lipid lowering agents *e.g*., HMG-CoA reductase inhibitors such as simvastatin and lovastatin which are marketed as ZOCOR^{®} and MEVACOR^{®} in lactone pro-drug form and function as inhibitors after administration, and pharmaceutically acceptable salts of dihydroxy open ring acid HMG-CoA reductase inhibitors such as atorvastatin (particularly the calcium salt sold in LIPITOR^{®}), rosuvastatin (particularly the calcium salt sold in CRESTOR^{®}), pravastatin (particularly the sodium salt sold in PRAVACHOL^{®}), fluvastatin (particularly the sodium salt sold in LESCOL^{®}), crivastatin, and pitavastatin; a cholesterol absorption inhibitor such as ezetimibe (ZETIA^{®}) and ezetimibe in combination with any other lipid lowering agents such as the HMG-CoA reductase inhibitors noted above and particularly with simvastatin (VYTORIN^{®}) or with atorvastatin calcium; niacin in immediate-release or controlled release forms and/or with an HMG-CoA reductase inhibitor; niacin receptor agonists such as acipimox and acifran, as well as niacin receptor partial agonists; metabolic altering agents including insulin and insulin mimetics (*e.g*., insulin degludec, insulin glargine, insulin lispro), dipeptidyl peptidase-IV (DPP-4) inhibitors (*e.g*., sitagliptin, alogliptin, omarigliptin, linagliptin, vildagliptin); insulin sensitizers, including (i) PPARy agonists, such as the glitazones (e.g., pioglitazone, mitoglitazone, lobeglitazone, rosiglitazone, and balaglitazone), and other PPAR ligands, including (1) PPARα/γ dual agonists (*e.g*., chiglitazar, muraglitazar, aleglitazar, sodelglitazar, and naveglitazar); (2) PPARα agonists such as fenofibric acid derivatives (*e.g*., gemfibrozil, clofibrate, ciprofibrate, fenofibrate, bezafibrate), (3) selective PPAR γ modulators (SPPAR γ M's), (*e.g.,* such as those disclosed in WO 02/060388, WO 02/08188, WO 2004/019869, WO 2004/020409, WO 2004/020408, and WO 2004/066963); and (4) PPAR γ partial agonists; (ii) biguanides, such as metformin and its pharmaceutically acceptable salts, in particular, metformin hydrochloride, and extended-release formulations thereof, such as Glumetza^{™}, Fortamet^{™}, and GlucophageXR^{™}; and (iii) protein tyrosine phosphatase-1 B (PTP-1 B) inhibitors; insulin or insulin analogs (*e.g*., insulin detemir, insulin glulisine, insulin degludec, insulin glargine, insulin lispro and inhalable formulations of each); leptin and leptin derivatives and agonists; amylin and amylin analogs (*e.g.,* pramlintide); sulfonylurea and non-sulfonylurea insulin secretagogues (*e.g.,* tolbutamide, glyburide, glipizide, glimepiride, mitiglinide, meglitinides, nateglinide and repaglinide); α-glucosidase inhibitors (*e.g*., acarbose, voglibose and miglitol); glucagon receptor antagonists; incretin mimetics, such as GLP-1, GLP-1 analogs, derivatives, and mimetics; and GLP-1 receptor agonists (*e.g*., dulaglutide, semaglutide, albiglutide, exenatide, liraglutide, lixisenatide, taspoglutide, including intranasal, transdermal, and once-weekly formulations thereof); bile acid sequestering agents (*e.g*., colestilan, colestimide, colesevalam hydrochloride, colestipol, cholestyramine, and dialkylaminoalkyl derivatives of a cross-linked dextran), acyl CoA:cholesterol acyltransferase inhibitors, (*e.g*., avasimibe); antiobesity compounds; agents intended for use in inflammatory conditions, such as aspirin, non-steroidal anti-inflammatory drugs or NSAIDs, glucocorticoids, and selective cyclooxygenase-2 or COX-2 inhibitors; glucokinase activators (GKAs); inhibitors of 11 β-hydroxysteroid dehydrogenase type 1, (*e.g.,* such as those disclosed in U.S. Patent No. 6,730,690); inhibitors of fructose 1,6-bisphosphatase, (*e.g.,* such as those disclosed in U.S. Patent Nos. 6,054,587; 6,110,903; 6,284,748; 6,399,782; and 6,489,476); inhibitors of acetyl CoA carboxylase-1 or 2 (ACC1 or ACC2); AMP-activated Protein Kinase (AMPK) activators; other agonists of the G-protein-coupled receptors: (i) GPR-109, (ii) GPR-119, and (iii) GPR-40; SSTR3 antagonists (*e.g*., such as those disclosed in WO 2009/001836); neuromedin U receptor agonists (*e.g*., such as those disclosed in WO 2009/042053, including, but not limited to, neuromedin S (NMS)); SCD modulators; GPR-105 antagonists (*e.g.,* such as those disclosed in WO 2009/000087); SGLT inhibitors (*e.g.,* empagliflozin, dapagliflozin, canagliflozin, ertugliflozin, remogloflozin, tofogliflozin, and ipragliflozin); inhibitors of acyl coenzyme A:diacylglycerol acyltransferase 1 and 2 (DGAT-1 and DGAT-2); inhibitors of fatty acid synthase; inhibitors of acyl coenzyme A:monoacylglycerol acyltransferase 1 and 2 (MGAT-1 and MGAT-2); agonists of the TGR5 receptor (also known as GPBAR1, BG37, GPCR19, GPR131, and M-BAR); ileal bile acid transporter inhibitors; PACAP, PACAP mimetics, and PACAP receptor 3 agonists; PPAR agonists; protein tyrosine phosphatase-1 B (PTP-1 B) inhibitors; IL-1β antibodies, (*e.g*., gevokizumab and canakinumab); and bromocriptine mesylate and rapid-release formulations thereof; or with other drugs beneficial for the treatment of the above-mentioned conditions or disorders including the free-acid, free-base, and pharmaceutically acceptable salt forms of the above active agents where chemically possible.

Examples of additional active agents which may be employed in treating inflammatory disorders include but are not limited to steroidal and non-steroidal anti-inflammatory agents, glucocorticoids, and therapeutic hormones. In particular embodiments, in treating hidradenitis suppurativa (*acne inversa*), the additional active agent can be an antibiotic, an injectable steroid, a therapeutic hormone, a TNF inhibitor (e.g., infliximab, adalimumab, etanercept, golimumab, certolizumab), a pain medication (e.g., codeine, hydrocodone, morphine, pregabalin, gabapentin, Intralesional triamcinolone, a corticosteroid, naproxen, ketoprofen, diclofenac, ibuprofen, acetaminophen). In other embodiments, in treating an inflammatory bowel disease, the additional active agent can be methotrexate, a TNF inhibitor, an oral sphingosine 1-phosphate receptor modulator (e.g., fingolimod, siponimod, ozanimod, ponesimod) or a selective JAK inhibitor (e.g., tofacitinib, baricitinib, upadacitinib). In some embodiments, in treating osteoarthritis, the additional active agent can be a pain medication (examples listed above). In other embodiments, in treating gouty arthritis, the additional active agent can be colcichine, a non-steroidal anti-inflammatory agent, or a glucocorticoid.

### Methods of Preparing the Compounds of the Disclosure

The compounds described herein can be prepared according to the procedures of the following schemes and examples, using appropriate materials and are further exemplified by the following specific examples. The examples also include methods for testing such compounds in cellular assays. The compounds illustrated in the examples are not, however, to be construed as forming the only genus that is considered as the disclosure.

The examples further illustrate details for the preparation of the compounds of the present disclosure. Those skilled in the art will readily understand that known variations of the conditions and processes of the following preparative procedures can be used to prepare these compounds. For instance, in some cases, the order of carrying out the steps of reaction schemes may be varied to facilitate the reaction or to avoid unwanted reaction products. Starting materials and intermediates for the final compounds are purchased, made from known procedures, or as otherwise illustrated. The examples are provided for the purpose of further illustration only and are not intended to be limitations on the disclosure.

NMR data were obtained on a 300 MHz or 400 MHz instrument in CDCl₃, DMSO-*d₆*, or Methanol-*d₄* with the chemical shifts reported relative to tetramethylsilane standard. Resonance signals are reported by the following abbreviations: s = singlet, d = doublet, t = triplet, q = quartet, dd = doublet of doublets, m = multiplet or overlap of nonequivalent resonances. Coupling constants (*J*) are reported in Hertz (Hz).

Throughout the synthetic schemes and examples, abbreviations and acronyms may be used with the following meanings unless otherwise indicated:

### Abbreviations

| **Abbreviation** | **Definition** |
|---|---|
| 1Inda | (S)-2-amino-3-(1H-indazol-1-yl)propanoic acid |
| 1Indolin | (S)-2-amino-3-(indolin-1-yl)propanoic acid |
| 1Nal | 3-(1-Naphthyl)-L-alanine |
| 3AzaPhe4AcPip | (S)-3-(6-(4-acetylpiperazin-1-yl)pyridin-3-yl)-2-aminopropanoic acid |
| 3AzaTyrEtNAc | (S)-3-(6-(2-acetamidoethoxy)pyridin-3-yl)-2-aminopropanoic acid |
| 3Pal | 3-Pyridylalanine or 3-pyridyl-L-alanine |
| 3Pal4Ph4CO2H | (S)-4-(5-(2-amino-2-carboxyethyl)pyridin-2-yl)benzoic acid |
| 4Pal | 4-Pyridylalanine or 4-pyridyl-L-alanine |
| A | L-Alanine |
| AA | Amino acid |
| AbuMph | (S)-2-amino-4-morpholinobutanoic acid |
| AcOH | Acetic acid |
| AEF | (S)-2-amino-3-(6-(2-aminoethoxy)pyridin-3-yl)propanoic acid |
| Aib | Aminoisobutyric acid or α-methylalanine |
| AlaPip4 | (S)-2-amino-3-(piperidin-4-yl)propanoic acid |
| AlaTHP4 | (S)-2-amino-3 -(tetrahydro-2H-pyran-4-yl)propanoic acid |
| Alloc-OSu | *N*-(Allyloxycarbonyloxy)succinimide |
| aMeNle | (S)-2-amino-2-methylhexanoic acid |
| ASCVD | Atherosclerotic cardiovascular disease |
| Bip4CO2H | L-Biphenylalanine-4-carboxylic acid or (S)-4'-(2-amino-2-carboxyethyl)-[1,1'-biphenyl]-4-carboxylic acid |
| BnBr | Benzyl bromide |
| Boc | *tert*-Butoxy-carbonyl |
| Boc₂O | Di-*tert*-butyl dicarbonate |
| CANTOS | Canakinumab Anti-inflammatory Thrombosis Outcomes Study |
| CDCl₃ | Deuterated chloroform |
| Cha | (S)-2-amino-3-cyclohexylpropanoic acid |
| Cha44F2 | (S)-2-amino-3-(4,4-difluorocyclohexyl)propanoic acid |
| CRP | C-reactive protein |
| D | L-Aspartic acid |
| dA | D-Alanine |
| Dab | L-2,4-diaminobutyric acid |
| daMeDab | (R)-2,4-diamino-2-methylbutanal |
| Dap | L-2,3-diaminopropionic acid |
| DapAc | (S)-3-acetamido-2-aminopropanoic acid |
| DapMe2 | (S)-2-amino-3-(dimethylamino)propanoic acid |
| DCE | Dichloroethane |
| DCM | Dichloromethane |
| dDab | D-2,4-diaminobutyric acid |
| dDabMe3 | [(R)-3-amino-3-carboxypropyl]-trimethylazanium |
| dDap | D-2,3-diaminopropionic acid |
| DIAD | Diisopropyl azodicarboxylate |
| DIC | *N,N*'-Diisopropylcarbodiimide |
| DIPEA | *N,N*-Diisopropylethylamine or Hunig's base |
| dK | D-Lysine |
| dL | D-Leucine |
| dLysAc | (R)-6-acetamido-2-aminohexanoic acid |
| DMA | N,N-Dimethylacetamide |
| DMAP | 4-Dimethylaminopyridine |
| DMF | *N,N-*Dimethylformamide |
| DMSO | Dimethylsulfoxide |
| dNle | D-Norleucine |
| dNMeA | D-*N*-methyl-Alanine |
| dNva | D-Norvaline |
| dOrn | D-Ornithine |
| dS | D-Serine |
| E | L-Glutamic acid |
| ES | Electrospray |
| EtOAc | Ethyl acetate |
| F | L-Phenylalanine |
| F4bcpA | 3-[4-[(S)-2-amino-2-carboxyethyl]phenyl]bicyclo[1.1.1]penta ne-1-carboxylic acid |
| F4pcCCA | (1R,4s)-4-(4-((S)-2-amino-2-carboxyethyl)phenyl)cyclohexane-1-carboxylic acid |
| F4ptCCA | (1S,4r)-4-(4-((S)-2-amino-2-carboxyethyl)phenyl)cyclohexane-1-carboxylic acid |
| Fmoc | 9*H*-fluoren-9-ylmethoxycarbonyl |
| G | Glycine |
| h | hour |
| h3Pal5CN | (S)-2-amino-4-(5-cyanopyridin-3-yl)butanoic acid |
| h4Pal | (S)-2-amino-4-(pyridin-4-yl)butanoic acid |
| HATU | 1-[Bis(dimethylamino)methylene]-1H-1,2,3-triazolo[4,5-b]pyridinium 3-oxid hexafluorophosphate or N-[(Dimethylamino)-1H-1,2,3-triazolo-[4,5-b]pyridin-1-ylmethylene]-N-methylmethanaminium hexafluorophosphate N-oxide |
| hCha | (S)-2-amino-4-cyclohexylbutanoic acid |
| hF | L-homoPhenylalanine |
| hF2CF3 | (S)-2-amino-4-(2-(trifluoromethyl)phenyl)butanoic acid |
| hF3CF3 | (S)-2-amino-4-(3-(trifluoromethyl)phenyl)butanoic acid |
| hF4CF3 | (S)-2-amino-4-(4-(trifluoromethyl)phenyl)butanoic acid |
| HFIP | Hexafluoroisopropanol |
| HFpEF | Heart failure with preserved ejection fraction |
| hK | L-homoLysine |
| HPLC | High-performance liquid chromatography |
| hQ | L-homoGlutamine |
| hsCRP | High sensitivity C-reactive protein |
| hSOMe | (S)-2-amino-4-methoxybutanoic acid |
| hY | L-homoTyrosine |
| Im | 1*H*-imidazole |
| K | L-Lysine |
| L | L-Leucine |
| LDL | low-density lipoprotein |
| LysAc | (S)-6-acetamido-2-aminohexanoic acid |
| MACE | Major adverse cardiac events |
| mAF | (S)-2-amino-3-(3-(aminomethyl)phenyl)propanoic acid |
| mBip | (S)-2-amino-3-(3-phenylphenyl)propanoic acid |
| MeCN | Acetonitrile |
| MeOH | Methanol |
| min | Minute |
| MS | Mass spectrometry |
| mTyrOAl | (S)-2-amino-3-(3-prop-2-enoxyphenyl)propanoic acid |
| N | L-Asparagine |
| NIS | N-iodosuccinimide |
| Nle | L-Norleucine |
| NMP | *N*-Methyl-2-pyrrolidone |
| NMR | Nuclear magnetic resonance |
| Nva | L-Norvaline |
| NsCl | 4-Nitrobenzenesulfonyl chloride |
| o.n. | Over night |
| OMpe | β-methylpentyl ester |
| Orn | L-Ornithine |
| OSu | O-succinimide |
| Oxyma | Ethyl cyano(hydroxyimino)acetate |
| PE | Petroleum ether |
| PfTrpB-0B2 | Engineered tryptophan synthase from Pyrococcus furiosus |
| PfTrpB-4D 11 | Engineered tryptophan synthase from Pyrococcus furiosus |
| PfTrpB-7E6 | Engineered tryptophan synthase from Pyrococcus furiosus |
| Phe3NH2 | (S)-2-amino-3-(3-aminophenyl)propanoic acid |
| Phe4AcPip | (S)-2-amino-3-(4-(4-acetylpiperazin-1-yl)phenyl)propanoic acid |
| Phe4CH2NH2 | (S)-2-amino-3-[4-(aminomethyl)phenyl]propanoic acid |
| Phe4COOH | L-phenylalanine-4-carboxylic acid or (S)-4-(2-amino-2-carboxyethyl)benzoic acid |
| Phe4NH2 | (S)-2-amino-3-(4-aminophenyl)propanoic acid |
| Phe4Pyrim5CO2H | (S)-2-(4-(2-amino-2-carboxyethyl)phenyl)pyrimidine-5-carboxylic acid |
| PLP | 3-hydroxy-2-methyl-5-([phosphonooxy]methyl)-4-pyridinecarboxaldehyde or pyridoxal 5-phosphate |
| PPH₃ | Triphenylphosphine |
| Prot4NH2 | (2S,4R)-4-aminopyrrolidine-2-carboxylic acid |
| Prot4NHBn | (2S,4R)-4-(benzylamino)pyrrolidine-2-carboxylic acid |
| Prot4NHCH2CH2Ph | (2S,4R)-4-(phenethylamino)pyrrolidine-2-carboxylic acid |
| Prot4NHEt | (2S,4R)-4-(ethylamino)pyrrolidine-2-carboxylic acid |
| psi | Pound-force per square inch |
| PyAOP | (7-Azabenzotriazol-1-yloxy)tripyrrolidinophosphonium hexafluorophosphate |
| PyrimAla | (S)-2-amino-3-(pyrimidin-5-yl)propanoic acid |
| PyrimAla4AcPip | (S)-3-(2-(4-acetylpiperazin-1-yl)pyrimidin-5-yl)-2-aminopropanoic acid |
| Q | L-Glutamine |
| RP | Reverse phase |
| rpm | Revolutions per minute |
| rt | Room temperature |
| S | L-Serine |
| SerOMe | O-methyl-L-serine |
| sbMe1Nal | (2S,3S)-2-amino-3-(naphthalen-1-yl)butanoic acid |
| sbMeW4Cl | (2S,3 S)-2-amino-3-(4-chloro-1H-indol-3-yl)butanoic acid |
| sbMeW4F | (2S,3S)-2-amino-3-(4-fluoro-1H-indol-3-yl)butanoic acid |
| SFC | Supercritical fluid chromatography |
| SPPS | Solid-Phase Peptide Synthesis |
| *t*Bu | *tert*-Butyl |
| *t*-BuOH | *tert*-Butanol |
| Tba | (S)-2-amino-4,4-dimethylpentanoic acid |
| TBAB | Tetrabutylammonium bromide |
| TBAI | Tetrabutylammonium iodide |
| TEA | Triethylamine |
| TFA | Trifluoroacetic acid |
| THF | Tetrahydrofuran |
| TIS | Triisopropylsilane |
| Trp6Cl | (S)-2-amino-3-(6-chloro-1H-indol-3-yl)propanoic acid |
| Trp6F | (S)-2-amino-3-(6-fluoro-1H-indol-3-yl)propanoic acid |
| Trp7F | (S)-2-amino-3-(7-fluoro-1H-indol-3-yl)propanoic acid |
| Trp7az | (S)-2-amino-3-(1H-pyrrolo[2,3-b]pyridin-3-yl)propanoic acid |
| Trp7Me | (S)-2-amino-3-(7-methyl-1H-indol-3-yl)propanoic acid |
| TyrEtMor | (S)-2-amino-3 -(4-(2-morpholinoethoxy)phenyl)propanoic acid |
| TyrEtNAc | L-Tyrosine *O*-ethyl acetamide or (S)-3-(4-(2-acetamidoethoxy)phenyl)-2-aminopropanoic acid |
| UPLC | Ultra Performance Liquid Chromatography or Ultrahigh Pressure Liquid Chromatography |
| W | L-Tryptophan |

### Intermediate Syntheses:

### Synthetic Scheme 1

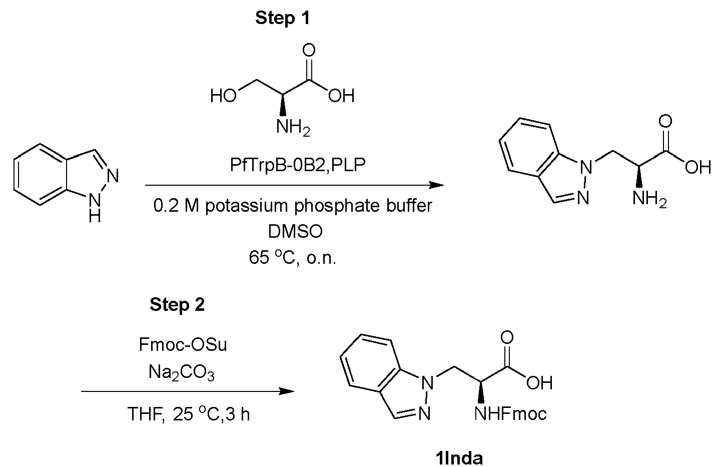

### 1Inda

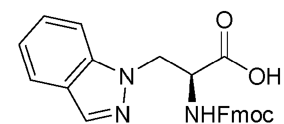

### (S)-2-((((9H-fluoren-9-yl)methoxy)carbonyl)amino)-3-(1H-indazol-1-yl)propanoic acid

Step 1: In to a 2-L 4-necked round-bottom flask purged and maintained with an inert atmosphere of nitrogen, was placed DMSO (150 mL), 1H-indazole (15 g, 127 mmol) and (S)-2-amino-3-hydroxypropanoic acid (14.01 g, 133 mmol), and pH 8 0.2 M potassium phosphate buffer (600 mL, 127 mmol) at 65 °C. [(4-formyl-5-hydroxy-6-methylpyridin-3-yl)methoxy] phosphonic acid (PLP) (96 mg, 0.00284 equiv) in H₂O (2 mL) and PfTrpB-0B2 (1.05 g, 10 wt%) in 0.2 M potassium phosphate buffer (2 mL) were added into the solution. The resulting solution was stirred for 16 h at 65 °C. The reaction progress was monitored by HPLC-MS.

Step 2: Into the resulting solution was added Na₂CO₃ (21.55 g, 2.0 eq) and Fmoc-OSu (37.68 g, 1.1 eq) in THF (150 mL) at 0 °C. The solution was then stirred for 2 h at 25 °C. The reaction progress was monitored by HPLC-MS. The solution was adjusted to pH 3 with hydrogen chloride (6 M). The solution was extracted with EtOAc (3 x 500 mL). The organic layers were combined and washed with H₂O (2 x 1 L) and NaCl (1 x 1 L). The organic layer was dried over anhydrous sodium sulfate and concentrated under vacuum. The crude product was slurried with DCM (15V) and the solids were collected by filtration. HPLC-MS: (ESI, m/z): 428 [M+H] +. ¹H NMR: (400 MHz, DMSO-*d₆*, ppm): δ 13.08 (s, 1H), 8.10 (d, J = 1.0 Hz, 1H), 7.87 (dt, J = 7.7, 1.0 Hz, 2H), 7.81 - 7.71 (m, 2H), 7.71 - 7.65 (m, 1H), 7.58 (d, J = 7.5 Hz, 1H), 7.54 (d, J = 7.5 Hz, 1H), 7.41 (dtd, J = 6.0, 4.4, 4.3, 3.2 Hz, 2H), 7.40 - 7.33 (m, 1H), 7.36 - 7.24 (m, 1H), 7.29 - 7.20 (m, 1H), 7.16 - 7.08 (m, 1H), 4.83 - 4.65 (m, 2H), 4.61 - 4.51 (m, 1H), 4.22 - 4.06 (m, 3H).

### Synthetic Scheme 2

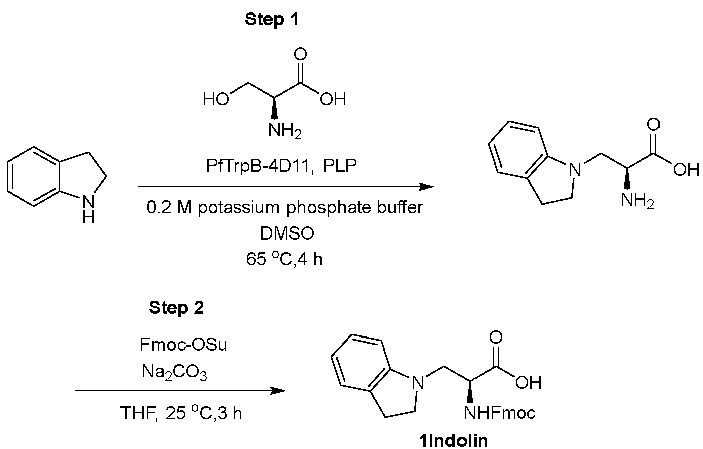

### 1Indolin

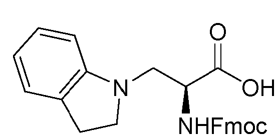

### (S)-2-((((9H-fluoren-9-yl)methoxy)carbonyl)amino)-3-(indolin-1-yl)propanoic acid

Step 1: Into a 10 L 3-necked round-bottom flask were added indoline (125 g, 1.05 mol, 1.00 eq.), DMSO (625 mL), pH 8 0.2 M potassium phosphate buffer (5625 mL) and PLP (778.32 mg, 2.94 mmol), L-serine (115.75 g, 1.10 mol, 1.05 eq.) and PfTrpB-4D11 (12.50 g) at 25 °C. The resulting solution was stirred for 4 h at 65 °C under nitrogen. The reaction progress was monitored by HPLC-MS.

Step 2: Into the above solution were added Na₂CO₃ (129 g, 2.00 eq.), Fmoc-OSu (306 g, 1.50 eq.) and THF (2.5 L) at 0 °C. The reaction mixture was stirred for 3 h at 25 °C under nitrogen. The solution was acidified to pH 3 with 6 M HCl. The mother liquid was collected by filtration, then extracted with EtOAc (3 x 1 L). The organic layers were combined and washed with H₂O (2 x 800 mL) and NaCl (1 x 800 mL), dried over Na₂SO₄ and concentrated under vacuum. The residue was purified by silica gel column chromatography, eluted with PE/EtOAc (3:1) to afford the crude product. Then the crude product was purified by preparative HPLC Reverse phase (C18), eluting with Acetonitrile/Water + 0.05% TFA. 50% to 70% gradient in 30 min. The mixture was concentrated to remove MeCN under vacuum at 20 °C, then extracted with EtOAc (3 x 1 L), dried over Na₂SO₄ and concentrated under vacuum. The pure product was slurried in hexanes (1 L) for 30 min. Solids were collected by filtration. This resulted in (2S)-3-(2,3-dihydroindol-1-yl)-2-[[(9H-fluoren-9-ylmethoxy)carbonyl]amino] propanoic acid. HPLC-MS: (ESI, m/z): 429 [M+H] +. ¹H NMR: (400 MHz, DMSO-*d₆*, ppm) δ 7.89 (d, J = 7.5 Hz, 2H), 7.74 - 7.67 (m, 2H), 7.41 (t, J = 7.5 Hz, 2H), 7.35 - 7.25 (m, 2H), 7.00 (ddd, J = 17.4, 7.5, 1.3 Hz, 2H), 6.61 - 6.48 (m, 2H), 4.35 - 4.18 (m, 4H), 3.47 - 3.28 (m, 4H), 2.87 (t, J = 8.2 Hz, 2H).

### Synthetic Scheme 3

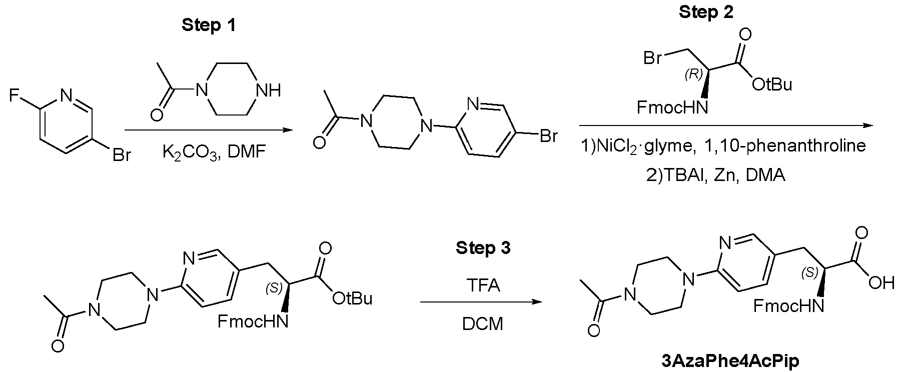

### 3AzaPhe4AcPip

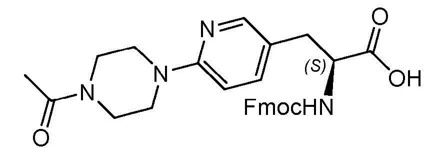

### (S)-2-((((9H-fluoren-9-yl)methoxy)carbonyl)amino)-3-(6-(4-acetylpiperazin-1-yl)pyridin-3-yl)propanoic acid

Step 1: To a mixture of 1-(piperazin-1-yl)ethan-1-one (21.85 g, 170 mmol) in DMF (150 mL) was added 5-bromo-2-fluoropyridine (15 g, 85 mmol) under argon at rt. The reaction was stirred at 100 °C for 2 h, then diluted with 300 mL EtOAc and washed with H₂O (3 x 80 mL), aqueous saturated NaCl (80 mL), dried over Na₂SO₄ and filtered. The filtrate was concentrated under reduced pressure. The residue was purified by silica gel column chromatography, eluting with a gradient of 0% - 100% EtOAc in PE. The fractions containing desired product were combined and concentrated under reduced pressure to afford 1-(4-(5-bromopyridin-2-yl)piperazin-1-yl)ethan-1-one. MS ESI calculated for C11H15BrN3O [M + H]⁺ 284.03 and 286.03, found 283.90 and 285.90.

Step 2: The mixture of nickel (II) chloride ethylene glycol dimethyl ether complex (0.696 g, 3.17 mmol) and 1,10-phenanthroline (0.571 g, 3.17 mmol) in DMA (2 mL) was heated at 50 °C for 0.5 hours. The mixture of 1-(4-(5-bromopyridin-2-yl)piperazin-1-yl)ethan-1-one (4.5 g, 15.84 mmol), tert-butyl (R)-2-((((9H-fluoren-9-yl)methoxy)carbonyl)amino)-3-bromopropanoate (7.78 g, 17.42 mmol) and tetrabutylammonium iodide (5.85 g, 15.84 mmol) in DMA (2 mL) were added at 25 °C. Then zinc powder (2.071 g, 31.7 mmol) was added. The resulting mixture was stirred for 1 h at 50 °C. The resulting mixture was poured into water (300 mL) and extracted with EtOAc (3 x 300 mL). The organic layer was washed with water (100 mL) and brine (2 x 80 mL), dried over anhydrous sodium sulfate and concentrated under reduced pressure. The residue was purified by column chromatography on silica gel eluting with DCM-MeOH (10:1) to afford tert-butyl (S)-2-((((9H-fluoren-9-yl)methoxy)carbonyl)amino)-3-(6-(4-acetylpiperazin-1-yl)pyridin-3-yl)propanoate. MS ESI calculated for C33H39N4O5 [M + H]⁺ 571.28, found 571.40.

Step 3: To a mixture of tert-butyl (S)-2-((((9H-fluoren-9-yl)methoxy)carbonyl)amino)-3-(6-(4-acetylpiperazin-1-yl)pyridin-3-yl)propanoate (7.3 g, 12.79 mmol) in DCM (70 mL) was added TFA (70 mL, 909 mmol) under argon at rt. The reaction was stirred at rt for 1 h then concentrated under reduced pressure. The residue was dissolved in THF (20 mL) and the resultant mixture was purified by reverse phase Combi-Flash with the following conditions: Column C18 silica gel column (330 g), 20-35 µm; Mobile Phase A: 5 mM aq. TFA; Mobile Phase B: MeCN; (Gradient: 0% B hold 10 min, up to 42.3% B within 35 min, 42.3% B hold 3.2 min; up to 95% B within 2 min, 95% B hold 10 min); Flow rate: 60 mL/min; Detector: UV 254 & 210 nm; RT: 35.32 min. The product-containing fractions were collected and concentrated under vacuum to afford (S)-2-((((9H-fluoren-9-yl)methoxy)carbonyl)amino)-3-(6-(4-acetylpiperazin-1-yl)pyridin-3-yl)propanoic acid. MS ESI calculated for C29H31N4O5 [M + H]⁺ 515.22, found 515.15. ¹H NMR (300 MHz, Methanol-*d₄*) δ 7.87 - 7.79 (m, 4H), 7.62 - 7.55 (m, 2H), 7.42 - 7.27 (m, 4H), 7.11 - 7.09 (m, 1H), 4.51 - 4.46 (m, 1H), 4.32 - 4.09 (m, 3H), 3.65 - 3.54 (m, 8H), 3.29 - 3.20 (m, 1H), 2.94 - 2.89 (m, 1H), 2.12 (s, 3H).

### Synthetic Scheme 4

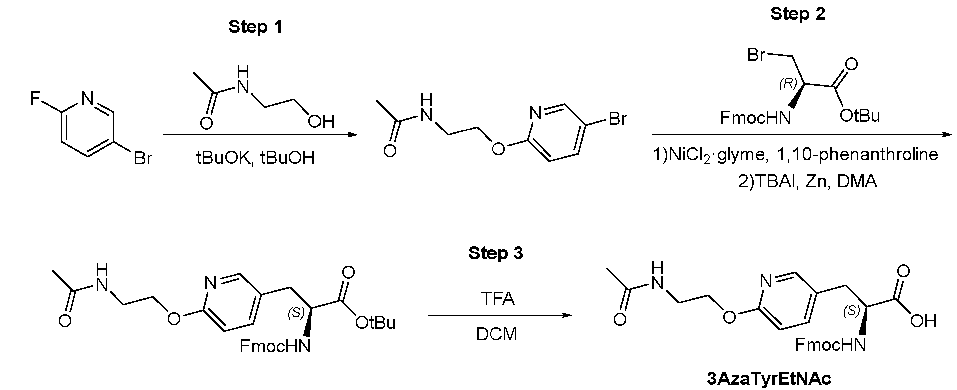

### 3AzaTyrEtNAc

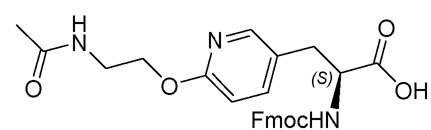

### (S)-2-((((9H-fluoren-9-yl)methoxy)carbonyl)amino)-3-(6-(2-acetamidoethoxy)pyridin-3-yl)propanoic acid

Step 1: To a mixture of N-(2-hydroxyethyl)acetamide (17.58 g, 170 mmol) in t-BuOH (150 mL) was added under argon at rt 5-bromo-2-fluoropyridine (15 g, 85 mmol) followed by potassium tert-butoxide (19.13 g, 170 mmol). The reaction was stirred at rt for 1 h then concentrated under reduced pressure. The residue was diluted with 500 mL EtOAc and washed with aqueous saturated NaHCO₃ (3 x 250 mL), aqueous saturated NaCl (250 mL), dried over Na₂SO₄ and filtered. The filtrate was concentrate under reduced pressure to afford crude N-(2-((5-bromopyridin-2-yl)oxy)ethyl)acetamide. MS ESI calculated for C9H12BrN2O2 [M + H]⁺ 259.00 and 261.00, found 258.90 and 261.90.

Step 2: The mixture of nickel (II) chloride ethylene glycol dimethyl ether complex (1.187 g, 5.40 mmol) and 1,10-phenanthroline (0.974 g, 5.40 mmol) in DMA (70 mL) was heated at 50 °C for 0.5 hours. The mixture of N-(2-((5-bromopyridin-2-yl)oxy)ethyl)acetamide (7 g, 27.0 mmol), tert-butyl (R)-2-((((9H-fluoren-9-yl)methoxy)carbonyl)amino)-3-bromopropanoate (13.26 g, 29.7 mmol) and tetrabutylammonium iodide (9.98 g, 27.0 mmol) in DMA (70 mL) were added at 25 °C. Then zinc powder (3.53 g, 54.0 mmol) was added and the resulting mixture was stirred for 1 h at 50 °C. The reaction mixture was diluted with 300 mL EtOAc and washed with aqueous saturated NaHCO₃ (3 x 80 mL), brine (80 mL), dried over Na₂SO₄ and filtered. The filtrate was concentrated under reduced pressure. The residue was purified by silica gel column chromatography, eluting with gradient 0% - 20% DCM in MeOH. The fractions containing the desired product were combined and concentrated under reduced pressure to afford tert-butyl (S)-2-((((9H-fluoren-9-yl) methoxy)carbonyl)amino)-3-(6-(2-acetamidoethoxy)pyridin-3-yl)propanoate. MS ESI calculated for C31H36N3O6 [M + H]⁺ 546.25, found 546.40.

Step 3: To a mixture of tert-butyl (S)-2-((((9H-fluoren-9-yl)methoxy)carbonyl)amino)-3-(6-(2-acetamidoethoxy)pyridin-3-yl)propanoate (8 g, 14.66 mmol) in DCM (80 mL) was added TFA (80 mL, 1038 mmol) under argon at rt. The reaction was stirred at rt for 1 h then concentrated under reduced pressure. The residue was purified by RP flash column chromatography with the following conditions: Column: C18 silica gel column (330 g), 20-35 µm; Mobile Phase A: 5 mM aq. NH₄HCO₃; Mobile Phase B: MeCN; (Gradient: 0% B hold 5 min, up to 45% B within 20 min, 45% B hold 10 min; up to 95% B within 15 min, 95% B hold 10 min); Flow rate: 60 mL/min; Detector: UV 254 & 210 nm; RT: 35.32 min. The product-containing fractions were collected and concentrated under reduced pressure to give (S)-2-((((9H-fluoren-9-yl)methoxy)carbonyl)amino)-3-(6-(2-acetamidoethoxy)pyridin-3-yl)propanoic acid. MS ESI calculated for C27H28N3O6 [M + H]⁺ 490.19, found 490.10. ¹H NMR (400 MHz, Methanol-*d₄*) δ 8.02 - 8.00 (m, 1H), 7.79 - 7.77 (m, 2H), 7.67 - 7.64 (m, 1H), 7.61 - 7.57 (m, 2H), 7.40 - 7.36 (m, 2H), 7.31 - 7.27 (m, 2H), 6.82 - 6.80 (m, 1H), 4.42 - 4.39 (m, 1H), 4.33 - 4.22 (m, 4H), 4.16 - 4.13 (m, 1H), 3.53 - 3.51 (m, 2H), 3.20 - 3.15 (m, 1H), 2.93 - 2.87 (m, 1H), 1.92 (s, 3H).

### Synthetic Scheme 5

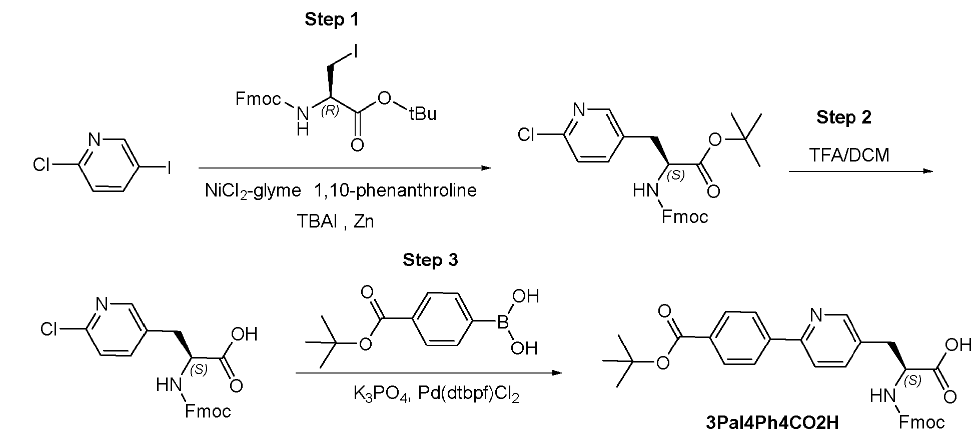

### 3Pal4Ph4CO2H

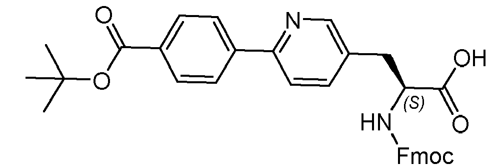

### (S)-2-((((9H-fluoren-9-yl)methoxy)carbonyl)amino)-3-(6-(4-(tert-butoxycarbonyl)phenyl)pyridin-3-yl)propanoic acid

Step 1: To a stirred solution of NiCl₂-glyme (0.918 g, 4.18 mmol) in DMA (100 mL) was added 1,10-phenanthroline (0.905 g, 4.18 mmol) at rt under nitrogen atmosphere. The resulted solution was stirred at 50 °C for 1 h. 2-Chloro-5-iodopyridine (5 g, 20.88 mmol), *tert*-butyl (*R*)-2-((((9*H*-fluoren-9-yl)methoxy)carbonyl)amino)-3-iodopropanoate (12.36 g, 25.06 mmol), TBAI (8.01 g, 20.88 mmol) and Zn (2.73 g, 41.8 mmol) were added to the mixture above at rt and the resulted mixture was stirred at 25 °C for 2 h. The reaction was quenched with H₂O (200 mL), extracted with EtOAc (2 x 500 mL). The combined organic layer was washed with brine (3 x 200 mL), dried over anhydrous Na₂SO₄ and filtered. The filtrate was concentrated under reduced pressure and the residue was purified by silica gel column chromatography, eluted with 0 - 30% EtOAc in PE to give *tert*-butyl (*S*)-2-((((9*H*-fluoren-9-yl)methoxy)carbonyl)amino)-3-(6-chloropyridin-3-yl)propanoate. MS ESI calculated for C₂₇H₂₈ClN₂O₄ [M + H]⁺ 479.17, found 479.20.

Step 2: To a stirred solution of *tert-*butyl (*S*)-2-((((9*H*-fluoren-9-yl)methoxy)carbonyl)amino)-3-(6-chloropyridin-3-yl)propanoate (5 g, 10.48 mmol) in DCM (5 mL) was added TFA (10 mL) at rt. The solution was stirred at 25 °C for 1 h. The solvent was concentrated under reduced pressure and the residue was purified by RP-flash with the following conditions: Column: Flash C18 (330 g); Mobile Phase A: water (0.1% TFA), Mobile Phase B: MeCN; (Gradient: 5% B hold 5 min, up to 30% B within 15 min, 30% B hold 5 min; up to 95% B within 20 min, 95% B hold 10 min); Flow rate: 90 mL/min; Detector: UV 210 nm; RT = 40 min. The product-containing fractions were collected and evaporated in vacuo to give (*S*)-2-((((9*H*-fluoren-9-yl)methoxy)carbonyl)amino)-3-(6-chloropyridin-3-yl)propanoic acid. MS ESI calculated for C₂₃H₂₀ClN₂O₄ [M + H]⁺ 423.10, found 423.10.

Step 3: To a stirred solution of (*S*)-2-((((9*H*-fluoren-9-yl)methoxy)carbonyl)amino)-3-(6-chloropyridin-3-yl)propanoic acid (3 g, 7.09 mmol) in THF (25 mL) and water (5 mL) were added (4-(*tert*-butoxycarbonyl)phenyl)boronic acid (1.890 g, 8.51 mmol) and K₃PO₄ (7.53 g, 35.5 mmol) at 25 °C under nitrogen. The resultant solution was stirred at 25 °C for 10 min. Pd(dtbpf)Cl₂ (0.694 g, 1.064 mmol) was added to the solution and the mixture was then stirred at 60 °C for 16 h. The reaction was cooled to rt and quenched with H₂O (200 mL) and extracted with EtOAc (2 x 500 mL). The combined organic layer was washed with brine (3 x 200 mL), dried over anhydrous Na₂SO₄, and filtered. The filtrate was concentrated under reduced pressure and the residue was purified by RP-flash with the following conditions: Column: Flash C18 (330 g); Mobile Phase A: water (0.1% TFA), Mobile Phase B: MeCN; (Gradient: 5% B hold 5 min, up to 60% B within 15 min, 60% B hold 15 min; up to 95% B within 10 min, 95% B hold 10 min); Flow rate: 90 mL/min; Detector: UV 210 nm; RT = 55 min. The product-containing fractions were collected and evaporated in vacuo to give (*S*)-2-((((9*H*-fluoren-9-yl)methoxy)carbonyl)amino)-3-(6-(4-(*tert*-butoxycarbonyl)phenyl)pyridin-3-yl)propanoic acid. MS ESI calculated for C₃₄H₃₃N₂O₆ [M + H]⁺ 565.23, found 565.15; ¹H NMR (400 MHz, Methanol-*d₄*) δ 8.66 (d, *J* = 1.9 Hz, 1H), 8.12 - 8.07 (m, 3H), 7.99 - 7.92 (m, 3H), 7.77 (d, *J* = 7.5 Hz, 2H), 7.59 - 7.56 (m, 2H), 7.37 - 7.33 (m, 2H), 7.30 - 7.22 (m, 2H), 4.60 - 4.56 (m, 1H), 4.29 - 4.27 (m, 2H), 4.14 - 4.10 (m, 1H), 3.45 - 3.41 (m, 1H), 3.14 - 3.10 (m, 1H), 1.62 (s, 9H).

### Synthetic Scheme 6

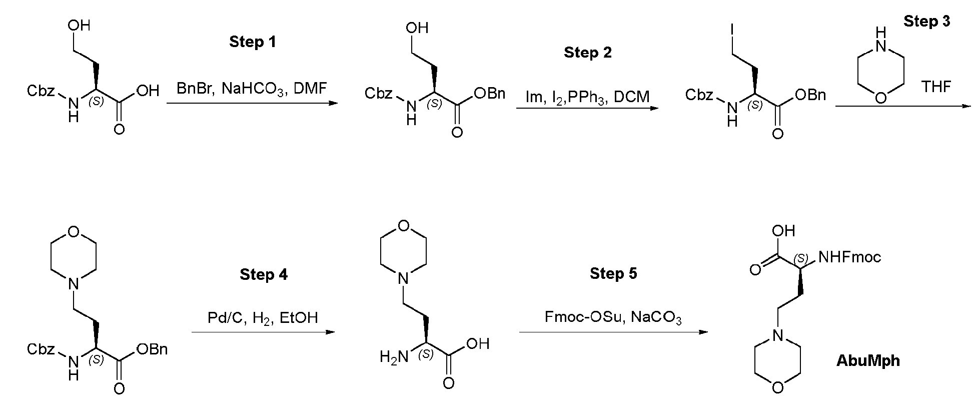

### AbuMph

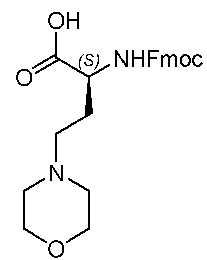

### (S)-2-((((9H-fluoren-9-yl)methoxy)carbonyl)amino)-4-morpholinobutanoic acid

Step 1: To a stirred mixture of ((benzyloxy)carbonyl)-L-homoserine (2.5 g, 9.87 mmol) and NaHCO₃ (2.488 g, 29.6 mmol) in DMF (50 mL) was added benzyl bromide (2.026 g, 11.85 mmol) at 0 °C under nitrogen atmosphere. The mixture was stirred at 25 °C for 16 h then quenched with water (200 mL), extracted with EtOAc (3 x 200 mL). The combined organic layer was washed with brine (3 x 100 mL), dried over anhydrous Na₂SO₄. After filtration, the filtrate was concentrated under reduced pressure and the residue was purified by a silica gel column chromatography, eluted with 0 - 45% EtOAc in PE to afford benzyl ((benzyloxy)carbonyl)-L-homoserinate. MS ESI calculated for C₁₉H₂₁NO₅Na [M + Na]⁺ 366.14, found 366.15.

Step 2: Under N₂ atmosphere in a two-neck 500 mL flask, I₂ (22.17 g, 87 mmol) was added to a solution of PPh₃ (17.19 g, 65.5 mmol) and 1*H*-imidazole (4.46 g, 65.5 mmol) in anhydrous CH₂Cl₂ (100 mL) at 0 °C. The reaction was warmed to 25 °C and stirred for 15 min. A solution of the benzyl ((benzyloxy)carbonyl)-*L*-homoserinate (15 g, 43.7 mmol) in CH₂Cl₂ (100 mL) was added over 10 minutes and then stirred at rt for 2 h. The mixture was washed by saturated Na₂S₂O₃solution (3 x 200 mL), followed by brine (3 x 50 mL). The organic layer was dried using Na₂SO₄, the filtrate was concentrated under reduced pressure and the residue was purified by silica gel column chromatography, eluted with 0 - 40% EtOAc in PE to afford benzyl (S)-2-(((benzyloxy)carbonyl)amino)-4-iodobutanoate. MS ESI calculated for C₁₉H₂₁INO₄ [M + H]⁺ 454.04, found 454.05. ¹H NMR (400 MHz, CDCl₃) δ 7.45 - 7.31 (m, 10H), 5.37 - 5.35 (m, 1H), 5.19 - 5.15 (m, 2H), 5.11 (s, 2H), 4.48 - 4.41 (m, 1H), 3.15 - 3.10 (m, 2H), 2.46 - 2.44 (m, 1H), 2.25 - 2.20 (m, 1H).

Step 3: To a stirred mixture of benzyl (*S*)-2-(((benzyloxy)carbonyl)amino)-4-iodobutanoate (13.6 g, 30.0 mmol) dissolved in THF (140 mL) was added morpholine (20.91 g, 240 mmol) at rt. The resulting mixture was stirred for 16 h at 50 °C. The reaction mixture was concentrated under reduced pressure and the residue was purified by silica gel column chromatography, eluted with 0 ~ 80% EtOAc in PE and the product-containing fractions were collected and concentrated under reduced pressure to afford benzyl (*S*)-2-(((benzyloxy)carbonyl)amino)-4-morpholinobutanoate. MS ESI calculated for C₂₃H₂₉N₂O₅ [M + H]⁺ 413.21, found 413.35. ¹H NMR (400 MHz, CDCl₃) δ 7.36 - 7.34 (m, 10H), 6.67 (s, 1H), 5.24 - 5.02 (m, 4H), 4.52 - 4.47 (m, 1H), 3.67 - 3.52 (m, 4H), 2.38 - 2.51 (m, 6H), 2.15 - 1.81 (m, 2H).

Step 4: The benzyl (S)-2-(((benzyloxy)carbonyl)amino)-4-morpholinobutanoate (6.5 g, 15.76 mmol) was dissolved in EtOH (65 mL) and the resulting mixture was evacuated and an atmosphere of nitrogen was applied at ambient temperature. Then Pd-C (2.6 g, dry, 20%wt) was added under nitrogen atmosphere. The suspension was degassed under vacuum and purged with H₂ several times, the reaction solution was stirred for 5 h at 25 °C under 1 atm H₂. The suspension was filtered and the filtrate was concentrated under reduced pressure to give crude (*S*)-2-amino-4-morpholinobutanoic acid. MS ESI calculated for C₈H₁₇N₂O₃ [M + H]⁺ 189.12, found 189.25.

Step 5: To a solution of (S)-2-amino-4-morpholinobutanoic acid (2.97 g, 15.76 mmol) in THF (25 mL) and water (25 mL) were added NaHCO₃ (6.63 g, 79 mmol) and Fmoc-OSu (4.85 g, 14.20 mmol) at 25 °C. The reaction mixture was stirred at 25 °C for 16 h. Upon completion, the pH value of the solution was adjusted to 3 with 1 M HCl. The mixture was purified by reverse phase flash chromatography with the following conditions: C18 column (120 g); Mobile Phase A: water (0.05% TFA), Mobile Phase B: MeCN; (Gradient: 5% B hold 5 min, up to 38 % B within 30 min, 38% B hold 2.6 min; up to 95% B within 2 min, 95% B hold 5 min); Flow rate: 60 mL/min; Detector: UV 210 nm; RT = 31 min. The product-containing fractions were collected and concentrated under reduced pressure to give (*S*)-2-((((9*H*-fluoren-9-yl)methoxy)carbonyl)amino)-4-morpholinobutanoic acid. MS ESI calculated for C₂₃H₂₇N₂O₅ [M - H]⁻ 411.19, found 411.30. ¹H NMR (300 MHz, Methanol-*d₄*) δ 7.81 - 7.79 (m, 2H), 7.68 - 7.65 (m, 2H), 7.42 - 7.37 (m, 2H), 7.33 - 7.29 (m, 2H), 4.48 - 4.45 (m, 2H), 4.42 - 4.23 (m, 2H), 4.01 - 3.77 (m, 4H), 3.33 - 3.30 (m, 2H), 3.29 - 3.14 (m, 4H), 2.45 - 2.05 (m, 2H).

### Synthetic Scheme 7

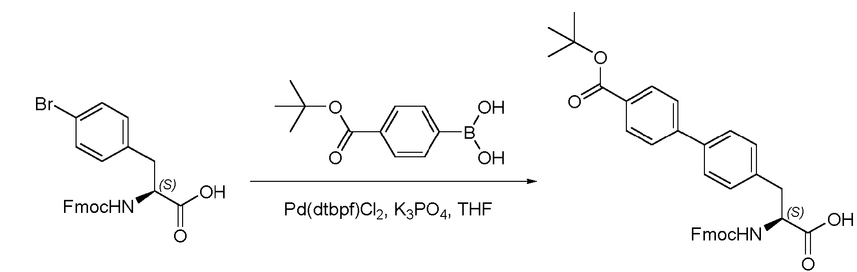

### Bip4CO2H

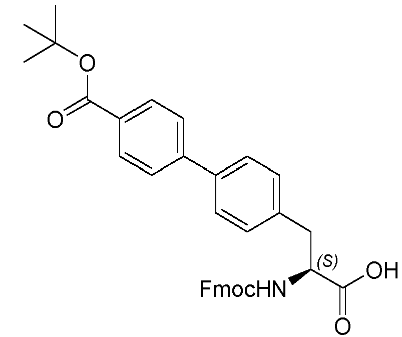

### (S)-2-((((9H-fluoren-9-yl)methoxy)carbonyl)amino)-3-(4'-(tert-butoxycarbonyl)-[1,1'-biphenyl]-4-yl)propanoic acid

Argon gas was bubbled through a mixture of (*S*)-2-((((9*H*-fluoren-9-yl)methoxy)carbonyl)amino)-3-(4-bromophenyl)propanoic acid (6 g, 12.87 mmol), (4-(*tert-*butoxycarbonyl)phenyl)boronic acid (4.29 g, 19.30 mmol) and K₃PO₄ (8.19 g, 38.6 mmol) in THF (40 mL) for 10 min, then [1,1'-bis(di-*tert*-butylphosphino)ferrocene]dichloropalladium (II) (0.839 g, 1.287 mmol) was added. After the resulting mixture was stirred at 50 °C for 16 h, it was diluted with EtOAc (300 mL) and washed with aqueous saturated NaHCO₃ (3 x 80 mL), brine (2 x 40 mL), dried over Na₂SO₄ and filtered. The filtrate was concentrated under reduced pressure. The residue was purified by silica gel column chromatography, eluted with gradient 0% - 50% EtOAc in PE. The product-containing fractions were collected and roto-evaporated *in vacuo.* The residue was re-purified by combi-Flash with the following conditions: Column: Column: C18 gel column (330 g), 20-35 µm; Mobile Phase A: 0.5% aq. TFA; Mobile Phase B: MeCN; (Gradient: 0% B hold 10 min, up to 62.3% B within 25 min, 62.3% B hold 6.2 min; up to 95% B within 2 min, 95% B hold 10 min); Flow rate: 90 mL/min; Detector: UV 254 & 210 nm; RT: 32.32 min. The product-containing fractions were collected and concentrated under reduced pressure to afford (*S*)-2-((((9*H*-fluoren-9-yl)methoxy)carbonyl)amino)-3-(4'-(*tert*-butoxycarbonyl)-[1,1'-biphenyl]-4-yl)propanoic acid. MS ESI calculated for C₃₅H₃₄NO₆ [M + 1]⁺ 564.23, found 564.15. ¹H NMR (300 MHz, Methanol-*d₄*) δ 7.97 - 7.95 (m, 2H), 7.78 - 7.76 (m, 2H), 7.61 - 7.53 (m, 6H), 7.38 - 7.21 (m, 2H), 4.51 - 4.11 (m, 4H), 3.32 - 3.25 (m, 1H), 3.03 - 2.95 (m, 1H), 1.61 (s, 9H).

### Synthetic Scheme 8

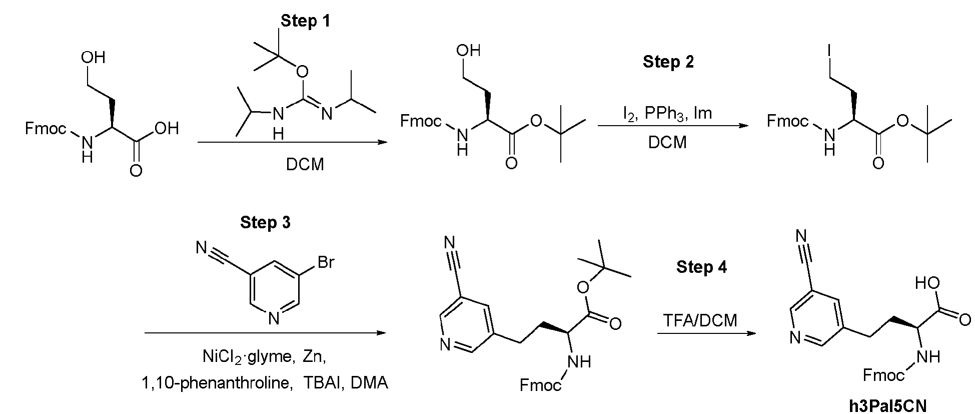

### h3Pal5CN

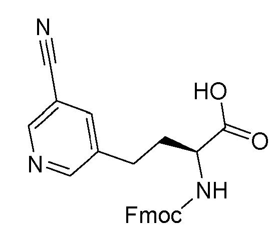

### (S)-2-((((9H-fluoren-9-yl)methoxy)carbonyl)amino)-4-(5-cyanopyridin-3-yl)butanoic acid

Step 1: To a stirred solution of (((9*H*-fluoren-9-yl)methoxy)carbonyl)-*L*-homoserine (15 g, 43.9 mmol) in DCM (200 mL) at rt was added *tert*-butyl (*Z*)-*N,N'*-diisopropylcarbamimidate (35.2 g, 176 mmol). The solution was stirred at 30 °C for 4 h. The solid was filtered out and the filtrate was concentrated under reduced pressure. The residue was purified by a silica gel column chromatography, eluted with 0 - 32% EtOAc in PE to afford *tert*-butyl (((9*H*-fluoren-9-yl)methoxy)carbonyl)-*L*-homoserinate. MS ESI calculated for C₂₃H₂₈NO₅ [M + H]⁺ 398.19, found 398.15.¹H NMR (300 MHz, CDCl₃) δ 7.81 - 7.74 (m, 2H), 7.64 - 7.56 (m, 2H), 7.45 - 7.37 (m, 2H), 7.37 - 7.28 (m, 2H), 5.61 (d, *J* = 7.7 Hz, 1H), 4.54 - 4.32 (m, 3H), 4.22 (t, *J* = 6.9 Hz, 1H), 3.76 - 3.52 (m, 2H), 2.25 - 2.07 (m, 1H), 1.71 - 1.55 (m, 1H), 1.48 (s, 9H).

Step 2: To a stirred solution of PPh₃ (12.67 g, 48.3 mmol) and 1H-imidazole (4.38 g, 64.4 mmol) in DCM (200 mL) was added I₂ (12.26 g, 48.3 mmol) at rt under nitrogen atmosphere. The mixture was stirred at rt for 10 min. *Tert*-butyl (((9*H*-fluoren-9-yl)methoxy)carbonyl)-*L-*homoserinate (12.8 g, 32.2 mmol) was added to the mixture and stirred at 25 °C for 2 h. The solvent was concentrated under reduced pressure and the residue was purified by silica gel column chromatography, eluted with 0 - 18% EtOAc in PE to afford *tert*-butyl (*S*)-2-((((9*H-*fluoren-9-yl)methoxy)carbonyl)amino)-4-iodobutanoate. MS ESI calculated for C₂₃H₂₆INO₄Na [M + Na]⁺ 530.09 found 530.20. ¹H NMR (300 MHz, CDCl₃) δ 7.83 - 7.71 (m, 2H), 7.65 - 7.56 (m, 2H), 7.47 - 7.36 (m, 2H), 7.36 - 7.28 (m, 2H), 5.35 (d, *J* = 8.3 Hz, 1H), 4.52 - 4.35 (m, 2H), 4.34 - 4.16 (m, 2H), 3.21 - 3.04 (m, 2H), 2.52 - 2.32 (m, 1H), 2.28 - 2.07 (m, 1H), 1.48 (s, 9H).

Step 3: To a stirred solution of 1,10-phenanthroline (1.083 g, 6.01 mmol) in DMA (300 mL) was added NiCl₂-glyme (1.321 g, 6.01 mmol) at rt under argon atmosphere. The mixture was stirred at 50 °C for 1 h then cooled to rt. Then *tert*-butyl (*S*)-2-((((9*H*-fluoren-9-yl)methoxy)carbonyl)amino)-4-iodobutanoate (15.25 g, 30.1 mmol), 5-bromonicotinonitrile (5.5 g, 30.1 mmol), TBAI (15.25 g, 30.1 mmol) and zinc (3.93 g, 60.1 mmol) were added to the mixture at rt and stirred at 35 °C for 2.5 h. The mixture was cooled to rt and diluted with water (500 mL) and EtOAc (800 mL). The solid was filtered out and the organic layer was separated. The organic layer was washed with brine (3 x 150 mL), dried over anhydrous Na₂SO₄, and filtered. The filtrate was concentrated under reduced pressure and the residue was purified by a silica gel column chromatography, eluted with 0 - 40% EtOAc in PE to afford *tert*-butyl (*S*)-2-((((9*H*-fluoren-9-yl)methoxy)carbonyl)amino)-4-(5-cyanopyridin-3-yl)butanoate. MS ESI calculated for C₂₉H₃₀N₃O₄ [M + H]⁺ 484.22, found 484.20.¹H NMR (300 MHz, CDCl₃) δ 8.75 (s, 1H), 8.66 (s, 1H), 7.87 - 7.71 (m, 3H), 7.61 (d, *J* = 7.4 Hz, 2H), 7.47 - 7.28 (m, 4H), 5.40 (d, *J* = 7.8 Hz, 1H), 4.55 - 4.37 (m, 2H), 4.36 - 4.18 (m, 2H), 2.85 - 2.59 (m, 2H), 2.26 - 2.08 (m, 1H), 2.02 - 1.82 (m, 1H), 1.49 (s, 9H).

Step 4: To a stirred solution of *tert*-butyl (*S*)-2-((((9*H*-fluoren-9-yl)methoxy)carbonyl)amino)-4-(5-cyanopyridin-3-yl)butanoate (7.5 g, 15.51 mmol) in DCM (50 mL) was added TFA (100 mL) at rt. The solution was stirred at 25 °C for 3 h then concentrated under reduced pressure. The residue was purified by RP-flash with the following conditions: C18 column, 330 g, 5% -5% in 10 min, 5% - 55% in 40 min, 55% -55% in 10 min, MeCN in water (0.05% TFA) to give (*S*)-2-((((9*H*-fluoren-9-yl)methoxy)carbonyl)amino)-4-(5-cyanopyridin-3-yl)butanoic acid. MS ESI calculated for C₂₅H₂₂N₃O₄ [M + H]⁺ 428.15, found 428.05. ¹H NMR (400 MHz, DMSO-*d₆*) δ 12.78 (br, 1H), 8.87 (s, 1H), 8.75 (s, 1H), 8.18 (s, 1H), 7.92 - 7.90 (m, 2H), 7.76 - 7.73 (m, 3H), 7.44 - 7.33 (m, 4H), 4.39 - 4.20 (m, 3H), 3.94 - 3.89 (m, 1H), 2.79 - 2.67 (m, 2H), 2.09 - 1.89 (m, 2H).

### Synthetic Scheme 9

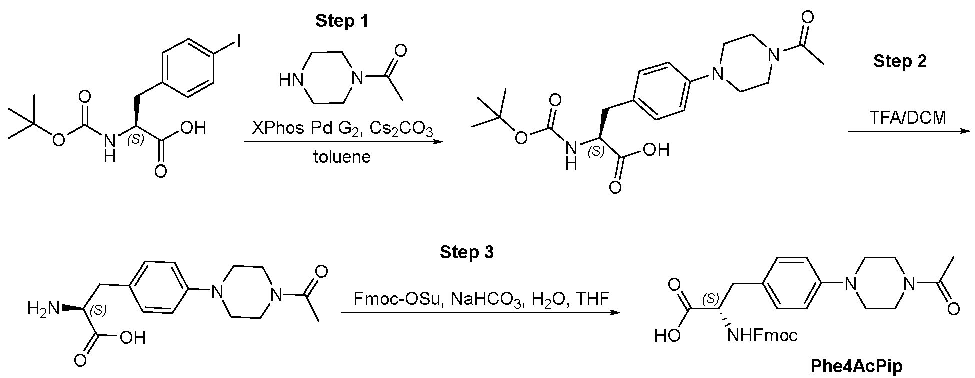

### Ph4AcPip

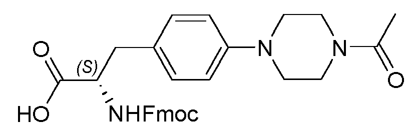

### (S)-2-((((9H-fluoren-9-yl)methoxy)carbonyl)amino)-3-(4-(4-acetylpiperazin-1-yl)phenyl)propanoic acid

Step 1: To a stirred solution of (*S*)-3-(4-bromophenyl)-2-((*tert-*butoxycarbonyl)amino)propanoic acid (6 g, 17.43 mmol) in toluene (180 mL) was added XPhos Pd G₂ (2.057 g, 2.61 mmol) at 25 °C under nitrogen. The resulting solution was stirred at 100 °C for 10 min. 1-(Piperazin-1-yl)ethan-1-one (2.234 g, 17.43 mmol) and Cs₂CO₃ (5.04 g, 26.1 mmol) were added and the resulting solution was stirred at 110 °C for 2 h. The reaction was cooled to rt and quenched with H₂O (500 mL), extracted with EtOAc (2 x 500 mL). The combined organic layer was washed with brine (3 x 200 mL), dried over anhydrous Na₂SO₄ and filtered. The filtrate was concentrated under reduced pressure and the residue was purified by silica gel column chromatography, eluted with 0 - 60% EtOAc in PE to give (*S*)-3-(4-(4-acetylpiperazin-1-yl)phenyl)-2-((*tert*-butoxycarbonyl)amino)propanoic acid. MS ESI calculated for C₂₀H₃₀N₃O₅[M + H]⁺ 392.21, found 392.25.¹H NMR (400 MHz, Methanol-*d₄*) δ 7.12 (d, *J* = 8 Hz, 2H), 6.89 (d, *J* = 8 Hz, 2H), 4.16 - 4.13 (m, 1H), 3.72 - 3.65 (m, 4H), 3.14 - 3.04 (m, 4H), 2.93 - 2.81 (m, 2H), 2.13 (s, 3H), 1.38 - 1.29 (m, 9H).

Step 2: To a stirred solution of (*S*)-3-(4-(4-acetylpiperazin-1-yl)phenyl)-2-((*tert-*butoxycarbonyl)amino)propanoic acid (10 g, 25.5 mmol) in DCM (30 mL) was added TFA (30 mL) at rt. The solution was stirred at 25 °C for 1 h then concentrated under reduced pressure. The crude (*S*)-3-(4-(4-acetylpiperazin-1-yl)phenyl)-2-aminopropanoic acid was used to the next step directly without any further purification. MS ESI calculated for C₁₅H₂₂N₃O₃[M + H]⁺ 292.16, found 292.20.

Step 3: To a stirred solution of (*S*)-3-(4-(4-acetylpiperazin-1-yl)phenyl)-2-(carboxyamino)propanoic acid (7 g, 20.87 mmol) in THF (25 mL) and water (25 mL) was added Fmoc-OSu (6.34 g, 18.79 mmol) at 25 °C under nitrogen. And then NaHCO₃ (8.77 g, 104 mmol) was added. The resulting mixture was stirred at 25 °C for 2 h. The pH was adjusted to 5 with 1 N HCl and extracted with EtOAc (2 x 200 mL). The combined organic layer was washed with brine (3 x 100 mL), dried over anhydrous Na₂SO₄ and filtered. The filtrate was concentrated under reduced pressure and the residue was purified by reverse phase flash chromatography with the following conditions: Column: C18 silica gel (330 g); Mobile Phase A: water (0.1% TFA), Mobile Phase B: MeCN; (Gradient: 5% B hold 5 min, up to 55% B within 15 min, 55% B hold 5 min; up to 95% B within 20 min, 95% B hold 5 min); Flow rate: 90 mL/min; Detector: UV 210 nm; RT = 45 min. The product-containing fractions were collected and concentrated *in vacuo* to give (*S*)-2-((((9*H*-fluoren-9-yl)methoxy)carbonyl)amino)-3-(4-(4-acetylpiperazin-1-yl)phenyl)propanoic acid. MS ESI calculated for C₃₀H₃₂N₃O₅ [M + H]⁺ 514.23, found 514.30. ¹H NMR (400 MHz, Methanol-*d₄*) δ 7.78 - 7.76 (m, 2H), 7.61 - 7.52 (m, 2H), 7.40 - 7.37 (m, 2H), 7.32 - 7.22 (m, 4H), 7.08 - 6.98 (m, 2H), 4.47 - 4.43 (m, 1H), 4.33 - 4.31 (m, 1H), 4.14 - 4.02 (m, 2H), 3.68 - 3.63 (m, 4H), 3.23 - 3.08 (m, 5H), 2.91 - 2.85 (m, 1H), 2.11 (s, 3H).

### Synthetic Scheme 10

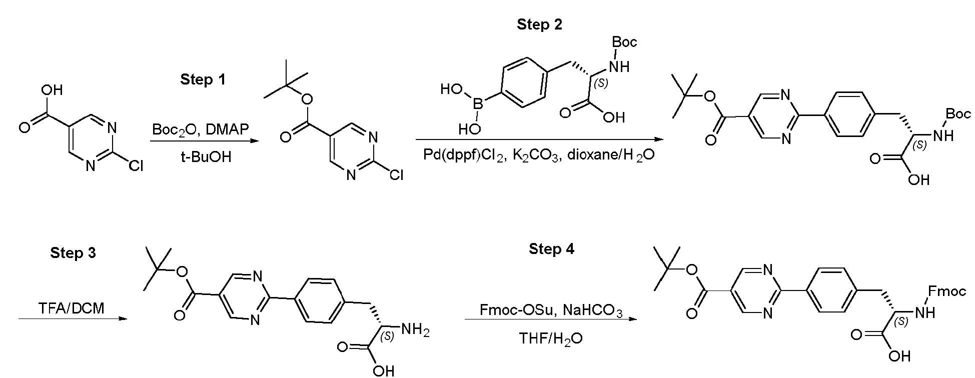

### Ph4Pyrim5CO2H

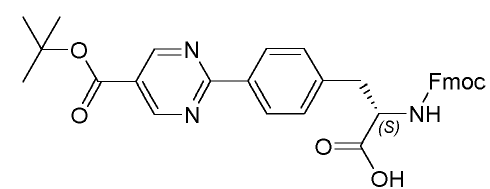

### (S)-2-((((9H-fluoren-9-yl)methoxy)carbonyl)amino)-3-(4-(5-(tert-butoxycarbonyl)pyrimidin-2-yl)phenyl)propanoic acid

Step 1: To a mixture of 2-chloropyrimidine-5-carboxylic acid (10 g, 63.1 mmol) in *t-*BuOH (100 mL) were added DMAP (0.771 g, 6.31 mmol) and Boc₂O (16.52 g, 76 mmol) under argon at rt. The resulting mixture was stirred at 50 °C for 16 h, then it was cooled to rt and concentrated under vacuum. The residue was purified by silica gel column chromatography, eluted with 0 - 60% EtOAc in PE to give *tert*-butyl 2-chloropyrimidine-5-carboxylate. MS ESI calculated for C₉H₁₂ClN₂O₂ [M + H]⁺ 215.05, found 214.95.

Step 2: To a mixture of *tert*-butyl 2-chloropyrimidine-5-carboxylate (4 g, 18.64 mmol) in 1,4-Dioxane (40 mL) and water (8 mL) were added (*S*)-3-(4-boronophenyl)-2-((*tert-*butoxycarbonyl)amino) propanoic acid (8.64 g, 28.0 mmol), PdCl₂(dppf) (1.364 g, 1.864 mmol) and K₂CO₃ (7.73 g, 55.9 mmol) under argon at rt. The resulting mixture was stirred at 80 °C for 3 h, then the pH was adjusted to 4 with 1 N HCl and extracted with EtOAc (2 x 300 mL). The combined organic layer was washed with brine (3 x 50 mL), dried over anhydrous Na₂SO₄ and filtered. The filtrate was concentrated under reduced pressure. The residue was purified by silica gel column chromatography, eluted with 0 -100% EtOAc in PE to give (*S*)-2-((*tert-*butoxycarbonyl) amino)-3-(4-(5-(*tert*-butoxycarbonyl) pyrimidin-2-yl) phenyl)propanoic acid. MS ESI calculated for C₂₃H₃₀N₃O₆ [M + H]⁺ 444.21, found 444.35.¹H NMR (400 MHz, CDCl₃) δ 9.27 (s, 2H), 8.42 (d, *J =* 7.8 Hz, 2H), 7.36 (d, *J =* 7.9 Hz, 2H), 4.69 - 4.68 (m, 1H), 3.29 - 3.24 (m, 2H), 1.63 (s, 9H), 1.44 (s, 9H).

Step 3: To a mixture of (*S*)-2-((*tert*-butoxycarbonyl)amino)-3-(4-(5-(*tert-*butoxycarbonyl)pyrimidin-2-yl)phenyl)propanoic acid (7 g, 15.78 mmol) in DCM (70 mL) was added TFA (14 mL, 182 mmol) under argon at rt. After stirring at rt for 1 h, the reaction was concentrated to obtain (*S*)-2-amino-3-(4-(5-(*tert*-butoxycarbonyl)pyrimidin-2-yl)phenyl)propanoic acid. MS ESI calculated for C₁₈H₂₂N₃O₄ [M + H]⁺ 344.15, found 344.20.

Step 4: To a mixture of (*S*)-2-amino-3-(4-(5-(*tert*-butoxycarbonyl) pyrimidin-2-yl) phenyl) propanoic acid (4 g, 11.65 mmol) in THF (40 mL) and H₂O (40 mL) were added NaHCO₃ (4.89 g, 58.2 mmol) and Fmoc-OSu (3.54 g, 10.48 mmol) under argon at rt. After stirring at rt for 1 h, the pH was adjusted to 4 with 1 N HCl and the solution was extracted with EtOAc (2 x 300 mL). The combined organic layer was washed with brine (150 mL), dried over anhydrous Na₂SO₄, and filtered. The filtrate was concentrated under reduced pressure and the residue was purified by RP-flash with the following conditions: Column: C18 column (330 g); Mobile Phase A: water (0.05% TFA); Mobile Phase B: MeCN; (Gradient: 0% B hold 5 min, up to 73% B within 30 min, 73% B hold 10 min; up to 95% B within 4 min, 95% B hold 10 min); Flow rate: 60 mL/min; Detector: UV 254 & 210 nm; RT: 35.32 min to give (*S*)-2-((((9*H*-fluoren-9-yl)methoxy)carbonyl)amino)-3-(4-(5-(*tert*-butoxycarbonyl) pyrimidin-2-yl) phenyl) propanoic acid. MS ESI calculated for C₃₃H₃₂N₃O₆ [M + H]⁺ 566.22, found 566.35.¹H NMR (300 MHz, DMSO-*d₆*) δ 12.85 (s, 1H), 9.24 (s, 2H), 8.37 (d, *J =* 8.0 Hz, 2H), 7.88 - 7.79 (m, 3H), 7.66 - 7.14 (m, 8H), 4.30 - 4.14 (m, 4H), 3.22 - 3.16 (m, 1H), 3.05 - 2.82 (m, 1H), 1.59 (s, 9H).

### Synthetic Scheme 11

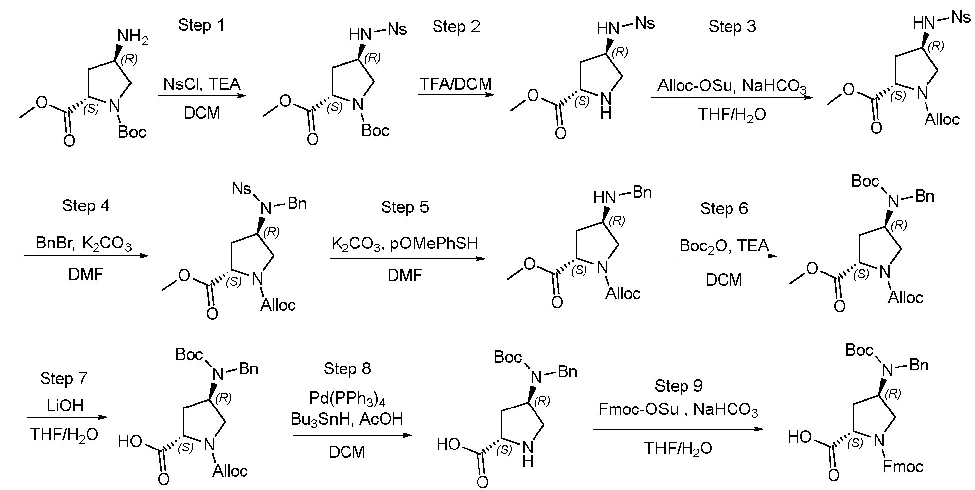

### Prot4NHBn

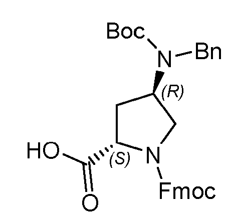

### (2S,4R)-1-(((9H-fluoren-9-yl)methoxy)carbonyl)-4-(benzylamino)pyrrolidine-2-carboxylic acid

Step 1: To a mixture of 1-(*tert*-butyl) 2-methyl (2S,4R)-4-aminopyrrolidine-1,2-dicarboxylate (10 g, 40.9 mmol) in DCM (100 mL) were added TEA (14.26 mL, 102 mmol) and NsCl (10.89 g, 49.1 mmol) at rt. The reaction was stirred at rt for 4 h. The resulting solution was quenched with water (150 mL) and extracted with EtOAc (3 x 300 mL). The organic layers were combined, washed with brine (2 x 150 mL), dried over anhydrous Na₂SO₄,and filtered. The filtrate was concentrated in vacuum. The residue was purified by silica gel chromatography, eluting with a gradient of EtOAc in PE from 0% to 45% afford 1-(*tert*-butyl) 2-methyl (2*S*,4*R*)-4-((4-nitrophenyl)sulfonamido)pyrrolidine-1,2-dicarboxylate. MS ESI calculated for C₁₇H₂₄N₃O₈S [M + H - Boc]⁺ 330.12, found 330.10.¹H NMR (400 MHz, CDCl₃) δ 8.18 - 8.16 (m, 1H), 7.91 - 7.87 (m, 1H), 7.79 - 7.75 (m, 2H), 4.40 - 4.29 (m, 1H), 4.18 - 4.15 (m, 1H), 3.72 (s, 3H), 3.66 - 3.62 (m, 1H), 3.26 - 3.19 (m, 1H), 2.29 - 2.15 (m, 2H), 1.38 (s, 9H).

Step 2: To a mixture of 1-(*tert*-butyl) 2-methyl (2*S*,4*R*)-4-((4-nitrophenyl)sulfonamido)pyrrolidine-1,2-dicarboxylate (17 g, 39.6 mmol) in DCM (60 mL) was added TFA (30 mL, 389 mmol) at ambient temperature. The reaction was stirred at ambient temperature for 2 h then concentrated under vacuum to afford methyl (2*S*,4*R*)-4-((4-nitrophenyl)sulfonamido)pyrrolidine-2-carboxylate. MS ESI calculated for C₁₂H₁₆N₃O₆S [M + H ]⁺ 330.07, found 330.10.

Step 3: To a solution of methyl (2*S*,4*R*)-4-((4-nitrophenyl)sulfonamido)pyrrolidine-2-carboxylate (18.5 g, 39.3 mmol) in THF (150 mL) and water (150 mL) were added NaHCO₃ (16.52 g, 197 mmol) and Alloc-OSu (5.46 mL, 35.4 mmol) at ambient temperature. The reaction was stirred at ambient temperature for 4 h then diluted with water (100 mL) and extracted with EtOAc (3 x 250 mL). The organic layers were combined, washed with brine (2 x 150 mL), dried over anhydrous Na₂SO₄,and filtered. The filtrate was concentrated in vacuum. The residue was purified by silica gel chromatography, eluting with a gradient of EtOAc in PE from 0% to 55% to afford 1-allyl 2-methyl (2*S*,4*R*)-4-((4-nitrophenyl)sulfonamido)pyrrolidine-1,2-dicarboxylate. MS ESI calculated for C₁₆H₂₀N₃O₈S [M + H]⁺ 414.09, found 413.95. ¹H NMR (300 MHz, CDCl₃) δ 8.18 - 8.15 (m, 1H), 7.91 - 7.88 (m, 1H), 7.82 - 7.77 (m, 2H), 5.84 - 5.70 (m, 1H), 5.30 - 5.16 (m, 2H), 4.58 - 4.52 (m, 2H), 4.50 - 4.48 (m, 1H), 4.47 - 4.15 (m, 1H), 3.78 - 3.73 (m, 4H), 3.32 - 3.28 (m, 1H), 2.30 - 2.19 (m, 2H).

Step 4: To a solution of 1-allyl 2-methyl (2*S*,4*R*)-4-((4-nitrophenyl)sulfonamido)pyrrolidine-1,2-dicarboxylate (8.1 g, 19.59 mmol) in DMF (100 mL) were added (bromomethyl)benzene (4.02 g, 23.51 mmol) and K₂CO₃ (8.12 g, 58.8 mmol) at ambient temperature. The reaction was stirred at ambient temperature for 4 h, then quenched with water (100 mL) and extracted with EtOAc (3 x 250 mL). The organic layers were combined, washed with brine (4 x 200 mL), dried over anhydrous Na₂SO₄, and filtered. The filtrate was concentrated under vacuum. The residue was purified by silica gel chromatography, eluting with a gradient of EtOAc in PE from 0% to 55% to afford 1-allyl 2-methyl (2*S*,4*R*)-4-((*N*-benzyl-4-nitrophenyl)sulfonamido)pyrrolidine-1,2-dicarboxylate. MS ESI calculated for C₂₃H₂₆N₃O₈S [M + H]⁺ 504.14, found 504.20.¹H NMR (300 MHz, CDCl₃) δ 7.88 - 7.84 (m, 1H), 7.68 - 7.67 (m, 2H), 7.66 - 7.55 (m, 1H), 7.29 - 7.23 (m, 5H), 5.85 - 5.75 (m, 1H), 5.25 - 5.18 (m, 2H), 4.19 - 4.15 (m, 6H), 3.73 - 3.25 (m, 5H), 2.23 - 2.15 (m, 2H).

Step 5: To a solution of 1-allyl 2-methyl (2*S*,4*R*)-4-((*N*-benzyl-4-nitrophenyl)sulfonamido)pyrrolidine-1,2-dicarboxylate (9.3 g, 18.47 mmol) in DMF (40 mL) were added 4-methoxybenzenethiol (3.11 g, 22.16 mmol) and K₂CO₃ (7.66 g, 55.4 mmol) at ambient temperature. The reaction was stirred at ambient temperature for 1 h, then diluted with water (100 mL) and extracted with EtOAc (3 x 150 mL). The organic layers were combined, washed with brine (2 x 100 mL), dried over anhydrous Na₂SO₄, and filtered. The residue was purified by RP-flash with the following conditions: Column: Flash C18 (330 g); Mobile Phase A: water (0.05% TFA), Mobile Phase B: MeCN; (Gradient: 5% B hold 5 min, up to 32% B within 15 min, 32% B hold 6 min; up to 95% B within 5 min, 95% B hold 5 min); Flow rate: 90 mL/min; Detector: UV 210 nm; RT = 36 min. The product-containing fractions were collected and concentrated in vacuum to give 1-allyl 2-methyl (2*S*,4*R*)-4-(benzylamino)pyrrolidine-1,2-dicarboxylate. MS ESI calculated for C₁₇H₂₃N₂O₄ [M + H]⁺ 319.16, found 319.10.¹H NMR (300 MHz, CDCl₃) δ 7.39 - 7.34 (m, 5H), 5.90 - 5.85 (m, 1H), 5.32 - 5.17 (m, 2H), 4.60 - 4.36 (m, 3H), 3.96 - 3.57 (m, 8H), 2.07 - 1.99 (m, 2H).

Step 6: To a solution of 1-allyl 2-methyl (2*S*,4*R*)-4-(benzylamino)pyrrolidine-1,2-dicarboxylate (4.5 g, 12.72 mmol) in DCM (40 mL) were added Boc₂O (4.16 g, 19.08 mmol) and TEA (5.32 mL, 38.2 mmol) at ambient temperature. The reaction was stirred at ambient temperature for 4 h then quenched with water (30 mL) and extracted with EtOAc (3 x 200 mL). The organic layers were combined, washed with brine (2 x 100 mL), dried over anhydrous Na₂SO₄,and filtered. The filtrate was concentrated under vacuum. The residue was purified by silica gel chromatography, eluting with a gradient of EtOAc in PE from 0% to 40% to afford 1-allyl 2-methyl (2*S*,4*R*)-4-(benzyl(*tert*-butoxycarbonyl)amino)pyrrolidine-1,2-dicarboxylate. MS ESI calculated for C₂₂H₃₁N₂O₆ [M + H]⁺ 419.21, found 419.15.¹H NMR (300 MHz, CDCl₃) δ 7.34 - 7.15 (m, 5H), 5.90 - 5.85 (m, 1H), 5.25 - 5.18 (m, 2H), 4.58 - 4.32 (m, 6H), 3.74 - 3.44 (m, 5H), 2.49 - 2.38 (m, 1H), 2.12 - 1.99 (m, 1H), 1.42 (s, 9H).

Step 7: To a mixture of 1-allyl 2-methyl (2*S*,4*R*)-4-(benzyl(*tert-*butoxycarbonyl)amino)pyrrolidine-1,2-dicarboxylate (3 g, 5.73 mmol) in THF (15 mL) was added LiOH (11.47 mL, 11.47 mmol, 1 M in water) at 0 °C. The reaction was stirred at ambient temperature for 2 h, then acidified with aqueous HCl to pH 3~4 and extracted with EtOAc (3 x 150 mL). The organic layers were combined, washed with brine (2 x 80 mL), dried over anhydrous Na₂SO₄, and filtered. The filtrate was concentrated under vacuum to afford (2*S*,4*R*)-1-((allyloxy)carbonyl)-4-(benzyl(*tert*-butoxycarbonyl)amino)pyrrolidine-2-carboxylic acid. MS ESI calculated for C₂₁H₂₉N₂O₆ [M + H]⁺ 405.19, found 405.30.¹H NMR (300 MHz, CDCl₃) δ 7.35 - 7.14 (m, 5H), 5.89 - 5.75 (m, 1H), 5.14 - 5.19 (m, 2H), 4.61 - 4.30 (m, 6H), 3.82 - 3.32 (m, 2H), 2.47 - 2.18 (m, 2H), 1.42 (s, 9H).

Step 8: To a mixture of (2*S*,4*R*)-1-((allyloxy)carbonyl)-4-(benzyl(*tert-*butoxycarbonyl)amino)pyrrolidine-2-carboxylic acid (2.6 g, 5.46 mmol) in DCM (150 mL) were added AcOH (0.751 mL, 13.11 mmol) and Pd(Ph₃P)₄ (0.126 g, 0.109 mmol) at ambient temperature. And then to the reaction was added Bu₃SnH (1.749 g, 6.01 mmol). After the reaction was stirred at ambient temperature for 4 h, it was concentrated under reduced pressure to afford (2*S*,4*R*)-4-(benzyl(*tert*-butoxycarbonyl)amino)pyrrolidine-2-carboxylic acid. MS ESI calculated for C₁₇H₂₅N₂O₄ [M + H]⁺ 321.17, found 321.15.

Step 9: To a mixture of (2*S*,4*R*)-4-(benzyl(*tert*-butoxycarbonyl)amino)pyrrolidine-2-carboxylic acid (3.2 g, 4.99 mmol) in THF (50 mL) and water (50 mL) was added NaHCO₃ (2.098 g, 24.97 mmol) to a pH of 8~9. And then Fmoc-OSu (1.516 g, 4.49 mmol) was added to the reaction. The resulting mixture was stirred at ambient temperature for 4 h then acidified with aqueous HCl to pH 3~4 and extracted with EtOAc (3 x 200 mL). The organic layers were combined, washed with brine (2 x 100 mL), dried over anhydrous Na₂SO₄,and filtered. The filtrate was concentrated under vacuum to afford crude product. The residue was purified by RP-flash with the following conditions: Column: Flash C18 (330 g); Mobile Phase A: water (0.05% TFA), Mobile Phase B: MeCN; (Gradient: 5% B hold 5 min, up to 70% B within 25 min, 70% B hold 8 min; up to 95% B within 2 min, 95% B hold 5 min); Flow rate: 90 mL/min; Detector: UV 210 nm; RT = 45 min. The product-containing fractions were collected and concentrated in vacuum to give (2*S*,4*R*)-1-(((9*H*-fluoren-9-yl)methoxy)carbonyl)-4-(benzyl(*tert-*butoxycarbonyl)amino)pyrrolidine-2-carboxylic acid. MS ESI calculated for C₃₂H₃₅N₂O₆ [M + H]⁺ 543.24, found 543.20.¹H NMR (400 MHz, DMSO-*d₆*) δ 12.93 (s, 2H), 7.88 (d, *J =* 7.6 Hz, 2H), 7.61 - 7.57 (m, 2H), 7.43 - 7.18 (m, 9H), 4.43 - 4.15 (m, 8H), 3.53 - 3.25 (m, 2H), 2.51 - 2.50 (m, 1H), 2.03 - 1.92 (m, 1H), 1.37 (s, 2H).

### Synthetic Scheme 12

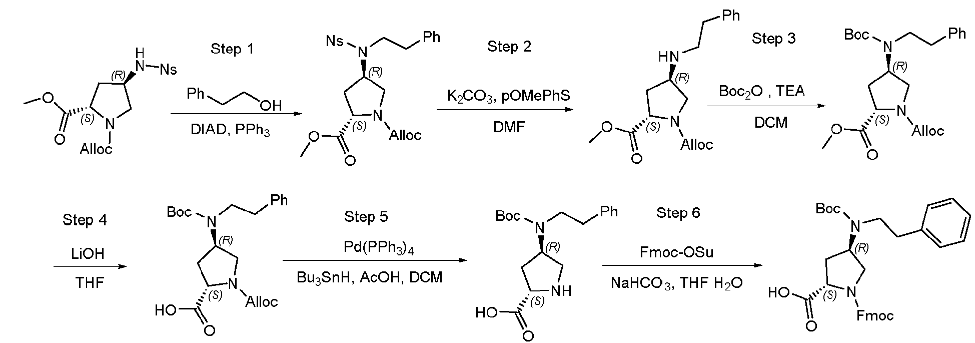

### Prot4NHCH2CH2Ph

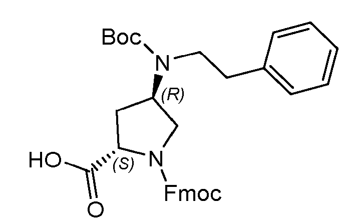

### (2S,4R)-1-(((9H-fluoren-9-yl)methoxy)carbonyl)-4-(phenethylamino)pyrrolidine-2-carboxylic acid

Step 1: To a mixture of 1-allyl 2-methyl (2*S*,4*R*)-4-((4-nitrophenyl)sulfonamido)pyrrolidine-1,2-dicarboxylate (7.2 g, 17.42 mmol), DIAD (5.08 ml, 26.1 mmol), and PPh₃ (6.85 g, 26.1 mmol) in THF (70 mL) was added 2-phenylethan-1-ol (3.19 g, 26.1 mmol) at ambient temperature. The reaction was stirred at ambient temperature for 4 h, then quenched with water (100 mL) and extracted with EtOAc (3 x 250 mL). The organic layers were combined, washed with brine (4 x 200 mL), dried over anhydrous Na₂SO₄, and filtered. The filtrate was concentrated under reduced pressure. The residue was purified by silica gel chromatography, eluting with a gradient of EtOAc in PE from 0% to 55% to afford 1-allyl 2-methyl (2*S*,4*R*)-4-((4-nitro-*N*-phenethylphenyl)sulfonamido)pyrrolidine-1,2-dicarboxylate. MS ESI calculated for C₂₄H₂₈N₃O₈S [M + H]⁺ 518.15, found 518.20.

Step 2: To a mixture of 1-allyl 2-methyl (2*S*,4*R*)-4-((4-nitro-*N-*phenethylphenyl)sulfonamido)pyrrolidine-1,2-dicarboxylate (7.2 g, 13.91 mmol) in DMF (80 mL) were added 4-methoxybenzenethiol (1.950 g, 13.91 mmol) and K₂CO₃ (1.923 g, 13.91 mmol) at ambient temperature. The reaction was stirred at ambient temperature for 1 h, then diluted with water (200 mL) and extracted with EtOAc (3 x 150 mL). The organic layers were combined, washed with brine (2 x 500 mL), dried over anhydrous Na₂SO₄, and filtered. The filtrate was concentrated under reduced pressure and the residue was purified by RP-flash with the following conditions: Column: Flash C18 (330 g); Mobile Phase A: water (0.05% TFA), Mobile Phase B: MeCN; (Gradient: 5% B hold 5 min, up to 32% B within 15 min, 32% B hold 6 min; up to 95% B within 5 min, 95% B hold 5 min); Flow rate: 90 mL/min; Detector: UV 210 nm; RT = 36 min. The product-containing fractions were collected and concentrated in vacuum to give 1-allyl 2-methyl (2*S*,4*R*)-4-(phenethylamino)pyrrolidine-1,2-dicarboxylate. MS ESI calculated for C₁₈H₂₅N₂O₄ [M + H]⁺ 333.17, found 333.25.

Step 3: To a mixture of 1-allyl 2-methyl (2*S*,4*R*)-4-(phenethylamino)pyrrolidine-1,2-dicarboxylate (4 g, 12.03 mmol) in DCM (40 mL) were added Boc₂O (3.93 g, 18.04 mmol) and TEA (3.35 mL, 24.07 mmol) at ambient temperature. The reaction was stirred at ambient temperature for 4 h then quenched with water (30 mL) and extracted with EtOAc (3 x 200 mL). The organic layers were combined, washed with brine (2 x 100 mL), dried over anhydrous Na₂SO₄,and filtered. The filtrate was concentrated under reduced pressure. The residue was purified by silica gel chromatography, eluting with a gradient of EtOAc in PE from 0% to 40% to afford 1-allyl 2-methyl (2*S*,4*R*)-4-((*tert*-butoxycarbonyl)(phenethyl)amino)pyrrolidine-1,2-dicarboxylate. MS ESI calculated for C₂₃H₃₃N₂O₆ [M + H]⁺ 433.23, found 433.25.

Step 4: To a mixture of 1-allyl 2-methyl (2*S*,4*R*)-4-((*tert-*butoxycarbonyl)(phenethyl)amino)pyrrolidine-1,2-dicarboxylate (3.84 g, 8.88 mmol) in THF (30 mL) was added LiOH (17.76 mL, 17.76 mmol, 1 M in water) at 0 °C. The reaction was stirred at ambient temperature for 2 h then acidified with aqueous HCl to pH 3~4 and extracted with EtOAc (3 x 150 mL). The organic layers were combined, washed with brine (2 x 80 mL), dried over anhydrous Na₂SO₄, and filtered. The filtrate was concentrated under reduced pressure and the crude product was used to the next step directly without any further purification. MS ESI calculated for C₂₂H₃₁N₂O₆ [M + H]⁺ 419.21 found 419.35.

Step 5: To a mixture of (2*S*,4*R*)-1-((allyloxy)carbonyl)-4-((*tert-*butoxycarbonyl)(phenethyl)amino)pyrrolidine-2-carboxylic acid (7 g, 16.73 mmol) in DCM (150 mL) were added AcOH (2.411 g, 40.1 mmol) and Pd(Ph₃P)₄ (3.87 g, 3.35 mmol) at ambient temperature. And then to the reaction was added Bu₃SnH (5.36 g, 18.40 mmol). The reaction was stirred at ambient temperature for 4 h then concentrated under reduced pressure. The crude product was used to the next step directly without any further purification. MS ESI calculated for C₁₈H₂₇N₂O₄ [M + H]⁺ 335.19, found 335.25.

Step 6: To a mixture of (2*S*,4*R*)-4-((*tert*-butoxycarbonyl)(phenethyl)amino)pyrrolidine-2-carboxylic acid (5.4 g, 16.15 mmol) in THF (50 mL) and water (50 mL) was added NaHCO₃ (8.56 g, 81 mmol) at rt, and then Fmoc-OSu (4.89 g, 14.54 mmol) was added. After the reaction was stirred at ambient temperature for 4 h, it was acidified with aqueous HCl to pH 3~4 and extracted with EtOAc (3 x 400 mL). The organic layers were combined, washed with brine (2 x 200 mL), dried over anhydrous Na₂SO₄and filtered. The filtrate was concentrated under reduced pressure. The residue was purified by RP-flash with the following conditions: Column: Flash C18 (330 g); Mobile Phase A: water (0.05% TFA), Mobile Phase B: MeCN; (Gradient: 5% B hold 5 min, up to 70% B within 25 min, 70% B hold 8 min; up to 95% B within 2 min, 95% B hold 5 min); Flow rate: 90 mL/min; Detector: UV 210 nm; RT = 45 min. The product-containing fractions were collected and concentrated in vacuum to give (2*S*,4*R*)-1-(((9*H*-fluoren-9-yl)methoxy)carbonyl)-4-((*tert*-butoxycarbonyl)(phenethyl)amino)pyrrolidine-2-carboxylic acid. MS ESI calculated for C₃₃H₃₇N₂O₆Na [M + Na]⁺ 579.26, found 579.25.¹H NMR (300 MHz, Methanol-*d₄*) δ 7.78 - 7.76 (m, 2H), 7.63 - 7.57 (m, 2H), 7.44 - 7.24 (m, 6H), 7.20 - 7.17 (m, 3H), 4.51 - 4.28 (m, 4H), 4.25 - 4.15 (m, 1H), 3.60 - 3.41 (m, 1H), 3.39 - 3.31 (m, 3H), 2.82 - 2.77 (m, 2H), 2.45 - 2.44 (m, 1H), 1.98 - 1.94 (m, 1H), 1.46 (s, 9H).

### Synthetic Scheme 13

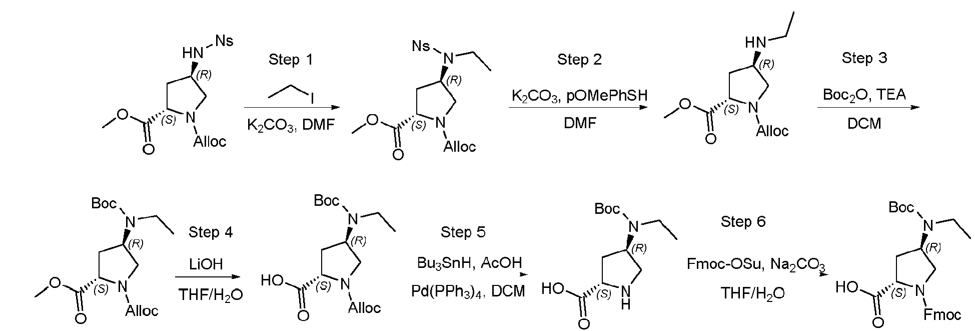

### Prot4NHEt

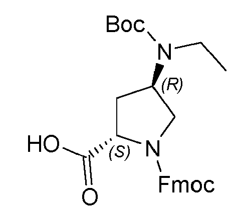

### (2S,4R)-1-(((9H-fluoren-9-yl)methoxy)carbonyl)-4-(ethylamino)pyrrolidine-2-carboxylic acid

Step 1: To a mixture of 1-allyl 2-methyl (2*S*,4*R*)-4-((4-nitrophenyl)sulfonamido)pyrrolidine-1,2-dicarboxylate (9.6 g, 23.22 mmol) in DMF (100 mL) were added iodoethane (4.35 g, 27.9 mmol) and K₂CO₃ (9.63 g, 69.7 mmol) at rt. The reaction was stirred at rt for 12 h, then quenched with water (80 mL) and extracted with EtOAc (3 x 200 mL). The organic layers were combined, washed with brine (4 x 150 mL), dried over anhydrous Na₂SO₄,and filtered. The filtrate was concentrated under reduced pressure. The residue was purified by silica gel chromatography, eluting with a gradient of EtOAc in PE from 0% to 55% to afford 1-allyl 2-methyl (2*S*,4*R*)-4-((*N*-ethyl-4-nitrophenyl)sulfonamido)pyrrolidine-1,2-dicarboxylate. MS ESI calculated for C₁₈H₂₄N₃O₈S [M + H]⁺ 442.12, found 442.05. ¹H NMR (300 MHz, CDCl₃) δ 8.02 - 8.01 (m, 1H), 7.73 - 7.62 (m, 3H), 5.95 - 5.78 (m, 1H), 5.29 - 5.18 (m, 2H), 4.58 - 4.43 (m, 4H), 3.76 - 3.73 (m, 4H), 3.38 - 3.29 (m, 3H), 2.29 - 2.26 (m, 2H), 1.26 - 1.20 (m, 3H).

Step 2: To a stirred mixture of 1-allyl 2-methyl (2*S*,4*R*)-4-((*N-*ethyl-4-nitrophenyl)sulfonamido)pyrrolidine-1,2-dicarboxylate (5.5 g, 12.46 mmol) and K₂CO₃ (5.17 g, 37.4 mmol) in DMF (80 mL) was added 4-methoxybenzenethiol (3.49 g, 24.92 mmol) at rt. After the mixture was stirred at rt for 1 h, it was quenched by water (200 mL), extracted with EtOAc (3 x 200 mL). The combined organic layer was washed with brine (3 x 100 mL), dried over anhydrous Na₂SO₄. After filtration, the filtrate was concentrated under reduced pressure and the residue was purified by a silica gel column chromatography, eluted with 0 - 3% MeOH in DCM to afford 1-allyl 2-methyl (2*S*,4*R*)-4-(ethylamino)pyrrolidine-1,2-dicarboxylate. MS ESI calculated for C₁₂H₂₁N₂O₄ [M + H]⁺ 257.14, found 257.15. ¹H NMR (300 MHz, CDCl₃) δ 6.02 - 5.76 (m, 1H), 5.40 - 5.11 (m, 2H), 4.68 - 4.38 (m, 3H), 3.91 - 3.77 (m, 1H), 3.77 - 3.67 (m, 3H), 3.61 - 3.42 (m, 1H), 3.42 - 3.21 (m, 1H), 2.68 (q, *J* = 7.2 Hz, 2H), 2.28 - 2.00 (m, 2H), 1.14 (t, *J* = 7.1 Hz, 3H).

Step 3: To a stirred solution of 1-allyl 2-methyl (2S,4R)-4-(ethylamino)pyrrolidine-1,2-dicarboxylate (2.9 g, 11.31 mmol) and TEA (3.15 mL, 22.63 mmol) in DCM (50 mL) was added Boc₂O (3.94 mL, 16.97 mmol) at rt. The resulting solution was stirred at 25 °C for 16 h then concentrated under reduced pressure. The residue was purified by a silica gel column chromatography, eluted with 0 - 30% EtOAc in PE to afford 1-allyl 2-methyl (2*S*,4*R*)-4-((*tert-*butoxycarbonyl)(ethyl)amino)pyrrolidine-1,2-dicarboxylate. MS ESI calculated for C₁₇H₂₉N₂O₆ [M + H]⁺ 357.19, found 357.30. ¹H NMR (400 MHz, CDCl₃) δ 6.01 - 5.79 (m, 1H), 5.37 - 5.14 (m, 2H), 4.70 - 4.40 (m, 4H), 3.85 - 3.75 (m, 1H), 3.73 (d, *J =* 7.8 Hz, 3H), 3.49 - 3.36 (m, 1H), 3.28 - 3.06 (m, 2H), 2.53 - 2.38 (m, 1H), 2.22 - 2.07 (m, 1H), 1.46 (s, 9H), 1.11 (t, *J =* 7.0 Hz, 3H).

Step 4: To a stirred solution of 1-allyl 2-methyl (2*S*,4*R*)-4-((*tert-*butoxycarbonyl)(ethyl)amino)pyrrolidine-1,2-dicarboxylate (3.8 g, 10.66 mmol) in THF (50 mL) was added LiOH (32 mL, 32.0 mmol, 1 N in water) at rt. After the solution was stirred at 25 °C for 2 h, the pH of the solution was adjusted to 3 with 1 N HCl and then extracted with EtOAc (3 x 100 mL). The combined organic layer was washed with brine (100 mL), dried over anhydrous Na₂SO₄. After filtration, the filtrate was concentrated under reduced pressure to give (2*S*,4*R*)-1-((allyloxy)carbonyl)-4-((*tert*-butoxycarbonyl)(ethyl)amino)pyrrolidine-2-carboxylic acid. MS ESI calculated for C₁₆H₂₇N₂O₆ [M + H]⁺ 343.18, found 343.30.

Step 5: To a stirred solution of (2*S*,4*R*)-1-((allyloxy)carbonyl)-4-((*tert-*butoxycarbonyl)(ethyl)amino)pyrrolidine-2-carboxylic acid (3.4 g, 9.93 mmol), AcOH (1.431 g, 23.83 mmol) and Pd(Ph₃P)₄ (2.295 g, 1.986 mmol) in DCM (150 mL) was added Bu₃SnH (3.18 g, 10.92 mmol) dropwise at rt. The resulting solution was stirred at 25 °C for 1 h then concentrated under reduced pressure to give (2*S,*4*R*)-4-((*tert-*butoxycarbonyl)(ethyl)amino)pyrrolidine-2-carboxylic acid. MS ESI calculated for C₁₂H₂₃N₂O₄ [M + H]⁺ 259.16, found 259.30.

Step 6: To a stirred solution of (2*S*,4*R*)-4-((*tert*-butoxycarbonyl)(ethyl)amino)pyrrolidine-2-carboxylic acid (2.5 g, 9.68 mmol) and Na₂CO₃ (3.08 g, 29.0 mmol) in THF (60 mL) and water (60 mL) was added Fmoc-OSu (3.26 g, 9.68 mmol) at rt. After the resulting mixture was stirred at 25 °C for 16 h, the pH was adjusted to 3 with 1 N HCl and then extracted with EtOAc (3 x 100 mL). The combined organic layer was washed with brine (2 x 50 mL), dried over anhydrous Na₂SO₄. After filtration, the filtrate was concentrated under reduced pressure and the residue was purified by RP-flash with the following conditions: 330 g C18 column, 5% - 5% in 5 min, 5% - 65% in 40 min, MeCN in water (0.05% TFA) to give (2*S*,4*R*)-1-(((9*H*-fluoren-9-yl)methoxy)carbonyl)-4-((*tert*-butoxycarbonyl)(ethyl)amino)pyrrolidine-2-carboxylic acid. MS ESI calculated for C₂₇H₃₃N₂O₆ [M + H]⁺ 481.23, found 481.10. ¹H NMR (400 MHz, DMSO-*d₆*) δ 12.98 (br, 1H), 7.90 (d, *J* = 7.6 Hz, 2H), 7.68 - 7.64 (m, 2H), 7.44 - 7.41 (m, 2H), 7.35 - 7.31 (m, 2H), 4.43 - 4.18 (m, 5H), 3.60 - 3.56 (m, 1H), 3.29 - 3.22 (m, 1H), 3.16 - 3.12 (m, 2H), 2.42 - 2.38 (m, 1H), 2.08 - 2.01 (m, 1H), 1.40 (s, 9H), 1.05 - 1.00 (m, 3H).

### Synthetic Scheme 14

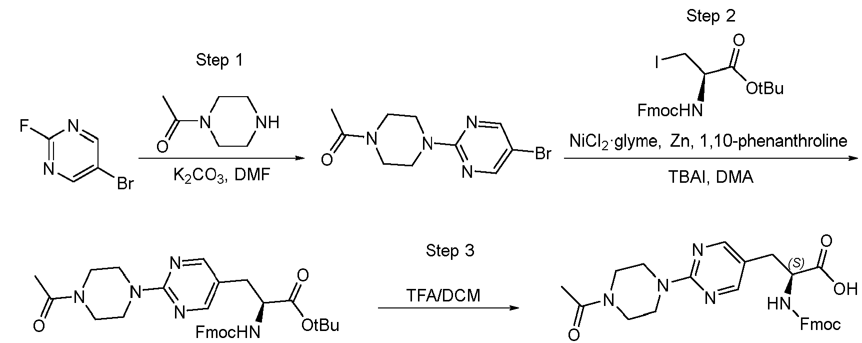

### PyrimAla4AcPip

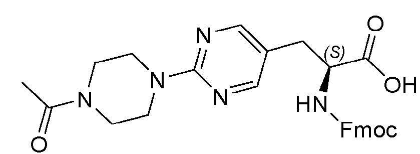

### (S)-2-((((9H-fluoren-9-yl)methoxy)carbonyl)amino)-3-(2-(4-acetylpiperazin-1-yl)pyrimidin-5-yl)propanoic acid

Step 1: To a mixture of 5-bromo-2-fluoropyrimidine (7 g, 39.6 mmol) in DMF (70 ml) were added 1-(piperazin-1-yl)ethan-1-one (10.14 g, 79 mmol) and K₂CO₃ (10.93 g, 79 mmol) at rt. The reaction mixture was stirred for 2 h at 100 °C then cooled back down to rt. The reaction mixture was extracted with 500 mL EtOAc, washed with H₂O (3 x 100 mL) and brine (80 mL), dried over Na₂SO₄, filtered and the filtrate was concentrated under reduced pressure. The residue was purified by silica gel column chromatography, eluting with gradient 0% - 80% EtOAc in PE. The fractions containing desired product were combined and concentrated under reduced pressure to afford 4-(5-bromopyrimidin-2-yl)piperazine-1-carbaldehyde. MS ESI calculated for C₁₀H₁₄BrN₄O [M + H]⁺ 285.03, 287.03, found 284.95, 286.95.¹H NMR (300 MHz, CDCl₃) δ 8.32 (s, 2H), 3.90 - 3.74 (m, 4H), 3.74 - 3.64 (m, 2H), 3.58 - 3.48 (m, 2H), 2.15 (s, 3H).

Step 2: A mixture of Nickel (II) chloride ethylene glycol dimethyl ether complex (0.693 g, 3.16 mmol) and 1,10-phenanthroline (0.569 g, 3.16 mmol) in DMA (50 mL) was heated at 50 °C for 0.5 hours. The solution of 1-(4-(5-bromopyrimidin-2-yl)piperazin-1-yl)ethan-1-one (4.5 g, 15.78 mmol), *tert*-butyl (*R*)-2-((((9*H*-fluoren-9-yl)methoxy)carbonyl)amino)-3-iodopropanoate (11.68 g, 23.67 mmol) and TBAI (5.83 g, 15.78 mmol) in DMA (50 mL) were added at 25 °C. Then Zn (2.064 g, 31.6 mmol) was added. After the resulting mixture was stirred for 24 h at 30 °C, the reaction mixture was filtrated and washed with DCM. The organic phase was concentrated under reduced pressure. The residue was purified by RP-flash with the following conditions: Column: C18 column (330 g); Mobile Phase A: water (0.05% TFA); Mobile Phase B: MeCN; (Gradient: 0% B hold 5 min, up to 82% B within 30 min, 82% B hold 6 min; up to 95% B within 2 min, 95% B hold 3 min); Flow rate: 60 mL/min; Detector: UV 254 & 210 nm; RT: 35 min to give *tert*-butyl (*S*)-2-((((9*H*-fluoren-9-yl)methoxy)carbonyl)amino)-3-(2-(4-acetylpiperazin-1-yl)pyrimidin-5-yl)propanoate. MS ESI calculated for C₃₂H₃₈N₅O₅ [M + H]⁺ 572.28, found 572.30.¹H NMR (300 MHz, CDCl₃) δ 8.19 (s, 2H), 7.77 (d, *J =* 7.6 Hz, 2H), 7.63 - 7.52 (m, 2H), 7.46 - 7.26 (m, 4H), 4.48 - 4.42 (m,2H), 4.35 - 4.30 (m, 1H), 4.22 - 4.17 (m, 1H), 3.89 - 3.81 (m, 4H), 3.71 - 3.68 (m, 2H), 3.56 - 3.52 (m, 2H), 3.08 - 3.02 (m, 1H), 2.92 - 2.87 (m, 1H), 2.17 (s, 3H), 1.48 (s, 9H).

Step 3: To a mixture of *tert*-butyl (*S*)-2-((((9*H*-fluoren-9-yl)methoxy)carbonyl)amino)-3-(2-(4-acetylpiperazin-1-yl)pyrimidin-5-yl)propanoate (4.6 g, 8.05 mmol) in DCM (40 mL) was added TFA (80 mL, 1038 mmol) under argon at rt. The reaction was stirred at rt for 3 h, then concentrated under reduced pressure. The residue was purified by RP-flash with the following conditions: Column: C18 gel column (330 g); Mobile Phase A: water (0.05% TFA); Mobile Phase B: MeCN; (Gradient: 0% B hold 5 min, up to 72% B within 30 min, 72% B hold 6 min; up to 95% B within 2 min, 95% B hold 10 min); Flow rate: 60 mL/min; Detector: UV 254 & 210 nm; RT: 35 min to give (*S*)-2-((((9*H*-fluoren-9-yl)methoxy)carbonyl)amino)-3-(2-(4-acetylpiperazin-1-yl)pyrimidin-5-yl)propanoic acid. MS ESI calculated for C₂₈H₃₀N₅O₅ [M + H]⁺ 516.22, found 516.35.¹H NMR (300 MHz, DMSO-*d₆*) δ 8.31 (s, 2H), 7.89 (d, *J =* 7.5 Hz, 2H), 7.81 (d, *J =* 8.6 Hz, 1H), 7.74 - 7.62 (m, 2H), 7.59 - 7.22 (m, 4H), 4.29 - 4.09 (m, 4H), 3.74 - 3.60 (m, 4H), 3.47 - 3.46 (m, 4H), 2.98 - 2.92 (m, 1H), 2.76 - 2.68 (m, 1H), 2.03 (s, 3H).

### Synthetic Scheme 15

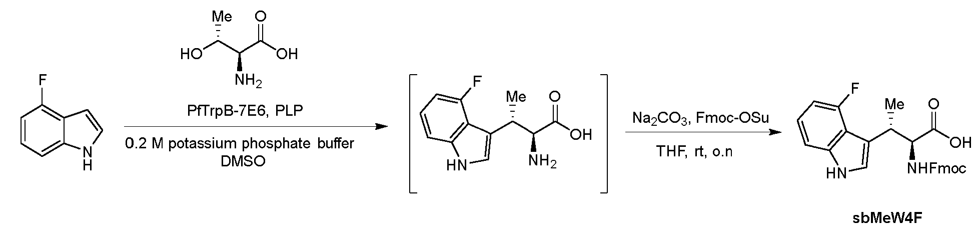

### sbMeW4F

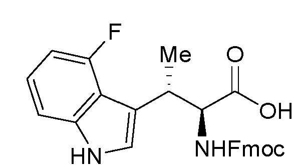

### (2S,3S)-2-amino-3-(4-fluoro-1H-indol-3-yl)butanoic acid

Into a 1-L 3-necked round-bottom flask purged and maintained with an inert atmosphere of nitrogen, was placed 4-fluoro-1H-indole (10 g, 1.00 eq), L-threonine (10.6 g, 1.20 eq), DMSO (100 mL), potassium phosphate buffer (0.2 M, 300 mL, pH = 7.4). The reaction mixture was heated to 65 °C, then PfTrpB-7E6 (2.5 g, 25 wt%) and 3-hydroxy-2-methyl-5-([phosphonooxy]methyl)-4-pyridinecarboxaldehyde (0.078 g, 0.004 eq) were added. The resulting solution was stirred overnight at 65 °C. The mixture was then cooled to rt and used directly in the next step.

Into the above reaction mixture, THF (100 mL), sodium carbonate (23.56 g, 3.0 eq.) and 2,5-dioxopyrrolidin-1-yl 9H-fluoren-9-ylmethyl carbonate (29.96 g, 1.20 eq.) were added at 0 °C. The resulting solution was stirred overnight at rt. The pH was adjusted to 4 by 3 M HCl and the solid precipitate was filtered away. The resulting solution was extracted with EtOAc (3 x 500 mL). The organic fractions were combined, and washed with brine (1 L), dried over anhydrous sodium sulfate and concentrated under vacuum. The mixture was applied onto a silica gel column with MeOH:DCM = 1:5. HPLC-MS: (ES, m/z): [M+1]: 459. ¹H NMR (300 MHz, DMSO-*d₆*) δ 12.60 (s, 1H), 11.15 (s, 1H), 7.87 (d, J = 7.6 Hz, 2H), 7.76 - 7.49 (m, 3H), 7.47 - 7.34 (m, 2H), 7.34 - 7.16 (m, 4H), 7.03 (td, J = 7.9, 5.0 Hz, 1H), 6.73 (dd, J = 11.8, 7.7 Hz, 1H), 4.36 (t, J = 8.5 Hz, 1H), 4.31 - 4.02 (m, 3H), 3.51 (q, J = 7.4 Hz, 1H), 1.31 (d, J = 7.0 Hz, 4H), 0.78 (s, 1H).

### Synthetic Scheme 16

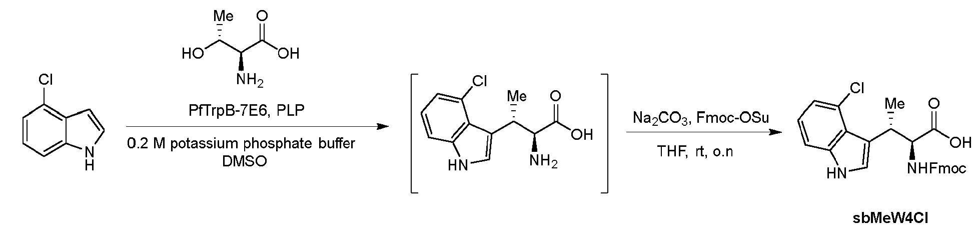

### sbMeW4Cl

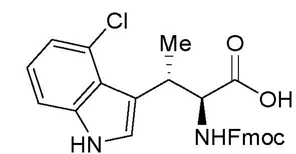

### (2S,3S)-2-amino-3-(4-chloro-1H-indol-3-yl)butanoic acid

Into a 1-L 3-necked round-bottom flask purged and maintained with an inert atmosphere of nitrogen, was placed 4-chloro-1H-indole (10g, 1.00 eq.), L-threonine (14.09 g, 1.8 eq.), DMSO (100 mL), potassium phosphate buffer (0.2 M, 300 mL, PH = 7.4), the reaction mixture was heated to 65 °C, then PfTrpB-7E6 (7.5g, 25 wt%) and 3-hydroxy-2-methyl-5-([phosphonooxy]methyl)-4-pyridinecarboxaldehyde (174 mg, 0.01 eq.) were added. The resulting solution was stirred for 36 h at 65 °C. The mixture was then cooled to rt and used directly in the next step.

Into the above reaction mixture, THF (100 mL), sodium carbonate (20.9 g, 3.0 eq.) and 2,5-dioxopyrrolidin-1-yl 9H-fluoren-9-ylmethyl carbonate (31.0 g, 1.40 eq.) were added at 0 °C. The resulting solution was stirred overnight at rt. The pH was adjusted to 4 by 3 M HCl and the solid precipitate was filtered away. The resulting solution was extracted with EtOAc (3 x 500 mL). The organic fractions were combined, and washed with brine (1 L), dried over anhydrous sodium sulfate and concentrated under vacuum. HPLC-MS: (ES, m/z): [M+1]: 475

### Synthetic Scheme 17

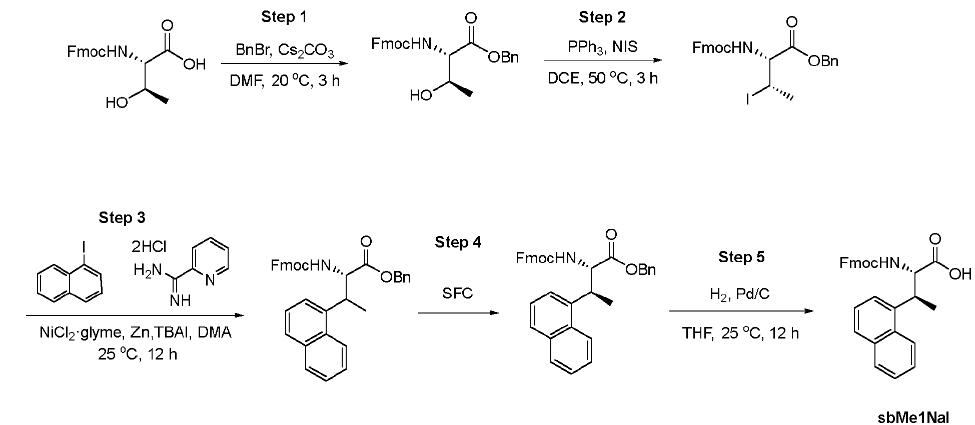

### sbMe1Nal

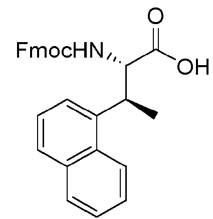

### (2S,3S)-2-amino-3-(naphthalen-1-yl)butanoic acid

Step 1: To a solution of (2S,3R)-2-((((9H-fluoren-9-yl)methoxy)carbonyl)amino)-3-hydroxybutanoic acid (250 g, 1.00 eq) in DMF (1.5 L) was added benzyl bromide (250 g, 2.00 eq) dropwise at 20 °C. Then, cesium carbonate (477 g, 2.00 eq) was added and the solution was stirred at 20°C for 3 h. The reaction was poured into ice H₂O (3 L) and extracted with EtOAc (500 mL x 3). The organic phase was washed with 3% LiCl solution (500 mL x 2) and brine (500 mL), dried over sodium sulfate and concentrated under vacuum at 40 °C. The crude product was triturated with methyl tert-butyl ether:PE = 6:1. ¹H NMR (400 MHz, CDCl₃): δ 7.77 (d, J = 7.6 Hz, 1H), 7.40 (d, J = 8.0 Hz, 1H), 7.31-7.36 (m, 10H), 5.65-5.71 m, 1H), 5.13-5.31 (m, 3H), 4.39-4.43 (m, 3H), 4.22-4.25 (m, 1H), 1.25 (d, J = 6.4 Hz, 3H)

Step 2: To a 3-neck round-bottom flask was added (2S,3R)-benzyl 2-((((9H-fluoren-9-yl)methoxy)carbonyl)amino)-3-hydroxybutanoate (125 g, 1.00 eq) and DCE (750 mL) with an inert atmosphere of nitrogen. The reaction was cooled to 0 °C followed by the addition of NIS (195 g, 3.00 eq) and PPh₃ (228 g, 3.00 eq). The temperature was raised to 50 °C and the reaction mixture was stirred for 3 h. The reaction was poured into ice H₂O (500 mL) and extracted with DCM (500 mL x 2). The organic phase was dried over sodium sulfate and concentrated under vacuum at 40°C. The residue was purified by silica gel column chromatography (PE/EtOAc =1/0 to 0/1). ¹H NMR (400 MHz, CDCl₃): δ 7.78 (d, *J* = 7.6 Hz, 2H), 7.68 (d, *J* = 7.2 Hz, 2H), 7.33-7.43 (m, 9H), 5.27-5.68 (m, 1H), 5.21-5.23 (m, 2H), 4.39-4.52 (m, 3H), 4.25-4.38 (m, 1H), 1.91-1.95 (m, 3H).

Step 3: To a 3-neck round-bottom flask was placed 2-((((9H-fluoren-9-yl) methoxy) carbonyl)amino)-3-iodobutanoate, 1-iodonaphthalene (42.2 g, 1.20 eq), TBAI (76.7 g, 1.50 eq), Zn (19.0 g, 2.10 eq) and DMA (750 mL). To a second 3-neck round-bottom flask was placed picolinimidamide.2HCl (42.2 g, 1.20 eq), NiCl₂.glyme (7.61 g, 0.25 eq) and DMA (750 mL) at 25 °C. Under argon, the contents of the second flask were added to the first flask. The resulting mixture was then stirred for 12 h at 25 °C. The reaction was poured into ice H₂O (3 L) and extracted with EtOAc (1 L x 2). The organic phase was dried over sodium sulfate and concentrated under vacuum at 40 °C. The crude product was purified by reversed-phase HPLC (MeCN:H₂O). HPLC-MS: [M+23]: 564. ¹H NMR (400 MHz, CDCl₃) δ: 8.17-8.24 (m, 1H), 8.15-8.17 (m, 1H), 7.77-7.87 (m, 2H), 7.76-7.77 (m, 4H), 7.30-7.41 (m, 10H), 5.30-5.38 (m, 1H), 4.96-5.04 (m, 3H), 4.85-4.87 (m, 1H), 4.30-4.34 (m, 1H), 4.18-4.26 (m, 4H), 1.43-1.45 (m, 3H).

Step 4: 143 g of (2S)-benzyl 2-((((9H-fluoren-9-yl) methoxy) carbonyl) amino)-3-(naphthalen-1-yl)butanoate was separated by SFC. The organic phase was concentrated under vacuum at 35 °C.

Peak 1: (2S,3R)-benzyl 2-((((9H-fluoren-9-yl)methoxy)carbonyl) amino)-3-(naphthalene-1-yl)butanoate. ¹H NMR (400 MHz, DMSO-*d₆*): δ 8.11-8.12 (m, 2H), 8.10-8.11 (m, 1H), 7.88-7.90 (m, 2H), 7.54-7.88 (m, 1H), 7.44-7.53 (m, 2H), 7.42-7.44 (m, 4H), 7.33-7.42 (m, 3H), 7.27-7.33 (m, 6H), 7.08-7.09 (m, 2H), 4.91-4.94 (m, 1H), 4.79- 4.82 (m, 1H), 4.58 (t, *J =* 8.0 Hz), 4.17-4.25 (m, 4H), 1.39 (d, *J =* 6.8 Hz, 3H). Peak 2: (2S,3S)-benzyl 2-((((9H-fluoren-9-yl)methoxy) carbonyl) amino)-3-(naphthalen-1-yl)butanoate. ¹H NMR (400 MHz, DMSO-*d₆*): δ 7.92-8.15 (m, 1H), 7.86-7.92 (m, 1H), 7.84-7.86 (m, 1H), 7.57-7.84 (m, 2H), 7.56-7.57 (m, 1H), 7.41-7.54 (m, 4H), 7.30-7.38 (m, 4H), 7.27-7.30 (m, 7H), 5.08-5.14 (m, 2H), 4.65 (t, *J =* 8.0 Hz), 4.23-4.26 (m, 1H), 4.05-4.18 (m, 3H), 1.30 (d, *J =* 6.8 Hz, 3H).

Step 5: To a 3-neck round-bottom flask was added (2S,3S)-benzyl 2-((((9H-fluoren-9-yl) methoxy) carbonyl)amino)-3-(naphthalen-1-yl)butanoate (40.0 g, 1.00 eq) and THF (200 mL). 10% wet Pd/C (7.00 g) was added and the reaction was purged 3 times with H₂ and stirred at 25 °C for 12 h under H₂(15 psi). The reaction was filtered through a celite pad and concentrated under vacuum at 35 °C. The crude product was triturated with PE at 25 °C for 1 h. After filtration, the filter cake was dissolved in MeCN (100 mL) and concentrated under vacuum at 35 °C to remove residual solvent. HPLC-MS: [M+23]: 474. ¹H NMR (400 MHz, DMSO-*d₆*) δ 12.78 (s, 1H), 8.23 (d, *J =* 7.6 Hz, 1H), 7.86-7.88 (m, 1H), 7.80-7.86 (m, 2H), 7.61-7.80 (m, 2H), 7.55-7.59 (m, 4H), 7.481-7.55 (m, 1H), 7.40-7.48 (m, 3H), 7.27-7.29 (m, 2H), 4.28-4.60 (m, 1H), 4.24-4.28 (m, 1H), 4.17-4.24 (m, 2H), 4.04-4.15 (m, 1H), 1.36 (d, *J =* 6.8 Hz, 3H).

### Synthetic Scheme 18

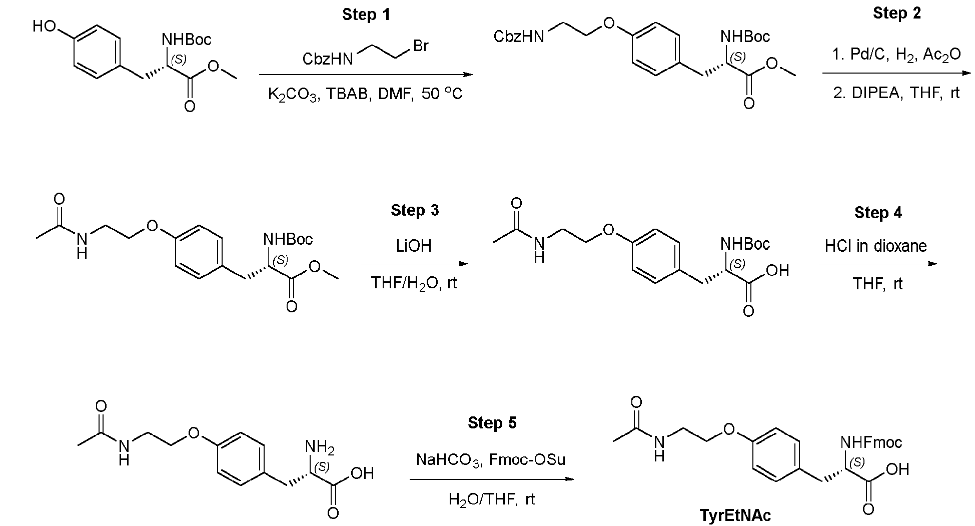

### TyrEtNAc

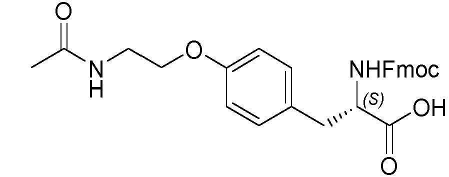

### L-Tyrosine O-ethyl acetamide or (S)-3-(4-(2-acetamidoethoxy)phenyl)-2-aminopropanoic acid

Step 1: To a stirred solution of methyl (tert-butoxycarbonyl)-L-tyrosinate (10.0 g, 33.9 mmol), benzyl (2-bromoethyl)carbamate (26.2 g, 102 mmol) and TBAB (5.46 g, 16.93 mmol) in DMF (150 mL) was added potassium carbonate (14.04 g, 102 mmol) at rt. The mixture was then stirred at 50 °C for 24 h. The mixture was cooled to rt, quenched with water (250 mL) and extracted with EtOAc (2 x 500 mL). The combined organic layers were washed with brine (3 x 150 mL), dried over anhydrous sodium sulfate and filtered. The filtrate was concentrated under reduced pressure, and the residue was purified by silica gel column chromatography, eluted with 0 - 30% EtOAc in PE. MS ESI calculated for C₂₅H₃₂N₂O₇ [M + Na]⁺ 495.22, found 495.10; ¹H NMR (300 MHz, CDCl₃) δ 7.38 - 7.32 (m, 5H), 7.04 (d, *J* = 8.4 Hz, 2H), 6.81 (d, *J* = 8.4 Hz, 2H), 5.31 (br, 1H), 5.21 (s, 2H), 4.97 (br, 1H), 4.56 - 4.53 (m, 1H), 4.03 (t, *J* = 5.0 Hz, 2H), 3.72 (s, 3H), 3.64 - 3.58 (m, 2H), 3.06 - 3.01 (m, 2H), 1.43 (s, 9H).

Step 2: To a stirred solution of methyl (*S*)-3-(4-(2-(((benzyloxy)carbonyl)amino)ethoxy)phenyl)-2-((*tert*-butoxycarbonyl)amino)propanoate (16.0 g, 33.9 mmol) and acetic anhydride (6.39 mL, 67.7 mmol) in THF (200 mL) was added Pd/C (3.60 g, 33.9 mmol, dry, 10%wt) at rt under nitrogen atmosphere. The mixture was degassed with hydrogen 3 times and stirred at 20 °C for 4 h. DIPEA (17.74 mL, 102 mmol) was added to the mixture and stirred at 20 °C for 1 h. The mixture was filtered, and the filtrate was concentrated under reduced pressure. The residue was purified by silica gel column chromatography, eluted with 0 - 3% MeOH in DCM. MS ESI calculated for C₁₉H₂₈N₂O₆ [M + Na]⁺ 403.19, found 403.10; ¹H NMR (300 MHz, CDCl₃) δ 7.05 (d, *J =* 8.4 Hz, 2H), 6.85 - 6.80 (m, 2H), 5.99 (br, 1H), 5.32 (br, 1H), 4.99 - 4.97 (m, 1H), 4.02 (t, *J =* 5.0 Hz, 2H), 3.72 (s, 3H), 3.69 - 3.63 (m, 2H), 3.10 - 2.89 (m, 2H), 2.02 (s, 3H), 1.42 (s, 9H).

Step 3: To a stirred solution of methyl (*S*)-3-(4-(2-acetamidoethoxy)phenyl)-2-((*tert-*butoxycarbonyl)amino)propanoate (12.5 g, 32.9 mmol) THF (100 mL) was added lithium hydroxide (65.7 mL, 65.7 mmol, 1 N in water) at rt. The solution was stirred at 20 °C for 2 h. The pH of the solution was adjusted to 3 with 1 N HCl. The aqueous layer was extracted with EtOAc (2 x 250 mL). The combined organic layers were washed with brine (150 mL), dried over anhydrous sodium sulfate, filtered, and concentrated under reduced pressure.

MS ESI [M + H]⁺: 367.10.

Step 4: To a stirred solution of (*S*)-3-(4-(2-acetamidoethoxy)phenyl)-2-((*tert-*butoxycarbonyl)amino)propanoic acid (12.5 g, 30.7 mmol) in THF (20 mL) was added 4 N HCl in dioxane (200 mL) at rt. The solution was stirred at 20 °C for 1 h. The solvent was concentrated under reduced pressure. MS ESI [M + H]⁺: 267.05.

Step 5: To a stirred mixture of (S)-3-(4-(2-acetamidoethoxy)phenyl)-2-aminopropanoic acid hydrochloride (9.50 g, 25.1 mmol) and NaHCO₃ (10.54 g, 126 mmol) in THF (100 mL) and water (100 mL) was added Fmoc-OSu (7.62 g, 22.59 mmol) at rt. The mixture was stirred at 20 °C for 1 h. The pH value of the solution was adjusted to 3 with 1 N HCl. The aqueous phase was extracted with EtOAc (2 x 500 mL). The combined organic layers were washed with brine (150 mL), dried over anhydrous sodium bicarbonate and filtered. The filtrate was concentrated under reduced pressure, and the residue was recrystallized from EtOAc (200 mL). The solid was collected by filtration and dried under vacuum. MS ESI [M + H]⁺: 489.05; ¹H NMR (300 MHz, Methanol-*d₄*) δ 7.79 (d, *J =* 7.6 Hz, 2H), 7.62-7.57 (m, 2H), 7.42-7.26 (m, 4H), 7.17-7.14 (m, 2H), 6.83 (d, *J* = 8.4 Hz, 2H), 4.41-4.31 (m, 2H), 4.29-4.10 (m, 2H), 3.96 (t, *J* = 4.8 Hz, 2H), 3.51 (t, *J* = 5.4 Hz, 2H), 3.19-3.13 (m, 1H), 2.92-2.84 (m, 1H), 1.94 (s, 3H).

### Synthetic Scheme 19

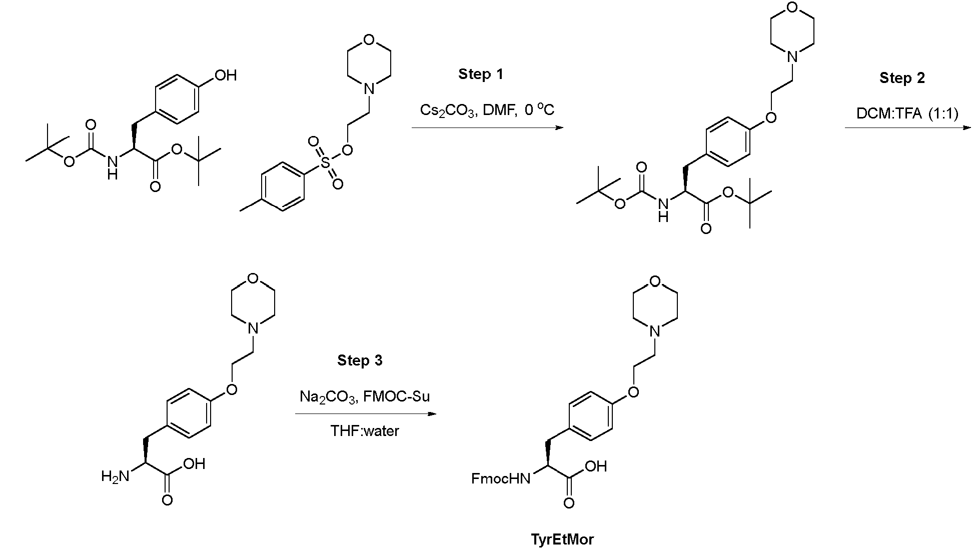

### TyrEtMor

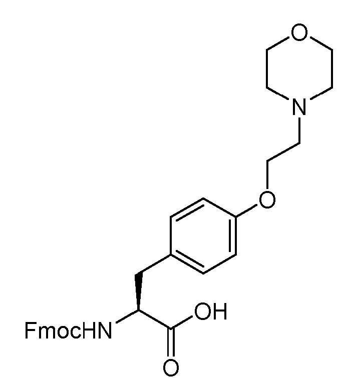

### (S)-2-((((9H-fluoren-9-yl)methoxy)carbonyl)amino)-3-(4-(2-morpholinoethoxy)phenyl)propanoic acid

Step 1: To a solution of tert-butyl (tert-butoxycarbonyl)-L-tyrosinate (400 mg, 1.185 mmol) in DMF (4.742 ml) at 0 °C was added cesium carbonate (1 g, 3.08 mmol). The reaction was stirred for 15 min and 2-morpholinoethyl 4-methylbenzenesulfonate (474 mg, 1.660 mmol) was added and stirred at 0 °C for 2.5 hours. The reaction was diluted with EtOAc and washed with 10% aqueous LiCl solution. The organic layer was washed with 1 M HCl, saturated aqueous NaHCO₃, and aqueous NaCl then dried with sodium sulfate, filtered, and concentrated. The crude material was purified by column chromatography using a RediSep Rf Gold silica 24 g prepackaged column on an Isco, eluting with a 0-100% gradient of EtOAc in hexanes over the course of 30 min. to give the product. MW: 450.576; MS ESI [M + H]⁺: 451.3.

Step 2: tert-butyl (S)-2-((tert-butoxycarbonyl)amino)-3-(4-(2-morpholinoethoxy)phenyl)propanoate (525 mg, 1.165 mmol) was dissolved in DCM (3 mL) at ambient temperature. TFA (3 mL) was added and allowed to stir at ambient temperature for 1.5 hours. The reaction was concentrated under reduced pressure and carried forward as crude. MW: 294.351, found MS ESI [M + H]⁺: 295.1.

Step 3: (S)-2-amino-3-(4-(2-morpholinoethoxy)phenyl)propanoic acid (354 mg, 1.203 mmol) was dissolved in Tetrahydrofuran (3 mL) and Water (3 mL) at ambient temperature. The reaction was chilled to 0 °C. Sodium carbonate (382 mg, 3.61 mmol) was added and the solution was stirred at 0 °C for 5 min. (9H-fluoren-9-yl)methyl (2,5-dioxopyrrolidin-1-yl) carbonate (406 mg, 1.203 mmol) was added and stirred for 2 h. THF was removed under reduced pressure. The aqueous solution was acidified with 1 N HCl to a pH of 3. The reaction was extracted with EtOAc, washed with water, dried with sodium sulfate, filtered, and concentrated. The crude material was purified by reverse phase column chromatography using a 50 g C18 prepackaged column on an Isco, eluting with a 10-100% gradient of TFA modified MeCN in TFA modified water over the course of 30 min. to give the product in fractions. The fractions were combined, frozen in a -78 °C dry ice/acetone bath, and lyophilized to furnish product. MW: 516.94. found MS ESI [M + H]⁺: 517.2.

### Synthetic Scheme 20

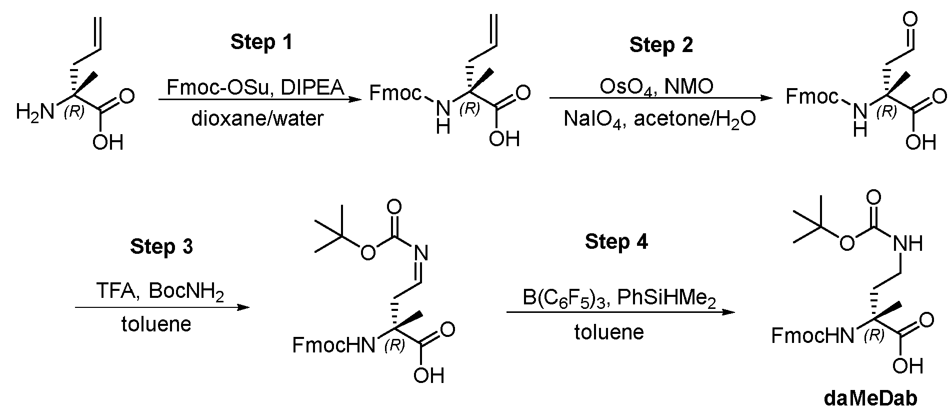

### daMeDab

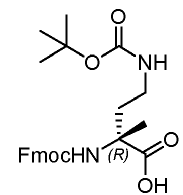

### (R)-2,4-diamino-2-methylbutanal

Step 1: To a stirred solution of (R)-2-amino-2-methylpent-4-enoic acid (1.3 g, 10.07 mmol) and DIPEA (5.27 mL, 30.2 mmol) in dioxane (20 mL) and water (20 mL) at room temperature was added Fmoc-OSu (3.73 g, 11.07 mmol). The resulting solution was stirred at 25 °C for 16 h. The pH was adjusted to 3 with 1 N HCl and the solution was purified by reverse phase flash column chromatography with the following conditions: C18 column, 330 g, 5% - 5% in 5 min, 5% - 50% in 30 min, 98% - 98% in 5 min, MeCN in water (0.05% TFA) to give (*R*)-2-((((9*H-*fluoren-9-yl)methoxy)carbonyl)amino)-2-methylpent-4-enoic acid. MS ESI calculated for C₂₁H₂₁NO₄Na [M + Na]⁺ 374.15, found 374.05. ¹H NMR (400 MHz, CDCl₃) *δ* 7.79 (d, *J =* 7.5 Hz, 2H), 7.61 (d, *J =* 7.5 Hz, 2H), 7.48 - 7.29 (m, 4H), 5.71 (s, 1H), 5.48 (s, 1H), 5.20 - 5.16 (m, 2H), 4.44 - 4.40 (m, 2H), 4.25 (t, *J =* 6.7 Hz, 1H), 2.82-2.71 (m, 2H).

Step 2: To a stirred solution of (*R*)-2-((((9*H*-fluoren-9-yl)methoxy)carbonyl)amino)-2-methylpent-4-enoic acid (3 g, 8.54 mmol) and *N*-methylmorpholine *N*-oxide (2.2 g, 9.39 mmol, 50% in water) in acetone (60 mL) was added OsO₄ (2.170 g, 0.854 mmol, 10% in water) at room temperature. The resulting solution was stirred at 25 °C for 4 h. Sodium periodate (2.00 g, 9.39 mmol) in water (20 mL) was added to the solution and the resulting mixture was stirred at 25 °C for 16 h. The reaction mixture was diluted with water (50 mL) and extracted with EtOAc (3 x 200 mL). The combined organic layers were washed with brine (2 x 100 mL), dried over anhydrous sodium sulfate, and filtered. The filtrate was then concentrated under reduced pressure to give (*R*)-2-((((9*H*-fluoren-9-yl)methoxy)carbonyl)amino)-2-methyl-4-oxobutanoic acid. MS ESI calculated for C₂₀H₂₀NO₅ [M + H]⁺ 354.13, found 354.00.

Step 3: To a stirred solution of (*R*)-2-((((9*H*-fluoren-9-yl)methoxy)carbonyl)amino)-2-methyl-4-oxobutanoic acid (3 g, 6.79 mmol) in toluene (120 mL) were added *tert*-butyl carbamate (4.77 g, 40.8 mmol) and TFA (2.32 g, 20.38 mmol) at room temperature. The resulting solution was stirred at 25 °C for 2 h. The solution was concentrated under reduced pressure to give (*R*,*Z*)-2-((((9*H*-fluoren-9-yl)methoxy)carbonyl)amino)-4-((*tert*-butoxycarbonyl)imino)-2-methylbutanoic acid. MS ESI calculated for C₂₅H₂₇N₂O₆ [M - H]⁺ 451.19, found 450.90.

Step 4: To a stirred solution of (*R*,*Z*)-2-((((9*H*-fluoren-9-yl)methoxy)carbonyl)amino)-4-((*tert*-butoxycarbonyl)imino)-2-methylbutanoic acid (3.1 g, 5.48 mmol) and dimethyl(phenyl)silane (1.87 g, 13.7 mmol) in toluene (120 mL) was added tris(pentafluorophenyl)borane (0.281 g, 0.548 mmol) at room temperature. The solution was stirred at 25 °C for 16 h. The solvent was concentrated under reduced pressure and the residue was purified by reverse phase flash chromatography with the following conditions: 330 g C18 column, 5% - 5% in 5 min, 5% - 55% in 30 min, 98% - 98% in 5 min, MeCN in water (0.05% TFA), RT = 35 min to give (*R*)-2-((((9*H*-fluoren-9-yl)methoxy)carbonyl)amino)-4-((*tert-*butoxycarbonyl)amino)-2-methylbutanoic acid. MS ESI calculated for C₂₅H₃₁N₂O₆ [M + H]⁺ 455.21, found 455.10; ¹H NMR (400 MHz, DMSO-*d₆*) *δ* 12.48 (s, 1H), 7.90 (d, *J =* 7.2 Hz, 2H), 7.73 (d, *J =* 7.2 Hz, 2H), 7.54 (s, 1H), 7.44 - 7.41 (m, 2H), 7.36 - 7.32 (m, 2H), 6.78 (s, 1H), 4.25 - 4.23 (m, 3H), 2.92 - 2.91 (m, 2H), 1.99 - 1.93 (m, 1H), 1.87 - 1.81 (m, 1H), 1.37 - 1.34 (m, 9H).

### Synthetic Scheme 21

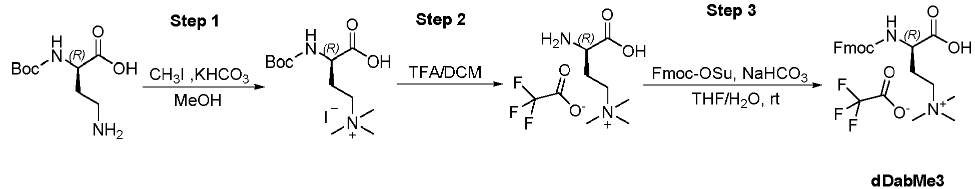

### dDabMe3

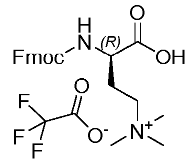

### [(R)-3-amino-3-carboxypropyl]-trimethylazanium

Step 1: To a mixture of (*R*)-4-amino-2-((*tert*-butoxycarbonyl)amino)butanoic acid (7 g, 32.1 mmol) in MeOH (30 mL) were added CH₃I (10.03 mL, 160 mmol) and KHCO₃ (16.05 g, 160 mmol) at ambient temperature. The reaction was warmed to 35 °C for 12 h. The reaction mixture was then filtered and the filtrate was concentrated under reduced pressure to afford crude product. The residue was dissolved in ethanol and then filtered again. The filtrate was concentrated under reduced pressure to afford (*R*)-3-((*tert*-butoxycarbonyl)amino)-3-carboxy-*N*,*N*,*N*-trimethylpropan-1-aminium iodide. MS ESI calculated for C₁₂H₂₅IN₂O₄ [M - I]⁺ 261.18, found 261.30.

Step 2: To a mixture of (*R*)-3-((*tert*-butoxycarbonyl)amino)-3-carboxy-*N*,*N*,*N-*trimethylpropan-1-aminium iodide (15 g, 23.18 mmol) in DCM (60 mL) was added TFA (30 mL, 389 mmol) at ambient temperature. The reaction was stirred at ambient temperature for 1 h then concentrated under reduced pressure to afford (*R*)-3-amino-3-carboxy-*N*,*N*,*N*-trimethylpropan-1-aminium 2,2,2-trifluoroacetate. MS ESI calculated for C₉H₁₇F₃N₂O₄ [M-CF₃COO]⁺ 161.13, found 161.30.

Step 3: To a mixture of (*R*)-3-amino-3-carboxy-*N*,*N*,*N*-trimethylpropan-1-aminium 2,2,2-trifluoroacetate (11.5 g, 20.97 mmol) in THF (40 mL) and water (40 mL) were added NaHCO₃ (8.81 g, 105 mmol) and Fmoc-OSu (6.37 g, 18.87 mmol). The reaction was stirred at ambient temperature for 2 h then acidified with aqueous HCl to pH 3-4 and filtered to afford crude product. The crude product was purified by reverse phase column chromatography with the following conditions: 330 g C18 column; Mobile Phase A: water (0.05% TFA), Mobile Phase B: MeCN; (Gradient: 0% B hold 5 min, up to 33% B within 19 min, 38% B hold 7 min; up to 95% B within 5 min, 95% B hold 5 min); Flow rate: 80 mL/min. The product-containing fractions were collected and lyophilized to afford (*R*)-3-((((9*H*-fluoren-9-yl)methoxy)carbonyl)amino)-3-carboxy-*N*,*N*,*N*-trimethylpropan-1-aminium 2,2,2-trifluoroacetate. MS ESI calculated for C₂₄H₂₇F₃N₂O₆ [M-CF₃COO]⁺ 383.20, found 383.25.¹H NMR (300 MHz, DMSO-*d₆*) *δ* 7.91 (d, *J* = 7.2 Hz, 2H), 7.79 - 7.71 (m, 3H), 7.46 - 7.41 (m, 2H), 7.37 - 7.32 (m, 2H), 4.42 - 4.22 (m, 3H), 4.07 - 4.01 (m, 1H), 3.99 - 3.41 (m, 1H), 3.33 - 3.28 (m, 1H), 3.07 (s, 9H), 2.21 - 2.16 (m, 1H), 2.08 - 2.04 (m, 1H). ¹⁹F-NMR (282 MHz, DMSO-*d₆*) *δ* 73.931.

### Synthetic Scheme 22

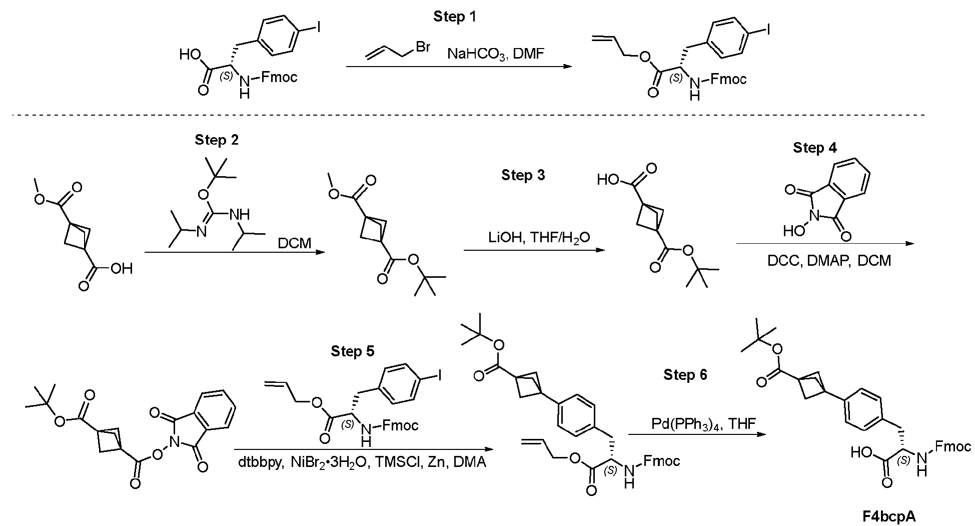

### F4cpA

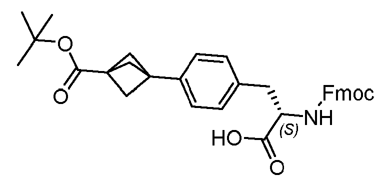

### 3-[4-[(S)-2-amino-2-carboxyethyl]phenyl]bicyclo[1.1.1]pentane-1-carboxylic acid

Step 1: To a stirred mixture of (*S*)-2-((*tert*-butoxycarbonyl)amino)-3-(4-iodophenyl)propanoic acid (3.91 g, 10 mmol) in DMF (40 mL) were added 3-bromoprop-1-ene (3.63 g, 30.0 mmol) and NaHCO₃ (0.840 g, 10.00 mmol) at 0 °C under an atmosphere of argon. The resulting mixture was stirred at 40 °C for 16 h. The reaction mixture was cooled to room temperature and quenched with water (200 mL) and extracted with EtOAc (2 x 200 mL). The combined organic layer was washed with brine (3 x 100 mL), dried over anhydrous sodium sulfate, and filtered. The filtrate was concentrated under reduced pressure and the residue was purified by silica gel column chromatography (eluted with 0 ~ 40% EtOAc in PE) and the product-containing fractions were collected and evaporated under reduced pressure to afford allyl (*S*)-2-((*tert*-butoxycarbonyl)amino)-3-(4-iodophenyl)propanoate. MS ESI calculated for C₂₇H₂₅INO₄ [M + H]⁺ 554.08, found 554.20.

Step 2: To a stirred solution of 3-(methoxycarbonyl)bicyclo[1.1.1]pentane-1-carboxylic acid (8 g, 47.0 mmol) in DCM (100 mL) was added *tert-*butyl (*Z*)-*N*,*N*-diisopropylcarbamimidate (37.7 g, 188 mmol) at room temperature. The resulting solution was stirred at 40 °C for 2 h. The mixture was then cooled to room temperature. The solid was filtered out and the filtrate was concentrated under reduced pressure to give 1-(*tert*-butyl) 3-methyl bicyclo[1.1.1]pentane-1,3-dicarboxylate. ¹H NMR (400 MHz, DMSO-*d₆*) *δ* 3.61 (s, 3H), 2.17 (s, 6H), 1.39 (s, 9H).

Step 3: To a stirred solution of 1-(*tert*-butyl) 3-methyl bicyclo[1.1.1]pentane-1,3-dicarboxylate (15 g, 39.8 mmol) in THF (150 mL) was added LiOH (119 mL, 119 mmol, 1 N in water) at room temperature. The resulting solution was stirred at 25 °C for 5 h. The pH of the solution was adjusted to 3 with 1 N HCl and then extracted with EtOAc (3 x 100 mL). The combined organic layer was washed with brine (2 x 50 mL), dried over anhydrous sodium sulfate, and filtered. The filtrate was then concentrated under reduced pressure to give 3-(*tert-*butoxycarbonyl)bicyclo[1.1.1]pentane-1-carboxylic acid. MS ESI calculated for C₁₁H₁₅O₄ [M - H]⁻ 211.10, found 211.10.

Step 4: To a stirred solution of 3-(*tert*-butoxycarbonyl)bicyclo[1.1.1]pentane-1-carboxylic acid (10 g, 28.3 mmol), 2-hydroxyisoindoline-1,3-dione (6.00 g, 36.7 mmol) and DMAP (0.345 g, 2.83 mmol) in DCM (100 mL) was added *N*,*N*'-dicyclohexylcarbodiimide (6.42 g, 31.1 mmol) at room temperature. The resulting mixture was stirred at 25 °C for 16 h. The solid was filtered out and the filtrate was concentrated under reduced pressure. The residue was purified by silica gel column chromatography, eluted with 0 - 40% EtOAc in PE to afford 1-(*tert*-butyl) 3-(1,3-dioxoisoindolin-2-yl) bicyclo[1.1.1]pentane-1,3-dicarboxylate. ¹H NMR (400 MHz, CDCl₃) *δ* 7.89 (dd, *J* = 5.5, 3.1 Hz, 2H), 7.79 (dd, *J* = 5.5, 3.1 Hz, 2H), 2.50 (s, 6H), 1.47 (s, 9H).

Step 5: To a stirred solution of NiBr₂·3H₂O (0.791 g, 2.90 mmol) in DMA (80 mL) was added 4,4'-di-tert-butyl-2,2'-dipyridine (0.973 g, 3.63 mmol) at room temperature under an atmosphere of nitrogen. After the resulting mixture was stirred at 50 °C for 30 min., it was cooled to room temperature. 1-(*Tert*-butyl) 3-(1,3-dioxoisoindolin-2-yl) bicyclo[1.1.1]pentane-1,3-dicarboxylate (4.8 g, 12.09 mmol), allyl (*S*)-2-((((9*H*-fluoren-9-yl)methoxy)carbonyl)amino)-3-(4-iodophenyl)propanoate (6.69 g, 12.09 mmol), trimethylsilyl chloride (0.131 g, 1.209 mmol), and zinc (3.95 g, 60.4 mmol) were added to the above mixture at room temperature. The resulting mixture was stirred at 25 °C for 2 h. The reaction was quenched with brine (150 mL) and extracted with EtOAc (3 x 100 mL). The combined organic layer was washed with brine (3 x 100 mL), dried over anhydrous sodium sulfate, and filtered. The filtrate was then concentrated under reduced pressure and the residue was purified by silica gel column chromatography, eluted with 0 - 40% EtOAc in PE to afford *tert*-butyl (*S*)-3-(4-(2-((((9*H*-fluoren-9-yl)methoxy)carbonyl)amino)-3-(allyloxy)-3-oxopropyl)phenyl)bicyclo[1.1.1]pentane-1-carboxylate. MS ESI calculated for C₃₇H₄₀NO₆ [M + H]⁺ 594.28, found 594.30.

Step 6: To a stirred solution of *tert*-butyl (*S*)-3-(4-(2-((((9*H*-fluoren-9-yl)methoxy)carbonyl)amino)-3-(allyloxy)-3-oxopropyl)phenyl)bicyclo[1.1.1]pentane-1-carboxylate (2.3 g, 1.55 mmol) and phenylsilane (0.335 g, 3.10 mmol) in THF (30 mL) was added Pd(Ph₃P)₄ (0.090 g, 0.077 mmol) at room temperature under a nitrogen atmosphere. The resulting mixture was stirred at room temperature for 1 h then concentrated under reduced pressure and the residue was purified by reverse phase column chromatography with the following conditions: C18 column, 330 g, 5% - 5% in 5 min, 5% - 70% in 40 min, MeCN in water (0.05% TFA) to give (*S*)-2-((((9*H*-fluoren-9-yl)methoxy)carbonyl)amino)-3-(4-(3-(*tert-*butoxycarbonyl)bicyclo[1.1.1]pentan-1-yl)phenyl)propanoic acid. MS ESI calculated for C₃₄H₃₄NO₆ [M - H]⁻ 552.25, found 552.30.¹H NMR (400 MHz, Methanol-*d₄*) *δ* 7.80 - 7.77 (m, 2H), 7.67 - 7.65 (m, 2H), 7.48 - 7.23 (m, 4H), 7.19 - 7.17 (m, 2H), 7.10 - 7.07 (m, 2H), 4.45 - 4.44 (m, 1H), 4.42 - 4.41 (m, 1H), 4.34 - 4.29 (m, 2H), 3.24 - 3.18 (m, 1H), 2.94 - 2.86 (m, 1H), 2.12 (s, 6H), 1.45 (s, 9H).

### Synthetic Scheme 23

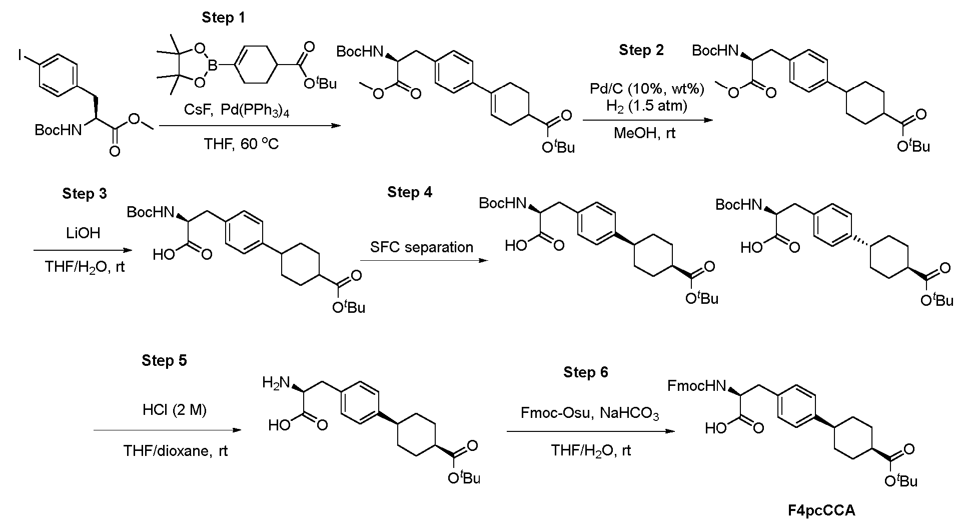

### F4pcCCA

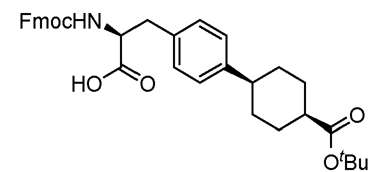

### (1R,4s)-4-(4-((S)-2-amino-2-carboxyethyl)phenyl)cyclohexane-1-carboxylic acid

Step 1: To a mixture of *tert*-butyl 4-(4,4,5,5-tetramethyl-1,3,2-dioxaborolan-2-yl) cyclohex-3-ene-1-carboxylate (29.5 g, 96 mmol) in THF (30 mL) was added methyl (*S*)-2-((*tert-*butoxycarbonyl) amino)-3-(4-iodophenyl) propanoate (15.5 g, 38.2 mmol), and Pd(Ph₃P)₄ (2.21 g, 1.91 mmol) at room temperature. The reaction was warmed to 60 °C for 4 h. The resulting solution was quenched with water (100 mL) and extracted with EtOAc (3 x 300 mL). The organic layers were combined, washed with brine (2 x 200mL), dried over anhydrous sodium sulfate, and filtered. The filtrate was concentrated under reduced pressure to afford crude product. The residue was purified by silica gel chromatography, eluting with a gradient of ethyl acetate in petroleum ether (0% to 25%) to afford *tert-*butyl 4'-((*S*)-2-((*tert-*butoxycarbonyl)amino)-3-methoxy-3-oxopropyl)-2,3,4,5-tetrahydro-[1,1'-biphenyl]-4-carboxylate. MS ESI calculated for C₂₆H₃₈NO₆Na [M + Na]⁺, 482.26 found 482.10.

Step 2: To a mixture of *tert*-butyl 4'-((*S*)-2-((*tert*-butoxycarbonyl) mino)-3-methoxy-3-oxopropyl)-2,3,4,5-tetrahydro-[1,1'-biphenyl]-4-carboxylate (16 g, 34.8 mmol) in methanol (160 mL) was added Pd-C (10% on carbon, wetted with ca.55% water, 5.3 g, 4.98 mmol) at room temperature. The solution was degassed with H₂ three times and stirred for 1 h at room temperature under the atmosphere of H₂ (1.5 atm). The resulting solution was filtered and the filtrate was concentrated under reduced pressure to afford *tert-*butyl(*S*)-4-(4-(2-((*tert-*butoxycarbonyl) amino)-3-methoxy-3-oxopropyl)phenyl)cyclohexane-1-carboxylate. MS ESI calculated for C₂₆H₄₁NO₆ [M + H]⁺, 462.28, found 462.30.

Step 3: To a stirred solution of *tert*-butyl(*S*)-4-(4-(2-((*tert*-butoxycarbonyl) amino)-3-methoxy-3-oxopropyl)phenyl)cyclohexane-1-carboxylate (15 g, 32.5 mmol) in THF (300 mL) was added LiOH (65.0 mL, 65.0 mmol) at room temperature. The solution was stirred at 20 °C for 1 h. The pH of the solution was adjusted to 3 with 1 N HCl. The reaction was concentrated under reduced pressure to give (*S*)-2-((*tert*-butoxycarbonyl)amino)-3-(4-(4-(*tert-*butoxycarbonyl)cyclohexyl)phenyl)propanoic acid. MS ESI calculated for C₂₅H₃₈NO₆Na [M + Na]⁺, 470.26, found 470.30.

Step 4: The (*S*)-2-((*tert*-butoxycarbonyl)amino)-3-(4-(4-(*tert*-butoxycarbonyl) cyclohexyl) phenyl) propanoic acid (14 g, 31.3 mmol) was separated using preparative supercritical fluid chromatography with the following conditions: Column: CHIRAL ART Cellulose-SB, 3 x 25 cm, 5µm; Mobile Phase A: CO₂, Mobile Phase B: MeOH (0.1% 2M NH₃-MEOH); Flow rate: 80 mL/min; Gradient: 10% B; 220 nm; retention time for peak 1: 7.45; retention time for peak 2: 8.38; to afford the first eluting peak (*S*)-2-((*tert*-butoxycarbonyl) amino)-3-(4-((*1s*,*4R*)-4-(*tert-*butoxycarbonyl) cyclohexyl) phenyl) propanoic acid. MS ESI calculated for C₂₅H₃₈NO₆Na [M + Na]⁺, 470.26 found 470.30. ¹H NMR (300 MHz, CDCl₃) *δ* 7.11 (d, J = 3.0 Hz, 4H), 4.27 (s, 1H), 3.18 - 3.12 (m, 1H), 2.91 (s, 1H), 2.60 (s, 1H), 2.49 (s, 1H), 2.20 (d, J = 10.9 Hz, 2H), 1.78 - 1.52 (m, 6H), 1.49 (d, J = 0.7 Hz, 9H), 1.36 (s, 9H).

And the second eluting peak (*S*)-2-((*tert*-butoxycarbonyl)amino)-3-(4-((*1r*,*4S*)-4-(*tert-*butoxycarbonyl)cyclohexyl)phenyl)propanoic acid. MS ESI calculated for C₂₅H₃₈NO₆Na [M + Na]⁺, 470.26 found 470.30. 1H NMR (300 MHz, CDCl₃) δ 7.14 - 7.06 (m, 4H), 4.25 (s, 1H), 3.14 (d, *J* = 12.3 Hz, 1H), 2.88 (s, 1H), 2.49 - 2.41 (m, 1H), 2.29 - 2.15 (m, 1H), 2.05 (d, *J =* 12.2 Hz, 2H), 1.90 (d, *J* = 12.1 Hz, 2H), 1.69 - 1.47 (m, 2H), 1.47 (s, 11H), 1.34 (s, 9H).

Step 5: To a solution of (*S*)-2-((*tert*-butoxycarbonyl) amino)-3-(4-((*1s,4R*)-4-(*tert-*butoxycarbonyl) cyclohexyl) phenyl) propanoic acid (8.3 g, 18.54 mmol) in THF (80 mL) was added HCl (9.27 mL, 18.54 mmol) in portions at room temperature. The reaction was concentrated under reduced pressure to afford (*S*)-2-amino-3-(4-((*1s,4R)*-4-(*tert-*butoxycarbonyl)cyclohexyl) phenyl)propanoic acid. MS ESI calculated for C₂₀H₃₀NO₄ [M + H]⁺, 348.21 found 348.25.

Step 6: To a stirred solution of (*S*)-2-amino-3-(4-((*1s,4R*)-4-(*tert*-butoxycarbonyl) cyclohexyl) phenyl) propanoic acid (6 g, 17.27 mmol) and NaHCO₃ (7.25 g, 86 mmol) in THF (60 mL) and water (60.0 mL) was added Fmoc-OSu (5.24 g, 15.54 mmol). The mixture was stirred at room temperature for 1 h. The pH of the solution was adjusted to 3 with 1 N HCl. The aqueous phase was extracted with EtOAc (2 x 200 mL). The combined organic layer was washed with brine (150 mL), dried over anhydrous sodium sulfate, and filtered. The filtrate was concentrated under reduced pressure, and the residue was purified by reverse phase column chromatography to give (*S*)-2-((((9*H*-fluoren-9-yl)methoxy)carbonyl)amino)-3-(4-((*1s,4R*)-4-(*tert*-butoxycarbonyl)cyclohexyl)phenyl)propanoic acid. MS ESI calculated for C₃₅H₄₁NO₆ [M + H]⁺, 570.28 found 570.15. ¹H NMR (300 MHz, Methanol-*d₄*) δ 7.81 (d, *J =* 7.5 Hz, 2H), 7.61 (d, *J =* 7.5 Hz, 2H), 7.43 - 7.27 (m, 4H), 7.16 (d, *J* = 7.8 Hz, 2H), 7.07 (d, *J* = 7.9 Hz, 2H), 4.45-4.33 (m, 2H), 4.22 - 4.06 (m, 2H), 3.26 - 3.14 (m, 1H), 2.96 - 2.88 (m, 1H), 2.59 (s, 1H), 2.48 (s, 1H), 2.15 (s, 2H), 1.61 (d, *J =* 5.5 Hz, 6H), 1.49 (s, 9H).

### Synthetic Scheme 24

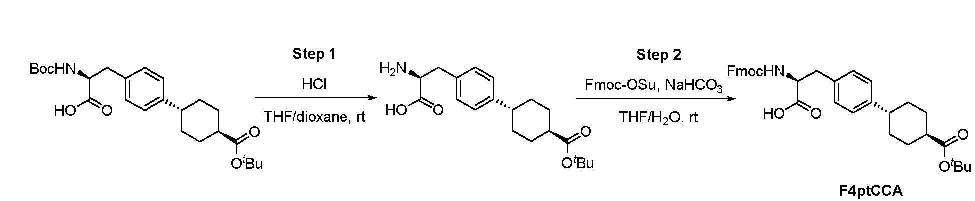

### F4ptCCA

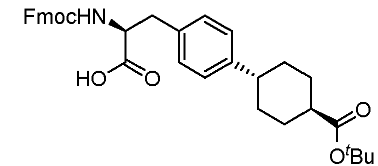

### (1S,4r)-4-(4-((S)-2-amino-2-carboxyethyl)phenyl)cyclohexane-1-carboxylic acid

Step 1: To a mixture of (*S*)-2-((*tert*-butoxycarbonyl) amino)-3-(4-((*1r,4S)*-4-(*tert-*butoxycarbonyl) cyclohexyl) phenyl) propanoic acid (3 g, 6.70 mmol) in THF (30 mL) was added hydrogen chloride (30 mL, 60.0 mmol) in portions at room temperature. The reaction was concentrated under reduced pressure to afford (*S*)-2-amino-3-(4-((*1r,4S*)-4-(*tert-*butoxycarbonyl)cyclohexyl)phenyl) propanoic acid. MS ESI calculated for C₂₀H₃₁NO₄ [M + H]⁺, 348.21, found 348.25.

Step 2: To a stirred solution of (*S*)-2-amino-3-(4-((*1r,4S*)-4-(*tert*-butoxycarbonyl) cyclohexyl)phenyl) propanoic acid (2 g, 5.76 mmol) (1.748 g, 5.18 mmol) and NaHCO₃ (2.418 g, 28.8 mmol) in THF (20 mL) and water (20 mL) was added Fmoc-OSu (1.748 g, 5.18 mmol) at room temperature. The mixture was stirred at 20 °C for 1 h. The pH of the solution was adjusted to 3 with 1 N HCl. The aqueous phase was extracted with EtOAc (2 x 200 mL). The combined organic layers were washed with brine (3 x 50 mL), dried over anhydrous sodium sulfate, and filtered. The filtrate was concentrated under reduced pressure and the residue was purified by reverse phase column chromatography to give (*S*)-2-((((9*H*-fluoren-9-yl) methoxy) carbonyl) amino)-3-(4-((*1r*,*4S*)-4-(*tert*-butoxycarbonyl) cyclohexyl) phenyl) propanoic acid. MS ESI calculated for C₃₅H₄₁NO₆ [M + H]⁺, 570.28, found 570.35. ¹H NMR (300 MHz, Methanol-*d*₄) δ 7.81 (d, *J* = 7.6 Hz, 2H), 7.61 (d, J = 7.5 Hz, 2H), 7.43 - 7.38 (m, 2H), 7.34 - 7.27 (m, 2H), 7.17 (d, *J* = 8.0 Hz, 2H), 7.08 (d, *J =* 7.8 Hz, 2H), 4.47 - 4.43 (m, 1H), 4.35 - 4.30 (m, 1H), 4.21 - 4.02 (m, 2H), 3.24 - 3.18 (m, 1H), 2.94 - 2.87 (m, 1H), 2.40 (s, 1H), 2.17 (d, *J* = 11.9 Hz, 1H), 1.96 (s, 3H), 1.82 - 1.55 (m, 2H), 1.47 (s, 13H).

### Preparation of Final Compounds:

### A. Generalized Procedure for Synthesizing Linear Peptide Precursors

Peptides in Table 1 were synthesized using standard solid-phase synthesis using Fmoc/tBu chemistry as exemplified in Chan, W.C.; White, P.D. "Fmoc Solid-Phase Synthesis: a Practical Approach", Oxford University Press, Oxford, 2000; Steward, J.; Young, J. "Solid Phase Peptide Synthesis", Pierce Chemical Company, Rockford, 1984.; Benoiton, N.L. "Chemistry of Peptide Synthesis", CRC Press, New York, 2006; and Lloyd-Williams, P.; Albericio, F.; Giralt, E. "Chemical Approaches to the Synthesis of Peptides and Proteins", CRC Press, New York, 1997.

During peptide chain elongation, the α-amino group of each amino acid was protected with a 9*H*-fluoren-9-ylmethoxycarbonyl group (Fmoc). To avoid any side reactions during the chain elongation steps, any reactive amino acid side chains also carry acid-labile protecting groups, effectively masking the reactive groups until removal upon treatment with strong acid. After completion of each coupling step, the Fmoc group of the *N*-terminal amino acid was removed with piperidine or 4-methylpiperidine and the resin was thoroughly washed to prepare for the coupling of the subsequent Fmoc-protected amino acid derivative.

The side chain protecting groups used were: *tert*-butyl (*t*Bu) for S, hY, Bip4CO2H, Phe4COOH, F4pcCCA, F4ptCCA, F4bcpA; *tert*-butoxy-carbonyl (Boc) for Dap, dDab, dK, dOrn, K, Prot4NH2, dDap, daMeDab; and, β-methylpentyl ester (OMpe) for D.

Fmoc-protected amino acids were typically obtained from vendors such as Sigma-Aldrich, Novabiochem, Chem-Impex, and Combi-Block.

### B. Synthetic Procedures used to Prepare Cyclic Peptides

### Synthetic Scheme 25

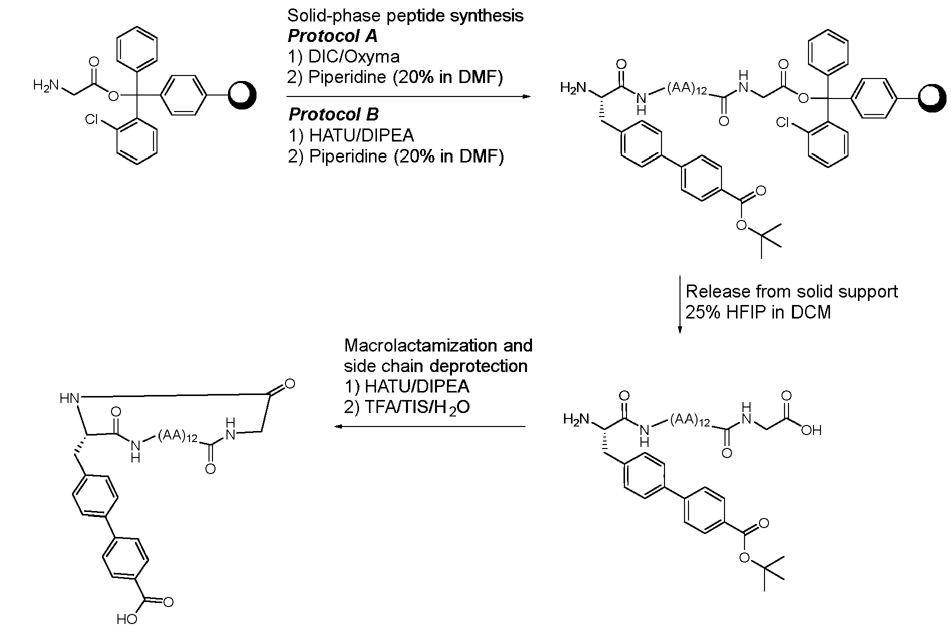

### Solid-Phase Synthesis of Peptides Protocol A

Peptides were synthesized on a Liberty Blue^{™} synthesizer from CEM Corporation, using standard solid-phase synthesis using Fmoc/tBu chemistry as summarized above in Scheme 25. *N*,*N*'-Diisopropylcarbodiimide (DIC) with ethyl cyano(hydroxyimino)acetate (Oxyma) were used as coupling agents to form the amide bond between the free amino terminus of the resin-bound protected peptide and the carboxylic acid of the Fmoc-protected amino acid.

H-Gly-loaded 2-chlorotrityl resin (200-400 mesh, 0.79 mmol/g loading, 1% cross-linked polystyrene, Novabiochem) was used for synthesis. All the amino acids were dissolved at a 0.2 M concentration in DMF (*N*,*N-*dimethylformamide). The amino acids were activated with equimolar amounts of Oxyma solution (0.5 M in DMF), and a 2-fold molar excess of DIC solution (1.0 M in DMF). Alternatively, amino acids were dissolved at a 0.125 M concentration in DMF (*N*,*N*-dimethylformamide). The amino acids were activated with equimolar amounts of Oxyma Pure solution (0.125 M in DMF; with 0.05 M DIEA), and a 2-fold molar excess of DIC solution (0.25 M in DMF). Reactions were typically performed at the 25 µmol scale.

Every synthesis cycle included: Fmoc-amino acid deprotection by 20% piperidine in DMF (90 °C microwave assisted heating, 2 min) and coupling (potentially repeated twice for difficult couplings) with Fmoc-protected amino acid/DIC/Oxyma (5, 5, and 10 eq respectively; 90 °C microwave assisted heating, 2 min or 4 min). Cycles of Fmoc deprotection and Fmoc-protected amino acid coupling were repeated with the desired monomers until the full linear peptide was formed.

### Solid-Phase Synthesis of Peptides Protocol B

Alternatively, peptides were synthesized on a Biotage^{®} Syro II peptide synthesizer using standard solid-phase synthesis using Fmoc/tBu chemistry as summarized above in Scheme 25. HATU with DIPEA were used as coupling agents to create the amide bond between the free amino terminus of the resin-bound protected peptide and the carboxylic acid of the Fmoc-protected amino acid. H-Gly-loaded 2-chlorotrityl resin (200-400 mesh, 0.79 mmol/g loading, 1% cross-linked polystyrene, Novabiochem) was used for synthesis. All the amino acids were dissolved at a 0.2 M concentration in 1: 1 DMF:NMP. Reactions were typically performed at the 12 µmol scale.

Every synthesis cycle included: (1) Coupling (repeated twice) with Fmoc-protected amino acid/HATU/DIPEA (4, 4 and 8 eq, respectively; rt; 15 min). The mixture was filtered, and the peptidyl resin was washed with DMF (2 x 1 mL); (2) Fmoc deprotection (repeated three times): 20% 4-methyl piperidine in DMF (1 mL; rt; 3 min). The mixture was filtered, and the peptidyl resin was washed with DMF (4 x 1 mL). Cycles of Fmoc deprotection and Fmoc-protected amino acid coupling were repeated with the desired monomers until the full linear peptide was formed.

### Selective Cleavage of Protected Peptide and Macrolactamization

For the cleavage of the protected linear peptide from the solid support, the peptidyl resin (~16 mg) was treated with 25% hexafluoroisopropanol (HFIP) in DCM for 20 min at rt, filtered, and the solvent was removed under reduced pressure. The resulting residue was dissolved in DMF (5 mL). HATU (0.44 eq) and DIPEA (2.5 eq) were added. The mixture was stirred for 5 min at rt. Then an additional 0.66 eq of HATU was added. Upon completion of the macrolactamization, monitored by UPLC-MS, the solvent was removed under reduced pressure.

### Final side chain deprotection

A solution of TFA/H₂O/TIS (90/8/2, v/v/v, 1 mL) was added to the crude protected cyclic peptide. The mixture was stirred for 10 min at rt. Cold diethyl ether (15 mL) was added to the solution. The peptide was precipitated by centrifugation (3200 rpm, -10 °C). The precipitate was washed with diethyl ether (2 x 10 mL) and dried under vacuum overnight to give the crude deprotected cyclic peptide as a solid.

### HPLC Purification

Purification was performed by preparative reversed-phase high performance liquid chromatography (RP-HPLC) on Waters X-Bridge Prep C18 OBD Prep column (130 Å, 5 pm, column size 19 * 100 mm) using a Waters MS-Directed AutoPurification HPLC/MS system. Mobile phase: (A) 0.16% TFA in HPLC water and (B) 0.16% TFA in HPLC acetonitrile; flow rate: 25 mL/min; UV wavelength λ = 215 nm; gradient: 25-50% B over 5 min. Alternatively purification was performed on Waters CSH-C18 Column (19 x 250mm, 5 uM) using an Agilent, with 1290 infinity II preparative LC system and LC-MSD XT mass spectrometer. Moblie phase: (A) 0.1% formic acid in HPLC water and (B) 0.1% formic acid in HPLC acetonitrile; flow rate: 25 mL/min; UV wavelength λ = 215 nm; gradient: 20% B over 2.5 min, 55% B over 2.5-20 min. UV absorbing fractions containing the target m/z ions were collected and the fractions containing product were confirmed by LC/MS.

Confirmation of identity and purity assessment of final compounds were performed by UPLC-MS, which was measured by a reverse phase Waters Acquity UPLC-MS system. Column: Waters XSelect CSH C18 Column (130 Å, 2.5 µm, column size 2.1 * 50 mm). Mobile phase: (A) 0.05% TFA in HPLC water and (B) 0.05% TFA in HPLC acetonitrile; injection volume: 1 µL; flow rate: 1 mL/min; UV wavelength λ = 215 nm; gradient: 5-100% B in 5 min. Lyophilization of combined fractions containing pure peptide resulted in the final cyclized product as a powder.

### BIOLOGICAL ASSAYS:

### Procedure for IL-6 Assay in MRC5 cells

The inhibition of IL-1β induced IL-6 secretion was evaluated in MRC5 cells. Recombinant human IL-1β (BioLegend 579404) at 2X EC₈₀ concentration was prepared in seeding medium, EMEM (ATCC 30-2003) with 0.025% BSA (Sigma A9576), 1X penicillin/streptomycin (Gibco 15070-063), 1X NEAA (Gibco 11140-050), 1X GlutaMax (Gibco 35050-061), and 1X sodium pyruvate (Gibco 11360-070). 20 µL of IL-1β was added to a 384-well collagen-coated plate (Corning 354664) and pre-incubated at ambient temperature for 1 hour with 200 nL of compound dispensed using an ECHO 555 liquid handler. Human lung fibroblast MRC5 cells (ATCC CCL-171) were added at a density of 3000 cells / 20 µL per well. The cells were prepared by passaging three times in growth medium, EMEM (ATCC 30-2003) with 10% fetal bovine serum (Gibco 16140-071), 1X penicillin/streptomycin (Gibco 15070-063), 1X NEAA (Gibco 11140-050), 1X GlutaMax (Gibco 35050-061), and 1X sodium pyruvate (Gibco 11360-070) in collagen-coated T175 flasks (Greiner 661950) and harvested in seeding medium after 5 minutes of 0.25% trypsin-EDTA (Gibco 25200-056) digestion. The 384-well collagen-coated plate, containing a final volume of 40 µL, was incubated at 37 °C, 5% CO₂ overnight. 5 µL of the conditioned medium was transferred to a 384-well AlphaLISA plate (PerkinElmer 6005350) for detection of IL-6 using the human AlphaLISA IL-6 kit (PerkinElmer AL223F) as per the manufacturer's protocol. 20 µL of the acceptor bead/biotinylated antibody mix was added to the 384-well AlphaLISA plate and incubated for 1 hour at ambient temperature. The donor bead mix was protected from light and 25 µL was added to the plate and incubated for 30 minutes at ambient temperature. The AlphaLISA plate was read on an EnVision multimode plate reader (Perkin Elmer model 2104) using the AlphaScreen setting (laser exc at 680 nm and emis at 570 nm). Dose response curves and IC₅₀ values were analyzed using a 4-parameter logistic equation in Spotfire software (Tibco, Palo Alto, CA).

The amino acid sequences, biological activities (MRC IC50s), calculated monoisotopic masses, molecular formulas, calculated molecular weights and mass spectral data ([M+H]+ or [M+2H]/2+) of Example Nos. 1-215 (SEQ ID NOS: 1-215) are provided below in Table 1.

**Table 1**

| **SEQ ID NO.** | **Sequence** | **MRC5 IC₅₀ (nM)** | **Exact Mass (Da)** | **Molecular Formula** | **Molecular Weight (g/mol)** | **Measured Mass (Da)** | **Observed ion** |
|---|---|---|---|---|---|---|---|
| **1** | 3AzaPhe4AcPip-W- Phe4COOH-G- Prot4NH2-SbMe1Nal- D-dNMeA-G- Bip4CO2H-4Pal-Dap- dA-h3Pal5CN | 0.0365 | 2047.87944 | C106H117N23O21 | 2049.2 | 1025.5 | [M+2H]/2+ |
| **2** | 3AzaPhe4AcPip-W- Phe4COOH-G- Prot4NH2-SbMe1Nal- D-dNMeA-G- Bip4CO2H-K-L-dA- h3Pal5CN | 0.0217 | 2054.94679 | C107H126N22O21 | 2056.28 | 1029.0 | [M+2H]/2+ |
| **3** | 3AzaPhe4AcPip-W- Phe4COOH-G- Prot4NH2-SbMe1Nal- D-dNMeA-G- Bip4CO2H-K-L-dA-hY | 0.05985 | 2044.95121 | C107H128N20O22 | 2046.29 | 1024.1 | [M+2H]/2+ |
| **4** | 3AzaPhe4AcPip-W- Phe4COOH-G- Prot4NH2-SbMe1Nal- D-dNMeA-G- Bip4CO2H-W-Dap-dA- h3Pal5CN | 0.0438 | 2085.89509 | C109H119N23O21 | 2087.25 | 1044.5 | [M+2H]/2+ |
| **5** | 3AzaPhe4AcPip-W- Phe4COOH-G- Prot4NH2-sbMe1Nal- D-dNMeA-G- Bip4CO2H-W-Dap-dA- hY | 0.6739 | 2075.8995 | C109H121N21O22 | 2077.25 | 1039.4 | [M+2H]/2+ |
| **6** | 3AzaPhe4AcPip-W- Phe4COOH-G- Prot4NH2-SbMeW4F- D-dNMeA-G- Bip4CO2H-K-L-dA-hY | 0.0666 | 2051.93703 | C105H126FN21O22 | 2053.25 | 1027.6 | [M+2H]/2+ |
| **7** | 3AzaPhe4AcPip-W- Phe4COOH-G- Prot4NH2-SbMeW4F- D-dNMeA-G- Bip4CO2H-K-L-dDab- hY | 0.1353 | 2080.96358 | C106H129FN22O22 | 2082.29 | 1042.2 | [M+2H]/2+ |
| **8** | 3AzaPhe4AcPip-W- Phe4COOH-G- Prot4NH2-SbMeW4F- D-dNMeA-G- Bip4CO2H-W-G-dDab- hY | 0.1401 | 2082.88533 | C107H119FN22O22 | 2084.22 | 1043.0 | [M+2H]/2+ |
| **9** | 3AzaTyrEtNAc-W- Phe4COOH-G- Prot4NH2-SbMeW4F- D-dNMeA-G- Bip4CO2H-K-L-dA-hY | 0.1182 | 2026.9054 | C103H123FN20O23 | 2028.2 | 1015.7 | [M+2H]/2+ |
| **10** | 3Pal4Ph4CO2H-W- Phe4COOH-G- Prot4NH2-sbMe1Nal- D-dNMeA-G- 3Pal4Ph4CO2H-W- Dap-dA-hY | 0.256 | 2070.83657 | C109H114N20O23 | 2072.19 | 1036.7 | [M+2H]/2+ |
| **11** | 3Pal4Ph4CO2H-W- Phe4COOH-G- Prot4NH2-sbMe1Nal- D-dNMeA-G- Bip4CO2H-W-Dap-dA- hY | 0.0908 | 2069.84132 | C110H115N19O23 | 2071.2 | 1036.7 | [M+2H]/2+ |
| **12** | Bip4CO2H-W- Phe4COOH-G- Prot4NH2-sbMe1Nal- D-dNMeA-G- 3Pal4Ph4CO2H-W- Dap-dA-hY | 0.0551 | 2069.84132 | C110H115N19O23 | 2071.2 | 1036.5 | [M+2H]/2+ |
| **13** | Bip4CO2H-W- Phe4COOH-G- Prot4NH2-SbMe1Nal- D-dNMeA-G- Bip4CO2H-1Inda-Dap- dA-hY | 0.03096 | 2069.84132 | C110H115N19O23 | 2071.2 | 1037.4 | [M+2H]/2+ |
| **14** | Bip4CO2H-W- Phe4COOH-G- Prot4NH2-SbMe1Nal- D-dNMeA-G- Bip4CO2H-1Indolin- Dap-dA-hY | 0.02974 | 2070.86172 | C111H118N18O23 | 2072.23 | 1037.4 | [M+2H]/2+ |
| **15** | [?] Bip4CO2H-W- Phe4COOH-G- Prot4NH2-sbMe1Nal- D-dNMeA-G- Bip4CO2H-1Nal-Dab- Aib-G | 0.6535 | 1987.82461 | C107H113N17O22 | 1989.14 | 997.4 | [M+2H]/2+ |
| **16** | Bip4CO2H-W- Phe4COOH-G- Prot4NH2-sbMe1Nal- D-dNMeA-G- Bip4CO2H-1Nal-Dab- Aib-hY | 0.175 | 2107.88212 | C115H121N17O23 | 2109.29 | 1057.7 | [M+2H]/2+ |
| **17** | Bip4CO2H-W- Phe4COOH-G- Prot4NH2-sbMe1Nal- D-dNMeA-G- Bip4CO2H-1Nal-Dap- Aib-G | 0.222 | 1973.80896 | C106H111N17O22 | 1975.12 | 989.2 | [M+2H]/2+ |
| **18** | Bip4CO2H-W- Phe4COOH-G- Prot4NH2-sbMe1Nal- D-dNMeA-G- Bip4CO2H-1Nal-Dap- Aib-h3Pal5CN | 0.0885 | 2103.86206 | C114H117N19O22 | 2105.26 | 2104.4 | [M+H]+ |
| **19** | Bip4CO2H-W- Phe4COOH-G- Prot4NH2-sbMe1Nal- D-dNMeA-G- Bip4CO2H-1Nal-L- Aib-G | 0.1179 | 2000.84501 | C109H116N16O22 | 2002.18 | 1002.8 | [M+2H]/2+ |
| **20** | Bip4CO2H-W- Phe4COOH-G- Prot4NH2-sbMe1Nal- D-dNMeA-G- Bip4CO2H-1Nal-Orn- Aib-G | 0.4401 | 2001.84026 | C108H115N17O22 | 2003.17 | 1004.1 | [M+2H]/2+ |
| **21** | Bip4CO2H-W- Phe4COOH-G- Prot4NH2-SbMe1Nal- D-dNMeA-G- Bip4CO2H-4Pal-Dap- dA-h3Pal5CN | 0.0234 | 2040.82601 | C108H112N20O22 | 2042.17 | 1022.0 | [M+2H]/2+ |
| **22** | Bip4CO2H-W- Phe4COOH-G- Prot4NH2-SbMe1Nal- D-dNMeA-G- Bip4CO2H-4Pal-Dap- dA-hY | 0.03251 | 2030.83042 | C108H114N18O23 | 2032.17 | 1017.4 | [M+2H]/2+ |
| **23** | Bip4CO2H-W- Phe4COOH-G- Prot4NH2-SbMe1Nal- D-dNMeA-G- Bip4CO2H-AlaTHP4- Dap-dA-h3Pal5CN | 0.0112 | 2047.85697 | C108H117N19O23 | 2049.2 | 1025.5 | [M+2H]/2+ |
| **24** | Bip4CO2H-W- Phe4COOH-G- Prot4NH2-SbMe1Nal- D-dNMeA-G- Bip4CO2H-AlaTHP4- Dap-dA-hY | 0.02912 | 2037.86139 | C108H119N17O24 | 2039.2 | 1021.1 | [M+2H]/2+ |
| **25** | Bip4CO2H-W- Phe4COOH-G- Prot4NH2-SbMe1Nal- D-dNMeA-G- Bip4CO2H-K-L-dA-hY | 0.05424 | 2037.89777 | C109H123N17O23 | 2039.24 | 1021.0 | [M+2H]/2+ |
| **26** | Bip4CO2H-W- Phe4COOH-G- Prot4NH2-SbMe1Nal- D-dNMeA-G- Bip4CO2H-K-L-dS-hY | 0.06881 | 2053.89269 | C109H123N17O24 | 2055.24 | 1029.0 | [M+2H]/2+ |
| **27** | Bip4CO2H-W- Phe4COOH-G- Prot4NH2-SbMe1Nal- D-dNMeA-G- Bip4CO2H-PyrimAla- Dap-dA-h3Pal5CN | 0.017 | 2041.82125 | C107H111N21O22 | 2043.15 | 1022.6 | [M+2H]/2+ |
| **28** | Bip4CO2H-W- Phe4COOH-G- Prot4NH2-SbMe1Nal- D-dNMeA-G- Bip4CO2H-PyrimAla- Dap-dA-hY | 0.03926 | 2031.82567 | C107H113N19O23 | 2033.16 | 1017.9 | [M+2H]/2+ |
| **29** | Bip4CO2H-W- Phc4COOH-G- Prot4NH2-SbMe1Nal- D-dNMeA-G- Bip4CO2II-Trp7az- Dap-dA-hY | 0.0205 | 2069.84132 | C110H115N19O23 | 2071.2 | 1036.6 | [M+2H]/2+ |
| **30** | Bip4CO2H-W- Phe4COOH-G- Prot4NH2-SbMe1Nal- D-dNMeA-G- Bip4CO2H-W-Dap-dA- h3Pal5CN | 0.0185 | 2078.84166 | C111H114N20O22 | 2080.21 | 1040.9 | [M+2H]/2+ |
| **31** | Bip4CO2H-W- Phe4COOH-G- Prot4NH2-SbMe1Nal- D-dNMeA-G- Bip4CO2H-W-Dap-dA- hY | 0.02075 | 2068.84607 | C111H116N18O23 | 2070.22 | 1035.7 | [M+2H]/2+ |
| **32** | Bip4CO2H-W- Phe4COOH-G- Prot4NH2-sbMe1Nal- D-dNMeA-G- Bip4CO2H-W-G-dDab- hY | 0.03296 | 2068.84607 | C111H116N18O23 | 2070.22 | 2070.1 | [M+H]+ |
| **33** | Bip4CO2H-W- Phe4COOH-G- Prot4NH2-SbMeW4Cl- A-dNMeA-G- Bip4CO2H-1Nal-L- Aib-G | 0.5443 | 1979.81146 | C106H114ClN17O20 | 1981.59 | 992.9 | [M+2H]/2+ |
| **34** | Bip4CO2H-W- Phe4COOH-G- Prot4NH2-SbMeW4Cl- D-dNMeA-G- Bip4CO2H-1Nal-Dap- Aib-h3Pal5CN | 0.0755 | 2126.81833 | C112H115ClN20O22 | 2128.69 | 1064.9 | [M+2H]/2+ |
| **35** | Bip4CO2H-W- Phe4COOH-G- Prot4NH2-SbMeW4Cl- D-dNMeA-G- Bip4CO2H-1Nal-L- Aib-G | 0.1598 | 2023.80129 | C107H114ClN17O22 | 2025.6 | 1012.9 | [M+2H]/2+ |
| **36** | Bip4CO2H-W- Phe4COOH-G- Prot4NH2-SbMeW4Cl- D-dNMeA-G- Bip4CO2H-1Nal-L- Aib-hY | 0.05222 | 2143.8588 | C115H122ClN17O23 | 2145.75 | 1074.6 | [M+2H]/2+ |
| **37** | Bip4CO2H-W- Phe4COOH-G- | 0.1031 | 2024.79654 | C106H113ClN18O22 | 2026.59 | 1014.2 | [M+2H]/2+ |
| | Prot4NH2-SbMeW4Cl- D-dNMeA-G- Bip4CO2H-1Nal-Orn- Aib-G | | | | | | |
| **38** | Bip4CO2H-W- Phe4COOH-G- Prot4NH2-SbMeW4Cl- D-dNMeA-G- Bip4CO2H-3Pal-S-dA- hY | 0.2167 | 2054.77071 | C106H111ClN18O24 | 2056.57 | 1029.0 | [M+2H]/2+ |
| **39** | Bip4CO2H-W- Phe4COOH-G- Prot4NH2-SbMeW4Cl- D-dNMeA-G- Bip4CO2H-4Pal-S-dA- hY | 0.232 | 2054.77071 | C106H111ClN18O24 | 2056.57 | 1029.1 | [M+2H]/2+ |
| **40** | Bip4CO2H-W- Phe4COOH-G- Prot4NH2-SbMeW4Cl- D-dNMeA-G- Bip4CO2H-AlaPip4-S- dA-hY | *0.5085* | 2060.81766 | C106H117ClN18O24 | 2062.62 | 1032.6 | [M+2H]/2+ |
| **41** | Bip4CO2H-W- Phe4COOH-G- Prot4NH2-SbMeW4Cl- D-dNMeA-G- Bip4CO2H-Dap-S-dA- hY | 0.4688 | 1992.75506 | C101H109ClN18O24 | 1994.51 | 998.6 | [M+2H]/2+ |
| **42** | Bip4CO2H-W- Phe4COOH-G- Prot4NH2-SbMeW4Cl- D-dNMeA-G- Bip4CO2H-K-L-dA-hY | 0.6266 | 2060.85405 | C107H121ClN18O23 | 2062.67 | 1032.3 | [M+2H]/2+ |
| **43** | Bip4CO2H-W- Phe4COOH-G- Prot4NH2-SbMeW4Cl- D-dNMeA-G- Bip4CO2H-K-L-dDab- hY | 0.2437 | 2089.8806 | C108H124ClN19O23 | 2091.71 | 1047.1 | [M+2H]/2+ |
| **44** | Bip4CO2H-W- Phe4COOH-G- Prot4NH2-SbMeW4Cl- D-dNMeA-G- Bip4CO2H-K-L-dS-hY | 0.1321 | 2076.84896 | C107H121ClN18O24 | 2078.66 | 1040.1 | [M+2H]/2+ |
| **45** | Bip4CO2H-W- Phe4COOH-G- Prot4NH2-SbMeW4Cl- D-dNMeA-G- Bip4CO2H-K-S-dA-hY | 0.174 | 2034.80201 | C104H115ClN18O24 | 2036.59 | 1019.1 | [M+2H]/2+ |
| **46** | Bip4CO2H-W- Phe4COOH-G- Prot4NH2-SbMeW4Cl- D-dNMeA-G- Bip4CO2H-K-S-dDab- hY | 0.1336 | 2063.82856 | C105H118ClN19O24 | 2065.63 | 1034.1 | [M+2H]/2+ |
| **47** | Bip4CO2H-W- Phe4COOH-G- Prot4NH2-SbMeW4Cl- D-dNMeA-G- Bip4CO2H-K-S-dS-hY | 0.1235 | 2050.79693 | C104H115ClN18O25 | 2052.58 | 1027.6 | [M+2H]/2+ |
| **48** | Bip4CO2H-W- Phe4COOH-G- Prot4NH2-SbMeW4Cl- D-dNMeA-G- Bip4CO2H-mAF-S-dA- hY | 0.04098 | 2082.80201 | C108H115ClN18O24 | 2084.63 | 1043.1 | [M+2H]/2+ |
| **49** | Bip4CO2H-W- Phe4COOII-G- Prot4NH2-SbMeW4Cl- D-dNMeA-G- Bip4CO2H-Orn-S-dA- hY | 0.4016 | 2020.78636 | C103H113ClN18O24 | 2022.56 | 1012.6 | [M+2H]/2+ |
| **50** | Bip4CO2H-W- Phe4COOH-G- Prot4NH2-SbMeW4Cl- D-dNMeA-G- Bip4CO2H-Phe3NH2- S-dA-hY | 0.5196 | 2068.78636 | C107H113ClN18O24 | 2070.6 | 1036.2 | [M+2H]/2+ |
| **51** | Bip4CO2H-W- Phe4COOH-G- Prot4NH2-SbMeW4Cl- D-dNMeA-G- Bip4CO2H- Phe4CH2NH2-S-dA- hY | 0.3199 | 2082.80201 | C108H115ClN18O24 | 2084.63 | 1043.5 | [M+2H]/2+ |
| **52** | Bip4CO2H-W- Phe4COOH-G- Prot4NH2-SbMeW4Cl- D-dNMeA-G- Bip4CO2H-Phe4NH2- S-dA-hY | 0.4537 | 2068.78636 | C107H113ClN18O24 | 2070.6 | 1036.1 | [M+2H]/2+ |
| **53** | Bip4CO2H-W- Phe4COOH-G- Prot4NH2-SbMeW4Cl- D-dNMeA-G- Bip4CO2H-W-Dap-dA- hY | 0.04179 | 2091.80235 | C109H114ClN19O23 | 2093.64 | 1047.0 | [M+2H]/2+ |
| **54** | Bip4CO2H-W- Phe4COOH-G- Prot4NH2-sbMeW4Cl- D-dNMeA-G- Bip4CO2H-W-G-dDab- hY | 0.06922 | 2091.80235 | C109H114ClN19O23 | 2093.64 | 1047.0 | [M+2H]/2+ |
| **55** | Bip4CO2H-W- Phe4COOH-G- Prot4NH2-sbMeW4Cl- E-dNMeA-G- Bip4CO2H-W-G-dDab- hY | 0.0493 | 2105.818 | C110H116ClN19O23 | 2107.66 | 1055.7 | [M+2H]/2+ |
| **56** | Bip4CO2H-W- Phe4COOH-G- Prot4NH2-sbMeW4Cl- N-dNMeA-G- Bip4CO2H-W-G-dDab- hY | 0.02065 | 2090.81833 | C109H115ClN20O22 | 2092.65 | 1046.8 | [M+2H]/2+ |
| **57** | Bip4CO2H-W- Phe4COOH-G- Prot4NH2-sbMeW4Cl- Q-dNMeA-G- Bip4CO2H-W-G-dDab- hY | 0.08728 | 2104.83398 | C110H117ClN20O22 | 2106.68 | 1055.2 | [M+2H]/2+ |
| **58** | Bip4CO2H-W- Phe4COOH-G- Prot4NH2-SbMeW4F- A-dNMeA-G- Bip4CO2H-K-L-dA-hY | 0.2475 | 2000.89377 | C106H121FN18O21 | 2002.2 | 1002.4 | [M+2H]/2+ |
| **59** | Bip4CO2H-W- Phe4COOH-G- Prot4NH2-SbMeW4F- D-dNMeA-G- Bip4CO2H-4Pal-L-dA- hY | 0.03617 | 2064.8523 | C109H117FN18O23 | 2066.2 | 1034.7 | [M+2H]/2+ |
| **60** | Bip4CO2H-W- Phe4COOH-G- Prot4NH2-SbMeW4F- D-dNMeA-G- Bip4CO2H-F-L-dA-hY | 0.06889 | 2063.85705 | C110H118FN17O23 | 2065.21 | 1034.2 | [M+2H]/2+ |
| **61** | Bip4CO2H-W- Phe4COOH-G- Prot4NH2-SbMeW4F- D-dNMeA-G- Bip4CO2H-h4Pal-L- dA-hY | 0.312 | 2078.86795 | C110H119FN18O23 | 2080.23 | 1041.5 | [M+2H]/2+ |
| **62** | Bip4CO2H-W- Phe4COOH-G- Prot4NH2-SbMeW4F- D-dNMeA-G- Bip4CO2H-hK-L-dA- hY | 0.283 | 2058.89925 | C108H123FN18O23 | 2060.24 | 1031.5 | [M+2H]/2+ |
| **63** | Bip4CO2H-W- Phe4COOH-G- Prot4NH2-SbMeW4F- D-dNMeA-G- Bip4CO2H-hQ-L-dA- hY | 0.06295 | 2058.86287 | C107H119FN18O24 | 2060.19 | 1031.2 | [M+2H]/2+ |
| **64** | Bip4CO2H-W- Phe4COOH-G- Prot4NH2-SbMeW4F- D-dNMeA-G- Bip4CO2H-K- AlaTHP4-dA- h3Pal5CN | 0.0182 | 2096.88975 | C109H121FN20O23 | 2098.25 | 1050.1 | [M+2H]/2+ |
| **65** | Bip4CO2H-W- Phe4COOH-G- Prot4NH2-SbMeW4F- D-dNMeA-G- Bip4CO2H-K- AlaTHP4-dA-hY | 0.0158 | 2086.89417 | C109H123FN18O24 | 2088.25 | 1045.0 | [M+2H]/2+ |
| **66** | Bip4CO2H-W- Phe4COOH-G- Prot4NH2-SbMeW4F- D-dNMeA-G- Bip4CO2H-K-L-dA- h3Pal5CN | 0.02985 | 2054.87918 | C107H119FN20O22 | 2056.21 | 2056.2 | [M+H]+ |
| **67** | Bip4CO2H-W- Phe4COOH-G- Prot4NH2-SbMeW4F- D-dNMeA-G- Bip4CO2H-K-L-dA-hY | 0.046 | 2044.8836 | C107H121FN18O23 | 2046.21 | 1024.7 | [M+2H]/2+ |
| **68** | Bip4CO2H-W- Phe4COOH-G- Prot4NH2-SbMeW4F- D-dNMeA-G- Bip4CO2H-K-L-dDab- hY | 0.296 | 2073.91015 | C108H124FN19O23 | 2075.25 | 1038.5 | [M+2H]/2+ |
| **69** | Bip4CO2H-W- Phe4COOH-G- Prot4NH2-SbMeW4F- D-dNMeA-G- Bip4CO2H-K-L-dS-hY | 0.3335 | 2060.87852 | C107H121FN18O24 | 2062.21 | 1032.4 | [M+2H]/2+ |
| **70** | Bip4CO2H-W- Phe4COOH-G- Prot4NH2-SbMeW4F- D-dNMeA-G- Bip4CO2H-K-L-G-hY | 0.09734 | 2030.86795 | C106H119FN18O23 | 2032.18 | 1017.5 | [M+2H]/2+ |
| **71** | Bip4CO2H-W- Phe4COOH-G- Prot4NH2-SbMeW4F- D-dNMeA-G- Bip4CO2H-K-Nva-dA- hY | 0.09256 | 2030.86795 | C106H119FN18O23 | 2032.18 | 1017.6 | [M+2H]/2+ |
| **72** | Bip4CO2H-W- Phe4COOH-G- Prot4NH2-SbMeW4F- D-dNMeA-G- Bip4CO2H-K-S-dA-hY | 0.10655 | 2018.83156 | C104H115FN18O24 | 2020.13 | 1011.5 | [M+2H]/2+ |
| **73** | Bip4CO2H-W- Phe4COOH-G- Prot4NH2-SbMeW4F- D-dNMeA-G- Bip4CO2H-mAF-L-dA- hY | 0.05088 | 2092.8836 | C111H121FN18O23 | 2094.25 | 1048.7 | [M+2H]/2+ |
| **74** | Bip4CO2H-W- Phe4COOII-G- Prot4NH2-SbMeW4F- | 0.296 | 2066.83156 | C108H115FN18O24 | 2068.17 | 1034.9 | [M+2H]/2+ |
| | D-dNMeA-G- Bip4CO2H-mAF-S-dA- hY | | | | | | |
| **75** | Bip4CO2H-W- Phe4COOH-G- Prot4NH2-SbMeW4F- D-dNMeA-G- Bip4CO2H-mBip-L- dA-hY | 0.1648 | 2139.88835 | C116H122FN17O23 | 2141.31 | 1071.5 | [M+2H]/2+ |
| **76** | Bip4CO2H-W- Phe4COOH-G- Prot4NH2-SbMeW4F- D-dNMeA-G- Bip4CO2H-mTyrOAl- L-dA-hY | 0.1217 | 2119.88327 | C113H122FN17O24 | 2121.28 | 1061.7 | [M+2H]/2+ |
| **77** | Bip4CO2H-W- Phe4COOH-G- Prot4NH2-sbMeW4F- D-dNMeA-G- Bip4CO2H-W-G-dDab- hY | 0.06011 | 2075.8319 | C109H114FN19O23 | 2077.18 | 1038.5 | [M+2H]/2+ |
| **78** | Bip4CO2H-W- Phe4COOH-G- Prot4NH2-SbMeW4F- D-dNMeA-G- Bip4CO2H-W-L-dA- hY | 0.07324 | 2102.86795 | C112H119FN18O23 | 2104.25 | 1052.9 | [M+2H]/2+ |
| **79** | Bip4CO2H-W- Phe4COOH-G- Prot4NH2-SbMeW4F- D-dNMeA-G- Bip4CO2H-W-Nva-dA- A | 0.1893 | 1982.81044 | C104H111FN18O22 | 1984.1 | 993.2 | [M+2H]/2+ |
| **80** | Bip4CO2H-W- Phe4COOH-G- Prot4NH2-SbMeW4F- N-dNMeA-G- Bip4CO2H-K-L-dA-hY | 0.2915 | 2043.89958 | C107H122FN19O22 | 2045.23 | 1023.9 | [M+2H]/2+ |
| **81** | Bip4CO2H-W- Phe4COOH-G- Prot4NH2-SbMeW4F- N-dNMeA-G- Bip4CO2H-K-L-dDab- hY | 0.2547 | 2072.92613 | C108H125FN20O22 | 2074.27 | 1038.1 | [M+2H]/2+ |
| **82** | Bip4CO2H-W- Phe4COOH-G- Prot4NHBn-SbMe1Nal- D-dNMeA-G- Bip4CO2H-W-Dap-dA- hY | 0.0157 | 2158.89302 | C118H122N18O23 | 2160.34 | 1081.0 | [M+2H]/2+ |
| **83** | Bip4CO2H-W- Phe4COOH-G- Prot4NHCH2CH2Ph- SbMe1Nal-D-dNMeA- G-Bip4CO2H-W-Dap- dA-hY | 0.0329 | 2172.90867 | C119H124N18O23 | 2174.36 | 1088.1 | [M+2H]/2+ |
| **84** | Bip4CO2H-W- Phe4COOH-G- Prot4NHEt-SbMe1Nal- D-dNMeA-G- Bip4CO2H-W-Dap-dA- hY | 0.0258 | 2096.87737 | C113H120N18O23 | 2098.27 | 1049.6 | [M+2H]/2+ |
| **85** | Phe4AcPip-W- Phe4COOH-G- Prot4NH2-sbMeW4Cl- D-dNMeA-G- Bip4CO2H-W-G-dDab- hY | 0.05433 | 2097.86053 | C108H120ClN21O22 | 2099.69 | 1050.9 | [M+2H]/2+ |
| **86** | Phe4Pyrim5CO2H-W- Phe4COOH-G- Prot4NH2-SbMe1Nal- D-dNMeA-G- | 0.0223 | 2070.83657 | C109H114N20O23 | 2072.19 | 1037.8 | [M+2H]/2+ |
| | Bip4CO2H-W-Dap-dA- hY | | | | | | |
| **87** | Phe4Pyrim5CO2H-W- Phe4COOH-G- Prot4NH2-SbMe1Nal- D-dNMeA-G- Bip4CO2H-W-Dap-dA- hY | 0.1415 | 2070.83657 | C109H114N20O23 | 2072.19 | 1037.9 | [M+2H]/2+ |
| **88** | PyrimAla4AcPip-W- Phe4COOH-G- Prot4NH2-sbMe1Nal- D-dNMeA-G- Bip4CO2H-W-Dap-dA- hY | 0.0945 | 2076.89475 | C108H120N22O22 | 2078.24 | 1039.7 | [M+2H]/2+ |
| **89** | TyrEtNAc-W- Phe4COOH-G- Prot4NH2-sbMe1Nal- A-dNMeA-G- Bip4CO2H-W-Dap-dA- hY | 0.271 | 2005.88279 | C107H119N19O21 | 2007.2 | 1004.6 | [M+2H]/2+ |
| **90** | TyrEtNAc-W- Phe4COOH-G- Prot4NH2-SbMe1Nal- D-dNMeA-G- Bip4CO2H-1Inda-G- dDab-h3Pal5CN | 0.0507 | 2060.86345 | C107H116N22O22 | 2062.2 | 1032.1 | [M+2H]/2+ |
| **91** | TyrEtNAc-W- Phe4COOH-G- Prot4NH2-SbMe1Nal- D-dNMeA-G- Bip4CO2H-1Indolin-G- dDab-h3Pal5CN | 0.0234 | 2061.88385 | C108H119N21O22 | 2063.23 | 1032.5 | [M+2H]/2+ |
| **92** | TyrEtNAc-W- Phe4COOH-G- Prot4NH2-sbMe1Nal- D-dNMeA-G- Bip4CO2H-1Nal-Dab- Aib-G | 0.457 | 1968.85116 | C104H116N18O22 | 1970.14 | 987.3 | [M+2H]/2+ |
| **93** | TyrEtNAc-W- Phe4COOH-G- Prot4NH2-sbMe1Nal- D-dNMeA-G- Bip4CO2H-1Nal-Dab- Aib-hY | 0.12 | 2088.90867 | C112H124N18O23 | 2090.29 | 1047.7 | [M+2H]/2+ |
| **94** | TyrEtNAc-W- Phe4COOH-G- Prot4NH2-sbMe1Nal- D-dNMeA-G- Bip4CO2H-1Nal-Dap- Aib-h3Pal5CN | 0.0639 | 2084.88861 | C111H120N20O22 | 2086.26 | 1043.4 | [M+2H]/2+ |
| **95** | TyrEtNAc-W- Phe4COOH-G- Prot4NH2-sbMe1Nal- D-dNMeA-G- Bip4CO2H-1Nal-L- Aib-G | 0.4222 | 1981.87156 | C106H119N17O22 | 1983.18 | 991.9 | [M+2H]/2+ |
| **96** | TyrEtNAc-W- Phe4COOH-G- Prot4NH2-sbMe1Nal- D-dNMeA-G- Bip4CO2H-1Nal-L- Aib-hY | 0.4637 | 2124.88535 | C112H125ClN18O23 | 2126.75 | 1063.7 | [M+2H]/2+ |
| **97** | TyrEtNAc-W- Phe4COOH-G- Prot4NH2-sbMe1Nal- D-dNMeA-G- Bip4CO2H-1Nal-L-dA- A | 0.2945 | 1981.87156 | C106H119N17O22 | 1983.18 | 992.8 | [M+2H]/2+ |
| **98** | TyrEtNAc-W- Phe4COOH-G- Prot4NH2-sbMe1Nal- D-dNMeA-G- | 0.494 | 1967.85591 | C105H117N17O22 | 1969.15 | 986.1 | [M+2H]/2+ |
| | Bip4CO2H-1Nal-Nva- dA-A | | | | | | |
| **99** | TyrEtNAc-W- Phe4COOH-G- Prot4NH2-sbMe1Nal- D-dNMeA-G- Bip4CO2H-1Nal-Orn- Aib-hY | 0.256 | 2102.92432 | C113H126N18O23 | 2104.32 | 1053.1 | [M+2H]/2+ |
| **100** | TyrEtNAc-W- Phe4COOH-G- Prot4NH2-sbMe1Nal- D-dNMeA-G- Bip4CO2H-4Pal-Dab- dA-hF4CF3 | 0.274 | 2077.86509 | C107H118F3N19O22 | 2079.19 | 2079.0 | [M+H]+ |
| **101** | TyrEtNAc-W- Phe4COOH-G- Prot4NH2-sbMe1Nal- D-dNMeA-G- Bip4CO2H-4Pal-Dab- dA-hY | 0.247 | 2025.87262 | C106H119N19O23 | 2027.19 | 2027.0 | [M+H]+ |
| **102** | TyrEtNAc-W- Phe4COOH-G- Prot4NH2-SbMe1Nal- D-dNMeA-G- Bip4CO2H-4Pal-G- dDab-h3Pal5CN | 0.0421 | 2021.85255 | C105H115N21O22 | 2023.16 | 1012.6 | [M+2H]/2+ |
| **103** | TyrEtNAc-W- Phe4COOH-G- Prot4NH2-sbMe1Nal- D-dNMeA-G- Bip4CO2H-4Pal-S-dA- hF2CF3 | 0.066 | 2064.83346 | C106H115F3N18O23 | 2066.15 | 1033.1 | [M+2H]/2+ |
| **104** | TyrEtNAc-W- Phe4COOH-G- Prot4NH2-sbMe1Nal- D-dNMeA-G- Bip4CO2H-4Pal-S-dA- hF3CF3 | 0.041 | 2064.83346 | C106H115F3N18O23 | 2066.15 | 1033.5 | [M+2H]/2+ |
| **105** | TyrEtNAc-W- Phe4COOH-G- Prot4NH2-sbMe1Nal- D-dNMeA-G- Bip4CO2H-4Pal-S-dA- hF4CF3 | 0.091 | 2064.83346 | C106H115F3N18O23 | 2066.15 | 1033.9 | [M+2H]/2+ |
| **106** | TyrEtNAc-W- Phe4COOH-G- Prot4NH2-SbMe1Nal- D-dNMeA-G- Bip4CO2H-AlaTHP4- G-dDab-h3Pal5CN | 0.0263 | 2028.88352 | C105H120N20O23 | 2030.2 | 1016.1 | [M+2H]/2+ |
| **107** | TyrEtNAc-W- Phe4COOH-G- Prot4NH2-sbMe1Nal- D-dNMeA-G- Bip4CO2H-K-G-Aib- hY | 0.05819 | 1976.87737 | C103H120N18O23 | 1978.16 | 989.7 | [M+2H]/2+ |
| **108** | TyrEtNAc-W- Phe4COOH-G- Prot4NH2-sbMe1Nal- D-dNMeA-G- Bip4CO2H-K-G-dA-hY | 0.102 | 1962.86172 | C102H118N18O23 | 1964.14 | 983.0 | [M+2H]/2+ |
| **109** | TyrEtNAc-W- Phe4COOH-G- Prot4NH2-sbMe1Nal- D-dNMeA-G- Bip4CO2H-K-G- dNMeA-hY | 0.07747 | 1976.87737 | C103H120N18O23 | 1978.16 | 989.0 | [M+2H]/2+ |
| **110** | TyrEtNAc-W- Phe4COOH-G- Prot4NH2-sbMe1Nal- D-dNMeA-G- Bip4CO2H-K-L-dA-hY | 0.1955 | 2018.92432 | C106H126N18O23 | 2020.24 | 2020.0 | [M+H]+ |
| **111** | TyrEtNAc-W- Phe4COOH-G- Prot4NH2-sbMe1Nal- D-dNMeA-G- Bip4CO2H-K-S-dA- hF4CF3 | 0.133 | 2044.86476 | C104H119F3N18O23 | 2046.16 | 2046.0 | [M+H]+ |
| **112** | TyrEtNAc-W- Phe4COOH-G- Prot4NH2-sbMe1Nal- D-dNMeA-G- Bip4CO2H-K-S-dA-hY | 0.06175 | 1992.87229 | C103H120N18O24 | 1994.16 | 1994.0 | [M+H]+ |
| **113** | TyrEtNAc-W- Phe4COOH-G- Prot4NH2-SbMe1Nal- D-dNMeA-G- Bip4CO2H-PyrimAla- G-dDab-h3Pal5CN | 0.0918 | 2022.8478 | C104H114N22O22 | 2024.15 | 1013.0 | [M+2H]/2+ |
| **114** | TyrEtNAc-W- Phe4COOH-G- Prot4NH2-SbMe1Nal- D-dNMeA-G- Bip4CO2H-Trp7az-G- dDab-h3Pal5CN | 0.0233 | 2060.86345 | C107H116N22O22 | 2062.2 | 1031.6 | [M+2H]/2+ |
| **115** | TyrEtNAc-W- Phe4COOH-G- Prot4NH2-sbMe1Nal- D-dNMeA-G- Bip4CO2H-W-Dab-dA- hF | 0.085 | 2047.89336 | C109H121N19O22 | 2049.24 | 1025.2 | [M+2H]/2+ |
| **116** | TyrEtNAc-W- Phe4COOH-G- Prot4NH2-sbMe1Nal- D-dNMeA-G- Bip4CO2H-W-Dab-dA- hY | 0.492 | 2063.88827 | C109H121N19O23 | 2065.24 | 2065.1 | [M+H]+ |
| **117** | TyrEtNAc-W- Phe4COOH-G- Prot4NH2-sbMe1Nal- D-dNMeA-G- Bip4CO2H-W-Dap- Aib-hY | 0.1595 | 2063.88827 | C109H121N19O23 | 2065.24 | 1034.5 | [M+2H]/2+ |
| **118** | TyrEtNAc-W- Phe4COOH-G- Prot4NH2-sbMe1Nal- D-dNMeA-G- Bip4CO2H-W-Dap-dA- A | 0.973 | 1943.83076 | C101H113N19O22 | 1945.09 | 973.7 | [M+2H]/2+ |
| **119** | TyrEtNAc-W- Phe4COOH-G- Prot4NH2-SbMe1Nal- D-dNMeA-G- Bip4CO2H-W-Dap-dA- hY | 0.06017 | 2049.87262 | C108H119N19O23 | 2051.21 | 1026.3 | [M+2H]/2+ |
| **120** | TyrEtNAc-W- Phe4COOH-G- Prot4NH2-SbMe1Nal- D-dNMeA-G- Bip4CO2H-W-Dap-dA- TyrEtMor | 0.86 | 2148.94104 | C113H128N20O24 | 2150.35 | 1076.1 | [M+2H]/2+ |
| **121** | TyrEtNAc-W- Phe4COOH-G- Prot4NH2-sbMe1Nal- D-dNMeA-G- Bip4CO2H-W-Dap- dNMeA-hY | 0.08498 | 2063.88827 | C109H121N19O23 | 2065.24 | 2064.7 | [M+H]+ |
| **122** | TyrEtNAc-W- Phe4COOH-G- Prot4NH2-SbMe1Nal- D-dNMeA-G- Bip4CO2H-W-G-dDab- h3Pal5CN | 0.0248 | 2059.8682 | C108H117N21O22 | 2061.21 | 1031.1 | [M+2H]/2+ |
| **123** | TyrEtNAc-W- Phe4COOH-G- Prot4NH2-sbMe1Nal- D-dNMeA-G- Bip4CO2H-W-G-dDab- hY | 0.04565 | 2049.87262 | C108H119N19O23 | 2051.21 | 1025.6 | [M+2H]/2+ |
| **124** | TyrEtNAc-W- Phe4COOH-G- Prot4NH2-sbMe1Nal- D-dNMeA-G- Bip4CO2H-W-L-dA-A | 0.636 | 1970.86681 | C104H118N18O22 | 1972.16 | 987.2 | [M+2H]/2+ |
| **125** | TyrEtNAc-W- Phe4COOH-G- Prot4NH2-SbMe1Nal- D-dNMeA-G- Bip4CO2H-W-L-dA- hY | 0.06403 | 2076.90867 | C111H124N18O23 | 2078.28 | 1039.7 | [M+2H]/2+ |
| **126** | TyrEtNAc-W- Phe4COOH-G- Prot4NH2-sbMe1Nal- D-dNMeA-G- Bip4CO2H-W-L-dDab- hY | 0.3705 | 2105.93522 | C112H127N19O23 | 2107.32 | 2107.0 | [M+H]+ |
| **127** | TyrEtNAc-W- Phe4COOH-G- Prot4NH2-sbMe1Nal- D-dNMeA-G- Bip4CO2H-W-Orn- Aib-hY | 0.4325 | 2091.91957 | C111H125N19O23 | 2093.29 | 2093.1 | [M+H]+ |
| **128** | TyrEtNAc-W- Phe4COOH-G- Prot4NH2-SbMe1Nal- D-dNMeA-G- Bip4CO2H-W-S-dA- hY | 0.090 | 2050.85664 | C108H118N18O24 | 2052.2 | 1026.4 | [M+2H]/2+ |
| **129** | TyrEtNAc-W- Phe4COOH-G- Prot4NH2-sbMe1Nal- N-dNMeA-G- Bip4CO2H-W-Dap-dA- hY | 0.08279 | 2048.88861 | C108H120N20O22 | 2050.23 | 1025.1 | [M+2H]/2+ |
| **130** | TyrEtNAc-W- Phe4COOH-G- Prot4NH2-SbMeW4Cl- D-dNMeA-G- Bip4CO2H-1Nal-Dap- Aib-h3Pal5CN | 0.114 | 2107.84488 | C109H118ClN21O22 | 2109.68 | 2109.0 | [M+H]+ |
| **131** | TyrEtNAc-W- Phe4COOH-G- Prot4NH2-SbMeW4Cl- D-dNMeA-G- Bip4CO2H-1Nal-L-dA- hY | 0.04984 | 2110.8697 | C111H123ClN18O23 | 2112.72 | 1056.5 | [M+2H]/2+ |
| **132** | TyrEtNAc-W- Phe4COOH-G- Prot4NH2-sbMeW4Cl- D-dNMeA-G- Bip4CO2H-1Nal-Orn- Aib-G | 0.3701 | 2005.82308 | C103H116ClN19O22 | 2007.59 | 1003.9 | [M+2H]/2+ |
| **133** | TyrEtNAc-W- Phe4COOH-G- Prot4NH2-SbMeW4Cl- D-dNMeA-G- Bip4CO2H-1Nal-S-dA- Phe4NH2 | 0.766 | 2069.818 | C107H116ClN19O23 | 2071.63 | 1036.9 | [M+2H]/2+ |
| **134** | TyrEtNAc-W- Phe4COOH-G- Prot4NH2-SbMeW4Cl- D-dNMeA-G- Bip4CO2H-4Pal-Dab- dA-hY | 0.3785 | 2048.8289 | C104H117ClN20O23 | 2050.61 | 1026.3 | [M+2H]/2+ |
| **135** | TyrEtNAc-W- Phe4COOH-G- Prot4NH2-SbMeW4Cl- D-dNMeA-G- Bip4CO2H-4Pal-L-dA- hY | 0.310 | 2061.8493 | C106H120ClN19O23 | 2063.65 | 1033.1 | [M+2H]/2+ |
| **136** | TyrEtNAc-W- Phe4COOH-G- Prot4NH2-SbMeW4Cl- D-dNMeA-G- Bip4CO2H-4Pal-Nva- dA-Cha44F2 | 0.396 | 2059.85119 | C104H120ClF2N19O22 | 2061.63 | 1031.8 | [M+2H]/2+ |
| **137** | TyrEtNAc-W- Phe4COOH-G- Prot4NH2-SbMeW4Cl- D-dNMeA-G- Bip4CO2H-K-L-dA- hF3CF3 | 0.09385 | 2093.87307 | C105H123ClF3N19O22 | 2095.66 | 1048.8 | [M+2H]/2+ |
| **138** | TyrEtNAc-W- Phe4COOH-G- Prot4NH2-SbMeW4Cl- D-dNMeA-G- Bip4CO2H-K-S-dA-hY | 0.2145 | 2015.82856 | C101H118ClN19O24 | 2017.58 | 1009.6 | [M+2H]/2+ |
| **139** | TyrEtNAc-W- Phe4COOH-G- Prot4NH2-SbMeW4Cl- D-dNMeA-G- Bip4CO2H-K-S-dDab- hY | 0.1325 | 2044.85511 | C102H121ClN20O24 | 2046.62 | 1024.2 | [M+2H]/2+ |
| **140** | TyrEtNAc-W- Phe4COOH-G- Prot4NH2-SbMeW4Cl- D-dNMeA-G- Bip4CO2H-W- AbuMph-dA-G | 0.515 | 2036.8289 | C103H117ClN20O23 | 2038.6 | 1019.2 | [M+2H]/2+ |
| **141** | TyrEtNAc-W- Phe4COOH-G- Prot4NH2-SbMeW4Cl- D-dNMeA-G- Bip4CO2H-W- AbuMph-dA-hY | 0.177 | 2156.88641 | C111H125ClN20O24 | 2158.75 | 1079.9 | [M+2H]/2+ |
| **142** | TyrEtNAc-W- Phe4COOH-G- Prot4NH2-SbMeW4Cl- D-dNMeA-G- Bip4CO2H-W-Dab-dA- hF | 0.159 | 2070.84963 | C107H119ClN20O22 | 2072.66 | 1037.1 | [M+2H]/2+ |
| **143** | TyrEtNAc-W- Phe4COOH-G- Prot4NH2-SbMeW4Cl- D-dNMeA-G- Bip4CO2H-W-Dab-dA- hY | 0.230 | 2086.84455 | C107H119ClN20O23 | 2088.66 | 1045.1 | [M+2H]/2+ |
| **144** | TyrEtNAc-W- Phe4COOH-G- Prot4NH2-SbMeW4Cl- D-dNMeA-G- Bip4CO2H-W-Dap-dA- hF | 0.07494 | 2056.83398 | C106H117ClN20O22 | 2058.64 | 1029.7 | [M+2H]/2+ |
| **145** | TyrEtNAc-W- Phe4COOH-G- Prot4NH2-SbMeW4Cl- D-dNMeA-G- Bip4CO2H-W-Dap-dA- hY | 0.07482 | 2072.8289 | C106H117ClN20O23 | 2074.64 | 1038.0 | [M+2H]/2+ |
| **146** | TyrEtNAc-W- Phe4COOH-G- Prot4NH2-SbMeW4Cl- D-dNMeA-G- Bip4CO2H-W- DapMe2-dA-hY | 0.114 | 2100.8602 | C108H121ClN20O23 | 2102.69 | 1051.7 | [M+2H]/2+ |
| **147** | TyrEtNAc-W- Phe4COOH-G- Prot4NH2-SbMeW4Cl- D-dNMeA-G- Bip4CO2H-W-hSOMe- dA-hY | 0.09264 | 2101.84421 | C108H120ClN19O24 | 2103.67 | 1052.5 | [M+2H]/2+ |
| **148** | TyrEtNAc-W- Phe4COOH-G- Prot4NH2-SbMeW4Cl- D-dNMeA-G- Bip4CO2H-W-L-dA- hY | 0.04831 | 2099.86495 | C109H122ClN19O23 | 2101.7 | 1051.3 | [M+2H]/2+ |
| **149** | TyrEtNAc-W- Phe4COOH-G- Prot4NH2-sbMeW4Cl- D-dNMeA-G- Bip4CO2H-W-L-dDab- hY | 0.81 | 2128.8915 | C110H125ClN20O23 | 2130.74 | 1065.9 | [M+2H]/2+ |
| **150** | TyrEtNAc-W- Phe4COOH-G- Prot4NH2-SbMeW4Cl- D-dNMeA-G- Bip4CO2H-W-S-dA-hF | 0.06331 | 2057.818 | C106H116ClN19O23 | 2059.62 | 1030.1 | [M+2H]/2+ |
| **151** | TyrEtNAc-W- Phe4COOH-G- Prot4NH2-SbMeW4Cl- D-dNMeA-G- Bip4CO2H-W-S-dA- hY | 0.07588 | 2073.81291 | C106H116ClN19O24 | 2075.62 | 1039.4 | [M+2H]/2+ |
| **152** | TyrEtNAc-W- Phe4COOH-G- Prot4NH2-SbMeW4Cl- D-dNMeA-G- Bip4CO2H-W- SerOMe-dA-hF | 0.06003 | 2071.83365 | C107H118ClN19O23 | 2073.65 | 1037.5 | [M+2H]/2+ |
| **153** | TyrEtNAc-W- Phe4COOH-G- Prot4NH2-SbMeW4Cl- D-dNMeA-G- Bip4CO2H-W- SerOMe-dA-hY | 0.088 | 2087.82856 | C107H118ClN19O24 | 2089.65 | 1045.1 | [M+2H]/2+ |
| **154** | TyrEtNAc-W- Phe4COOH-G- Prot4NH2-SbMeW4F- D-dNMeA-G- Bip4CO2H-1Nal-Dab- dA-hY | 0.196 | 2081.87885 | C109H120FN19O23 | 2083.23 | 1041.7 | [M+2H]/2+ |
| **155** | TyrEtNAc-W- Phe4COOH-G- Prot4NH2-SbMeW4F- D-dNMeA-G- Bip4CO2H-1Nal-S-dA- hY | 0.2203 | 2068.84722 | C108H117FN18O24 | 2070.19 | 1036.7 | [M+2H]/2+ |
| **156** | TyrEtNAc-W- Phe4COOH-G- Prot4NH2-SbMeW4F- D-dNMeA-G- Bip4CO2H-4Pal-L-dA- hY | 0.948 | 2045.87885 | C106H120FN19O23 | 2047.2 | 1024.2 | [M+2H]/2+ |
| **157** | TyrEtNAc-W- Phe4COOH-G- Prot4NH2-SbMeW4F- D-dNMeA-G- Bip4CO2H-4Pal-Nva- dA-hY | 0.403 | 2031.8632 | C105H118FN19O23 | 2033.17 | 1017.8 | [M+2H]/2+ |
| **158** | TyrEtNAc-W- Phe4COOH-G- Prot4NH2-SbMeW4F- D-dNMeA-G- Bip4CO2H-K-L-dA- h3Pal5CN | 0.0257 | 2035.90573 | C104H122FN21O22 | 2037.21 | 2036.4 | [M+H]+ |
| **159** | TyrEtNAc-W- Phe4COOH-G- Prot4NH2-SbMeW4F- D-dNMeA-G- Bip4CO2H-K-L-dA-hY | 0.1185 | 2025.91015 | C104H124FN19O23 | 2027.21 | 1014.9 | [M+2H]/2+ |
| **160** | TyrEtNAc-W- Phe4COOH-G- Prot4NH2-SbMeW4F- D-dNMeA-G- Bip4CO2H-K-L-dDab- hY | 0.5225 | 2054.9367 | C105H127FN20O23 | 2056.25 | 1029.4 | [M+2H]/2+ |
| **161** | TyrEtNAc-W- Phe4COOH-G- Prot4NH2-SbMeW4F- D-dNMeA-G- Bip4CO2H-K-L-dS-hY | 0.483 | 2041.90506 | C104H124FN19O24 | 2043.21 | 1022.9 | [M+2H]/2+ |
| **162** | TyrEtNAc-W- Phe4COOH-G- Prot4NH2-SbMeW4F- D-dNMeA-G- Bip4CO2H-W- AbuMph-dA-G | 0.749 | 2020.85845 | C103H117FN20O23 | 2022.15 | 1012.1 | [M+2H]/2+ |
| **163** | TyrEtNAc-W- Phe4COOH-G- Prot4NH2-SbMeW4F- D-dNMeA-G- Bip4CO2H-W-Dab-dA- hF | 0.156 | 2054.87918 | C107H119FN20O22 | 2056.21 | 1029.5 | [M+2H]/2+ |
| **164** | TyrEtNAc-W- Phe4COOH-G- Prot4NH2-SbMeW4F- D-dNMeA-G- Bip4CO2H-W-L-dA- AEF | 0.459 | 2112.92105 | C110H125FN20O23 | 2114.29 | 1058.0 | [M+2H]/2+ |
| **165** | TyrEtNAc-W- Phe4COOH-G- Prot4NH2-SbMeW4F- D-dNMeA-G- Bip4CO2H-W-L-dA- AlaPip4 | 0.781 | 2060.92613 | C107H125FN20O22 | 2062.26 | 1031.9 | [M+2H]/2+ |
| **166** | TyrEtNAc-W- Phe4COOH-G- Prot4NH2-SbMeW4F- D-dNMeA-G- Bip4CO2H-W-L-dA-hF | 0.1486 | 2067.89958 | C109H122FN19O22 | 2069.25 | 1035.7 | [M+2H]/2+ |
| **167** | TyrEtNAc-W- Phe4COOH-G- Prot4NH2-SbMeW4F- D-dNMeA-G- Bip4CO2H-W-L-dA- hY | 0.1357 | 2083.8945 | C109H122FN19O23 | 2085.25 | 1044.2 | [M+2H]/2+ |
| **168** | TyrEtNAc-W- Phe4COOH-G- Prot4NH2-SbMeW4F- D-dNMeA-G- Bip4CO2H-W-L-dDab- hY | 0.08471 | 2112.92105 | C110H125FN20O23 | 2114.29 | 1058.7 | [M+2H]/2+ |
| **169** | TyrEtNAc-W- Phe4COOH-G- Prot4NH2-SbMeW4F- D-dNMeA-G- Bip4CO2H-W-L-dK- hY | 0.1119 | 2140.95235 | C112H129FN20O23 | 2142.34 | 1072.7 | [M+2H]/2+ |
| **170** | TyrEtNAc-W- Phe4COOH-G- Prot4NH2-SbMeW4F- D-dNMeA-G- Bip4CO2H-W-L-dOrn- hY | 0.09664 | 2126.9367 | C111H127FN20O23 | 2128.31 | 1065.8 | [M+2H]/2+ |
| **171** | TyrEtNAc-W- Phe4COOII-G- Prot4NH2-SbMeW4F- | 0.1453 | 2099.88941 | C109H122FN19O24 | 2101.25 | 1052.2 | [M+2H]/2+ |
| | D-dNMeA-G- Bip4CO2H-W-L-dS-hY | | | | | | |
| **172** | TyrEtNAc-W- Phe4COOH-G- Prot4NH2-SbMeW4F- D-dNMeA-G- Bip4CO2H-W-S-dA- hY | 0.246 | 2057.84246 | C106H116FN19O24 | 2059.17 | 1031.2 | [M+2H]/2+ |
| **173** | TyrEtNAc-W- Phe4COOH-G- Prot4NH2-SbMeW4F- D-dNMeA-G- Bip4CO2H-W-S-dA- TyrEtNAc | 0.9592 | 2128.87958 | CI09H121FN20O25 | 2130.24 | 1066.3 | [M+2H]/2+ |
| **174** | 3AzaPhe4AcPip-W- Phe4COOH-G- Prot4NH2-SbMe1Nal- D-dNMeA-G- Bip4CO2H-Dab-L-dA- hCha | 0.015 | 2006.9719 | C105H130N20O21 | 2008.28 | 2008.99 | [M+H]+ |
| **175** | 3AzaPhe4AcPip-W- Phe4COOH-G- Prot4NH2-SbMe1Nal- D-dNMeA-G- Bip4CO2H-Dap-L-dA- hCha | 0.016 | 1992.9563 | C104H128N20O21 | 1994.25 | 1994.97 | [M+H]+ |
| **176** | 3AzaPhe4AcPip-W- Phe4COOH-G- Prot4NH2-SbMe1Nal- D-dNMeA-G- Bip4CO2H-hQ-L-dA- h3Pal5CN | 0.017 | 2068.9261 | C107H124N22O22 | 2070.26 | 2070.94 | [M+H]+ |
| **177** | 3AzaPhe4AcPip-W- Phe4COOH-G- Prot4NH2-SbMe1Nal- D-dNMeA-G- Bip4CO2H-K-L-dA- hCha | 0.013 | 2035.0032 | C107H134N20O21 | 2036.33 | 2037.01 | [M+H]+ |
| **178** | 3AzaPhe4AcPip-W- Phe4COOH-G- Prot4NH2-SbMe1Nal- D-dNMeA-G- Bip4CO2H-K-L-dL- h3Pal5CN | 0.019 | 2096.9937 | C110H132N22O21 | 2098.36 | 2099.02 | [M+H]+ |
| **179** | 3AzaPhe4AcPip-W- Phe4COOH-G- Prot4NH2-SbMe1Nal- D-dNMeA-G- Bip4CO2H-K-L-dNle- h3Pal5CN | 0.017 | 2096.9937 | C110H132N22O21 | 2098.36 | 2099.03 | [M+H]+ |
| **180** | 3AzaPhe4AcPip-W- Phe4COOH-G- Prot4NH2-SbMe1Nal- D-dNMeA-G- Bip4CO2H-K-L-dNva- h3Pal5CN | 0.021 | 2082.9781 | C109H130N22O21 | 2084.33 | 2085.01 | [M+H]+ |
| **181** | 3AzaPhe4AcPip-W- Phe4COOH-G- Prot4NH2-SbMe1Nal- D-dNMeA-G- Bip4CO2H-K-Tba-dA- h3Pal5CN | 0.015 | 2068.9624 | C108H128N22O21 | 2070.31 | 2070.98 | [M+H]+ |
| **182** | 3AzaPhe4AcPip-W- Phe4COOH-G- Prot4NH2-SbMe1Nal- D-dNMeA-G- Bip4CO2H-Orn-L-dA- hCha | 0.013 | 2020.9876 | C106H132N20O21 | 2022.30 | 2023.00 | [M+H]+ |
| **183** | 3AzaPhe4AcPip-W- Phe4COOH-G- Prot4NH2-SbMe1Nal- D-dNMeA-G- | 0.013 | 2054.9104 | C106H122N22O22 | 2056.24 | 2056.93 | [M+H]+ |
| | Bip4CO2H-Q-L-dA- h3Pal5CN | | | | | | |
| **184** | Bip4CO2H-Trp6Cl- Phe4COOH-G- Prot4NH2-SbMeW4F- D-dNMeA-G- Bip4CO2H-K-L-dA-hY | 0.018 | 2078.8446 | C107H120ClFN18O23 | 2080.66 | 2079.70 | [M+H]+ |
| **185** | Bip4CO2H-Trp7F- Phe4COOH-G- Prot4NH2-SbMeW4F- D-dNMeA-G- Bip4CO2H-K-L-dA-hY | 0.024 | 2062.8742 | C107H120F2N18O23 | 2064.20 | 1032.76 | [M+2H]/2+ |
| **186** | Bip4CO2H-Trp7Me- Phe4COOH-G- Prot4NH2-SbMeW4F- D-dNMeA-G- Bip4CO2H-K-L-dA-hY | 0.038 | 2058.8993 | C108H123FN18O23 | 2060.24 | 1030.33 | [M+2H]/2+ |
| **187** | Bip4CO2H-W- Phe4COOH-G- Prot4NH2-SbMeW4F- D-dNMeA-G- Bip4CO2H-K-L- dLysAc-hY | 0.009 | 2143.9520 | C112H130FN19O24 | 2145.34 | 2145.97 | [M+H]+ |
| **188** | F4bcpA-W- Phe4COOH-G- Prot4NH2-SbMe1Nal- D-dNMeA-G- Bip4CO2H-1Inda-G- dDab-hCha | 0.016 | 2049.9090 | C109H123N19O22 | 2051.26 | 2052.87 | [M+H]+ |
| **189** | F4bcpA-W- Phe4COOH-G- Prot4NH2-SbMe1Nal- D-dNMeA-G- Bip4CO2H-Cha-G- dDab-hCha | 0.019 | 2015.9498 | C108H129N17O22 | 2017.28 | 2017.49 | [M+H]+ |
| **190** | F4bcpA-W- Phe4COOH-G- Prot4NH2-SbMe1Nal- D-dNMeA-G- Bip4CO2H-Cha-G- dDab-hY | 0.018 | 2025.8978 | C108H123N17O23 | 2027.23 | 2028.19 | [M+H]+ |
| **191** | F4pcCCA-W- Phe4COOH-G- Prot4NH2-SbMe1Nal- D-dNMeA-G- Bip4CO2H-4Pal-G- dDab-hCha | 0.019 | 2026.9294 | C108H126N18O22 | 2028.26 | 2029.36 | [M+H]+ |
| **192** | F4ptCCA-Trp6F- Phe4COOH-G- Prot4NH2-SbMe1Nal- D-dNMeA-G- Bip4CO2H-4Pal-G- dDab-hCha | 0.015 | 2044.9200 | C108H125FN18O22 | 2046.25 | 2046.42 | [M+H]+ |
| **193** | F4ptCCA-Trp6F- Phe4COOH-G- Prot4NH2-SbMe1Nal- D-dNMeA-G- Bip4CO2H-4Pal-S- dDab-hCha | 0.012 | 2074.9306 | C109H127FN18O23 | 2076.28 | 2076.24 | [M+H]+ |
| **194** | F4ptCCA-Trp6F- Phe4COOH-G- Prot4NH2-SbMe1Nal- D-dNMeA-G- Bip4CO2H-4Pal-S- dDabMe3-hCha | 0.015 | 2117.9853 | C112H134FN18O23 | 2119.37 | 2119.29 | [M+H]+ |
| **195** | F4ptCCA-W- Phe4COOH-G- Prot4NH2-SbMe1Nal- D-dNMeA-G- Bip4CO2H-4Pal-G- dDab-aMeNle | 0.022 | 1986.8981 | C105H122N18O22 | 1988.20 | 1988.96 | [M+H]+ |
| **196** | F4ptCCA-W- Phe4COOH-G- Prot4NH2-SbMe1Nal- D-dNMeA-G- Bip4CO2H-4Pal-G- dDab-hCha | 0.030 | 2026.9294 | C108H126N18O22 | 2028.26 | 2029.60 | [M+H]+ |
| **197** | F4ptCCA-W- Phe4COOH-G- Prot4NH2-SbMe1Nal- D-dNMeA-G- Bip4CO2H-4Pal-G- dDab-Nle | 0.024 | 1972.8825 | C104H120N18O22 | 1974.17 | 1974.94 | [M+H]+ |
| **198** | Phe4AcPip-W- Phe4COOH-G- Prot4NH2-SbMe1Nal- D-dNMeA-G- Bip4CO2H-1Indolin-G- dDab-hY | 0.021 | 2076.9199 | C110H124N20O22 | 2078.28 | 1040.18 | [M+2H]/2+ |
| **199** | Phe4AcPip-W- Phe4COOH-G- Prot4NH2-SbMe1Nal- D-dNMeA-G- Bip4CO2H-4Pal-G- daMeDab-hCha | 0.024 | 2040.9563 | C108H128N20O21 | 2042.29 | 2043.02 | [M+H]+ |
| **200** | Phe4AcPip-W- Phe4COOH-G- Prot4NH2-SbMe1Nal- D-dNMeA-G- Bip4CO2H-4Pal-G- dDab-aMeNle | 0.034 | 1986.9093 | C104H122N20O21 | 1988.20 | 1988.97 | [M+H]+ |
| **201** | Phe4AcPip-W- Phe4COOH-G- Prot4NH2-SbMe1Nal- D-dNMeA-G- Bip4CO2H-4Pal-G- dDab-hCha | 0.030 | 2026.9406 | C107H126N20O21 | 2028.27 | 2028.77 | [M+H]+ |
| **202** | Phe4AcPip-W- Phe4COOH-G- Prot4NH2-SbMe1Nal- D-dNMeA-G- Bip4CO2H-Cha-G- dDab-hY | 0.029 | 2041.9403 | C108H127N19O22 | 2043.28 | 2044.64 | [M+H]+ |
| **203** | Phe4AcPip-W- Phe4COOH-G- Prot4NH2-SbMe1Nal- D-dNMeA-G- Bip4CO2H-Dab-L-dA- hCha | 0.012 | 2005.9767 | C106H131N19O21 | 2007.29 | 2008.04 | [M+H]+ |
| **204** | Phe4AcPip-W- Phe4COOH-G- Prot4NH2-SbMe1Nal- D-dNMeA-G- Bip4CO2H-DapAc-K- dA-h3Pal5CN | 0.009 | 2068.9261 | C107H124N22O22 | 2070.26 | 2071.00 | [M+H]+ |
| **205** | Phe4AcPip-W- Phe4COOH-G- Prot4NH2-SbMe1Nal- D-dNMeA-G- Bip4CO2H-DapAc-L- dDap-h3Pal5CN | 0.010 | 2068.9261 | C107H124N22O22 | 2070.26 | 2070.96 | [M+H]+ |
| **206** | Phe4AcPip-W- Phe4COOH-G- Prot4NH2-SbMe1Nal- D-dNMeA-G- Bip4CO2H-Dap-L-dA- hCha | 0.015 | 1991.9610 | C105H129N19O21 | 1993.26 | 1994.03 | [M+H]+ |
| **207** | Phe4AcPip-W- Phe4COOH-G- Prot4NH2-SbMe1Nal- D-dNMeA-G- Bip4CO2H-K-L-dA- hCha | 0.011 | 2034.0080 | C108H135N19O21 | 2035.34 | 2036.08 | [M+H]+ |
| **208** | Phe4AcPip-W- Phe4COOH-G- Prot4NH2-SbMe1Nal- D-dNMeA-G- Bip4CO2H-LysAc-L- dA-h3Pal5CN | 0.018 | 2095.9621 | C110H129N21O22 | 2097.33 | 2097.97 | [M+H]+ |
| **209** | Phe4AcPip-W- Phe4COOH-G- Prot4NH2-SbMe1Nal- D-dNMeA-G- Bip4CO2H-Orn-L-dA- hCha | 0.009 | 2019.9923 | C107H133N19O21 | 2021.32 | 2022.07 | [M+H]+ |
| **210** | TyrEtNAc-Trp6Cl- Phe4COOH-G- Prot4NH2-SbMe1Nal- D-dNMeA-G- Bip4CO2H-Trp7az-S- dDab-hCha | 0.020 | 2104.8915 | C108H125C1N20O23 | 2106.72 | 1053.44 | [M+2H]/2+ |
| **211** | TyrEtNAc-Trp6F- Phe4COOH-G- Prot4NH2-SbMe1Nal- D-dNMeA-G- Bip4CO2H-1Indolin-G- dDab-hY | 0.027 | 2069.8789 | C108H120FN19O23 | 2071.22 | 2071.32 | [M+H]+ |
| **212** | TyrEtNAc-Trp6F- Phe4COOH-G- Prot4NH2-SbMe1Nal- D-dNMeA-G- Bip4CO2H-Trp7az-S- dDab-hCha | 0.025 | 2088.9211 | C108H125FN20O23 | 2090.27 | 1045.22 | [M+2H]/2+ |
| **213** | TyrEtNAc-Trp6F- Phe4COOH-G- Prot4NH2-SbMe1Nal- D-dNMeA-G- Bip4CO2H-Trp7az-S- dDabMe3-hCha | 0.018 | 2131.9758 | C111H132FN20O23 | 2133.35 | 2133.32 | [M+H]+ |
| **214** | TyrEtNAc-W- Phe4COOH-G- Prot4NH2-SbMe1Nal- D-dNMeA-G- Bip4CO2H-Trp7az-G- dDab-hCha | 0.020 | 2040.9199 | C107H124N20O22 | 2042.25 | 1022.67 | [M+2H]/2+ |
| **215** | TyrEtNAc-W- Phe4COOH-G- Prot4NH2-SbMe1Nal- D-dNMeA-G- Bip4CO2H-Trp7az-G- dDab-hY | 0.024 | 2050.8679 | C107H118N20O23 | 2052.20 | 1026.43 | [M+2H]/2+ |

## Claims

1. A compound of Formula (I) 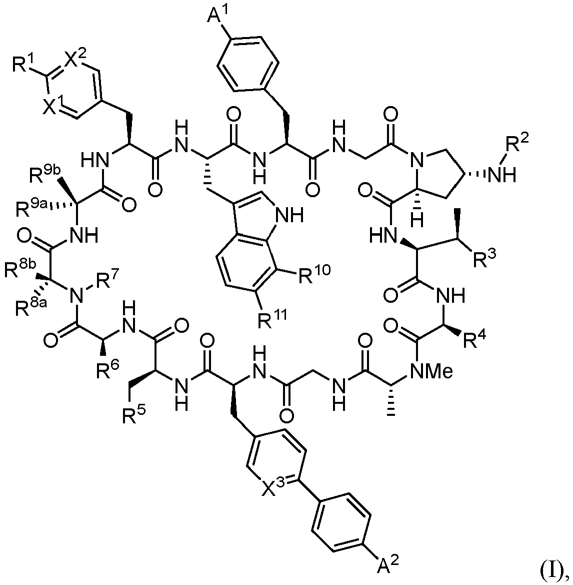 wherein:
R¹ is CH₃C(O)NH-CH₂CH₂-O- or C¹;
C¹ is:
(i) a 5- to 6-membered monocyclic aryl or heteroaryl, wherein said heteroaryl contains 1 to 2 heteroatoms selected from the group consisting of N, O, and S; or
(ii) a 5- to 6-membered mono- or bicyclic, saturated cycloalkyl or heterocycloalkyl containing 1 to 2 heteroatoms selected from the group consisting of N, O, and S; or
(iii) a 5- to 6-membered mono- or bicyclic cycloalkyl;
wherein C¹ is unsubstituted or substituted by 1 to 3 R^{C1} substituents independently selected from the group consisting of halo, C₁-C₃ alkyl, C₁-C₃ fluoroalkyl, carboxy, C₁-C₃ alkoxy, and C₂-C₃ acyl;
R² is H, C₁-C₃ alkyl, benzyl, or phenyl-CH₂CH₂-;
R³ is a 9- to 10-membered bicyclic aryl or heteroaryl, wherein said heteroaryl contains 1 to 2 heteroatoms selected from the group consisting of N, O, and S;
wherein R³ is unsubstituted or substituted by 1 to 3 R^{3a} substituents independently selected from the group consisting of halo, C₁-C₃ alkyl, C₁-C₃ fluoroalkyl, hydroxy and C1-C3 alkoxy;
R⁴ is C₁-C₃ alkyl, HO₂C-(CH₂)ₘ-, H₂NC(O)-(CH₂)ₘ-, (CH₃)₂NC(O)-(CH₂)ₘ-, or tetrazolyl-(CH₂)ₘ-;
R⁵ is amino, H₂N(CH₂)ₙ-, H₂NC(O)-(CH₂)ₙ-, CH₃C(O)NH-, CH₃C(O)NH(CH₂)ₙ-, C⁵, or C⁵-CH₂-,
C⁵ is:
(ii) a 5- to 6-membered monocyclic aryl or heteroaryl, wherein said heteroaryl contains 1 to 2 heteroatoms selected from the group consisting of N, O, and S;
(ii) a 9- to 10-membered bicyclic aryl or heteroaryl, wherein said bicyclic heteroaryl contains 1 to 3 heteroatoms selected from the group consisting of N, O, and S;
(iii) a 5- to 6-memberered monocyclic or 9-to 10-membered heterocycloalkyl, wherein said heterocycloalkyl is saturated or partially unsaturated, and contains 1 to 2 heteroatoms selected from the group consisting of N, O, and S;
(iv) a 5- to 6-membered monocyclic cycloalkyl;
(v) 2,3-dihydroindolyl;
wherein C⁵ is unsubstituted or substituted by 1 to 3 R^{C5} substituents independently selected from the group consisting of halo, amino, hydroxy, C₁-C₃ alkyl, C₁-C₃ fluoroalkyl, C₁-C₃ alkoxy, H₂N-(CH₂)k-, H₂NC(O)-(CH₂)k-, H₂C=CH-CH₂O-, and phenyl;
R⁶ is H, C₁-C₅ alkyl, H₂N(CH₂)ₚ-, HOCH₂-, (CH₃)₂NCH₂-, H₃CO-(CH₂)_{q}-, or C⁶-CH₂-;
C⁶ is 5- or 6-membered monocyclic, saturated heterocycloalkyl containing 1 to 2 heteroatoms selected from the group consisting of N, O, and S; and wherein C⁶ is unsubstituted or substituted by 1 to 3 R^{C6} substituents independently selected from the group consisting of halo, C₁-C₃ alkyl, C₁-C₃ fluoroalkyl, hydroxy and C₁-C₃ alkoxy;
R⁷ is H or C₁-C₃ alkyl;
R^{8a} is H, C₁-C₅ alkyl, HOCH₂-, H₂N(CH2)ᵣ-, (CH₃)₃N⁺(CH2)ᵣ-,or CH₃C(O)NH(CH2)ᵣ-;
R8b is H or C₁-C₃ alkyl;
R9a is H or C₁-C₃ alkyl;
R9b is H, C₁-C₅ alkyl, C⁹-CH₂-, or C⁹-CH₂CH₂-;
C⁹ is:
(i) a 5- to 6-membered monocyclic aryl or heteroaryl, wherein said heteroaryl contains 1 to 2 heteroatoms selected from the group consisting of N, O, and S; or
(ii) a 5- to 6-membered monocyclic, saturated cycloalkyl or heterocycloalkyl, wherein the heterocycloalkyl contains 1 to 2 heteroatoms selected from the group consisting of N, O, and S;
wherein C⁹ is unsubstituted or substituted by 1 to 3 R^{C9} substituents independently selected from the group consisting of halo, amino, hydroxy, cyano, C₁-C₃ alkyl, C1-C3 fluoroalkyl, C₁-C₃ alkoxy, H₂N-(CH₂)k-, H₂NC(O)-(CH₂)k-, H₂NCH₂CH₂O-, CH₃C(O)NH-CH₂CH₂O-, and morpholinyl-CH₂CH₂O-;
R¹⁰ is H, halo, or C₁-C₃ alkyl;
R¹¹ is H, halo, or C₁-C₃ alkyl;
each occurrence of subscript k is independently 1 or 2;
subscript m is 1 or 2;
subscript n is 1, 2, 3, or 4;
subscript p is 1, 2, 3, or 4;
subscript q is 1 or 2;
subscript r is 1, 2, 3, or 4;
X¹, X², and X³ are independently C(H) or N; and
A¹ and A² are independently selected from the group consisting of HO2C-, H₂NC(O)-, CH₃C(O)N(H)-, H₂NS(O)2-, CH₃S(O)₂N(H)-, tetrazolyl, and 5-oxo oxadiazolyl; or
a pharmaceutically acceptable salt thereof.

2. The compound of claim 1 or a pharmaceutically acceptable salt thereof, wherein:
R⁵ is amino, H₂N(CH₂)ₙ-, H₂NC(O)-(CH₂)ₙ-, C⁵, or C⁵-CH₂-,
C⁵ is:
(i) a 5- to 6-membered monocyclic aryl or heteroaryl, wherein said heteroaryl contains 1 to 2 heteroatoms selected from the group consisting of N, O, and S:
(ii) a 9- to 10-membered bicyclic aryl or heteroaryl, wherein said bicyclic heteroaryl contains 1 to 3 heteroatoms selected from the group consisting of N, O, and S;
(iii) a 5- to 6-memberered monocyclic or 9-to 10-membered heterocycloalkyl, wherein said heterocycloalkyl is saturated or partially unsaturated, and contains 1 to 2 heteroatoms selected from the group consisting of N, O, and S; or
(iv) 2,3-dihydroindolyl;
wherein C⁵ is unsubstituted or substituted by 1 to 3 R^{C5} substituents independently selected from the group consisting of halo, amino, hydroxy, C₁-C₃ alkyl, C₁-C₃ fluoroalkyl, C₁-C₃ alkoxy, H₂N-(CH₂)k-, H₂NC(O)-(CH₂)k-, H₂C=CH-CH₂O-, and phenyl;
R⁶ is H, C2-CS alkyl, H₂N(CH₂)ₚ-, HOCH₂-, (CH₃)₂NCH₂-, H₃CO-(CH₂)_{q}-, or C⁶-CH₂-;
R^{8a} is H, C₁-C₃ alkyl, HOCH₂-, or H₂N(CH₂)ᵣ-;
R9a is H;
R9b is H, C₁-C₃ alkyl, C⁹-CH₂-, or C⁹-CH₂CH₂-;
R10 is H;
R¹¹ is H;
subscript p is 1, 2, or 3; and
subscript r is 2, 3, or 4.

3. The compound of claim 1, or a pharmaceutically acceptable salt thereof, wherein: C¹ is phenyl, pyrimidyl, or piperazinyl, wherein C¹ is unsubstituted or substituted by 1 to 2 R^{C1} substituents;
R³ is naphthyl or indolyl, wherein R³ is unsubstituted or substituted by 1 to 2 R^{3a} substituents;
C⁵ is phenyl, pyridyl, pyrimidyl, naphthyl, indolyl, 7-azaindolyl, indazolyl, 2,3-dihydroindolyl, piperidinyl, tetrahydropyranyl, or cyclohexyl, wherein C⁵ is unsubstituted or substituted by 1 to 2 RCS;
C⁶ is tetrahydropyranyl or morpholinyl; wherein C⁶ is unsubstituted or substituted by 1 to 2 RC6; and
R9a is H or methyl;
C⁹ is phenyl, pyridyl, cyclohexyl, morpholinyl, or piperidinyl, wherein C⁹ is unsubstituted or substituted by 1 to 2 R^{C9}.

4. The compound of any of claims 1 to 3, or a pharmaceutically acceptable salt thereof, wherein X¹ and X² are C(H); and R¹ is phenyl substituted by carboxy or R¹ is CH₃C(O)NH-CH₂CH₂-O-.

5. The compound of any of claims 1 to 4, or a pharmaceutically acceptable salt thereof, wherein R² is H.

6. The compound of any of claims 1 to 5, or a pharmaceutically acceptable salt thereof, wherein R³ is indolyl substituted by one halo or R³ is naphthyl.

7. The compound of any of claims 1 to 6, or a pharmaceutically acceptable salt thereof, wherein R⁴ is HO₂C-(CH₂)ₘ-.

8. The compound of any of claims 1 to 7, or a pharmaceutically acceptable salt thereof, wherein X³ is C(H).

9. The compound of any of claims 1 to 8, or a pharmaceutically acceptable salt thereof, wherein R⁵ is H₂N(CH₂)ₙ-, indole, 7-azaindole, naphthyl or pyridyl.

10. The compound of any of claims 1 to 9, or a pharmaceutically acceptable salt thereof, wherein R⁶ is H, HOCH₂-, C₂-C₅ alkyl or H₂N(CH₂)ₚ-.

11. The compound of any of claims 1 to 10, or a pharmaceutically acceptable salt thereof, wherein R⁷ is H.

12. The compound of any of claims 1 to 11, or a pharmaceutically acceptable salt thereof, wherein:
R^{8a} is methyl or H₂NCH₂CH₂-; and R^{8b} is H.

13. The compound of any of claims 1 to 12, or a pharmaceutically acceptable salt thereof, wherein R^{9b} is 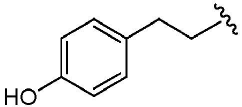 or

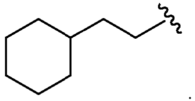

14. The compound of any of claims 1 to 13, or a pharmaceutically acceptable salt thereof, wherein
R¹⁰ is H; and R¹¹ is H, F or Cl.

15. The compound of any of claims 1 to 14, or a pharmaceutically acceptable salt thereof, wherein A¹ and A² are both HO2C-.

16. The compound of claim 1 or a pharmaceutically acceptable salt thereof, wherein:
R¹ is 4-CH₃C(O)-piperazin-1-yl, CH₃C(O)NH-CH₂CH₂-O-, 5-CO₂H-pyrimidin-2-yl, or 4-CO₂H-phenyl;
R² is H, ethyl, benzyl, or phenyl-CH₂CH₂-;
R³ is naphth-1-yl, 4-fluoroindol-3-yl, or 4-chloroindol-3-yl;
R⁴ is methyl, HO₂C-(CH₂)ₘ-, or H₂NC(O)-(CH₂)ₘ-;
R⁵ is amino, H₂N(CH₂)ₙ-, naphth-1-yl, indol-3-yl, 7-aza-indol-3-yl, indazol-1-yl, 2,3-dihydroindol-1-yl, pyrid-3-yl, pyrid-4-yl, piperidin-4-yl, 3-aminomethylphenyl, 4-aminomethylphenyl, 3-aminophenyl, 4-aminophenyl, phenyl, pyrid-4-yl-CH₂-, pyrimidin-5-yl, tetrahydropyran-4-yl-, H₂NC(O)-(CH₂)₂-, 3-biphenyl, 3-CH₂=CH-CH₂O-phenyl, CH₃C(O)NH-, CH₃C(O)NH(CH₂)₃-, or cyclohexyl;
R⁶ is H, (CH₃)₂CHCH₂-, (CH₃)₃CCH₂-, H₂N(CH₂)ₚ-, HOCH₂-, H3CCH2CH2-, morpholin-4-yl-CH₂-, tetrahydropyran-4-yl-CH₂-, (CH₃)₂NCH₂-, or H₃CO-(CH₂)_{q}-;
R⁷ is H or methyl;
R^{8a} is H, methyl, HOCH₂-, H₂N(CH2)ᵣ-, (CH₃)₃NCH₂CH₂-, or CH3C(O)NH(CH2)4-;
R8b is H or methyl;
R9a is H or methyl;
R^{9b} is H, methyl, H₃CCH₂CH₂CH₂-, 4-HO-phenyl-CH₂CH₂-, phenyl-CH₂CH₂-, cyclohexyl-CH₂CH₂-, 5-NC-pyrid-3-yl-CH₂CH₂-, 4-F₃C-phenyl-CH₂-, 3-F₃C-phenyl-CH₂-, 2-F₃C-phenyl-CH₂-, morpholin-4-yl-CH₂CH₂O-phenyl-CH₂-, 4-aminophenyl-CH₂-, 4,4-difluorocyclohexyl-CH₂-, 4-H₂NCH₂CH₂O-phenyl-CH₂-, 4-H₂NCH₂CH₂O-pyrid-3-yl-CH₂-, piperidin-4-yl-CH₂-, or 4-CH₃C(O)NH-CH₂CH₂O-phenyl-CH₂-;
R¹⁰ is H, fluoro, or methyl;
R¹¹ is H, fluoro, or chloro;
A¹ and A² are both HO2C-; and
X¹, X², and X³ are C(H).

17. The compound of claim 1 selected from the group consisting of (SEQ ID NOS 78, 71-72, 67, 65, 70, 69, 68, 66, 64, 77, 79, 209, 193, 215, 212, and 210 respectively, in order of appearance): 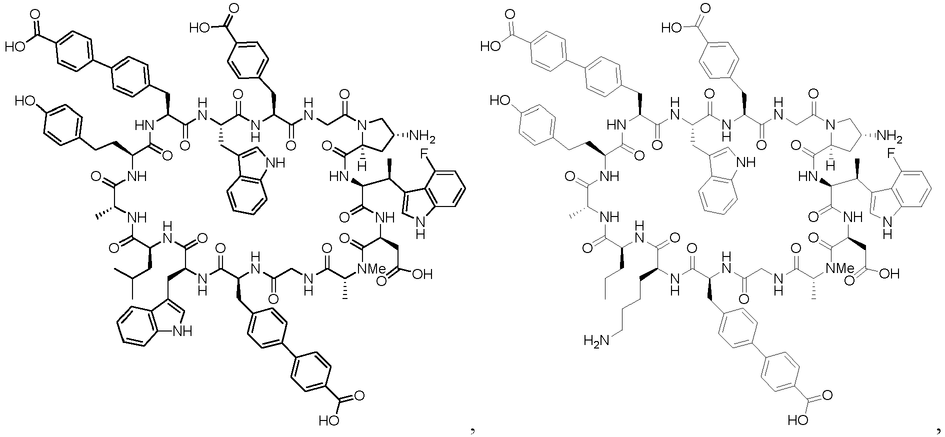 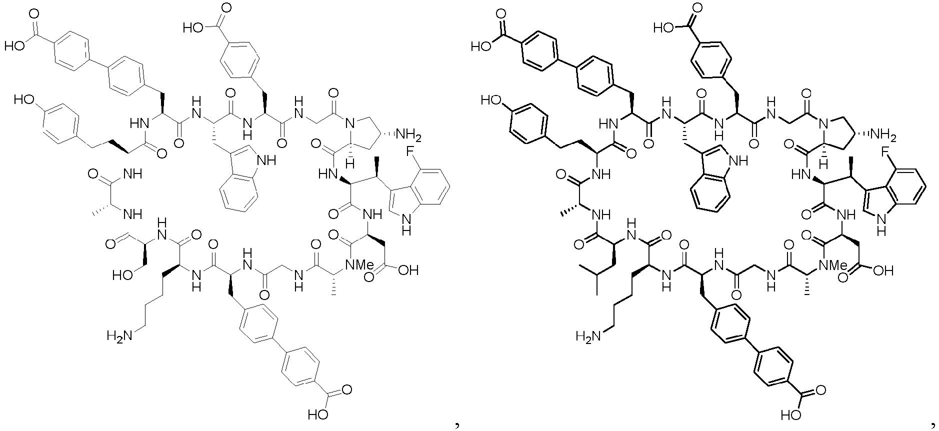 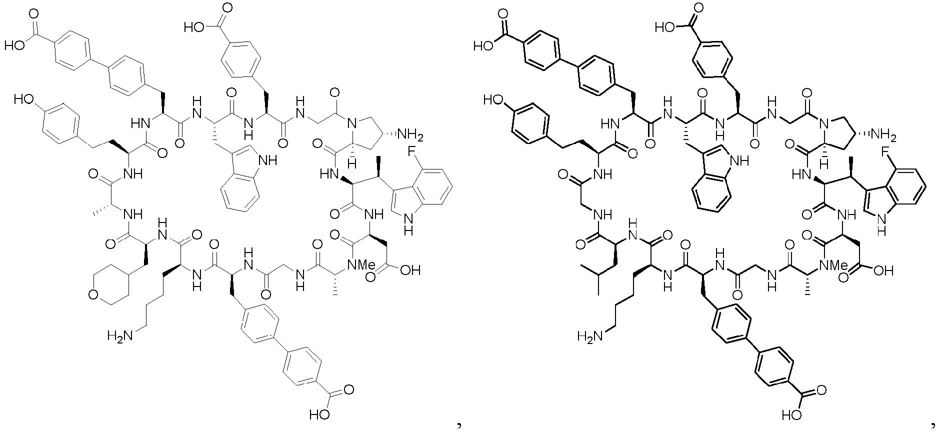 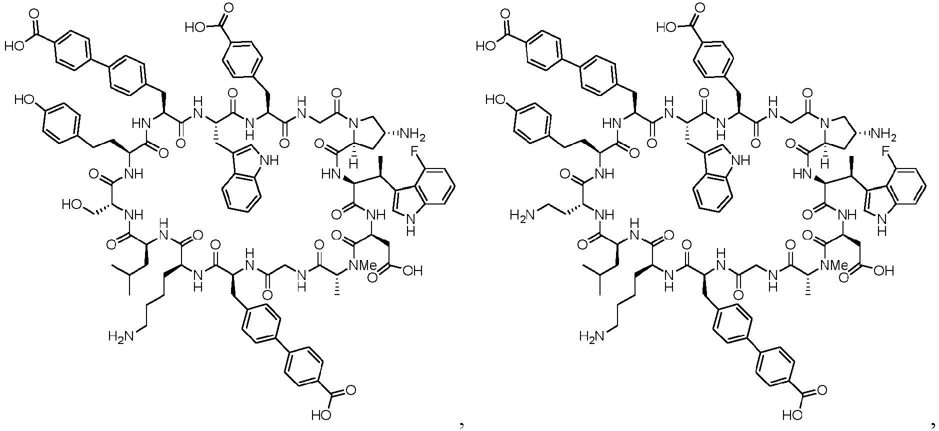 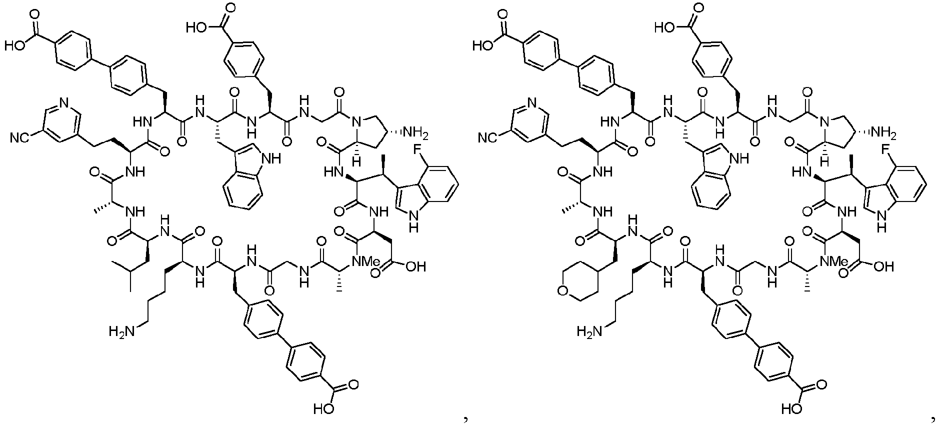 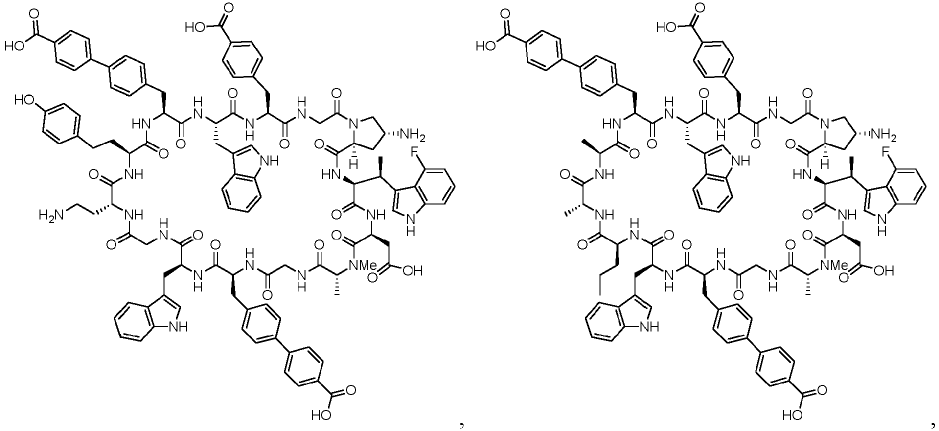 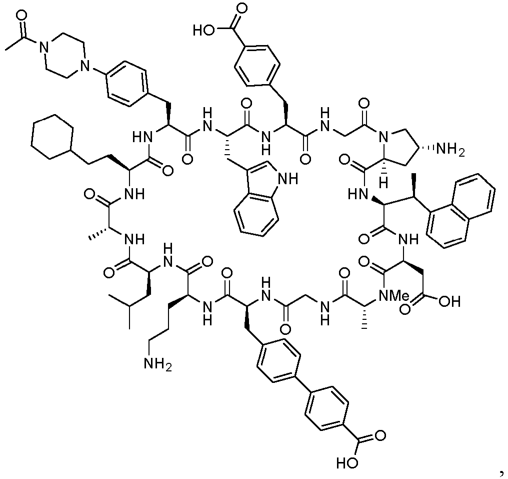 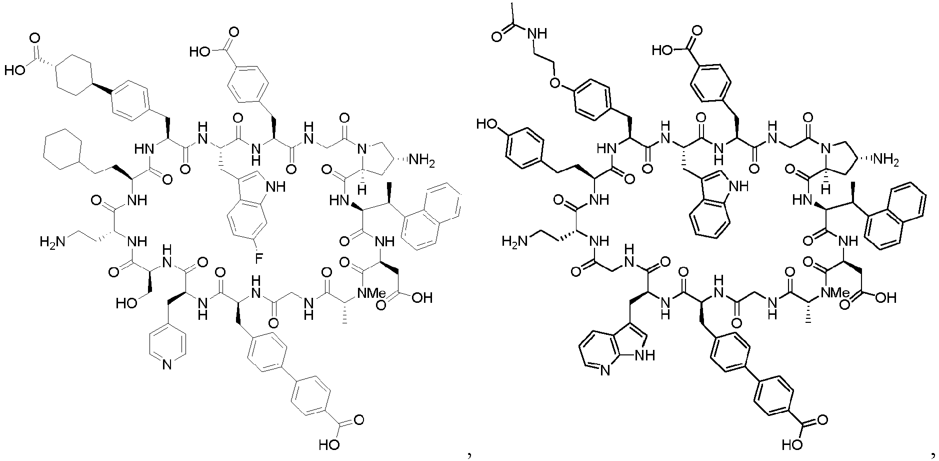 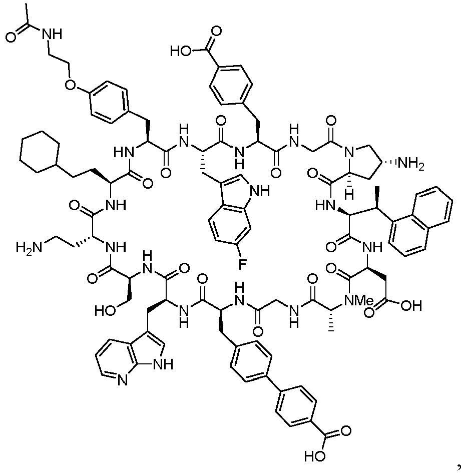 and 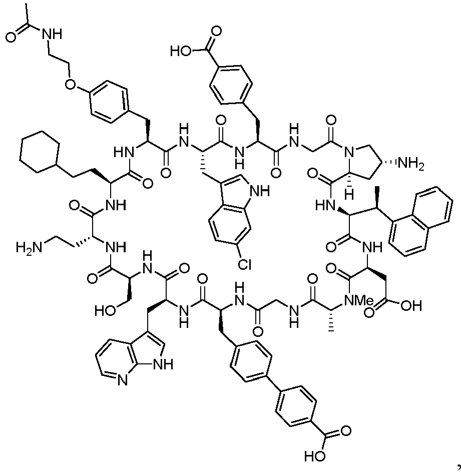 or a pharmaceutically acceptable salt thereof.

18. The compound of claim 1, which is: 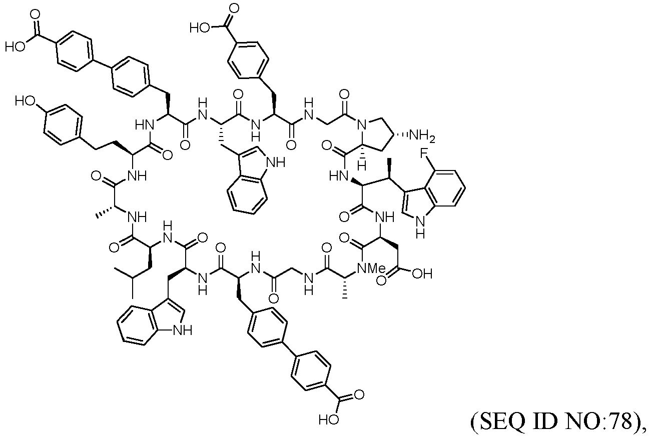 or a pharmaceutically acceptable salt thereof.

19. The compound of claim 1, which is: 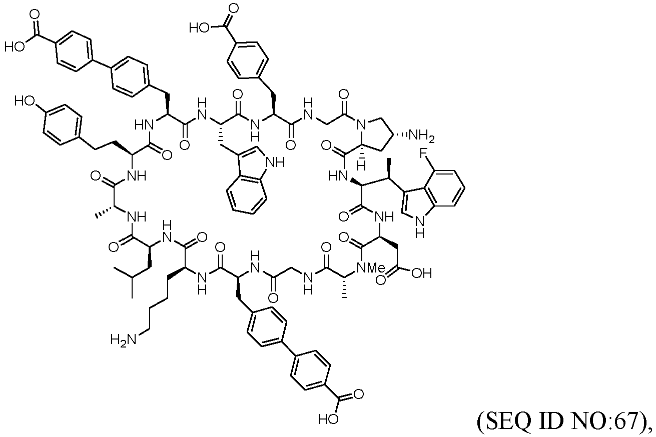 or a pharmaceutically acceptable salt thereof.

20. The compound of claim 1, which is: 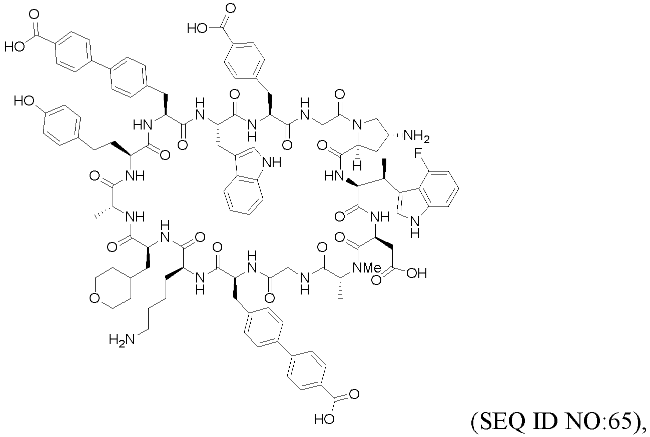 or a pharmaceutically acceptable salt thereof.

21. The compound of claim 1, which is: 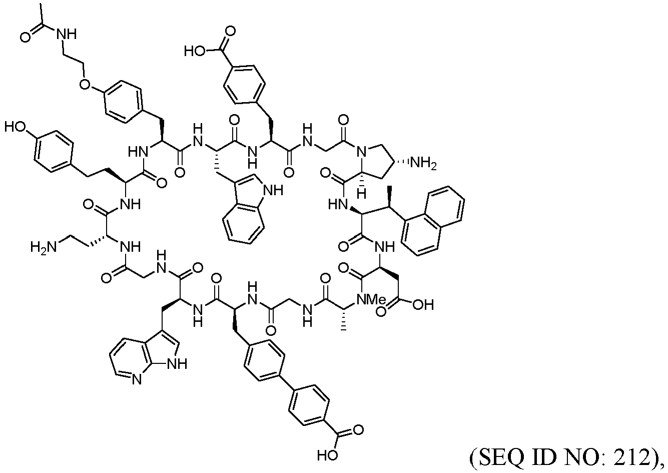 or a pharmaceutically acceptable salt thereof.

22. The compound of claim 1, which is:

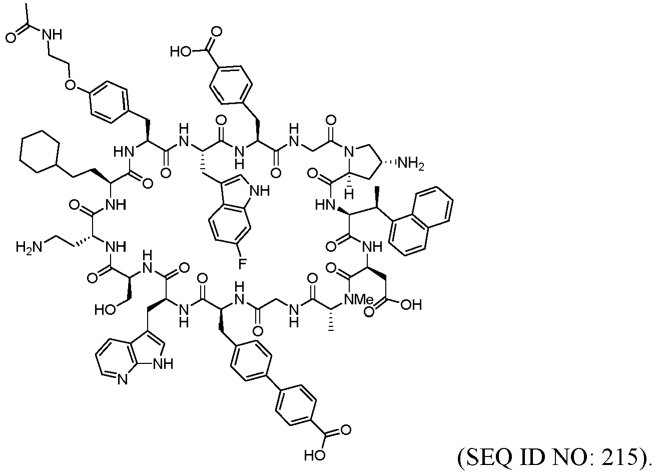

23. A pharmaceutical composition comprising the compound of any one of claims 1 to 22, or a pharmaceutically acceptable salt thereof, and a pharmaceutically acceptable carrier.

24. A compound of any one of claims 1 to 22, or a pharmaceutically acceptable salt thereof, for use in therapy.

25. A compound of any one of claims 1 to 22, or a pharmaceutically acceptable salt thereof, for use in treating atherosclerosis, or vascular inflammation.

## Patentansprüche

1. Eine Verbindung der Formel (I) 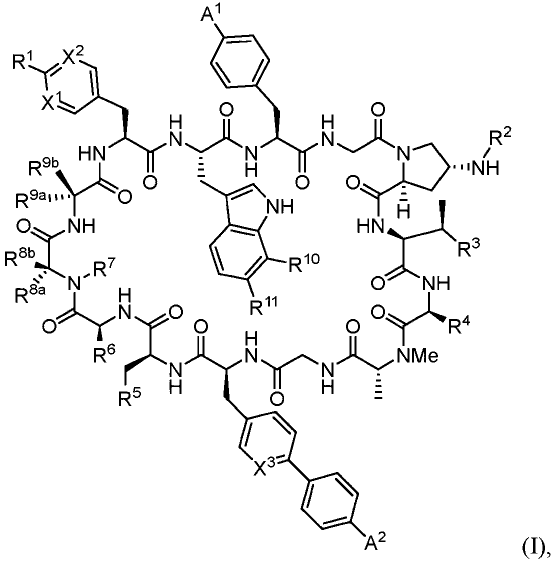 wobei:
R¹ CH₃C(O)NH-CH₂CH₂-O- oder C¹ ist,
C¹ ist:
(i) ein 5- bis 6-gliedriges monocyclisches Aryl oder Heteroaryl, wobei das Heteroaryl 1 bis 2 Heteroatome enthält, ausgewählt aus der Gruppe bestehend aus N, O und S, oder
(ii) ein 5- bis 6-gliedriges mono- oder bicyclisches gesättigtes Cycloalkyl oder Heterocycloalkyl, das 1 bis 2 Heteroatome enthält, ausgewählt aus der Gruppe bestehend aus N, O und S, oder
(iii) ein 5- bis 6-gliedriges mono- oder bicyclisches Cycloalkyl,
wobei C¹ unsubstituiert oder substituiert ist durch 1 bis 3 R^{C1}-Substituenten, unabhängig ausgewählt aus der Gruppe bestehend aus Halogen, C₁-C₃-Alkyl, C₁-C₃-Fluoralkyl, Carboxy, C₁-C₃-Alkoxy und C₂-C₃-Acyl,
R² H, C₁-C₃-Alkyl, Benzyl oder Phenyl-CH₂CH₂- ist,
R³ ein 9- bis 10-gliedriges bicyclisches Aryl oder Heteroaryl ist, wobei das Heteroaryl 1 bis 2 Heteroatome enthält, ausgewählt aus der Gruppe bestehend aus N, O und S,
wobei R³ unsubstituiert oder substituiert ist durch 1 bis 3 R^{3a}-Substituenten, unabhängig ausgewählt aus der Gruppe bestehend aus Halogen, C₁-C₃-Alkyl, C₁-C₃-Fluoralkyl, Hydroxy und C₁-C₃-Alkoxy,
R⁴ C₁-C₃-Alkyl, HO₂C-(CH₂)ₘ-, H₂NC(O)-(CH₂)ₘ-, (CH₃)₂NC(O)-(CH₂)ₘ- oder Tetrazolyl-(CH₂)ₘ- ist,
R⁵ Amino, H₂N(CH₂)ₙ-, H₂NC(O)-(CH₂)ₙ-, CH₃C(O)NH-, CH₃C(O)NH(CH₂)ₙ-, C⁵ oder C⁵-CH₂- ist,
C⁵ ist:
(i) ein 5- bis 6-gliedriges monocyclisches Aryl oder Heteroaryl, wobei das Heteroaryl 1 bis 2 Heteroatome enthält, ausgewählt aus der Gruppe bestehend aus N, O und S,
(ii) ein 9- bis 10-gliedriges bicyclisches Aryl oder Heteroaryl, wobei das bicyclische Heteroaryl 1 bis 3 Heteroatome enthält, ausgewählt aus der Gruppe bestehend aus N, O und S,
(iii) ein 5- bis 6-gliedriges monocyclisches oder 9- bis 10-gliedriges Heterocycloalkyl, wobei das Heterocycloalkyl gesättigt oder teilweise ungesättigt ist und 1 bis 2 Heteroatome enthält, ausgewählt aus der Gruppe bestehend aus N, O und S,
(iv) ein 5- bis 6-gliedriges monocyclisches Cycloalkyl,
(v) 2,3-Dihydroindolyl,
wobei C⁵ unsubstituiert oder substituiert ist durch 1 bis 3 R^{C5}-Substituenten, unabhängig ausgewählt aus der Gruppe bestehend aus Halogen, Amino, Hydroxy, C₁-C₃-Alkyl, C₁-C₃-Fluoralkyl,
C₁-C₃-Alkoxy, H₂N-(CH₂)ₖ-, H₂NC(O)-(CH₂)ₖ-, H₂C=CH-CH₂O- und Phenyl,
R⁶ H, C₁-C₅-Alkyl, H₂N(CH₂)ₚ-, HOCH₂-, (CH₃)₂NCH₂-, H₃CO-(CH₂)_{q}- oder C⁶-CH₂- ist,
C⁶ 5- oder 6-gliedriges monocyclisches gesättigtes Heterocycloalkyl ist, das 1 bis 2 Heteroatome enthält, ausgewählt aus der Gruppe bestehend aus N, O und S, und wobei C⁶ unsubstituiert oder substituiert ist durch 1 bis 3 R^{C6}-Substituenten, unabhängig ausgewählt aus der Gruppe bestehend aus Halogen, C₁-C₃-Alkyl, C₁-C₃-Fluoralkyl, Hydroxy und C₁-C₃-Alkoxy,
R⁷ H oder C₁-C₃-Alkyl ist,
R^{8a} H, C₁-C₅-Alkyl, HOCH₂-, H₂N(CH2)ᵣ-, (CH₃)₃N⁺(CH₂)ᵣ- oder CH₃C(O)NH(CH₂)ᵣ- ist,
R^{8b} H oder C₁-C₃-Alkyl ist,
R^{9a} H oder C₁-C₃-Alkyl ist,
R^{9b} H, C₁-C₅-Alkyl, C⁹-CH₂- oder C⁹-CH₂CH₂- ist,
C⁹ ist:
(i) ein 5- bis 6-gliedriges monocyclisches Aryl oder Heteroaryl, wobei das Heteroaryl 1 bis 2 Heteroatome enthält, ausgewählt aus der Gruppe bestehend aus N, O und S, oder
(ii) ein 5- bis 6-gliedriges monocyclisches gesättigtes Cycloalkyl oder Heterocycloalkyl ist, wobei das Heterocycloalkyl 1 bis 2 Heteroatome enthält, ausgewählt aus der Gruppe bestehend aus N, O und S,
wobei C⁹ unsubstituiert oder substituiert ist durch 1 bis 3 R^{C9}-Substituenten, unabhängig ausgewählt aus der Gruppe bestehend aus Halogen, Amino, Hydroxy, Cyano, C₁-C₃-Alkyl, C₁-C₃-Fluoralkyl, C₁-C₃-Alkoxy, H₂N-(CH₂)k-, H₂NC(O)-(CH₂)k-, H₂NCH₂CH₂O-, CH₃C(O)NH-CH₂CH₂O- und Morpholinyl-CH₂CH₂O-,
R¹⁰ H, Halogen oder C₁-C₃-Alkyl ist,
R¹¹ H, Halogen oder C₁-C₃-Alkyl ist,
jedes Vorkommen des Indexes k unabhängig 1 oder 2 ist,
der Index m 1 oder 2 ist,
der Index n 1, 2, 3 oder 4 ist,
der Index p 1, 2, 3 oder 4 ist,
der Index q 1 oder 2 ist,
der Index r 1, 2, 3 oder 4 ist,
X¹, X² und X³ unabhängig C(H) oder N sind, und
A¹ und A² unabhängig ausgewählt sind aus der Gruppe bestehend aus HO₂C-, H₂NC(O)-, CH₃C(O)N(H)-, H₂NS(O)₂-, CH₃S(O)₂N(H)-, Tetrazolyl und 5-Oxooxadiazolyl, oder
ein pharmazeutisch annehmbares Salz davon.

2. Die Verbindung nach Anspruch 1 oder ein pharmazeutisch annehmbares Salz davon, wobei:
R⁵ Amino, H₂N(CH₂)ₙ-, H₂NC(O)-(CH₂)₂-, C⁵ oder C⁵-CH₂- ist,
C⁵ ist:
(i) ein 5- bis 6-gliedriges monocyclisches Aryl oder Heteroaryl, wobei das Heteroaryl 1 bis 2 Heteroatome enthält, ausgewählt aus der Gruppe bestehend aus N, O und S,
(ii) ein 9- bis 10-gliedriges bicyclisches Aryl oder Heteroaryl, wobei das bicyclische Heteroaryl 1 bis 3 Heteroatome enthält, ausgewählt aus der Gruppe bestehend aus N, O und S,
(iii) ein 5- bis 6-gliedriges monocyclisches oder 9- bis 10-gliedriges Heterocycloalkyl, wobei das Heterocycloalkyl gesättigt oder teilweise ungesättigt ist und 1 bis 2 Heteroatome enthält, ausgewählt aus der Gruppe bestehend aus N, O und S, oder
(iv) 2,3-Dihydroindolyl,
wobei C⁵ unsubstituiert oder substituiert ist durch 1 bis 3 R^{C5}-Substituenten, unabhängig ausgewählt aus der Gruppe bestehend aus Halogen, Amino, Hydroxy, C₁-C₃-Alkyl, C₁-C₃-Fluoralkyl, C₁-C₃-Alkoxy, H₂N-(CH₂)k-, H₂NC(O)-(CH₂)ₖ-, H₂C=CH-CH₂O- und Phenyl,
R⁶ H, C₂-C₅-Alkyl, H₂N(CH₂)ₚ-, HOCH₂-, (CH₃)₂NCH₂-, H₃CO-(CH₂)_{q}- oder C⁶-CH₂- ist,
R^{8a} H, C₁-C₃-Alkyl, HOCH₂- oder H₂N(CH₂)ᵣ- ist,
R^{9a} H ist,
R^{9b} H, C₁-C₃-Alkyl, C⁹-CH₂- oder C⁹-CH₂CH₂- ist,
R¹⁰ H ist,
R¹¹ H ist,
der Index p 1, 2 oder 3 ist, und
der Index r 2, 3 oder 4 ist.

3. Die Verbindung nach Anspruch 1 oder ein pharmazeutisch annehmbares Salz davon, wobei:
C¹ Phenyl, Pyrimidyl oder Piperazinyl ist, wobei C¹ unsubstituiert oder substituiert ist durch 1 bis 2 R^{C1}-Substituenten,
R³ Naphthyl oder Indolyl ist, wobei R³ unsubstituiert oder substituiert ist durch 1 bis 2 R^{3a}-Substituenten,
C⁵ Phenyl, Pyridyl, Pyrimidyl, Naphthyl, Indolyl, 7-Azaindolyl, Indazolyl, 2,3-Dihydroindolyl, Piperidinyl, Tetrahydropyranyl oder Cyclohexyl ist, wobei C⁵ unsubstituiert oder substituiert ist durch 1 bis 2 R^{C5},
C⁶ Tetrahydropyranyl oder Morpholinyl ist, wobei C⁶ unsubstituiert oder substituiert ist durch 1 bis 2 R^{C6}, und
R^{9a} H oder Methyl ist,
C⁹ Phenyl, Pyridyl, Cyclohexyl, Morpholinyl oder Piperidinyl ist, wobei C⁹ unsubstituiert oder substituiert ist durch 1 bis 2 R^{C9}.

4. Die Verbindung nach einem der Ansprüche 1 bis 3 oder ein pharmazeutisch annehmbares Salz davon, wobei X¹ und X² C(H) sind und R¹ Phenyl ist, das durch Carboxy substituiert ist, oder R¹ CH₃C(O)NH-CH₂CH₂-O- ist.

5. Die Verbindung nach einem der Ansprüche 1 bis 4 oder ein pharmazeutisch annehmbares Salz davon, wobei R² H ist.

6. Die Verbindung nach einem der Ansprüche 1 bis 5 oder ein pharmazeutisch annehmbares Salz davon, wobei R³ Indolyl ist, das durch ein Halogen substituiert ist, oder R³ Naphthyl ist.

7. Die Verbindung nach einem der Ansprüche 1 bis 6 oder ein pharmazeutisch annehmbares Salz davon, wobei R⁴ HO₂C-(CH₂)ₘ- ist.

8. Die Verbindung nach einem der Ansprüche 1 bis 7 oder ein pharmazeutisch annehmbares Salz davon, wobei X³ C(H) ist.

9. Die Verbindung nach einem der Ansprüche 1 bis 8 oder ein pharmazeutisch annehmbares Salz davon, wobei R⁵ H₂N(CH₂)ₙ-, Indol, 7-Azaindol, Naphthyl oder Pyridyl ist.

10. Die Verbindung nach einem der Ansprüche 1 bis 9 oder ein pharmazeutisch annehmbares Salz davon, wobei R⁶ H, HOCH₂-, C₂-C₅-Alkyl oder H₂N(CH₂)ₚ- ist.

11. Die Verbindung nach einem der Ansprüche 1 bis 10 oder ein pharmazeutisch annehmbares Salz davon, wobei R⁷ H ist.

12. Die Verbindung nach einem der Ansprüche 1 bis 11 oder ein pharmazeutisch annehmbares Salz davon, wobei:
R^{8a} Methyl oder H₂NCH₂CH₂- ist und R^{8b} H ist.

13. Die Verbindung nach einem der Ansprüche 1 bis 12 oder ein pharmazeutisch annehmbares Salz davon, wobei R^{9b} 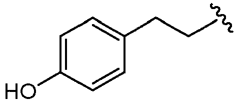 oder 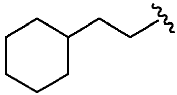 ist.

14. Die Verbindung nach einem der Ansprüche 1 bis 13 oder ein pharmazeutisch annehmbares Salz davon, wobei
R¹⁰ H ist und R¹¹ H, F oder Cl ist.

15. Die Verbindung nach einem der Ansprüche 1 bis 14 oder ein pharmazeutisch annehmbares Salz davon, wobei A¹ und A² beide HO₂C- sind.

16. Die Verbindung nach Anspruch 1 oder ein pharmazeutisch annehmbares Salz davon, wobei:
R¹ 4-CH₃C(O)-Piperazin-1-yl, CH₃C(O)NH-CH₂CH₂-O-, 5-CO₂H-Pyrimidin-2-yl oder 4-CO₂H-Phenyl ist,
R² H, Ethyl, Benzyl oder Phenyl-CH₂CH₂ ist,
R³ Naphth-1-yl, 4-Fluorindol-3-yl oder 4-Chlorindol-3-yl ist,
R⁴ Methyl, HO₂C-(CH₂)ₘ- oder H₂NC(O)-(CH₂)ₘ- ist,
R⁵ Amino, H₂N(CH₂)ₙ-, Naphth-1-yl, Indol-3-yl, 7-Azaindol-3-yl, Indazol-1-yl, 2,3-Dihydroindol-1-yl, Pyrid-3-yl, Pyrid-4-yl, Piperidin-4-yl, 3-Aminomethylphenyl, 4-Aminomethylphenyl, 3-Aminophenyl, 4-Aminophenyl, Phenyl, Pyrid-4-yl-CH₂-, Pyrimidin-5-yl, Tetrahydropyran-4-yl-, H₂NC(O)-(CH₂)₂-, 3-Biphenyl,
3-CH₂=CH-CH₂O-Phenyl, CH₃C(O)NH-, CH₃C(O)NH(CH₂)₃- oder Cyclohexyl ist,
R⁶ H, (CH₃)₂CHCH₂-, (CH₃)₃CCH₂-, H₂N(CH₂)ₚ-, HOCH₂-, H₃CCH₂CH₂-, Morpholin-4-yl-CH₂-, Tetrahydropyran-4-yl-CH₂-, (CH₃)₂NCH₂- oder H₃CO-(CH₂)_{q}- ist,
R⁷ H oder Methyl ist,
R^{8a} H, Methyl, HOCH₂-, H₂N(CH2)ᵣ-, (CH₃)₃NCH₂CH₂- oder CH₃C(O)NH(CH₂)₄- ist,
R^{8b} H oder Methyl ist,
R^{9a} H oder Methyl ist,
R^{9b} H, Methyl, H₃CCH₂CH₂CH₂-, 4-HO-Phenyl-CH₂CH₂-, Phenyl-CH₂CH₂-, Cyclohexyl-CH₂CH₂-, 5-NC-Pyrid-3-yl-CH₂CH₂-, 4-F₃C-Phenyl-CH₂-, 3-F₃C-Phenyl-CH₂-, 2-F₃C-Phenyl-CH₂-, Morpholin-4-yl-CH₂CH₂O-phenyl-CH₂-, 4-Aminophenyl-CH₂-, 4,4-Difluorcyclohexyl-CH₂-, 4-H₂NCH₂CH₂O-Phenyl-CH₂-, 4-H₂NCH₂CH₂O-Pyrid-3-yl-CH₂-, Piperidin-4-yl-CH₂- oder 4-CH₃C(O)NH-CH₂CH₂O-Phenyl-CH₂- ist,
R¹⁰ H, Fluor oder Methyl ist,
R¹¹ H, Fluor oder Chlor ist,
A¹ und A² beide HO₂C- sind, und
X¹, X² und X³ C(H) sind.

17. Die Verbindung nach Anspruch 1, ausgewählt aus der Gruppe bestehend aus (SEQ ID NO. 78, 71-72, 67, 65, 70, 69, 68, 66, 64, 77, 79, 209, 193, 215, 212 bzw. 210, in der Reihenfolge des Auftretens): 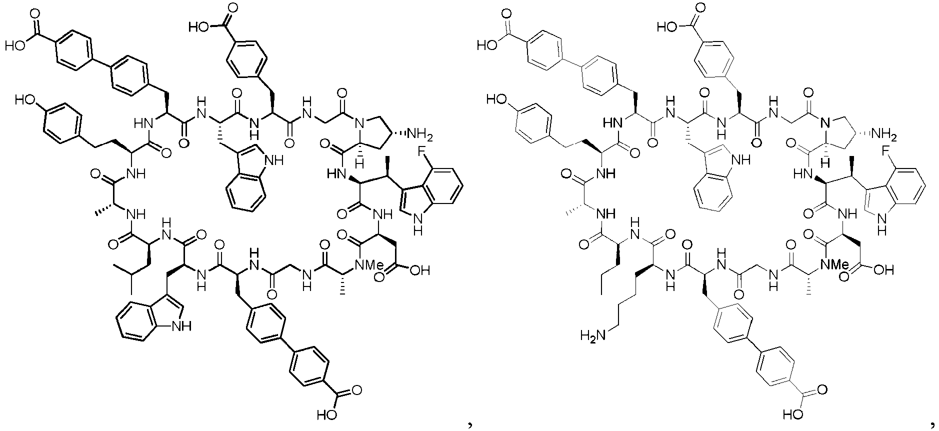 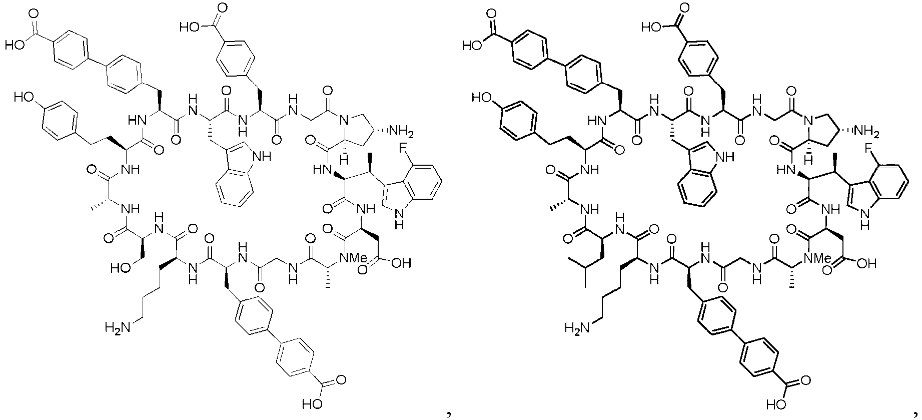 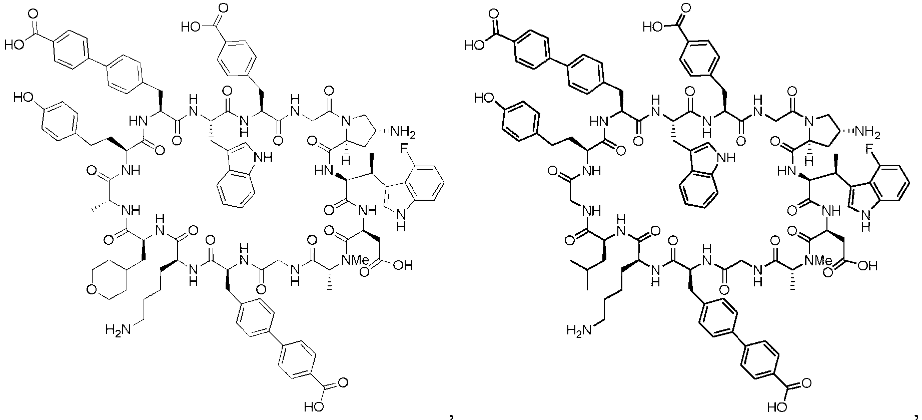 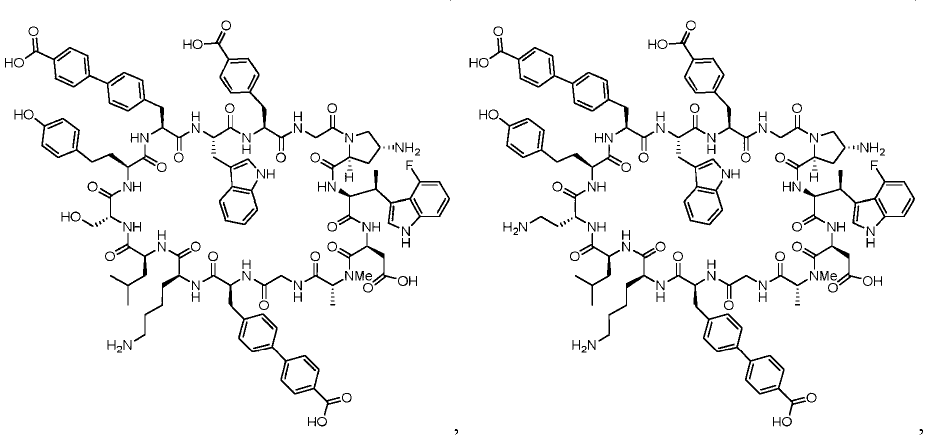 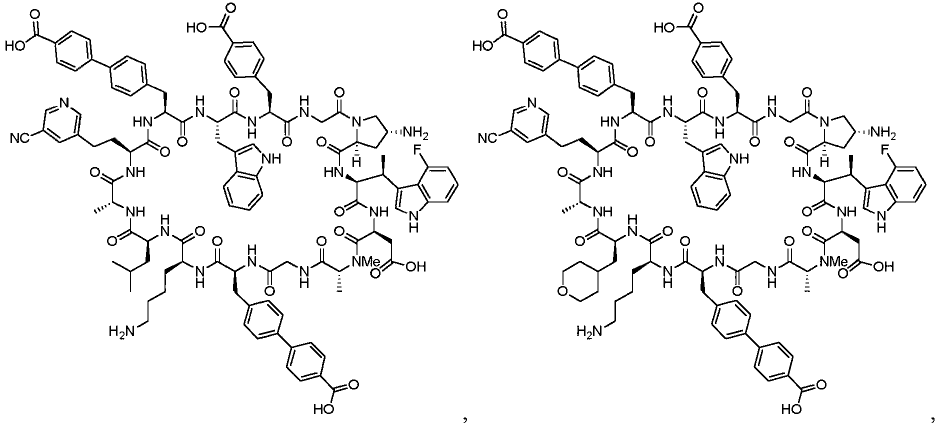 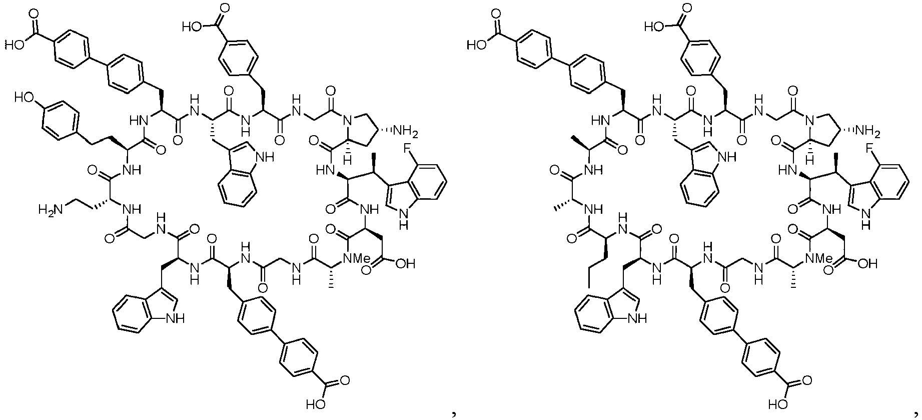 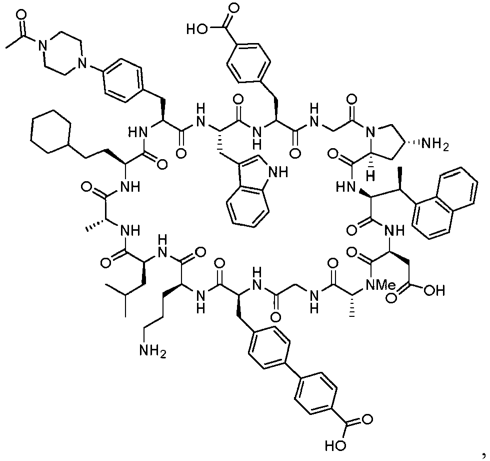 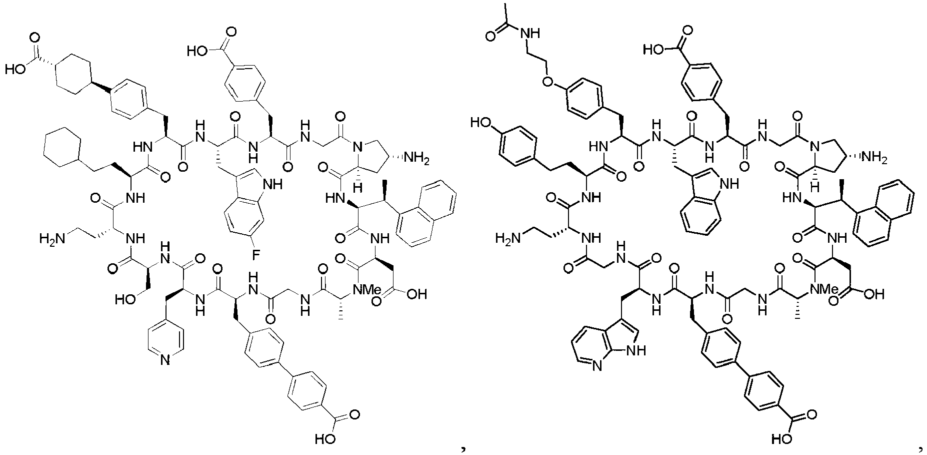 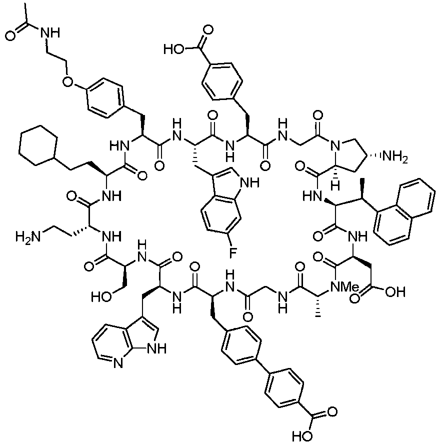 und 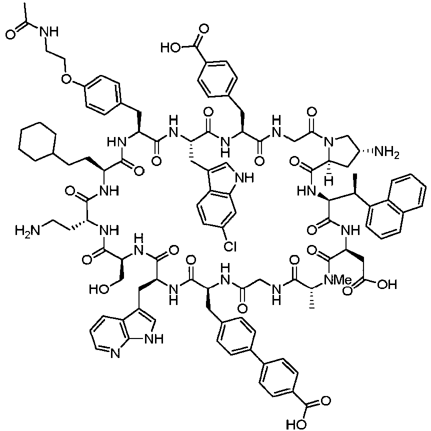 , oder ein pharmazeutisch annehmbares Salz davon.

18. Die Verbindung nach Anspruch 1, die ist: 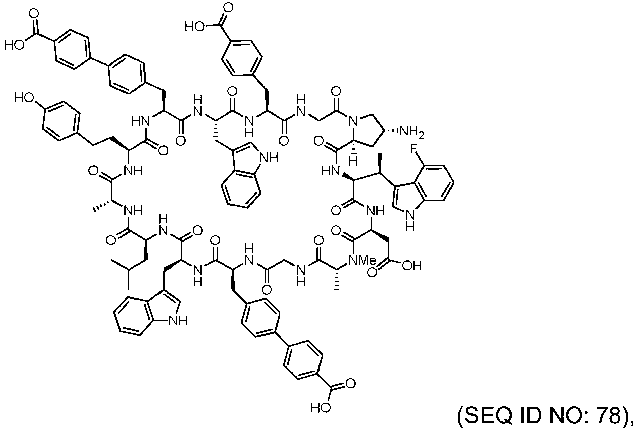 oder ein pharmazeutisch annehmbares Salz davon.

19. Die Verbindung nach Anspruch 1, die ist: 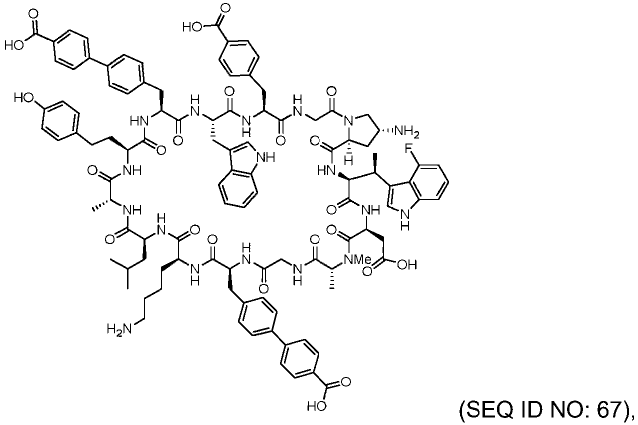 oder ein pharmazeutisch annehmbares Salz davon.

20. Die Verbindung nach Anspruch 1, die ist: 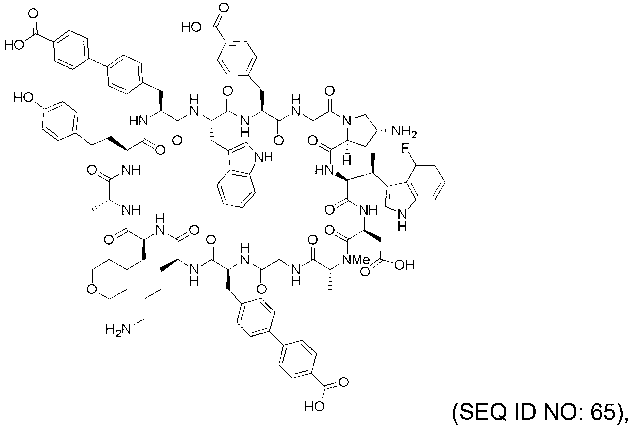 oder ein pharmazeutisch annehmbares Salz davon.

21. Die Verbindung nach Anspruch 1, die ist: 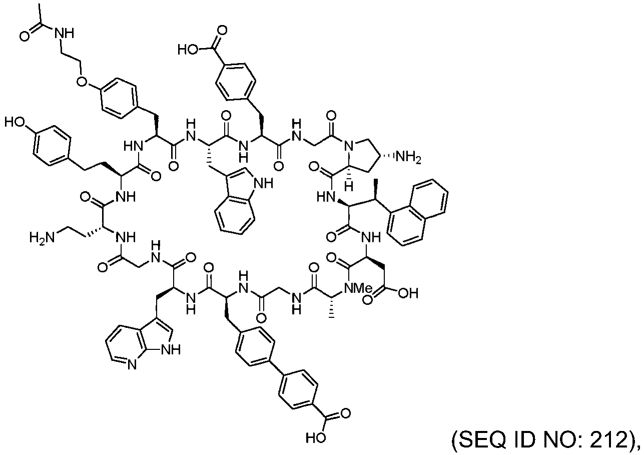 oder ein pharmazeutisch annehmbares Salz davon.

22. Die Verbindung nach Anspruch 1, die ist:

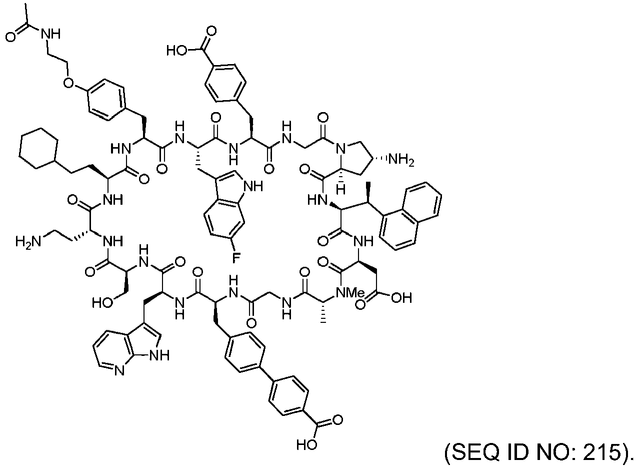

23. Eine pharmazeutische Zusammensetzung, die die Verbindung nach einem der Ansprüche 1 bis 22 oder ein pharmazeutisch annehmbares Salz davon und einen pharmazeutisch annehmbaren Träger umfasst.

24. Eine Verbindung nach einem der Ansprüche 1 bis 22 oder ein pharmazeutisch annehmbares Salz davon zur Verwendung in der Therapie.

25. Eine Verbindung nach einem der Ansprüche 1 bis 22 oder ein pharmazeutisch annehmbares Salz davon zur Verwendung bei der Behandlung von Atherosklerose oder Gefäßentzündung.

## Revendications

1. Composé de formule (I) 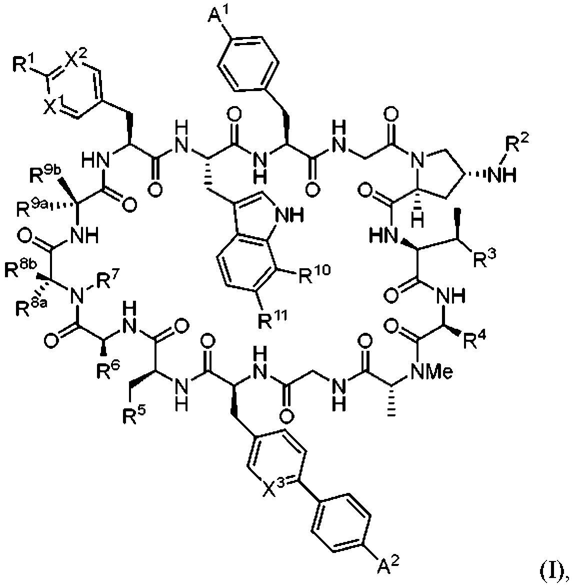
R¹ étant CH₃C(O)NH-CH₂CH₂-O- ou C¹ ;
C¹ étant :
(i) un aryle ou hétéroaryle monocyclique à 5 à 6 chaînons, ledit hétéroaryle contenant 1 à 2 hétéroatomes choisis dans le groupe constitué par N, O, et S ; ou
(ii) un cycloalkyle ou hétérocycloalkyle saturé monocyclique ou bicyclique à 5 à 6 chaînons contenant 1 à 2 hétéroatomes choisis dans le groupe constitué par N, O, et S ; ou
(iii) un cycloalkyle monocyclique ou bicyclique à 5 à 6 chaînons ;
C¹ étant non substitué ou substitué par 1 à 3 R^{C1} substituants indépendamment choisis dans le groupe constitué par halogéno, C₁-C₃ alkyle, C₁-C₃ fluoroalkyle, carboxy, C₁-C₃ alcoxy, et C₂-C₃ acyle ;
R² étant H, C₁-C₃ alkyle, benzyle, ou phényl-CH₂CH₂- ;
R³ étant un aryle ou hétéroaryle bicyclique à 9 à 10 chaînons, ledit hétéroaryle contenant 1 à 2 hétéroatomes choisis dans le groupe constitué par N, O, et S ;
R³ étant non substitué ou substitué par 1 à 3 R^{3a} substituants indépendamment choisis dans le groupe constitué par halogéno, C₁-C₃ alkyle, C₁-C₃ fluoroalkyle, hydroxy et C₁-C₃ alcoxy ;
R⁴ étant C₁-C₃ alkyle, HO₂C-(CH₂)ₘ-, H₂NC(O)-(CH₂)ₘ-, (CH₃)₂NC(O)-(CH₂)ₘ-, ou tétrazolyl- (CH₂)ₘ- ;
R⁵ étant amino, H₂N(CH₂)ₙ-, H₂NC(O)-(CH₂)ₙ-, CH₃C(O)NH-, CH₃C(O)NH (CH₂)ₙ-, C⁵, ou C⁵-CH₂-,
C⁵ étant :
(ii) un aryle ou hétéroaryle monocyclique à 5 à 6 chaînons, ledit hétéroaryle contenant 1 à 2 hétéroatomes choisis dans le groupe constitué par N, O, et S ;
(ii) un aryle ou hétéroaryle bicyclique à 9 à 10 chaînons, ledit hétéroaryle bicyclique contenant 1 à 3 hétéroatomes choisis dans le groupe constitué par N, O, et S ;
(iii) un hétérocycloalkyle à 5 à 6 chaînons monocyclique ou à 9 à 10 chaînons, ledit hétérocycloalkyle étant saturé ou partiellement insaturé, et contenant 1 à 2 hétéroatomes choisis dans le groupe constitué par N, O, et S ;
(iv) un cycloalkyle monocyclique à 5 à 6 chaînons ;
(v) 2,3-dihydroindolyle ;
C⁵ étant non substitué ou substitué par 1 à 3 R^{C5}, substituants indépendamment choisis dans le groupe constitué par halogéno, amino, hydroxy, C₁-C₃ alkyle, C₁-C₃ fluoroalkyle, C₁-C₃ alcoxy, H₂N-(CH₂)ₖ-, H₂NC(O)-(CH₂)ₖ-, H₂C=CH-CH₂O-, et phényle ;
R⁶ étant H, C₁-C₅ alkyle, H₂N(CH₂)ₚ-, HOCH₂-, (CH₃)₂NCH₂-, H₃CO-(CH₂)_{q}- , ou C⁶-CH₂- ;
C⁶ étant hétérocycloalkyle saturé, monocyclique à 5 ou 6 chaînons contenant 1 à 2 hétéroatomes choisis dans le groupe constitué par N, O, et S ; et C⁶ étant non substitué ou substitué par 1 à 3 R^{C6} substituants indépendamment choisis dans le groupe constitué par halogéno, C₁-C₃ alkyle, C₁-C₃ fluoroalkyle, hydroxy et C₁-C₃ alcoxy ;
R⁷ étant H ou C₁-C₃ alkyle ;
R^{8a} étant H, C₁-C₅ alkyle, HOCH₂-, H₂N(CH₂)ᵣ-, (CH₃)₃N⁺(CH₂)ᵣ-, or CH₃C(O)NH (CH₂)ᵣ- ;
R^{8b} étant H ou C₁-C₃ alkyle ;
R^{9a} étant H ou C₁-C₃ alkyle ;
R^{9b} étant H, C₁-C₅ alkyle, C⁹-CH₂-, ou C⁹-CH₂CH₂- ;
C⁹ étant :
(i) un aryle ou hétéroaryle monocyclique à 5 à 6 chaînons, ledit hétéroaryle contenant 1 à 2 hétéroatomes choisis dans le groupe constitué par N, O, et S ; ou
(ii) un cycloalkyle ou hétérocycloalkyle saturé, monocyclique à 5 à 6 chaînons, l'hétérocycloalkyle contenant 1 à 2 hétéroatomes choisis dans le groupe constitué par N, O, et S ;
C⁹ étant non substitué ou substitué par 1 à 3 R^{C9} substituants indépendamment choisis dans le groupe constitué par halogéno, amino, hydroxy, cyano, C₁-C₃ alkyle, C₁-C₃ fluoroalkyle, C₁-C₃ alcoxy, H₂N-(CH₂)ₖ-, H₂NC(O)-(CH₂)ₖ-, H₂NCH₂CH₂O-, CH₃C(O)NH-CH₂CH₂O-, et morpholinyl-CH₂CH₂O- ;
R¹⁰ étant H, halogéno, ou C₁-C₃ alkyle ;
R¹¹ étant H, halogéno, ou C₁-C₃ alkyle ;
chaque occurrence de l'indice k étant indépendamment 1 ou 2 ;
l'exposant m étant 1 ou 2 ;
l'exposant n étant 1, 2, 3, ou 4 ;
l'exposant p étant 1, 2, 3, ou 4 ;
l'exposant q étant 1 ou 2 ;
l'exposant r étant 1, 2, 3, ou 4 ;
X¹, X², et X³ étant indépendamment C(H) ou N ; et
A¹ et A² étant indépendamment choisis dans le groupe constitué par HO₂C-, H₂NC(O)-, CH₃C(O)N(H)-, H₂NS(O)₂-, CH₃S(O)₂N(H)-, tétrazolyle, et 5-oxo oxadiazolyle ; ou
sel pharmaceutiquement acceptable correspondant.

2. Composé selon la revendication 1 ou sel pharmaceutiquement acceptable correspondant,
R⁵ étant amino, H₂N(CH₂)ₙ-, H₂NC(O)-(CH₂)ₙ-, C⁵, ou C⁵-CH₂-,
C⁵ étant :
(i) un aryle ou hétéroaryle monocyclique à 5 à 6 chaînons, ledit hétéroaryle contenant 1 à 2 hétéroatomes choisis dans le groupe constitué par N, O, et S :
(ii) un aryle ou hétéroaryle bicyclique à 9 à 10 chaînons, ledit hétéroaryle bicyclique contenant 1 à 3 hétéroatomes choisis dans le groupe constitué par N, O, et S ;
(iii) un hétérocycloalkyle à 5 à 6 chaînons monocyclique ou à 9 à 10 chaînons, ledit hétérocycloalkyle étant saturé ou partiellement insaturé, et contenant 1 à 2 hétéroatomes choisis dans le groupe constitué par N, O, et S ; ou
(iv) 2,3-dihydroindolyle ;
C⁵ étant non substitué ou substitué par 1 à 3 R^{C5}, substituants indépendamment choisis dans le groupe constitué par halogéno, amino, hydroxy, C₁-C₃ alkyle, C₁-C₃ fluoroalkyle, C₁-C₃ alcoxy, H₂N-(CH₂)ₖ-, H₂NC(O)-(CH₂)ₖ-, H₂C=CH-CH₂O-, et phényle ;
R⁶ étant H, C₂-C₅ alkyle, H₂N(CH₂)ₚ-, HOCH₂-, (CH₃)₂NCH₂-, H₃CO-(CH₂)_{q}-, ou C⁶-CH₂- ;
R^{8a} étant H, C₁-C₃ alkyle, HOCH₂-, ou H₂N(CH₂)ᵣ- ;
R^{9a} étant H ;
R^{9b} étant H, C₁-C₃ alkyle, C⁹-CH₂-, ou C⁹-CH₂CH₂- ;
R¹⁰ étant H ;
R¹¹ étant H ;
l'exposant p étant 1, 2, ou 3 ; et
l'exposant r étant 2, 3, ou 4.

3. Composé selon la revendication 1, ou sel pharmaceutiquement acceptable correspondant,
C¹ étant phényle, pyrimidyle, ou pipérazinyle, C¹ étant non substitué ou substitué par 1 à 2 R^{C1} substituants ;
R³ étant naphtyle ou indolyle, R³ étant non substitué ou substitué par 1 à 2 R^{3a} substituants ;
C⁵ étant phényle, pyridinyle, pyrimidyle, naphtyle, indolyle, 7-azaindolyle, indazolyle, 2,3-dihydroindolyle, pipéridinyle, tétrahydropyranyle, ou cyclohexyle, C⁵ étant non substitué ou substitué par 1 à 2 R^{C5}, ;
C⁶ étant tétrahydropyranyle ou morpholinyle ; C⁶ étant non substitué ou substitué par 1 à 2 R^{C6} ; et
R^{9a} étant H ou méthyle ;
C⁹ étant phényle, pyridinyle, cyclohexyle, morpholinyle, ou pipéridinyle, C⁹ étant non substitué ou substitué par 1 à 2 R^{C9}.

4. Composé selon l'une quelconque des revendications 1 à 3, ou sel pharmaceutiquement acceptable correspondant, X¹ et X² étant C(H) ; et R¹ étant phényle substitué par carboxy ou R¹ étant CH₃C(O)NH-CH₂CH₂-O-.

5. Composé selon l'une quelconque des revendications 1 à 4, ou sel pharmaceutiquement acceptable correspondant, R² étant H.

6. Composé selon l'une quelconque des revendications 1 à 5, ou sel pharmaceutiquement acceptable correspondant, R³ étant indolyle substitué par un halogéno ou R³ étant naphtyle.

7. Composé selon l'une quelconque des revendications 1 à 6, ou sel pharmaceutiquement acceptable correspondant, R⁴ étant HO₂C-(CH₂)ₘ- .

8. Composé selon l'une quelconque des revendications 1 à 7, ou sel pharmaceutiquement acceptable correspondant, X³ étant C(H).

9. Composé selon l'une quelconque des revendications 1 à 8, ou sel pharmaceutiquement acceptable correspondant, R⁵ étant H₂N(CH₂)ₙ-, indole, 7-azaindole, naphtyle ou pyridinyle.

10. Composé selon l'une quelconque des revendications 1 à 9, ou sel pharmaceutiquement acceptable correspondant, R⁶ étant H, HOCH₂- , C₂-C₅ alkyle ou H₂N(CH₂)ₚ-.

11. Composé selon l'une quelconque des revendications 1 à 10, ou sel pharmaceutiquement acceptable correspondant, R⁷ étant H.

12. Composé selon l'une quelconque des revendications 1 à 11, ou sel pharmaceutiquement acceptable correspondant,
R^{8a} étant méthyle ou H₂NCH₂CH₂- ; et R^{8b} étant H.

13. Composé selon l'une quelconque des revendications 1 à 12, ou sel pharmaceutiquement acceptable correspondant, R^{9b} étant 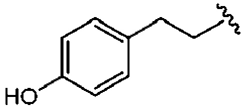 ou

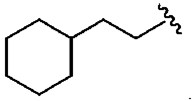

14. Composé selon l'une quelconque des revendications 1 à 13, ou sel pharmaceutiquement acceptable correspondant,
R¹⁰ étant H ; et R¹¹ étant H, F ou Cl.

15. Composé selon l'une quelconque des revendications 1 à 14, ou sel pharmaceutiquement acceptable correspondant, A¹ et A² étant tous deux HO₂C-.

16. Composé selon la revendication 1 ou sel pharmaceutiquement acceptable correspondant,
R¹ étant 4-CH₃C(O)-pipérazin-1-yle, CH₃C(O) NH-CH₂CH₂-O-, 5-CO₂H-pyrimidin-2-yle, ou 4-CO₂H-phényle ;
R² étant H, éthyle, benzyle, ou phényl-CH₂CH₂- ;
R³ étant napht-1-yle, 4-fluoroindol-3-yle, ou 4-chloroindol-3-yle ;
R⁴ étant méthyle, HO₂C-(CH₂)ₘ-, ou H₂NC(O)-(CH₂)ₘ- ;
R⁵ étant amino, H₂N(CH₂)ₙ-, napht-1-yle, indol-3-yle, 7-aza-indol-3-yle, indazol-1-yle, 2,3-dihydroindol-1-yle, pyrid-3-yle, pyrid-4-yle, pipéridin-4-yle, 3-aminométhylphényle, 4-aminométhylphényle, 3-aminophényle, 4-aminophényle, phényle, pyrid-4-yl-CH₂-, pyrimidin-5-yle, tétrahydropyran-4-yl-, H₂NC(O)-(CH₂)₂-, 3-biphényle, 3-CH₂=CH-CH₂O-phényle, CH₃C(O)NH-,, CH₃C(O) NH (CH₂)₃-, ou cyclohexyle ;
R⁶ étant H, (CH₃)₂CHCH₂-, (CH₃)₃CCH₂-, H₂N(CH₂)ₚ-, HOCH₂-, H₃CCH₂CH₂- , morpholin-4-yl-CH₂-, tétrahydropyran-4-yl-CH₂-, (CH₃)₂NCH₂-, ou H₃CO-(CH₂)_{q}- ;
R⁷ étant H ou méthyle ;
R^{8a} étant H, méthyle, HOCH₂-, H₂N(CH₂)ᵣ-, (CH₃)₃NCH₂CH₂-, ou CH₃C(O) NH (CH₂)₄- ;
R^{8b} étant H ou méthyle ;
R^{9a} étant H ou méthyle ;
R^{9b} étant H, méthyle, H₃CCH₂CH₂CH₂-, 4-HO-phényl-CH₂CH₂-, phényl-CH₂CH₂-, cyclohexyl-CH₂CH₂-, 5-NC-pyrid-3-yl-CH₂CH₂-, 4-F₃C-phényl-CH₂-, 3-F₃C-phényl-CH₂-, 2-F₃C-phényl-CH₂-, morpholin-4-yl-CH₂CH₂O-phényl-CH₂-, 4-aminophényl-CH₂-, 4,4-difluorocyclohexyl-CH₂-, 4-H₂NCH₂CH₂O-phényl-CH₂-, 4-H₂NCH₂CH₂O-pyrid-3-yl-CH₂-, pipéridin-4-yl-CH₂-, ou 4-CH₃C(O)NH-CH₂CH₂O-phényl-CH₂- ;
R¹⁰ étant H, fluoro, ou méthyle ;
R¹¹ étant H, fluoro, ou chloro ;
A¹ et A² étant tous deux HO₂C- ; et
X¹, X², et X³ étant C(H).

17. Composé selon la revendication 1 choisi dans le groupe constitué par (SEQ ID NOS 78, 71-72, 67, 65, 70, 69, 68, 66, 64, 77, 79, 209, 193, 215, 212, et 210 respectivement, dans l'ordre d'apparence) : 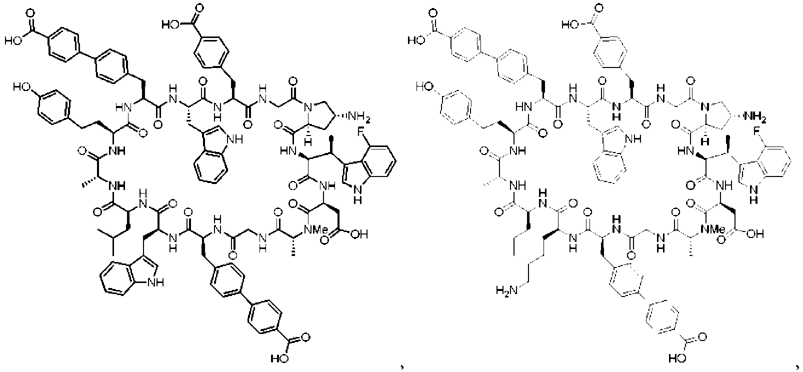 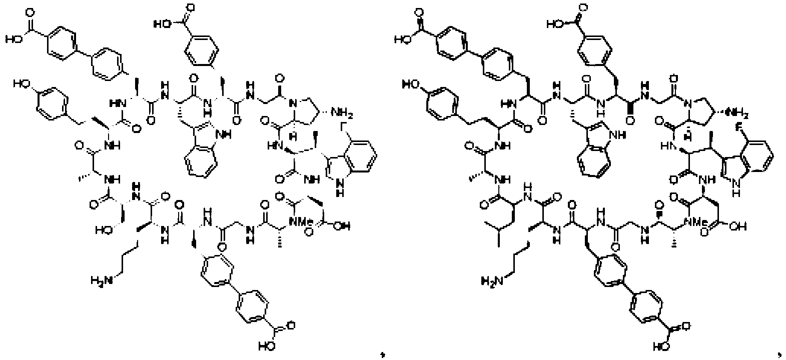 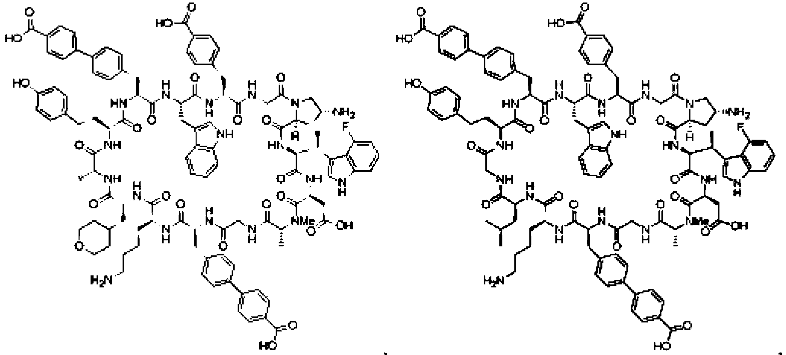 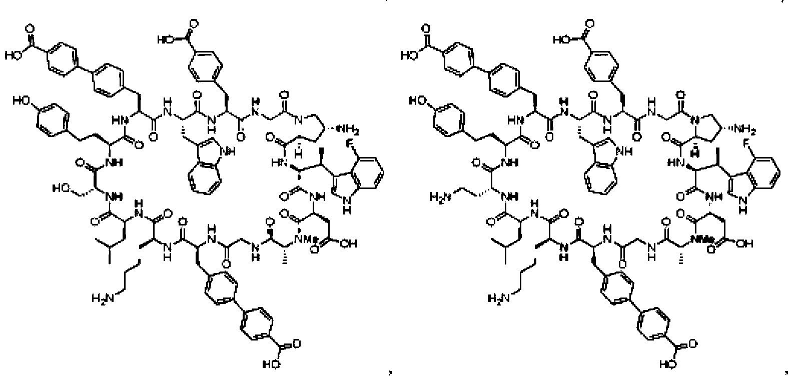 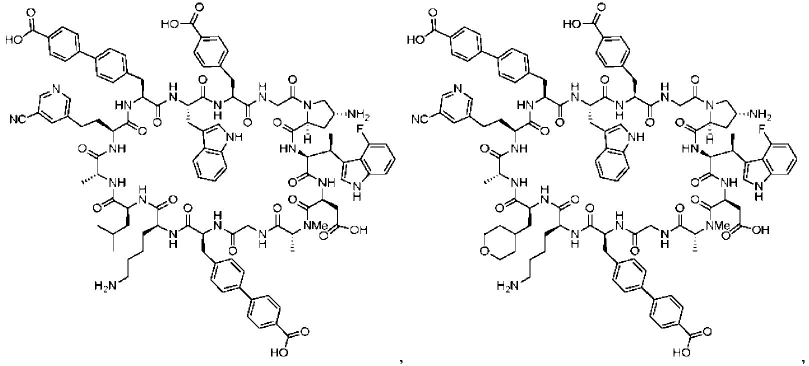 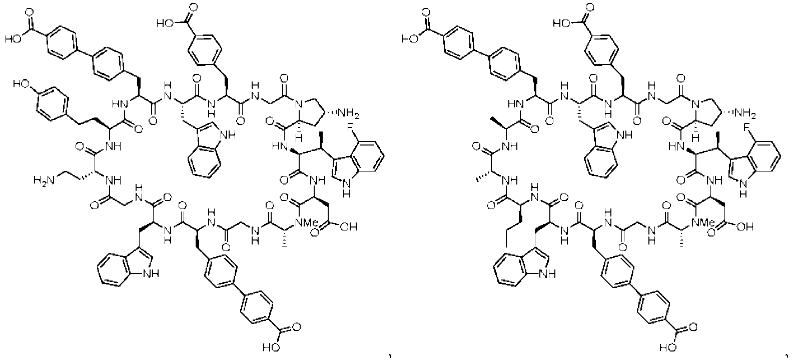 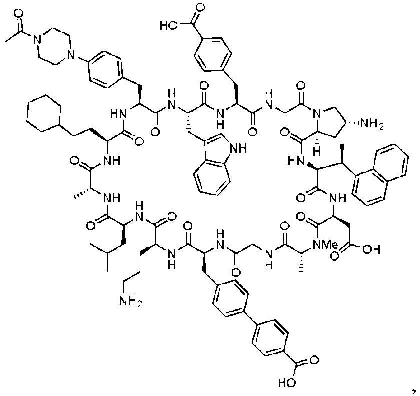 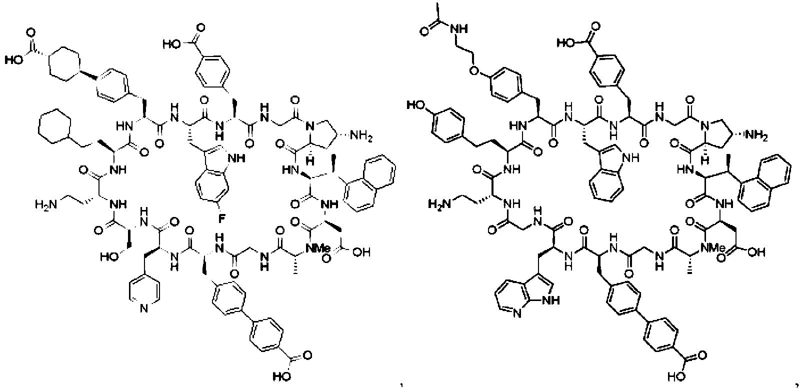 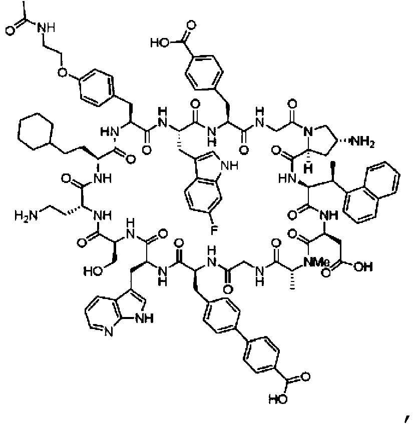 et 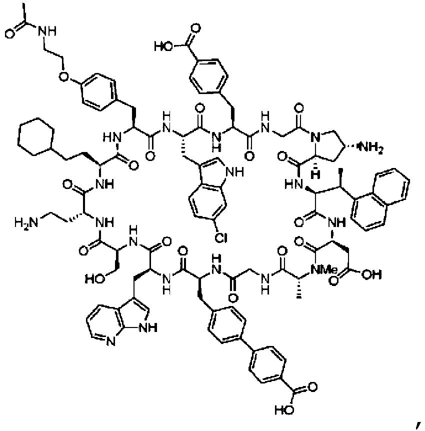 ou sel pharmaceutiquement acceptable correspondant.

18. Composé selon la revendication 1, qui est : 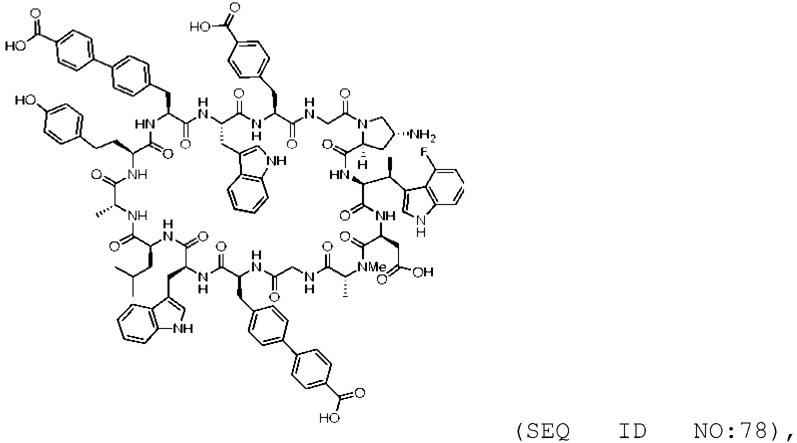 ou sel pharmaceutiquement acceptable correspondant.

19. Composé selon la revendication 1, qui est : 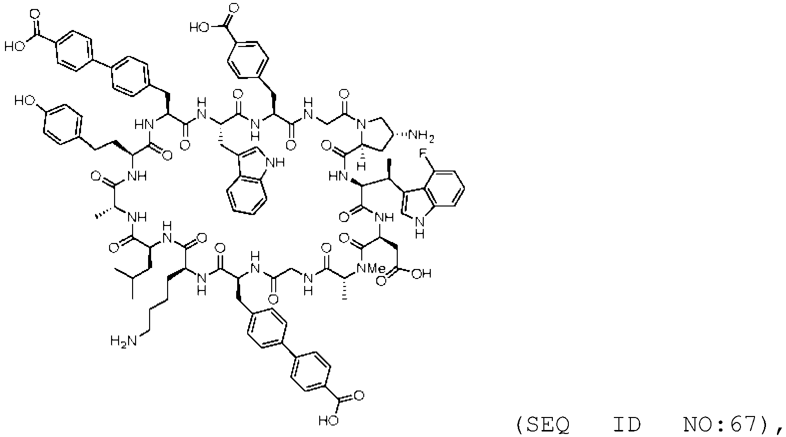 ou sel pharmaceutiquement acceptable correspondant.

20. Composé selon la revendication 1, qui est : 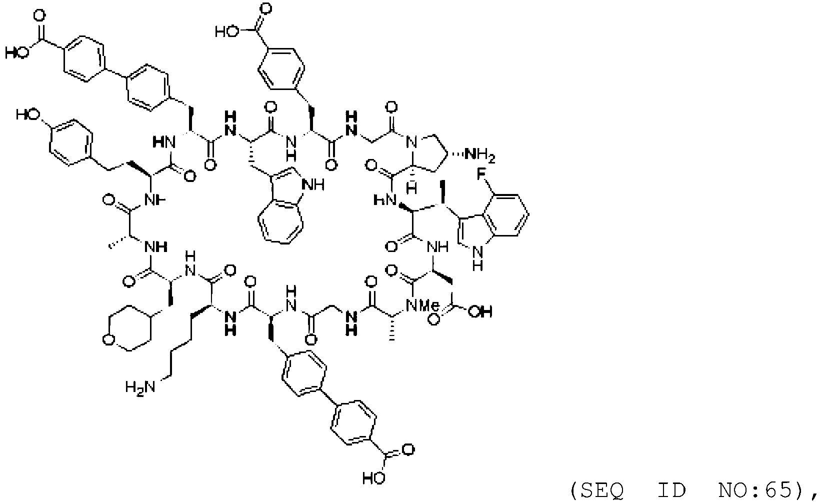 ou sel pharmaceutiquement acceptable correspondant.

21. Composé selon la revendication 1, qui est : 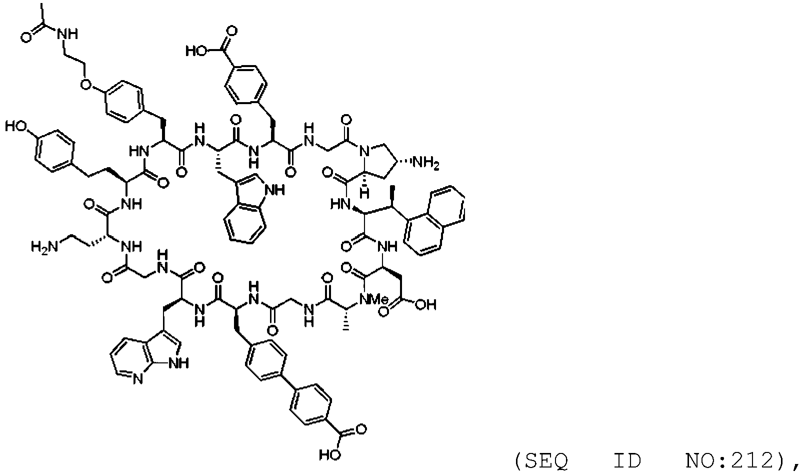 ou sel pharmaceutiquement acceptable correspondant.

22. Composé selon la revendication 1, qui est :

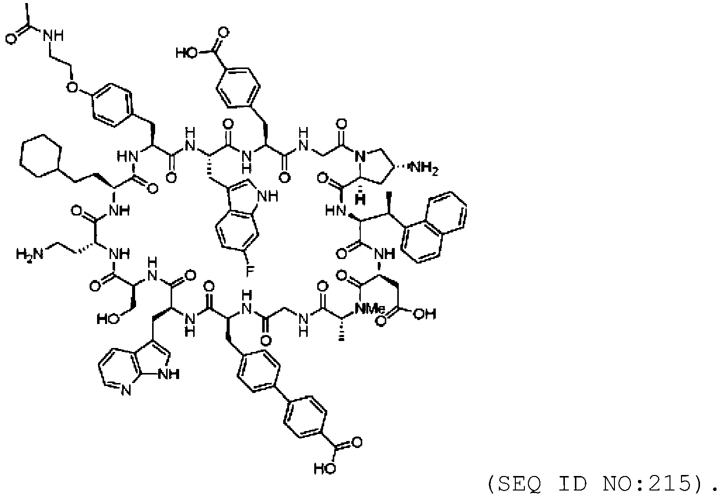

23. Composition pharmaceutique comprenant le composé selon l'une quelconque des revendications 1 à 22, ou un sel pharmaceutiquement acceptable correspondant, et un support pharmaceutiquement acceptable.

24. Composé selon l'une quelconque des revendications 1 à 22, ou sel pharmaceutiquement acceptable correspondant, pour une utilisation en thérapie.

25. Composé selon l'une quelconque des revendications 1 à 22, ou sel pharmaceutiquement acceptable correspondant, pour une utilisation dans le traitement de l'athérosclérose, ou d'une inflammation vasculaire.
